(19) Europäisches Patentamt
European Patent Office
Office européen des brevets

(11) **EP 4 134 098 A1**

(12) **EUROPEAN PATENT APPLICATION**
published in accordance with Art. 153(4) EPC

(43) Date of publication:
**15.02.2023 Bulletin 2023/07**

(21) Application number: **21785563.4**

(22) Date of filing: **09.04.2021**

(51) International Patent Classification (IPC):
***A61K 45/00*** (1974.07) ***A61K 31/437*** (2000.01)
***A61K 31/573*** (2000.01) ***A61K 31/661*** (2000.01)
***A61K 39/395*** (1980.01) ***A61P 35/00*** (2000.01)
***A61P 43/00*** (2000.01)

(52) Cooperative Patent Classification (CPC):
**A61K 31/437; A61K 31/573; A61K 31/661; A61K 39/395; A61K 45/00; A61P 35/00; A61P 43/00**

(86) International application number:
**PCT/JP2021/014973**

(87) International publication number:
**WO 2021/206158 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.04.2020 JP 2020070915**

(71) Applicant: **ONO Pharmaceutical Co., Ltd.**
**Osaka-shi, Osaka 541-8526 (JP)**

(72) Inventors:
- **TAKEDA, Kazuhiko**
  **Mishima-gun, Osaka 618-8585 (JP)**
- **MIYATA, Ryohei**
  **Mishima-gun, Osaka 618-8585 (JP)**
- **KOIKE, Tomoya**
  **Mishima-gun, Osaka 618-8585 (JP)**
- **KATSUBE, Akira**
  **Mishima-gun, Osaka 618-8585 (JP)**

(74) Representative: **Grünecker Patent- und Rechtsanwälte PartG mbB**
**Leopoldstraße 4**
**80802 München (DE)**

(54) **METHOD OF CANCER THERAPY**

(57) The object of the present invention is, when suppressing the progression of, suppressing the recurrence of and/or treating cancer by a STING agonistic compound, to provide a prescription capable of avoiding the induction of inflammatory cytokine production caused by the same agent.

The present invention relates to prescriptions that a STING agonistic compound is administered in combination with an adrenal corticosteroid when suppressing the progression of, suppressing the recurrence of and/or treating cancer, and methods for avoiding the induction of inflammatory cytokine production by prescription with further administering in combination with an adrenal corticosteroid when administering a STING agonistic compound at reduced dosage in combination with an anti-neoplastic agent.

*FIG. 4*

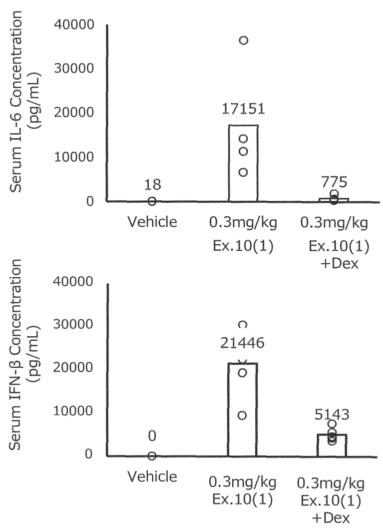


EP 4 134 098 A1

## Description

[Technical field]

The present invention relates a pharmaceutical agent containing a STING agonistic compound, as an active ingredient, prescribed in suppressing the progression of, suppressing the recurrence of and/or treating cancer (hereinafter, may be abbreviated as a "STING agonist").

[Background art]

It is known that STING (Stimulation of Interferon Genes) is an endoplasmic reticulum localized type four-transmembrane protein and is involved in innate immunity. When foreign double-stranded DNAs appear in cytoplasm due to infection or the like, cyclic GMP-AMP synthase (cGAS) is activated and cyclic GMP-AMP (cGAMP) is synthesized. This cGAMP binds to STING on endoplasmic reticulum and induces type I interferon (IFN) production. On the other hand, it is known that cyclic dinucleotides such as cyclic Di-GMP, which were first identified as a second messenger of bacteria and later confirmed to also exist in mammals, also directly bind to STING to activate it (Non-Patent Literature 1).

Furthermore, STING is also known to be involved in autoimmune diseases and tumor immunity. For example, it has been indicated that abnormal host DNAs leak from nucleus and activate STING to induce pro-inflammatory responses, which have been implicated in autoimmune disease. The STING pathway also detects tumor-derived DNAs to promote T cell responses to tumors. It is known that a STING agonistic compound administered to mouse tumors induces adaptive immune response to cause tumor regression (Non-Patent Literature 2), and that an activating molecule of the STING pathway enhances IFN production to exhibit antiviral effects. (Non-Patent Literature 3).

However, when this STING agonist is prescribed, it is concerned about the possibility of developing cytokine release syndrome immediately after prescription.

[Citation List]

[Non-patent literature]

Non-Patent Literature 1: Devaux L. et. al., Curr. Opi. Microbiol. 41, 21-28 (2018)
Non-Patent Literature 2: Corrales L. et. al., Cell Rep. 11 (7), 1018- 1030 (2015)
Non-Patent Literature 3: Sali T.M. et. Al., PLoS Pathog., 11 (12): e1005324

[Summary of Invention]

[Technical problem]

The object of the present invention is to provide a prescription capable of avoiding the induction of inflammatory cytokine production caused by STING agonists.

[Solution to problem]

In order to find the prescription which could solve the above object, the present inventors have diligently studied the prescription of STING agonist to find that they can be administered in combination with an adrenal corticosteroid. Furthermore, they found the prescription in which the concern of cytokine release syndrome is more reduced by administering the STING agonist at reduced dosage in combination with an anti-neoplastic agent without any concerns of developing cytokine release syndrome, and further in combination with a corticosteroid, and completed the present inventions.

That is, constitution of the present invention is as follows.

[1] A compound represented by the general formula (I)

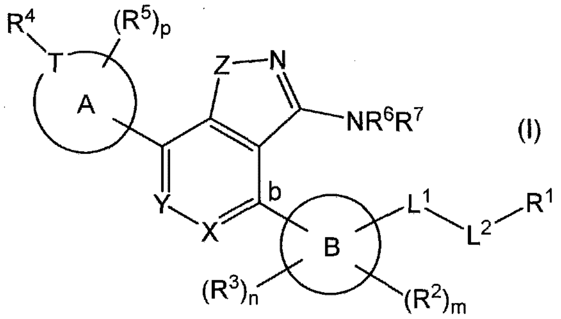

(I)

[wherein X and Y represent -CH= or a nitrogen atom (provided that both X and Y do not represent -CH=, simultaneously), respectively, Z represents an oxygen atom or sulfur atom, T represents a carbon atom or nitrogen atom, Ring A represents a 5 to 7-membered monocycle, Ring B represents a 5 to 7-membered monocycle or 8 to 10-membered bicycle, $L^1$ represents a bond, -O-, -CONH-, -CO-, $-CO_2-$, -S-, $-SO_2-$ or -SO-, $L^2$ represents a bond, C1-3 alkylene group, C3-7 cycloalkylene group or phenylene group, $R^1$ represents a hydrogen atom, halogen atom, hydroxyl group, cyano group, $N(R^{1a})_2$ (herein, two $R^{1a}$s each independently represent a hydrogen atom or C1-4 alkyl group), C1-4 alkyl group, carboxy group, C1-4 alkoxycarbonyl group, C1-4 haloalkyl group, methyl-$d_3$ group, C3-7 cycloalkyl group, phenyl group or 3 to 7-membered monocyclic non-aromatic heterocycle, $R^2$ represents a hydrogen atom, halogen atom, hydroxyl group, oxo group, nitro group, cyano group, C1-4 alkoxy group or $-CH_2NR^{2a}R^{2b}$ or $NR^{2a}R^{2b}$ (herein, $R^{2a}$ represents a hydrogen atom or C1-4 alkyl group, and $R^{2b}$ represents a hydrogen atom), m represents an integer of 0 or 1, $R^3$ represents a hydrogen atom, halogen atom, hydroxyl group, C1-4 alkyl group, C1-4 alkoxy group, C1-4 haloalkyl group, C1-4 haloalkoxy group or amino group, n represents an integer of 1 to 16 (herein, if n is two or more, the groups represented by a plurality of $R^3$s may be the same or different), $R^4$ represents a hydrogen atom, C1-4 alkyl group or carboxy group, $R^5$ represents a C1-4 alkyl group, p represents an integer of 0 to 5 (herein, if p is two or more, the groups represented by a plurality of $R^5$s may be the same or different), $R^6$ represents a hydrogen atom or C1-4 alkyl group, $R^7$ is a hydrogen atom, and b represents the bonding position of Ring B.], an N-oxide thereof, a prodrug thereof, a pharmaceutically acceptable salt thereof, or a solvate thereof;

[2] the compound, N-oxide thereof, prodrug thereof, pharmaceutically acceptable salt thereof, or solvate thereof, according to the preceding item [1], wherein Ring A is (a) a C5-6 monocyclic carbocycle or (b) a 5 to 6-membered monocyclic heterocycle containing 1 to 4 heteroatoms selected from an oxygen atom, nitrogen atom and sulfur atom;

[3] the compound, N-oxide thereof, prodrug thereof, pharmaceutically acceptable salt thereof, or solvate thereof, according to the preceding item [1] or [2], wherein Ring B is (a) a C5-6 monocyclic carbocycle or (b) a 5 to 6-membered monocyclic heterocycle containing 1 to 4 heteroatoms selected from an oxygen atom, nitrogen atom and sulfur atom;

[4] the compound, N-oxide thereof, prodrug thereof, pharmaceutically acceptable salt thereof, or solvate thereof, according to the preceding item [1] or [3], wherein Ring A is (a) a benzene ring or (b) a 5 to 6-membered monocyclic aromatic heterocycle containing 1 to 4 heteroatoms selected from an oxygen atom, nitrogen atom and sulfur atom;

[5] the compound, N-oxide thereof, prodrug thereof, pharmaceutically acceptable salt thereof, or solvate thereof, according to any one of the preceding items [1], [2] and [4], wherein Ring B is (a) a benzene ring or (b) a 5 to 6-membered monocyclic aromatic heterocycle containing 1 to 4 heteroatoms selected from an oxygen atom, nitrogen atom and sulfur atom;

[6] the compound, N-oxide thereof, prodrug thereof, pharmaceutically acceptable salt thereof, or solvate thereof, according to any one of the preceding items [1], [3] and [5], wherein Ring A is a 5 to 6-membered monocyclic aromatic nitrogen-containing heterocycle containing 1 to 4 nitrogen atoms, without any other heteroatoms;

[7] the compound, N-oxide thereof, prodrug thereof, pharmaceutically acceptable salt thereof, or solvate thereof, according to any one of the preceding items [1] to [6], wherein Z is an oxygen atom;

[8] the compound, N-oxide thereof, prodrug thereof, pharmaceutically acceptable salt thereof, or solvate thereof, according to any one of the preceding items [1] to [7], wherein X is a nitrogen atom, and Y is -CH=;

[9] the compound, N-oxide thereof, prodrug thereof, pharmaceutically acceptable salt thereof, or solvate thereof, according to any one of the preceding items [1] to [8], wherein

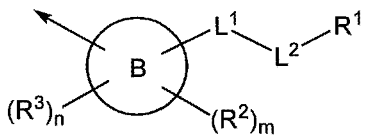

[wherein the arrow is bound to the carbon atom represented by b in the general formula (I), and other symbols represent the same meanings as described above.] of the general formula (I) is the group represented by the following formula (Ib)

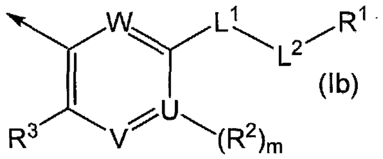

(Ib)

[wherein U represents a nitrogen atom or carbon atom (herein, if U represents a nitrogen atom, m represents 0, and if U represents a carbon atom, m represents 1), W represents $-CR^3=$ or a nitrogen atom, V represents -CH= or a nitrogen atom, and if the formula (Ib) has a plurality of $R^3$s, the groups represented thereby may be the same or different, and other symbols represent the same meanings as described above.];

[10] the compound, N-oxide thereof, prodrug thereof, pharmaceutically acceptable salt thereof, or solvate thereof, according to the preceding item [1], wherein the compound represented by the general formula (I) is the compound represented by the general formula (II)

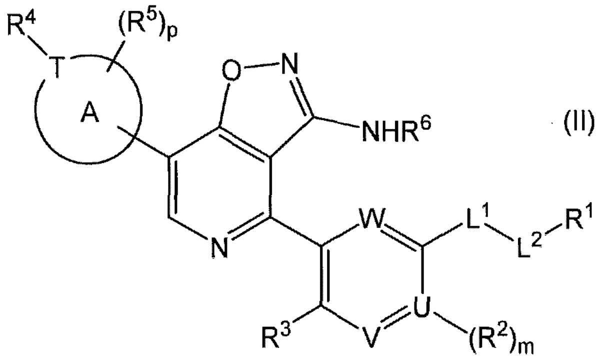

(II)

[wherein all symbols represent the same meanings as described above.];
[11] the compound, N-oxide thereof, prodrug thereof, pharmaceutically acceptable salt thereof or solvate thereof, according to any one of the preceding items [1] to [10], wherein T is a nitrogen atom;
[12] the compound, N-oxide thereof, prodrug thereof, pharmaceutically acceptable salt thereof or solvate thereof, according to any one of the preceding items [9] to [11], wherein U is a carbon atom;
[13] the compound, N-oxide thereof, prodrug thereof, pharmaceutically acceptable salt thereof or solvate thereof, according to any one of the preceding items [1], [3], [5] and [7] to [12], wherein Ring A is pyrazole, triazole (e.g., 1,2,3-triazole and 1,2,4-triazole), tetrazole, oxazole, isoxazole, imidazole, thiazole or isothiazole;
[14] the compound, N-oxide thereof, prodrug thereof, pharmaceutically acceptable salt thereof or solvate thereof, according to the preceding item [1], wherein the compound represented by the general formula (I) is the compound represented by the general formula (III)

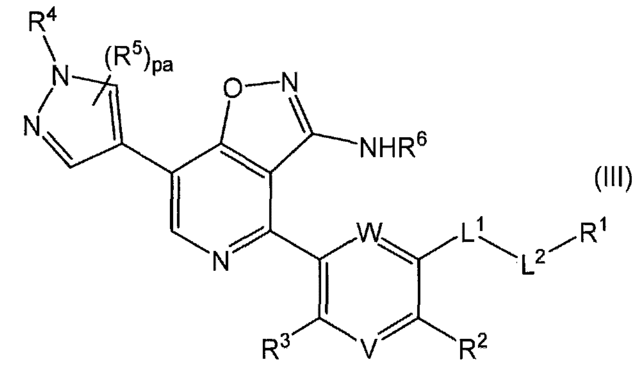

(III)

[wherein pa represents an integer of 0 to 2, and other symbols represent the same meanings as described above.];

[15] the compound, N-oxide thereof, prodrug thereof, pharmaceutically acceptable salt thereof or solvate thereof, according to any one of the preceding items [1] to [14], wherein $L^2$ in any of the general formula (I), general formula (II) and general formula (III) (hereinafter, may be abbreviated as "the general formula (I) or the like") is a bond or C1-3 alkylene group;

[16] the compound, N-oxide thereof, prodrug thereof, pharmaceutically acceptable salt thereof or solvate thereof, according to any one of the preceding items [1] to [15], wherein $L^1$ in any of the general formula (I) or the like is -O-, -CONH-, -CO-, -COz-, -S-, $-SO_2-$ or -SO-;

[17] the compound, N-oxide thereof, prodrug thereof, pharmaceutically acceptable salt thereof or solvate thereof, according to any one of the preceding items [1] to [15], wherein $L^1$ in the general formula (I) or the like is -CONH- (provided that the left side of the group is bound to Ring B), -CO-, $-CO_2-$, -S-, -SOz- or -SO-;

[18] the compound, N-oxide thereof, prodrug thereof, pharmaceutically acceptable salt thereof or solvate thereof, according to any one of the preceding items [1] to [17], wherein $R^1$ is a hydrogen atom, hydroxyl group, C1-4 alkyl group or carboxy group;

[19] the compound, N-oxide thereof, prodrug thereof, pharmaceutically acceptable salt thereof or solvate thereof, according to any one of the preceding items [1] to [17], wherein $R^1$ is a hydrogen atom or C1-4 alkyl group;

[20] the compound, N-oxide thereof, prodrug thereof, pharmaceutically acceptable salt thereof or solvate thereof, according to any one of the preceding items [1] to [19], wherein $R^2$ is a nitro group or $NR^{2a}R^{2b}$;

[21] the compound, N-oxide thereof, prodrug thereof, pharmaceutically acceptable salt thereof or solvate thereof, according to any one of the preceding items [1] to [20], wherein both of $R^{2a}$ and $R^{2b}$ are hydrogen atoms;

[22] the compound, N-oxide thereof, prodrug thereof, pharmaceutically acceptable salt thereof or solvate thereof, according to any one of the preceding items [1] to [21], wherein $R^3$ is a hydrogen atom, halogen atom or hydroxyl group;

[23] the compound, N-oxide thereof, prodrug thereof, pharmaceutically acceptable salt thereof or solvate thereof, according to any one of the preceding items [1] to [22], wherein $R^4$ is a hydrogen atom;

[24] the compound, N-oxide thereof, prodrug thereof, pharmaceutically acceptable salt thereof or solvate thereof, according to any one of the preceding items [1] to [23], wherein $R^6$ is a hydrogen atom;

[25] the compound, N-oxide thereof, prodrug thereof, pharmaceutically acceptable salt thereof or solvate thereof, according to any one of the preceding items [1] to [24], wherein p and pa are integers of 0 or 1;

[26] the compound, N-oxide thereof, prodrug thereof, pharmaceutically acceptable salt thereof or solvate thereof, according to the preceding item [1], wherein the compound represented by the general formula (I) is the compound selected from the group consisting of

(1) 4-(4-amino-2-fluoro-5-methoxyphenyl)-7-(1*H*-pyrazol-4-yl)isoxazolo[5,4-*c*]pyridin-3-amine,
(2) 4-(4-amino-2-fluoro-5-methoxyphenyl)-7-(1*H*-pyrazol-4-yl)isoxazolo[4,5-*c*]pyridin-3-amine,
(3) 4-(4-amino-3-methoxyphenyl)-7-(1*H*-pyrazol-4-yl)isoxazolo[4,5-c]pyridin-3-amine,
(4) 4-(4-amino-2-fluoro-5-(methylthio)phenyl)-7-(1*H*-pyrazol-4-yl)isoxazolo[4,5-*c*]pyridin-3-amine,
(5) 4-(4-amino-2-fluoro-5-(methoxy-$d_3$)phenyl)-7-(1*H*-pyrazol-4-yl)isoxazolo[4,5-*c*]pyridin-3-amine,
(6) 4-(4-amino-2-fluoro-5-(methylsulfonyl)phenyl)-7-(1*H*-pyrazol-4-yl)isoxazolo[4,5-*c*]pyridin-3-amine,
(7) 4-(4-amino-5-(ethylthio)-2-fluorophenyl)-7-(1*H*-pyrazol-4-yl)isoxazolo[4,5-*c*]pyridin-3-amine,
(8) 4-(4-amino-2-fluoro-5-(methylsulfinyl)phenyl)-7-(1*H*-pyrazol-4-yl)isoxazolo[4,5-*c*]pyridin-3-amine,
(9) 4-(4-amino-2-fluoro-3-methoxyphenyl)-7-(1*H*-pyrazol-4-yl)isoxazolo[4,5-*c*]pyridin-3-amine,
(10) methyl 2-amino-5-(3-amino-7-(1*H*-pyrazol-4-yl)isoxazolo[4,5-*c*]pyridin-4-yl)-4-fluorobenzoate,
(11) 2-amino-5-(3-amino-7-(1*H*-pyrazol-4-yl)isoxazolo[4,5-*c*]pyridin-4-yl)-4-fluorobenzoic acid,
(12) 2-amino-5-(3-amino-7-(1*H*-pyrazol-4-yl)isoxazolo[4,5-*c*]pyridin-4-yl)-4-fluorobenzamide,
(13) 4-(4-amino-2-fluoro-5-methoxyphenyl)-7-(3-methyl-1*H*-pyrazol-4-yl)isoxazolo[4,5-*c*]pyridin-3-amine,

(14) 1-(2-amino-5-(3-amino-7-(1*H*-pyrazol-4-yl)isoxazolo[4,5-*c*]pyridin-4-yl)-4-fluorophenyl)ethan-1 -one,
(15) 4-(4-amino-2-chloro-5-(methylthio)phenyl)-7-(1*H*-pyrazol-4-yl)isoxazolo[4,5-*c*]pyridin-3-amine,
(16) ethyl 2-amino-5-(3-amino-7-(1*H*-pyrazol-4-yl)isoxazolo[4,5-*c*]pyridin-4-yl)-4-fluorobenzoate,
(17) 2-amino-5-(3-amino-7-(1*H*-pyrazol-4-yl)isoxazolo[4,5-*c*]pyridin-4-yl)-4-fluoro-*N*-methylbenzamide,
(18) 1-(2-amino-5-(3-amino-7-(1*H*-pyrazol-4-yl)isoxazolo[4,5-*c*]pyridin-4-yl)-4-fluorophenyl)propan-1-one,
(19) 2-amino-5-(3-amino-7-(1*H*-pyrazol-4-yl)isoxazolo[4,5-*c*]pyridin-4-yl)-*N*-ethyl-4-fluorobenzamide,
(20) 1-(2-amino-5-(3-amino-7-(1*H*-pyrazol-4-yl)isoxazolo[4,5-*c*]pyridin-4-yl)phenyl)ethan-1-one,
(21) methyl 2-amino-5-(3-amino-7-(1*H*-pyrazol-4-yl)isoxazolo[4,5-*c*]pyridin-4-yl)benzoate,
(22) 2-amino-5-(3-amino-7-(1*H*-pyrazol-4-yl)isoxazolo[4,5-*c*]pyridin-4-yl)-*N*-propylbenzamide,
(23) 1-(2-amino-5-(3-amino-7-(1*H*-pyrazol-4-yl)isoxazolo[4,5-*c*]pyridin-4-yl)-4-fluorophenyl)butan-1-one,
(24) 2-amino-5-(3-amino-7-(1*H*-pyrazol-4-yl)isoxazolo[4,5-*c*]pyridin-4-yl)-4-fluoro-N-propylbenzamide,
(25) 1-(2-amino-5-(3-amino-7-(1*H*-pyrazol-4-yl)isoxazolo[4,5-*c*]pyridin-4-yl)phenyl)butan-1-one,
(26) 2-hydroxyethyl 2-amino-5-(3-amino-7-(1*H*-pyrazol-4-yl)isoxazolo[4,5-*c*]pyridin-4-yl)-4-fluorobenzoate,
(27) 2-amino-5-(3-amino-7-(1*H*-pyrazol-4-yl)isoxazolo[4,5-*c*]pyridin-4-yl)benzamide,
(28) 2-amino-5-(3-amino-7-(1*H*-pyrazol-4-yl)isoxazolo[4,5-*c*]pyridin-4-yl)-N-methylbenzamide,
(29) 1-(2-amino-5-(3-amino-7-(1*H*-pyrazol-4-yl)isoxazolo[4,5-*c*]pyridin-4-yl)-4-hydroxyphenyl)ethan-1-one,
(30) 2-amino-5-(3-amino-7-(1*H*-pyrazol-4-yl)isoxazolo[4,5-*c*]pyridin-4-yl)-*N*-ethylbenzamide,
(31) 1-(2-amino-5-(3-amino-7-(1*H*-pyrazol-4-yl)isoxazolo[4,5-*c*]pyridin-4-yl)phenyl)propan-1-one,
(32) 2-amino-5-(3-amino-7-(1*H*-pyrazol-4-yl)isoxazolo[4,5-*c*]pyridin-4-yl)-4-chloro-N-ethylbenzamide,
(33) 4-(2-fluoro-5-methoxy-4-nitrophenyl)-7-(1*H*-pyrazol-4-yl)isoxazolo[4,5-*c*]pyridin-3-amine,
(34) 1-(2-amino-5-(3-amino-7-(1*H*-pyrazol-4-yl)isothiazolo[4,5-*c*]pyridin-4-yl)-4-fluorophenyl)ethan-1-one, and
(35) 4-(4-amino-2-fluoro-5-(trifluoromethyl)phenyl)-7-(pyrazol-4-yl)isoxazolo[4,5-c]pyridin-3-amine;

[1-1] a compound represented by the general formula (I-1)

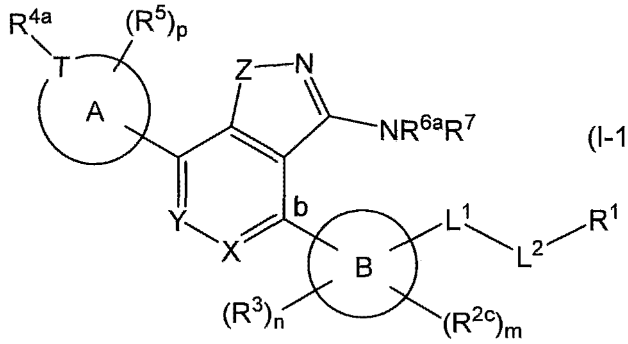

(I-1)

[wherein X and Y represent -CH= or a nitrogen atom (provided that both X and Y do not represent -CH=, simultaneously), respectively, Z represents an oxygen atom or sulfur atom, T represents a carbon atom or nitrogen atom, Ring A represents a 5 to 7-membered monocycle, Ring B represents a 5 to 7-membered monocycle or 8 to 10-membered bicycle, $L^1$ represents a bond, -O-, -CONH-, -CO-, $-CO_2-$, -S-, $-SO_2-$ or -SO-, $L^2$ represents a bond, C1-3 alkylene group, C3-7 cycloalkylene group or phenylene group, $R^1$ represents a hydrogen atom, halogen atom, hydroxyl group, cyano group, $N(R^{1a})_2$ (herein, two $R^{1a}$s each independently represent a hydrogen atom or C1-4 alkyl group), C1-4 alkyl group, carboxy group, C1-4 alkoxycarbonyl group, C1-4 haloalkyl group, methyl-$d_3$ group, C3-7 cycloalkyl group, phenyl group or 3 to 7-membered monocyclic non-aromatic heterocycle, $R^{2c}$ represents a hydrogen atom, hydroxyl group, halogen atom, oxo group, nitro group, cyano group, C1-4 alkoxy group or $-CH_2NR^{2d}R^{2e}$ or $NR^{2d}R^{2e}$ (herein, $R^{2d}$ is a hydrogen atom, C1-4 alkyl group or $R^{FR}$, and $R^{2e}$ represents a hydrogen atom), m represents an integer of 0 or 1, $R^3$ represents a hydrogen atom, halogen atom, hydroxyl group, C1-4 alkyl group, C1-4 alkoxy group, C1-4 haloalkyl group, C1-4 haloalkoxy group or amino group, n represents an integer of 1 to 16 (herein, if n is two or more, the groups represented by a plurality of $R^3$s may be the same or different), $R^{4a}$ represents a hydrogen atom, C1-4 alkyl group, carboxy group or $R^{FR}$, $R^5$ represents a C1-4 alkyl group, p represents an integer of 0 to 5 (herein, if p is two or more, the groups represented by a plurality of $R^5$s may be the same or different), $R^{6a}$ represents a hydrogen atom, C1-4 alkyl group or $R^{FR}$, wherein $R^{FR}$ represents

(i)

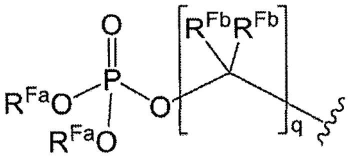

[wherein $R^{Fa}$ each independently represents a hydrogen atom, C1-4 alkyl group, C3-6 cycloalkyl group, -(CHz)zOH, $-CR^{Fb}{}_2OC(=O)$-(C1-4 alkyl), $-CR^{Fb}{}_2OC(=O)O$-(C1-4 alkyl) or benzyl group, $R^{Fb}$ each independently represents a hydrogen atom or C1-4 alkyl group, and q represents an integer of 1 or 2.] (hereinafter, the same group may be collectively referred to as a "phosphonooxyalkyl group"),
(ii)

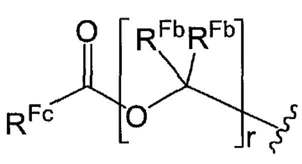 , 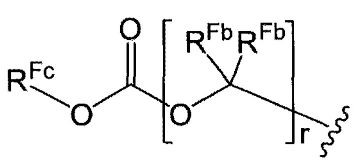 or 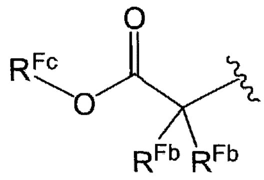

[wherein r represents an integer of 0 or 1,
$R^{Fc}$ represents

(a) $-L^3-R^8$ [wherein $L^3$ is a bond, linear or branched C1-4 alkylene group, C3-6 cycloalkyl group,

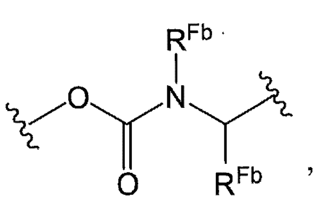 , 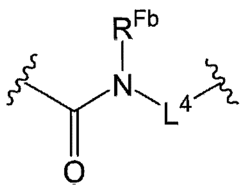 or 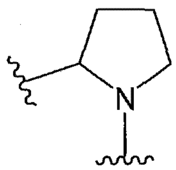

[wherein $L^4$ represents a linear or branched C1-4 alkylene group, and $R^{Fb}$ represents the same meaning as described above.],
$R^8$ represents a C1-4 alkyl group, amino group,

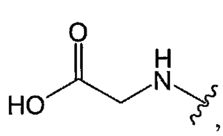 , 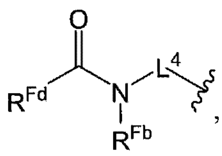 , 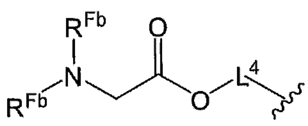 , 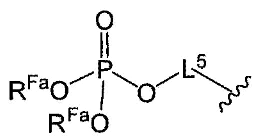 ,

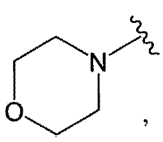 , 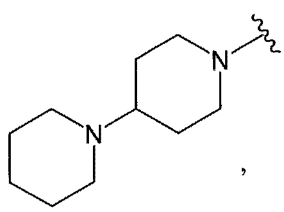 , 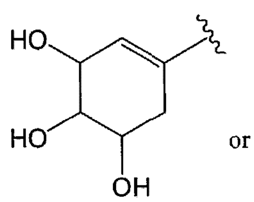 or 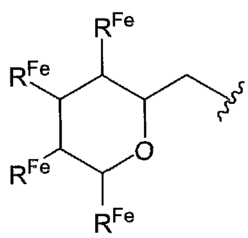

[wherein $R^{Fd}$ represents a C1-4 alkyl group which may be substituted with a halogen atom, hydroxyl group, cyano group, C1-4 alkyl group, C1-4 alkoxy group, or C1-4 haloalkyl group, and $L^5$ represents a bond or linear C1-4 alkylene group which may be substituted with one or two $R^{Fb}$s (provided that two adjacent carbon atoms in the group may be replaced by - $C(=O)NR^{Fb}$-, and two $R^{Fb}$s bonded to the same carbon atom may form a ring), $R^{Fe}$ each independently represents a hydroxyl group or amino group, and other symbols represent the same meanings as described above.].], or

(b)

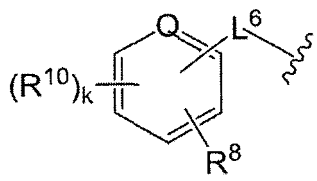

[wherein Q represents -N= or -CH=, $L^6$ represents a bond, $-NR^{Fb}-$, or linear or branched C1-4 alkylene group, $R^{10}$ represents a halogen atom, hydroxyl group, cyano group, C1-4 alkyl group, C1-4 alkoxy group or C1-4 haloalkyl group, k represents an integer from 0 to 3, other symbols represent the same meanings as described above, and herein, the plurality of $R^{10}$s may be the same or different.].],

(iii)

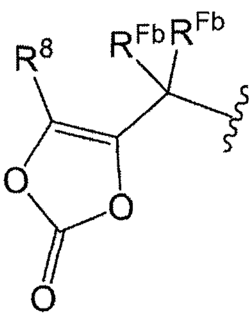

[wherein all symbols represent the same meanings as described above.], or

(iv) a free radical group producing a compound represented by the general formula (I) or N-oxide thereof, as a result of decomposition in vivo,

$R^7$ represents a hydrogen atom and b represents the bonding position of Ring B, provided that two or more of $R^{2d}$, $R^{4a}$ and $R^{6a}$ do not represent $R^{FR}$, simultaneously.], an N-oxide thereof, a pharmaceutically acceptable salt thereof or a solvate thereof;

[1-2] the compound, N-oxide thereof, pharmaceutically acceptable salt thereof or solvate thereof, according to the preceding item [1-1], wherein Ring A is (a) a C5-6 monocyclic carbocycle or (b) a 5 to 6-membered monocyclic heterocycle containing 1 to 4 heteroatoms selected from an oxygen atom, nitrogen atom and sulfur atom;

[1-3] the compound, N-oxide thereof, pharmaceutically acceptable salt thereof or solvate thereof, according to the preceding item [1-1] or [1-2], wherein Ring B is (a) a C5-6 monocyclic carbocycle or (b) a 5 to 6-membered monocyclic heterocycle containing 1 to 4 heteroatoms selected from an oxygen atom, nitrogen atom and sulfur atom;

[1-4] the compound, N-oxide thereof, pharmaceutically acceptable salt thereof or solvate thereof, according to the preceding item [1-1] or [1-3], wherein Ring A is (a) a benzene ring or (b) a 5 to 6-membered monocyclic aromatic heterocycle containing 1 to 4 heteroatoms selected from an oxygen atom, nitrogen atom and sulfur atom;

[1-5] the compound, N-oxide thereof, pharmaceutically acceptable salt thereof or solvate thereof, according to any one of the preceding items [1-1], [1-2] and [1-4], wherein Ring B is (a) a benzene ring or (b) a 5 to 6-membered monocyclic aromatic heterocycle containing 1 to 4 heteroatoms selected from an oxygen atom, nitrogen atom and sulfur atom;

[1-6] the compound, N-oxide thereof, pharmaceutically acceptable salt thereof or solvate thereof, according to any one of the preceding items [1-1], [1-3] and [1-5], wherein Ring A is a 5 to 6-membered monocyclic aromatic nitrogen-containing heterocycle containing 1 to 4 nitrogen atoms, without any other heteroatoms;

[1-7] the compound, N-oxide thereof, pharmaceutically acceptable salt thereof or solvate thereof, according to any one of the preceding items [1-1] to [1-6], wherein Z is an oxygen atom;

[1-8] the compound, N-oxide thereof, pharmaceutically acceptable salt thereof or solvate thereof, according to any one of the preceding items [1-1] to [1-7], wherein X is a nitrogen atom and Y is -CH=;

[1-9] the compound, N-oxide thereof, pharmaceutically acceptable salt thereof, or solvate thereof, according to any one of the preceding items [1-1] to [1-8], wherein

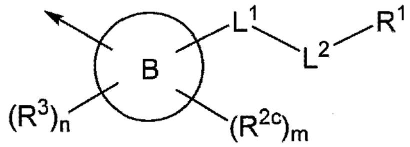

[wherein the arrow is bound to the carbon atom represented by b in the general formula (I-1), and other symbols represent the same meanings as described above.] of the general formula (I-1) is the group represented by the formula (Ib-1)

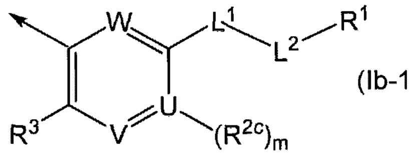

(Ib-1)

[wherein all symbols represent the same meanings as described above.];

[1-10] the compound, N-oxide thereof, pharmaceutically acceptable salt thereof, or solvate thereof, according to the preceding item [1-1], wherein the compound represented by the general formula (I-1) is the compound represented by the general formula (II-1)

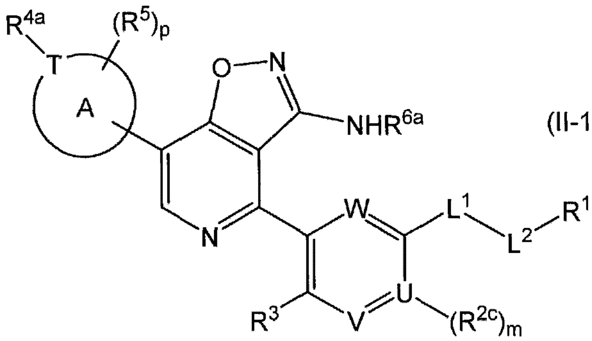

(II-1)

[wherein all symbols represent the same meanings as described above.];

[1-11] the compound, N-oxide thereof, pharmaceutically acceptable salt thereof or solvate thereof, according to any one of the preceding items [1-1] to [1-10], wherein T is a nitrogen atom;

[1-12] the compound, N-oxide thereof, pharmaceutically acceptable salt thereof or solvate thereof, according to any one of the preceding items [1-9] to [1-11], wherein U is a carbon atom;

[1-13] the compound, N-oxide thereof, pharmaceutically acceptable salt thereof or solvate thereof, according to any one of the preceding items [1-1], [1-3], [1-5] and [1-7] to [1-12], wherein Ring A is pyrazole, triazole (e.g., 1,2,3-triazole and 1,2,4-triazole), tetrazole, oxazole, isoxazole, imidazole, thiazole or isothiazole;

[1-14] the compound, N-oxide thereof, pharmaceutically acceptable salt thereof or solvate thereof, according to the preceding item [1-1], wherein the compound represented by the general formula (I-1) is the compound represented by the general formula (III-1)

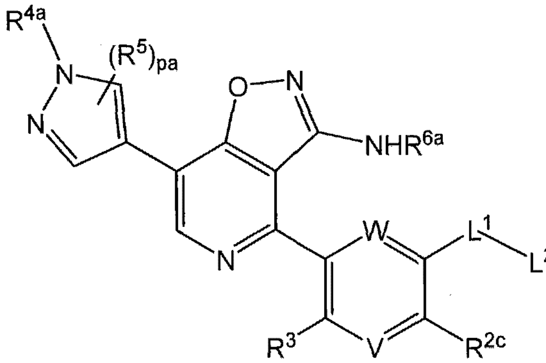

(III-1)

[wherein all symbols represent the same meanings as described above.];

[1-15] the compound, N-oxide thereof, pharmaceutically acceptable salt thereof or solvate thereof, according to any one of the preceding items [1-1] to [1-14], wherein $L^2$ in any of the general formula (I-1), general formula (II-1) and general formula (III-1) (hereinafter, may be abbreviated as "the general formula (I-1) or the like") is a bond or C1-3 alkylene group; [1-16] the compound, N-oxide thereof, pharmaceutically acceptable salt thereof or solvate thereof, according to any one of the preceding items [1-1] to [1-15], wherein $L^1$ in any of the general formula (I-1) or the like is -O-, -CONH-, -CO-, -COz-, -S-, $-SO_2-$ or -SO-;

[1-17] the compound, N-oxide thereof, pharmaceutically acceptable salt thereof or solvate thereof, according to any one of the preceding items [1-1] to [1-15], wherein $L^1$ in any of the general formula (I-1) or the like is -CONH- (provided that the left side of the group is bound to Ring B), -CO-, $-CO_2-$, -S-, $-SO_2-$ or -SO-;

[1-18] the compound, N-oxide thereof, pharmaceutically acceptable salt thereof or solvate thereof, according to any one of the preceding items [1-1] to [1-17]; wherein $R^1$ is a hydrogen atom, hydroxyl group, C1-4 alkyl group or carboxy group;

[1-19] the compound, N-oxide thereof, pharmaceutically acceptable salt thereof or solvate thereof, according to any one of the preceding items [1-1] to [1-17]; wherein $R^1$ is a hydrogen atom or C1-4 alkyl group;

[1-20] the compound, N-oxide thereof, pharmaceutically acceptable salt thereof or solvate thereof, according to any one of the preceding items [1-1] to [1-19], wherein $R^{2c}$ is a nitro group or $NR^{2d}R^{2e}$;

[1-21] the compound, N-oxide thereof, pharmaceutically acceptable salt thereof or solvate thereof, according to any one of the preceding items [1-1] to [1-20], wherein $R^3$ is a hydrogen atom, halogen atom or hydroxyl group;

[1-22] the compound, N-oxide thereof, pharmaceutically acceptable salt thereof or solvate thereof, according to any one of the preceding items [1-1] to [1-21], wherein $R^{2d}$ is a hydrogen atom or $R^{FR}$;

[1-23] the compound, N-oxide thereof, pharmaceutically acceptable salt thereof or solvate thereof, according to any one of the preceding items [1-1] to [1-22], wherein both of $R^{4a}$ and $R^{6a}$ are hydrogen atoms;

[1-24] the compound, N-oxide thereof, pharmaceutically acceptable salt thereof or solvate thereof, according to any one of the preceding items [1-1] to [1-21], wherein $R^{4a}$ is a hydrogen atom or $R^{FR}$;

[1-25] the compound, N-oxide thereof, pharmaceutically acceptable salt thereof or solvate thereof, according to any one of the preceding items [1-1] to [1-21] and [1-24], wherein both of $R^{2d}$ and $R^{6a}$ are hydrogen atoms;

[1-26] the compound, N-oxide thereof, pharmaceutically acceptable salt thereof or solvate thereof, according to any one of the preceding items [1-1] to [1-21], wherein $R^{6a}$ is a hydrogen atom or $R^{FR}$;

[1-27] the compound, N-oxide thereof, pharmaceutically acceptable salt thereof or solvate thereof according to any one of the preceding items [1-1] to [1-21] and [1-26], wherein both of $R^{2d}$ and $R^{4a}$ are hydrogen atoms;

[1-28] the compound, N-oxide thereof, pharmaceutically acceptable salt thereof or solvate thereof according to any one of the preceding items [1-1] to [1-21], wherein any one of $R^{2d}$, $R^{4a}$ and $R^{6a}$ represents $R^{FR}$;

[1-29] the compound, N-oxide thereof, pharmaceutically acceptable salt thereof or solvate thereof according to any one of the preceding items [1-1] to [1-21], wherein $R^{4a}$ represents $R^{FR}$ and both of $R^{2d}$ and $R^{6a}$ are hydrogen atoms;

[1-30] the compound, N-oxide thereof, pharmaceutically acceptable salt thereof or solvate thereof, according to any one of the preceding items [1-1] to [1-29], wherein $R^{FR}$ is

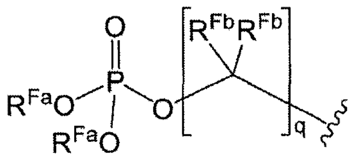

[wherein all symbols represent the same meanings as described above.];

[1-31] the compound, N-oxide thereof, pharmaceutically acceptable salt thereof or solvate thereof, according to the preceding item [1-30], wherein

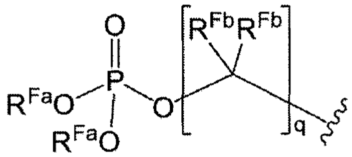

[wherein all symbols represent the same meanings as described above.] is

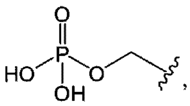, 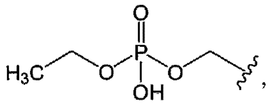, 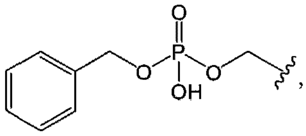, 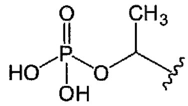,

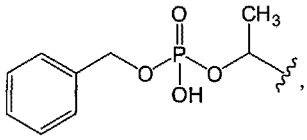, 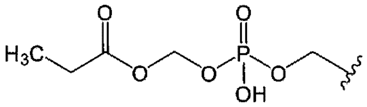, 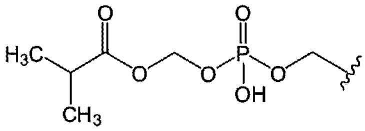,

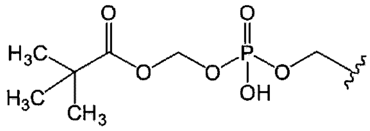 or 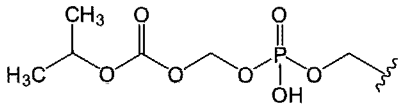 ;

[1-32] the compound, N-oxide thereof, pharmaceutically acceptable salt thereof or solvate thereof, according to any one of the preceding items [1-1] to [1-29], wherein $R^{FR}$ is

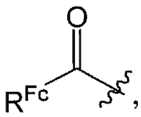, 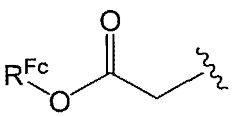, 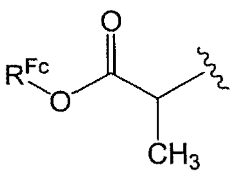, 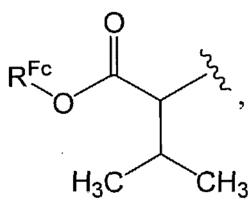,

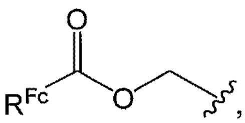, 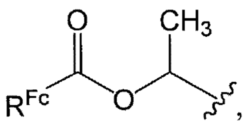, 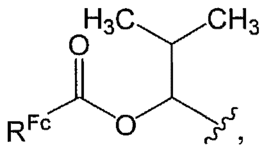,

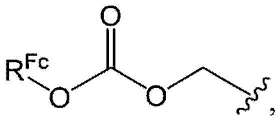, 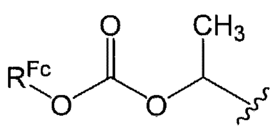 or 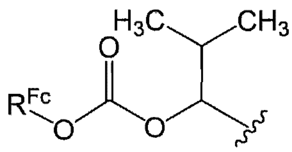

[wherein $R^{Fc}$ represents the same meanings as the preceding items.];

[1-33] the compound, N-oxide thereof, pharmaceutically acceptable salt thereof or solvate thereof, according to any one of the preceding items [1-1] to [1-29] and [1-32], wherein $R^{Fc}$ is

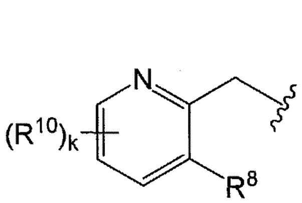 or 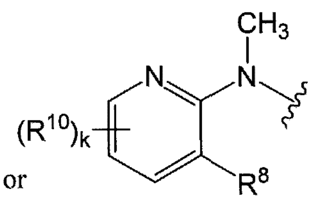

[wherein all symbols represent the same meanings as described above.];

[1-34] the compound, N-oxide thereof, pharmaceutically acceptable salt thereof or solvate thereof, according to any one of the preceding items [1-1] to [1-29] and [1-32], wherein $R^{Fc}$ is

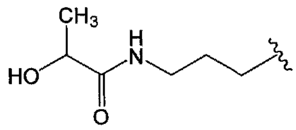,
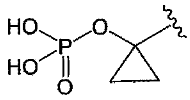,
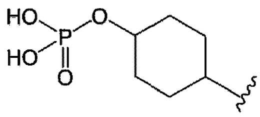,
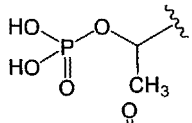,
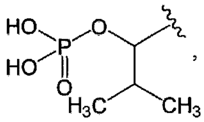,
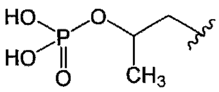,
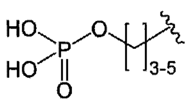,
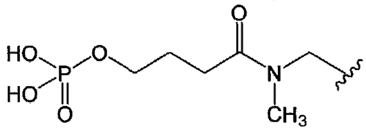,
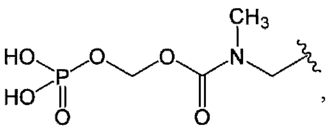,
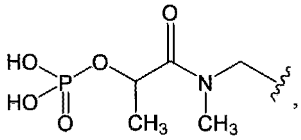,
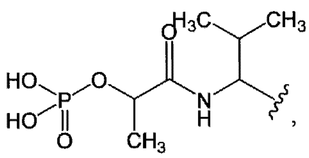,
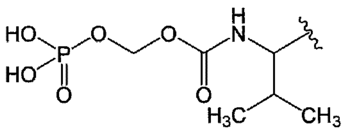,
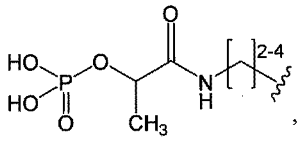,
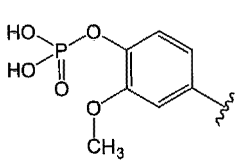,
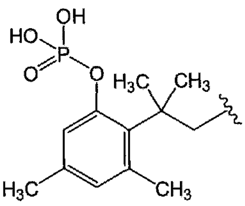,
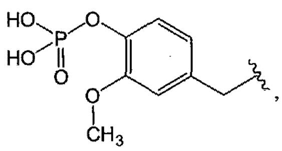,
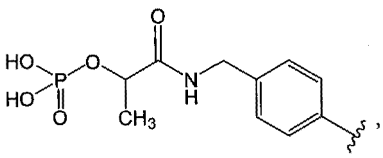,
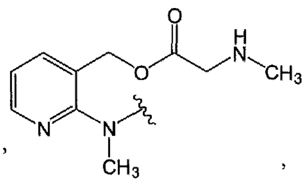,
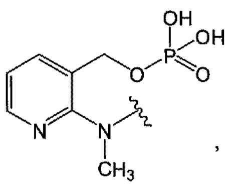,
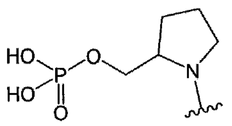,
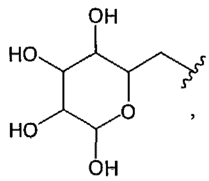,
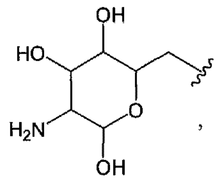,
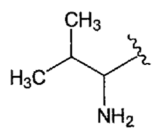 or

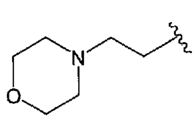

;

[1-35] the compound, N-oxide thereof, pharmaceutically acceptable salt thereof or solvate thereof, according to any one of the preceding items [1-1] to [1-29], wherein $R^{FR}$ is

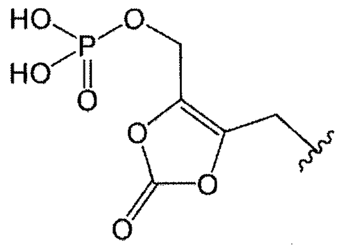

;

[1-36] the pharmaceutically acceptable salt of the compound according to any one of the preceding items [1-1] to [1-31], or solvate thereof, wherein $R^{FR}$ is

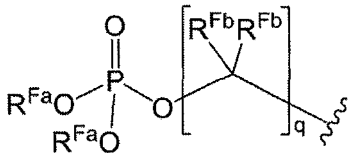

[wherein all symbols represent the same meanings as described above.], and the pharmaceutically acceptable salt described in any one of the preceding items [1-1] to [1-31] is an alkali metal salt (e.g., a lithium salt, sodium salt or potassium salt), alkaline earth metal salt (e.g., a calcium salt and magnesium salt), zinc salt, ammonium salt or organic amine salt, formed together with the same group;

[1-37] the pharmaceutically acceptable salt of the compound according to the preceding item

[1-36] or solvate thereof, wherein the organic amine salt is an aliphatic amine salt (e.g., methylamine salt, dimethylamine salt, cyclopentylamine salt, trimethylamine salt, triethylamine salt, dicyclohexylamine salt, monoethanolamine salt, diethanolamine salt, triethanolamine salt, procaine salt, meglumine salt, diethanolamine salt, tris(hydroxymethyl)aminomethane salt or ethylenediamine salt, etc.), aralkylamine salt (e.g., benzylamine salt, phenethylamine salt, TV, N-dibenzylethylenediamine salt or benetamine salt, etc.), heterocyclic aromatic amine salt (e.g., piperidine salt, pyridine salt, picoline salt, quinoline salt or isoquinoline salt, etc.), quaternary ammonium salt (e.g., tetramethylammonium salt, tetraethylammonium salt, benzyltrimethylammonium salt, benzyltriethylammonium salt, benzyltributylammonium salt, methyltrioctylammonium salt or tetrabutylammonium salt, etc.), basic amino acid salt (e.g., arginine salt or lysine salt, etc.) or N-methyl-D-glucamine salt;

[1-38] the compound, N-oxide thereof, pharmaceutically acceptable salt thereof or solvate thereof, according to any one of the preceding items [1-1] to [1-37], wherein, wherein p and pa are integers of 0 or 1;

[1-39] the compound, N-oxide thereof, pharmaceutically acceptable salt thereof or solvate thereof, according to the preceding item [1-1], wherein the compound represented by the general formula (I-1) is the compound selected from the group consisting of

(1) 4-(4-amino-2-fluoro-5-methoxyphenyl)-7-(1*H*-pyrazol-4-yl)isoxazolo[5,4-*c*]pyridin-3-amine,
(2) 4-(4-amino-2-fluoro-5-methoxyphenyl)-7-(1*H*-pyrazol-4-yl)isoxazolo[4,5-*c*]pyridin-3-amine,
(3) 4-(4-amino-3-methoxyphenyl)-7-(1*H*-pyrazol-4-yl)isoxazolo[4,5-*c*]pyridin-3-amine,
(4) 4-(4-amino-2-fluoro-5-(methylthio)phenyl)-7-(177-pyrazol-4-yl)isoxazolo[4,5-*c*]pyridin-3-amine,
(5) 4-(4-amino-2-fluoro-5-(methoxy-$d_3$)phenyl)-7-(1*H*pyrazol-4-yl)isoxazolo[4,5-*c*]pyridin-3-amine,
(6) 4-(4-amino-2-fluoro-5-(methylsulfonyl)phenyl)-7-(1*H*-pyrazol-4-yl)isoxazolo[4,5-*c*]pyridin-3-amine,
(7) 4-(4-amino-5-(ethylthio)-2-fluorophenyl)-7-(1*H*-pyrazol-4-yl)isoxazolo[4,5-*c*]pyridin-3-amine,
(8) 4-(4-amino-2-fluoro-5-(methylsulfinyl)phenyl)-7-(1*H*-pyrazol-4-yl)isoxazolo[4,5-*c*]pyridin-3-amine,
(9) 4-(4-amino-2-fluoro-3-methoxyphenyl)-7-(1*H*-pyrazol-4-yl)isoxazolo[4,5-*c*]pyridin-3-amine,
(10) methyl 2-amino-5-(3-amino-7-(1*H*-pyrazol-4-yl)isoxazolo[4,5-*c*]pyridin-4-yl)-4-fluorobenzoate,
(11) 2-amino-5-(3-amino-7-(1*H*-pyrazol-4-yl)isoxazolo[4,5-*c*]pyridin-4-yl)-4-fluorobenzoic acid,
(12) 2-amino-5-(3-amino-7-(1*H*-pyrazol-4-yl)isoxazolo[4,5-*c*]pyridin-4-yl)-4-fluorobenzamide,
(13) 4-(4-amino-2-fluoro-5-methoxyphenyl)-7-(3-methyl-1*H*-pyrazol-4-yl)isoxazolo[4,5-*c*]pyridin-3-amine,
(14) methyl 2-amino-5-(3-amino-7-(1-((phosphonooxy)methyl)-1*H*-pyrazol-4-yl)isoxazolo[4,5-*c*]pyridin-4-yl)-4-fluorobenzoate,
(15) 1-(2-amino-5-(3-amino-7-(1*H*-pyrazol-4-yl)isoxazolo[4,5-*c*]pyridin-4-yl)-4-fluorophenyl)ethan-1-one,
(16) 4-(4-amino-2-chloro-5-(methylthio)phenyl)-7-(1*H*-pyrazol-4-yl)isoxazolo[4,5-*c*]pyridin-3-amine,
(17) ethyl 2-amino-5-(3-amino-7-(1*H*-pyrazol-4-yl)isoxazolo[4,5-*c*]pyridin-4-yl)-4-fluorobenzoate,

(18) (4-(4-(5-acetyl-4-amino-2-fluorophenyl)-3-aminoisoxazolo[4,5-*c*]pyridin-7-yl)-1*H*-pyrazol-1-yl)methyl dihydrogen phosphate,
(19) ethyl 2-amino-5-(3-amino-7-(1-((phosphonooxy)methyl)-1*H*-pyrazol-4-yl)isoxazolo[4,5-*c*]pyridin-4-yl)-4-fluorobenzoate,
(20) 2-amino-5-(3-amino-7-(1*H*-pyrazol-4-yl)isoxazolo[4,5-*c*]pyridin-4-yl)-4-fluoro-*N*-methylbenzamide,
(21) 1-(2-amino-5-(3-amino-7-(1*H*-pyrazol-4-yl)isoxazolo[4,5-*c*]pyridin-4-yl)-4-fluorophenyl)propan-1-one,
(22) 2-amino-5-(3-amino-7-(1*H*-pyrazol-4-yl)isoxazolo[4,5-*c*]pyridin-4-yl)-*N*-ethyl-4-fluorobenzamide,
(23) 1-(2-amino-5-(3-anaino-7-(1*H*-pyrazol-4-yl)isoxazolo[4,5-*c*]pyridin-4-yl)phenyl)ethan-1-one,
(24) methyl 2-amino-5-(3-amino-7-(1*H*-pyrazol-4-yl)isoxazolo[4,5-*c*]pyridin-4-yl)benzoate,
(25) 2-amino-5-(3-amino-7-(1*H*-pyrazol-4-yl)isoxazolo[4,5-*c*]pyridin-4-yl)-*N*-propylbenzamide,
(26) 1-(2-amino-S-(3-amino-7-(1*H*-pyrazol-4-yl)isoxazolo[4,5-*c*]pyridin-4-yl)-4-fluorophenyl)butan-1-one,
(27) 2-amino-5-(3-amino-7-(1*H*-pyrazol-4-yl)isoxazolo[4,5-*c*]pyridin-4-yl)-4-fluoro-*N*-propylbenzamide,
(28) 1-(2-amino-5-(3-amino-7-(1*H*-pyrazol-4-yl)isoxazolo[4,5-*c*]pyridin-4-yl)phenyl)butan-1-one,
(29) 2-hydroxyethyl 2-amino-5-(3-amino-7-(1*H*-pyrazol-4-yl)isoxazolo[4,5-*c*]pyridin-4-yl)-4-fluorobenzoate,
(30) 2-amino-5-(3-amino-7-(1*H*-pyrazol-4-yl)isoxazolo[4,5-*c*]pyridin-4-yl)benzamide,
(31) 2-amino-5-(3-amino-7-(1*H*-pyrazol-4-yl)isoxazolo[4,5-*c*]pyridin-4-yl)-*N*-methylbenzamide,
(32) (4-(3-amino-4-(4-amino-5-(ethylcarbamoyl)-2-fluorophenyl)isoxazolo[4,5-*c*]pyridin-7-yl)-1*H*-pyrazol-1-yl)methyl dihydrogen phosphate,
(33) 1-(2-amino-5-(3-amino-7-(1*H*-pyrazol-4-yl)isoxazolo[4,5-*c*]pyridin-4-yl)-4-hydroxyphenyl)ethan-1-one,
(34) 2-amino-5-(3-amino-7-(1*H*-pyrazol-4-yl)isoxazolo[4,5-*c*]pyridin-4-yl)-*N*-ethylbenzamide,
(35) 1-(2-amino-5-(3-amino-7-(1*H*-pyrazol-4-yl)isoxazolo[4,5-*c*]pyridin-4-yl)phenyl)propan-1-one,
(36) 2-amino-5-(3-amino-7-(1*H*-pyrazol-4-yl)isoxazolo[4,5-*c*]pyridin-4-yl)-4-chloro-*N*-ethylbenzamide,
(37) (4-(3-amino-4-(4-amino-2-fluoro-5-(methylthio)phenyl)isoxazolo[4,5-*c*]pyridin-7-yl)-1*H*-pyrazol-1-yl)methyl dihydrogen phosphate,
(38) (4-(3-amino-4-(4-amino-2-fluoro-5-propionylphenyl)isoxazolo[4,5-*c*]pyridin-7-yl)-1*H*-pyrazol-1-yl)methyl dihydrogen phosphate,
(39) (4-(4-(3-acetyl-4-aminophenyl)-3-aminoisoxazolo[4,5-*c*]pyridin-7-yl)-1*H*-pyrazol-1-yl)methyl dihydrogen phosphate,
(40) 4-(2-fluoro-5-methoxy-4-nitrophenyl)-7-(1*H*-pyrazol-4-yl)isoxazolo[4,5-*c*]pyridin-3-amine,
(41) (4-(3-amino-4-(4-amino-2-fluoro-5-(methylsulfonyl)phenyl)isoxazolo[4,5-*c*]pyridin-7-yl)-1*H*-pyrazol-1-yl)methyl dihydrogen phosphate,
(42) (4-(3-amino-4-(4-amino-5-(ethylcarbamoyl)-2-chlorophenyl)isoxazolo[4,5-*c*]pyridin-7-yl)-1*H*-pyrazol-1-yl)methyl dihydrogen phosphate,
(43) 1-(2-amino-5-(3-amino-7-(1*H*-pyrazol-4-yl)isothiazolo[4,5-*c*]pyridin-4-yl)-4-fluorophenyl)ethan-1-one,
(44) 4-(4-amino-2-fluoro-5-(trifluoromethyl)phenyl)-7-(1*H*-pyrazol-4-yl)isoxazolo[4,5-*c*]pyridin-3-amine,
(45) (4-(4-(5-acetyl-4-amino-2-fluorophenyl)-3-aminoisoxazolo[4,5-*c*]pyridin-7-yl)-1*H*-pyrazol-1-yl)methyl (((2R,3S,4S,5R)-3,4,5,6-tetrahydroxytetrahydro-2*H*-pyran-2-yl)methyl)carbonate,
(46) ((((4-(4-(5-acetyl-4-amino-2-fluorophenyl)-3-aminoisoxazolo[4,5-*c*]pyridin-7-yl)-1*H*-pyrazol-1-yl)methoxy)(hydroxy)phosphoryl)oxy)methyl isopropyl carbonate,
(47) 2-((2-((((4-(4-(5-acetyl-4-amino-2-fluorophenyl)-3-aminoisoxazolo[4,5-*c*]pyridin-7-yl)-1*H*-pyrazol-1-yl)methoxy)carbonyl)(mEthyl)amino)pyridin-3-yl)mεthoxy)-*N*-methyl-2-oxoethane-1-ammonium chloride,
(48) (4-(4-(5-acetyl-4-amino-2-fluorophenyl)-3-aminoisoxazolo[4,5-*c*]pyridin-7-yl)-1*H*-pyrazol-1-yl)methyl methyl(3-((phosphonooxy)methyl)pyridin-2-yl)carbamate,
(49) (4-(4-(5-acetyl-4-amino-2-fluorophenyl)-3-aminoisoxazolo[4,5-*c*]pyridin-7-yl)-1*H*-pyrazol-1-yl)methyl (((2R,3S,4R,5R)-5-amino-3,4,6-trihydroxytetrahydro-2*H*-pyran-2-yl)methyl) carbonate,
(50) (4-(4-(5-acetyl-4-amino-2-fluorophenyl)-3-aminoisoxazolo[4,5-*c*]pyridin-7-yl)-1*H*-pyrazol-1-yl)methyl [1,4'-bipiperidine]-1'-carboxylate,
(51) (4-(4-(5-acetyl-4-amino-2-fluorophenyl)-3-aminoisoxazolo[4,5-*c*]pyridin-7-yl)-1*H*-pyrazol-1-yl)methyl (2-morpholinoethyl) carbonate,
(52) (4-(4-(5-acetyl-4-amino-2-fluorophenyl)-3-aminoisoxazolo[4,5-*c*]pyridin-7-yl)-1*H*-pyrazole-1-carbonyl)glycine,
(53) (2-(((1-(4-(4-(5-acetyl-4-amino-2-fluorophenyl)-3-aminoisoxazolo[4,5-*c*]pyridin-7-yl)-1*H*-pyrazol-1-yl)ethoxy)carbonyl)(methyl)amino)pyridin-3-yl)methyl methylglycinate,
(54) methyl 4-(4-(5-acetyl-4-amino-2-fluorophenyl)-3-aminoisoxazolo[4,5-*c*]pyridin-7-yl)-1*H*-pyrazole-1-carboxylate,
(55) 1-(4-(4-(5-acetyl-4-amino-2-fluorophenyl)-3-aminoisoxazolo[4,5-*c*]pyridin-7-yl)-1*H*-pyrazol-1-yl)ethyl dihydrogen phosphate,
(56) (2-(4-(4-(5-acetyl-4-amino-2-fluorophenyl)-3-aminoisoxazolo[4,5-*c*]pyridin-7-yl)-*N*-methyl-1*H*-pyrazole-1-carboxamido)pyridin-3-yl)methyl methylglycinate, and
(57) (2-(4-(4-(5-acetyl-4-amino-2-fluorophenyl)-3-aminoisoxazolo[4,5-*c*]pyridin-7-yl)-*N*-methyl-1*H*-pyrazole-1-carboxamido)pyridin-3-yl)methyl dihydrogen phosphate;

[1-40] the pharmaceutically acceptable salt of the compound or N-oxide thereof according to any one of the preceding items [1-1] to [1-39], or solvate thereof, wherein the pharmaceutically acceptable salt of the compound or N-oxide thereof described in any one of the preceding items [1-1] to [1-39] is an alkali metal salt (e.g., a lithium salt, sodium salt or potassium salt);
[1-41] the solvate of the compound, N-oxide thereof or pharmaceutically acceptable salt thereof according to any one of the preceding items [1-1] to [1-40], wherein the solvate of the compound, N-oxide thereof or pharmaceutically acceptable salt thereof described in any one of the preceding items [1-1] to [1-40] is a hydrate;
[1-42] a hydrate of (4-(4-(5-acetyl-4-amino-2-fluorophenyl)-3-aminoisoxazolo[4,5-*c*]pyridin-7-yl)-1*H*-pyrazol-1-yl)methyl dihydrogen phosphate;
[1-43] the hydrate according to the preceding item [1-42], which is a clathrate hydrate;
[1-44] the hydrate according to the preceding item [1-42] or [1-43], to which two or three water molecules per one molecule of (4-(4-(5-acetyl-4-amino-2-fluorophenyl)-3-aminoisoxazolo[4,5-*c*]pyridin-7-yl)-1*H*-pyrazol-1-yl)methyl dihydrogen phosphate are coordinated;
[2-1] an agent for suppressing the progression of, suppressing the recurrence of and/or treating cancer, containing a STING agonistic compound as an active ingredient, being further administered in combination with an adrenal corticosteroid when

(1) administering the agent containing the STING agonistic compound as an active ingredient (hereinafter, may be abbreviated as a "STING agonist"), or
(2) administering the STING agonist in combination with one or two or more kinds of anti-I neoplastic agents;

[2-2] the agent according to the preceding item [2-1], being further administered in combination with the adrenal corticosteroid when administering the STING agonist;
[2-3] an agent for suppressing the induction of inflammatory cytokine production in blood or tissue in suppressing the progression of, suppressing the recurrence of and/or treating cancer by administering (1) the STING agonist or (2) administering the STING agonist in combination with one or two or more kinds of anti-neoplastic agents, which is an adrenal corticosteroid;
[2-4] an agent for suppressing the progression of, suppressing the recurrence of and/or treating cancer, containing a STING agonistic compound as an active ingredient, being further administered in combination with one or two or more kinds of drugs selected from the group consisting of an adrenal corticosteroid, IL-6 inhibitor and TNF-alpha inhibitor when

(1) administering the STING agonist, or
(2) administering the STING agonist in combination with one or two or more kinds of anti-neoplastic agents;

[2-5] the agent according to the preceding item [2-4], being further administered in combination with one or two or more kinds of drugs selected from the group consisting of the adrenal corticosteroid, IL-6 inhibitor and TNF-alpha inhibitor when administering the STING agonist;
[2-6] an agent for suppressing the induction of inflammatory cytokine production in blood or tissue in suppressing the progression of, suppressing the recurrence of and/or treating cancer by administering (1) the STING agonist or (2) administering the STING agonist in combination with one or two or more kinds of anti-neoplastic agents, which is an adrenal corticosteroid, IL-6 inhibitor or TNF-alpha inhibitor;
[2-7] the agent according to any one of the preceding items [2-1] to [2-6], wherein the STING agonistic compound is

(1) the compound, N-oxide thereof, prodrug thereof, pharmaceutically acceptable salt thereof or solvate thereof, according to any one of the preceding items [1] to [26], or
(2) the compound, N-oxide thereof, pharmaceutically acceptable salt thereof or solvate thereof, according to any one of the preceding items [1-1] to [1-44] (hereinafter, the compounds of the present items (1) and (2) may be collectively abbreviated as the "compound pertaining to the present invention");

[2-8] the agent according to any one of the preceding items [2-1] to [2-6], wherein the STING agonistic compound is any of STING agonistic compounds described in the specification of the patent application selected from the group consisting of WO2015/185565, WO2017/093933, WO2017/175147, WO2017/175156, WO2019/069275, WO2019/069270, WO2019/069269, WO2016/096174, WO2017/186711, WO2019/129880, WO2014/093936,

WO2014/189805, WO2014/189806, WO2016/145102, WO2017/075477, WO2017/106740, WO2018/009466, WO2018/198076, WO2018/200812, WO2017/027645, WO2017/027646, WO2018/067423, WO2018/118665, WO2018/118664, WO2018/208667, WO2019/027858, WO2019/027857, WO2019/125974, WO2019/195124, WO2019/195063, WO2017/011622, WO2016/164619, WO2019/046511, WO2019/051489, WO2019/051488, WO2017/161349, WO2018/009648, WO2018/013887, WO2018/013908, WO2019/046511, WO2019/051489, WO2019/051488, WO2019/161171, WO2020/014127, WO2018/013924, WO2018/060323, WO2018/172206, WO2019/185476, WO2019/185477, WO2017/123669, WO2017/123657, WO2018/045204, WO2020/010092, WO2020/010155, WO2018/100558, WO2019/092660, WO2019/180683, WO2018/098203, WO2018/138685, WO2018/138684, WO2019/118839, WO2020/016782, WO2018/065360, WO2018/152450, WO2018/152453, WO2019/232392, WO2018/156625, US2017/0146519, WO2018/234808, WO2018/234807, WO2018/234805, WO2019/243823, WO2019/243825, WO2019/023459, WO2019/046500, WO2019/046498, WO2019/046496, WO2019/074887, WO2019/160884, WO2019/173587, WO2019/043634, US2017/0050967, WO2019/165032, WO2019/158731, WO2019/134705, WO2019/134707, WO2019/123338, WO2019/123339, WO2019/123340, WO2019/122202, WO2019/100061, WO2020/010451, WO2020/006432, WO2019/238786, WO2019/227007, WO2019/219820, WO2019/211799, WO2019/193542, WO2019/193533, WO2019/193543, WO2019/123340, WO2019/123339, WO2019/123338, WO2019/183578, WO2019/175776, WO2019/170912, WO2020/028565, WO2020/028566, WO2020/038387, WO2020/042995, WO2020/050406, WO2020/057546, WO2020/072492, WO2020/074004, WO2020/092127, WO2020/106736, WO2020/117623, WO2020/117624, WO2020/117625, WO2020/115676, WO2020/124059, WO2020/135715, WO2020/146237, WO2020/151682, WO2020/156363, WO2020/163415, WO2020/178768, WO2020/178769, WO2020/178770, WO2020/202091, WO2020/194160, WO2020/221038, WO2020/232375, WO2020/232378, WO2020/227421, WO2020/252240, WO2020/236586, WO2020/243519, WO2020/249773, WO2021/009362, WO2021/009365, WO2021/000770, WO2021/014365, WO2021/013234, WO2021/013250, WO2021/026009, WO2021/035257, WO2021/035258, WO2021/037179 and WO2021/042024;

[2-9] the agent according to any one of the preceding items [2-1] to [2-6], wherein the STING agonistic compound is ADU-S100 (CAS No.1638241-89-0), MK-1454, MK-2118, SB11285, GSK3745417, BMS-986301, E7766, TAK-676, CRD5500, MAVU-104, SYNB1891, SB11325, SB11396, TTI-10001, exoSTING, VTX-001, SRCB-0074, ISMA-101 or BI-13874456;

[2-10] the agent according to the preceding item [2-8], wherein the STING agonistic compound described in the patent application identified as WO2018/067423 is a compound identified by the number selected from the group consisting of CAS No.2218503-83-2, 2218505-09-8, 2218505-08-7, 2218503-88-7, 2218504-006, 2218504-44-8, 2218504-06-2 and 2218504-10-8;

[2-11] the agent according to the preceding item [2-8], wherein the STING agonistic compound described in the patent application identified as WO2018/100558 is a compound identified by CAS registry number selected from the group consisting of 2228934-37-8, 2228891-92-5, 2228891-91-4, 2228891-93-6, 2228891-97-0, 2228891-94-7, 2228892-02-0, 2228892-01-9, 2228893-53-4, 2228892-08-6, 2228892-16-6, 2228892-15-5, 2228892-09-7, 2228892-61-1, 2228892-60-0, 2228892-59-7, 2228892-69-9, 2228892-68-8, 2228892-94-0, 2228892-93-9, 2228893-00-1, 2228892-99-5, 2228893-32-9, 2228893-31-8, 2228893-13-6, 2228893-12-5, 2228893-17-0, 2228893-16-9, 2228893-44-3 and 2228893-43-2;

[2-12] the agent according to the preceding item [2-8], wherein the STING agonistic compound described in the patent application identified as WO2018/060323 is a compound identified by CAS registry number selected from the group consisting of 2211044-08-3, 2211044-07-2, 2308490-32-4, 2211044-10-7, 2308490-31-3, 2211044-12-9, 2308490-29-9 and 2211044-14-1;

[2-13] the agent according to the preceding item [2-8], wherein the STING agonistic compound described in the patent application identified as WO2017/093933 is a compound identified by CAS registry number selected from the group consisting of 2099072-25-8, 2099072-26-9, 2099072-21-4, 2099072-22-5, 2099073-79-5, 2099072-28-1, 2099072-29-2, 2099072-30-5, 2099072-27-0, 2099072-31-6, 2099072-23-6, 2099072-24-7, 2099072-32-7, 2099072-33-8 and 2099072-34-9;

[2-14] the agent according to any one of the preceding items [2-1] to [2-6], wherein the STING agonistic compound is

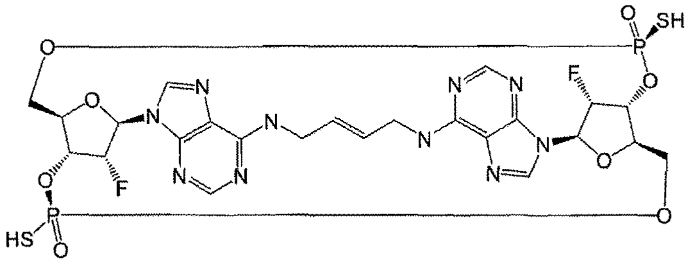

;

[2-15] the agent according to the preceding item [2-7], wherein when the STING agonistic compound is

(1) the compound, N-oxide thereof, prodrug thereof, pharmaceutically acceptable salt thereof or solvate thereof, according to any one of the preceding items [1] to [26], or

(2) the compound, N-oxide thereof, pharmaceutically acceptable salt thereof or solvate thereof, according to any one of the preceding items [1-1] to [1-44], the compound is administered to an adult at about 0.03 to about 10.0 mg/kg (body weight) per dose or about 2.4 to about 800 mg per dose every 1, 2, 3, 4, 6 or 8 weeks by intravenous injection or intravenous drip infusion;

[2-16] the agent according to the preceding item [2-15], wherein the STING agonistic compound is administered to an adult at 0.03 mg/kg, 0.04 mg/kg, 0.05 mg/kg, 0.06 mg/kg, 0.07 mg/kg, 0.08 mg/kg, 0.09 mg/kg or 0.1 mg/kg (body weight) per dose;

[2-17] the agent according to the preceding item [2-15], wherein the STING agonistic compound is administered to an adult at 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg (body weight) per dose;

[2-18] the agent according to the preceding item [2-15], wherein the STING agonistic compound is administered to an adult at 1.1 mg/kg, 1.2 mg/kg, 1.3mg/kg, 1.4 mg/kg, 1.5 mg/kg, 1.6 mg/kg, 1.7 mg/kg, 1.8 mg/kg, 1.9 mg/kg or 2.0 mg/kg (body weight) per dose;

[2-19] the agent according to the preceding item [2-15], wherein the STING agonistic compound is administered to an adult at 2.1 mg/kg, 2.2 mg/kg, 2.3mg/kg, 2.4 mg/kg, 2.5 mg/kg, 2.6 mg/kg, 2.7 mg/kg, 2.8 mg/kg, 2.9 mg/kg or 3.0 mg/kg (body weight) per dose;

[2-20] the agent according to any one of the preceding items [2-15] to [2-19], wherein the STING agonistic compound is administered an arbitrary time only selected from 1 to 16 times (preferably, only once, only twice, only three times, only four times, only six times, only eight times, only ten times, only twelve times, only fourteen times or only sixteen times);

[2-21] the agent according to any one of the preceding items [2-1], [2-3], [2-4] and [2-6] to [2-20], wherein the antineoplastic agent is one or more kinds of agents selected from an alkylating agent, platinum preparation, antimetabolite (e.g., antifolate, pyridine metabolism inhibitor and purine metabolism inhibitor), ribonucleotide reductase inhibitor, nucleotide analog, topoisomerase inhibitor, microtubule polymerization inhibitor, microtubule depolymerization inhibitor, anti-tumor antibiotic, cytokine preparation, anti-hormonal drug, molecular targeting drug and tumor immunotherapeutic drug (preferably, an agent without any concerns of developing cytokine release syndrome);

[2-22] the agent according to the preceding item [2-21], wherein the anti-neoplastic agent is a molecular targeting drug, which is one or more kinds of agents selected from an ALK inhibitor, BCR-ABL inhibitor, EGFR inhibitor, B-RAF inhibitor, VEGFR inhibitor, FGFR inhibitor, c-MET inhibitor, AXL inhibitor, MEK inhibitor, CDK inhibitor, BTK inhibitor, BCL-2 inhibitor, PI3K-$\delta/\gamma$ inhibitor, JAK-1/2 inhibitor, TGFbR1 inhibitor, Cancer cell sternness kinase inhibitor, SYK/FLT3 dual inhibitor, ATR inhibitor, WEE1 kinase inhibitor, multiple tyrosine kinase inhibitor, mTOR inhibitor, HDAC inhibitor, PARP inhibitor, aromatase inhibitor, EZH2 inhibitor, galectin-3 inhibitor, STAT3 inhibitor, DNMT inhibitor, SMO inhibitor, HSP90 inhibitor, $\gamma$-tubulin specific inhibitor, HIF2$\alpha$ inhibitor, glutaminase inhibitor, E3 ligase inhibitor, NRF2 activator, arginase inhibitor, cell cycle inhibitor, IAP antagonist, anti-CD40 antibody, anti-CD70 antibody, anti-HER1 antibody, anti-HER2 antibody, anti-HER3 antibody, anti-VEGF antibody, anti-VEGFR1 antibody, anti-VEGFR2 antibody, anti-CD20 antibody, anti-CD30 antibody, anti-CD38 antibody, anti-TNFRSF10B antibody, anti-TNFRSF10A antibody, anti-MUC1 antibody, anti-MUC5AC antibody, anti-MUC16 antibody, anti-DLL4 antibody, anti-fucosyl GM1 antibody, anti-gpNMB antibody, anti-Mesothelin antibody, anti-MMP9 antibody, anti-GD2 antibody, anti-MET antibody, anti-FOLR1 antibody, anti-CD79b antibody, anti-DLL3 antibody, anti-CD51 antibody, anti-EPCAM antibody, anti-CEACAM5 antibody, anti-CEACAM6 antibody, anti-FGFR2 antibody, anti-CD44 antibody, anti-PSMA antibody, anti-Endoglin antibody, anti-IGF1R antibody, anti-TNFSF11 antibody, anti-GUCY2C, anti-SLC39A6 antibody, anti-SLC34A2 antibody, anti-NCAM1 antibody, anti-ganglioside GD3 antibody, anti-AMHR2 antibody, anti-CD37 antibody, anti-IL1RAP antibody, anti-PDGFR2 antibody, anti-CD200 antibody, anti-TAG-72

antibody, anti-SLITRK6 antibody, anti-DPEP3 antibody, anti-CD19 antibody, anti-NOTCH2/3 antibody, anti-tenascin C antibody, anti-AXL antibody, anti-STEAP1 antibody, anti-CTAA16 antibody, anti-CLDN18 antibody, anti-GM3 antibody, anti-PSCA antibody, anti-FN extra domain B antibody, anti-HAVCR1 antibody, anti-TNFRSF4 antibody, anti-HER1-MET bispecific antibody, anti-EPCAM-CD3 bispecific antibody, anti-Ang2-VEGF bispecific antibody, anti-HER2-CD3 bispecific antibody, anti-HER3-IGF1R bispecific antibody, anti-PMSA-CD3 bispecific antibody, anti-HER1-LGR5 bispecific antibody, anti-SSTR2-CD3 bispecific antibody, anti-CD30-CD16A bispecific antibody, anti-CEA-CD3 bispecific antibody, anti-CD3-CD 19 bispecific antibody, anti-IL3RA-CD3 bispecific antibody, anti-GPRC5D-CD3 bispecific antibody, anti-CD20-CD3 bispecific antibody, anti-TNFRSF17-CD3 bispecific antibody, anti-CLEC12A-CD3 bispecific antibody, anti-HER2-HER3 bispecific antibody, anti-FAP antibody/IL-2 fusion protein and anti-CEA antibody/IL-2 fusion protein;

[2-23] the agent according to the preceding item [2-21], wherein the anti-neoplastic agent is a tumor immunotherapeutic drug, which is one or more kinds of agents selected from an anti-PD-1 antibody, anti-PD-L1 antibody, PD-1 antagonist, PD-L1/VISTA antagonist, PD-L1/TIM3 antagonist, anti-PD-L2 antibody, PD-L1 fusion protein, PD-L2 fusion protein, anti-CTLA-4 antibody, anti-LAG-3 antibody, anti-TIM3 antibody, anti-KIR antibody, anti-BTLA antibody, anti-TIGIT antibody, anti-VISTA antibody, anti-CD137 antibody, anti-CSF-1R antibody/CSF-1R inhibitor, anti-OX40 antibody, OX40L antibody, anti-HVEM antibody, anti-CD27 antibody, anti-GITR antibody/GITR fusion protein, anti-CD28 antibody, anti-CCR4 antibody, anti-B7-H3 antibody, anti-ICOS agonistic antibody, anti-CD4 antibody, anti-DEC-205 antibody/NY-ESO-1 fusion protein, anti-SLAMF7 antibody, anti-CD73 antibody, PEGylated IL-2, IDO inhibitor, TLR agonist, adenosine A2A receptor antagonist, anti-NKG2A antibody, anti-CSF-1 antibody, immunopotentiator, IL-15 super agonist, soluble LAG3, anti-CD47 antibody/CD47 antagonist and IL-12 antagonist;

[2-24] the agent according to the preceding item [2-21], wherein the anti-neoplastic agent is a tumor immunotherapeutic drug, which is an anti-PD-1 antibody, anti-PD-L1 antibody or anti-CTLA-4 antibody;

[2-25] the agent according to the preceding item [2-24], wherein the tumor immunotherapeutic drug is an anti-PD-1 antibody, which is any one of antibody selected from Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, GLS010, CS1003, BAT-1306, AK105, AK103, BI 754091, LZM009, CMAB819, Sym021, SSI-361, JY034, HX008, ISU106, and CX-188;

[2-26] the agent according to the preceding item [2-24], wherein the tumor immunotherapeutic drug is an anti-PD-L1 antibody, which is any one of antibody selected from Atezolizumab, Avelumab, Durvalumab, Manelimab, Pacmilimab, Envafolimab, Cosibelimab, Sugemalimab, BMS-936559, STI-1014, HLX20, SHR-1316, MSB2311, BGB-A333, KL-A167, AK106, AK104, ZKAB001, FAZ053, CBT-502 and JS003;

[2-27] the agent according to the preceding item [2-24], wherein the tumor immunotherapeutic drug is an anti-CTLA-4 antibody, which is any one of antibody selected from Ipilimumab, Zalifrelimab, Nurulimab and Tremelimumab,

[2-28] the agent according to the preceding item [2-25], wherein if the anti-PD-1 antibody is Nivolumab, it is administered to an adult at (1) 1 mg/kg (body weight) per dose every 3 weeks, (2) 3 mg/kg (body weight) per dose every 2 weeks, (3) 2 mg/kg (body weight) per dose every 3 weeks, (4) 80 mg per dose every 3 weeks, (5) 240 mg per dose every 2 weeks, (6) 360 mg per dose every 3 weeks, or (7) 480 mg per dose every 4 weeks, of Nivolumab, by intravenous drip infusion;

[2-29] the agent according to the preceding item [2-25], wherein if the anti-PD-1 antibody is Pembrolizumab, it is administered to an adult at (1) 200 mg per dose every 3 weeks, (2) 400 mg per dose every 6 weeks, or (3) 2 mg/kg (body weight) per dose (up to 200 mg per dose) every 3 weeks, of Pembrolizumab, by intravenous drip infusion;

[2-30] the agent according to the preceding item [2-25], wherein if the anti-PD-1 antibody is Cemiplimab-rwlc, it is administered to an adult at 350 mg of Cemiplimab-rwlc per dose every 3 weeks by intravenous drip infusion;

[2-31] the agent according to the preceding item [2-26], wherein if the anti-PD-L1 antibody is Avelumab, it is administered to an adult at 10 mg/kg (body weight) of Avelumab per dose every 2 weeks by intravenous drip infusion;

[2-32] the agent according to the preceding item [2-26], wherein if the anti-PD-L1 antibody is Atezolizumab, it is administered to an adult at (1) 840 mg per dose every 2 weeks, (2) 1200 mg per dose every 3 weeks, or (3) 1680 mg per dose every 4 weeks, of Atezolizumab, by intravenous drip infusion;

[2-33] the agent according to the preceding item [2-26], wherein if the anti-PD-L1 antibody is Durvalumab, it is administered to an adult at 10 mg/kg (body weight) of Durvalumab per dose every 2 weeks by intravenous drip infusion;

[2-34] the agent according to the preceding item [2-27], wherein if the anti-CTLA-4 antibody is Ipilimumab, it is administered to an adult at (1) 3 mg/kg (body weight) once per day or (2) 1 mg/kg (body weight) once per day, every 3 weeks four times, of Ipilimumab, by intravenous drip infusion;

[2-35] the agent according to any one of the preceding items [2-24] to [2-34], wherein the anti-PD-1 antibody, anti-PD-L1 antibody or anti-CTLA-4 antibody is administered over 30 minutes, 60 minutes, 30 to 60 minutes or 60 minutes or more by intravenous drip infusion;

[2-36] the agent according to any one of the preceding items [2-1] to [2-35], wherein the adrenal corticosteroid is administered by intravenous injection;
[2-37] the agent according to any one of the preceding items [2-1] to [2-36], wherein the adrenal corticosteroid is administered before each administration of the STING agonist;
[2-38] the agent according to any one of the preceding items [2-1] to [2-37], wherein the adrenal corticosteroid is administered at an arbitrary timing between just before and about 2 hours before each administration of the STING agonist;
[2-39] the agent according to any one of the preceding items [2-1] to [2-38], wherein the adrenal corticosteroid is administered at about 30 minutes, about 1 hour, about 90 minutes or about 2 hours before each administration of the STING agonist;
[2-40] the agent according to any one of the preceding items [2-1] to [2-36], wherein the adrenal corticosteroid is administered after each administration of the STING agonist;
[2-41] the agent according to any one of the preceding items [2-1] to [2-36], wherein the adrenal corticosteroid is administered just after each administration of the STING agonist;
[2-42] the agent according to any one of the preceding items [2-1] to [2-36], wherein the adrenal corticosteroid is administered concomitantly with each administration of the STING agonist;
[2-43] the agent according to any one of the preceding items [2-1] to [2-35], wherein if the adrenal corticosteroid is administered orally, it is administered at an arbitrary timing on at least one day before each administration of the STING agonist;
[2-44] the agent according to any one of the preceding items [2-1] to [2-43], wherein the adrenal corticosteroid is one or two or more kinds of agents containing an active ingredient selected from cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone palmitate, dexamethasone sodium metasulfobenzoate, paramethasone, paramethasone acetate, betamethasone, betamethasone dipropionate, betamethasone valerate, betamethasone acetate, betamethasone butyrate propionate and betamethasone sodium phosphate;
[2-45] the agent according to any one of the preceding items [2-1] to [2-43], wherein the adrenal corticosteroid is one or two or more kinds of agents containing an active ingredient selected from hydrocortisone sodium phosphate, hydrocortisone sodium succinate, prednisolone sodium succinate, methylprednisolone sodium succinate, dexamethasone, dexamethasone sodium phosphate and betamethasone sodium phosphate;
[2-46] the agent according to the preceding item [2-45], wherein if the active ingredient of the adrenal corticosteroid is hydrocortisone sodium phosphate, it is administered to an adult at 100 to 1000 mg of hydrocortisone per dose, 1 to 4 times per day by intravenous injection or intravenous drip infusion;
[2-47] the agent according to the preceding item [2-45], wherein if the active ingredient of the adrenal corticosteroid is hydrocortisone sodium succinate, it is administered to an adult

(1) at 50 to 100 mg of hydrocortisone per dose, 1 to 4 times per day by intravenous injection or intravenous drip infusion, or
(2) in an emergency, at 100 to 200 mg of hydrocortisone per dose by intravenous injection or intravenous drip infusion;

[2-48] the agent according to the preceding item [2-45], wherein if the active ingredient of the adrenal corticosteroid is prednisolone sodium succinate, it is administered to an adult at

(1) 10 to 50 mg of prednisolone per dose every 3 to 6 hours by intravenous injection, or
(2) 20 to 100 mg of prednisolone per dose once or twice per day by intravenous drip infusion;

[2-49] the agent according to the preceding item [2-45], wherein if the active ingredient of the adrenal corticosteroid is methylprednisolone sodium succinate, it is administered slowly to an adult at 125 to 2000 mg of methylprednisolone per dose by intravenous injection or intravenous drip infusion;
[2-50] the agent according to the preceding item [2-45], wherein if the active ingredient of the adrenal corticosteroid is dexamethasone sodium phosphate, it is administered to an adult at

(1) 1.65 to 6.6 mg of dexamethasone per dose every 3 to 6 hours by intravenous injection, or
(2) 1.65 to 8.3 mg of dexamethasone per dose once or twice per day by intravenous drip infusion;

[2-51] the agent according to the preceding item [2-45], wherein if the active ingredient of the adrenal corticosteroid is betamethasone sodium phosphate, it is administered to an adult at

(1) 2 to 8 mg of betamethasone per dose every 3 to 6 hours by intravenous injection, or
(2) 2 to 10 mg of betamethasone per dose once or twice per day by intravenous drip infusion;

[2-52] the agent according to the preceding item [2-45], wherein if the active ingredient of the adrenal corticosteroid is dexamethasone, it is administered orally to an adult at 0.5 to 8 mg of dexamethasone per day in 1 to 4 divided doses;
[2-53] the agent according to any one of the preceding items [2-1] or [2-52], wherein the cancer is solid cancer or hematological cancer;
[2-54] the agent according to the preceding item [2-53], wherein the cancer is solid cancer, which is one or more cancers selected from malignant melanoma (e.g., malignant melanoma in skin, oral mucosal epithelium or orbit, etc.), non-small cell lung cancer (e.g., squamous non-small cell lung cancer and non-squamous non-small cell lung cancer), small cell lung cancer, head and neck cancer (e.g., oral cancer, nasopharyngeal cancer, oropharyngeal cancer, hypopharyngeal cancer, laryngeal cancer, salivary gland cancer and tongue cancer), renal cell cancer (e.g., clear cell renal cell cancer), breast cancer, ovarian cancer (e.g., serous ovarian cancer and ovarian clear cell adenocarcinoma), nasopharyngeal cancer, uterine cancer (e.g., cervical cancer and endometrial cancer), anal cancer (e.g., anal canal cancer), colorectal cancer (e.g., high-frequency microsatellite instability (hereinafter, abbreviated as "MSI-H") and/or mismatch repair defect (hereinafter, abbreviated as "dMMR") positive colorectal cancer), rectal cancer, colon cancer, hepatocellular carcinoma, esophageal cancer, gastric cancer, esophagogastric junction cancer, pancreatic cancer, urine urothelial cancer (e.g., bladder cancer, upper urinary tract cancer, ureteral cancer, renal pelvis cancer and urethral cancer), prostate cancer, fallopian tube cancer, primary peritoneal cancer, malignant pleural mesothelioma, gallbladder cancer, bile duct cancer, biliary tract cancer, skin cancer (e.g., uveal melanoma and Merkel cell carcinoma), testicular cancer (germ cell tumor), vaginal cancer, vulvar cancer, penile cancer, small intestine cancer, endocrine system cancer, thyroid cancer, parathyroid cancer, adrenal carcinoma, spinal tumor, neuroblastoma, medulloblastoma, ocular retinoblastoma, neuroendocrine tumor, brain tumor (e.g., glioma (e.g., glioblastoma and gliosarcoma) and meningioma) and squamous cell carcinoma;
[2-55] the agent according to the preceding item [2-53], wherein the cancer is solid cancer, which is bone/soft tissue sarcoma (e.g., Ewing sarcoma, pediatric rhabdomyosarcoma, endometrial leiomyosarcoma, chondrosarcoma, lung sarcoma, osteosarcoma and congenital fibrosarcoma) or Kaposi's sarcoma;
[2-56] the agent according to the preceding item [2-53], wherein the cancer is hematological cancer, which is one or more cancers selected from multiple myeloma, malignant lymphoma (e.g., non-Hodgkin lymphoma (e.g., B-cell non-Hodgkin's lymphoma (e.g., precursor B-cell lymphoblastic lymphoma, precursor B-cell acute lymphoblastic leukemia, chronic B-lymphoid leukemia (small lymphocytic lymphoma), B-cell precursor leukemia, B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B-cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM-monoclonal gammopathy of undetermined significance, $\mu$ heavy chain disease, $\lambda$ heavy chain disease, $\alpha$ heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma with MYC and BCL2 and/or BCL6 rearrangement, high-grade B-cell lymphoma, not otherwise specified and B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell large granular lymphoblastic leukemia, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T-cell lymphoma, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the gastrointestinal tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with T follicular helper phenotype, anaplastic large cell lymphoma, ALK positive, anaplastic large cell lymphoma, ALK negative and breast implant-associated anaplastic large-cell lymphoma)) and Hodgkin lymphoma (e.g., classic Hodgkin lymphoma (e.g., nodular sclerosis, mixed cellularity, lymphocyte-rich and lymphopenic) and nodular lymphoid predominant Hodgkin lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome and chronic myelogenous leukemia), central nervous system malignant lymphoma, and myeloproliferative syndromes;

[2-57] the agent according to any one of the preceding items [2-1] to [2-53], wherein the cancer is pediatric cancer or unknown primary cancer;

[2-58] the agent according to any one of the preceding items [2-1] to [2-57], wherein the cancer is cancer on which the therapeutic effects of other anti-neoplastic agents are insufficient or not sufficient;

[2-59] the agent according to any one of the preceding items [2-1] to [2-58], wherein the cancer is worsened after treatment with other anti-neoplastic agents;

[2-60] the agent according to any one of the preceding items [2-1] to [2-57], wherein a patient with cancer without any histories of treatment with other anti-neoplastic agents;

[2-61] the agent according to any one of the preceding items [2-1] to [2-60], which is prescribed in postoperative adjuvant therapy or preoperative adjuvant therapy;

[2-62] the agent according to any one of the preceding items [2-1] to [2-61], wherein the cancer is incurable or unresectable, metastatic, recurrent, refractory and/or distant metastatic;

[2-63] the agent according to any one of the preceding items [2-1] to [2-62], wherein the ratio of PD-L1-expressing tumor cells among tumor cells in tumor tissue (hereinafter, abbreviated as "TPS") or the value given by dividing the number of PD-L1 positive cells (tumor cells, lymphocytes and macrophages) by the total number of tumor cells and multiplying by 100 (hereinafter, abbreviated as "CPS") is 50% or more, 25% or more, 10% or more, 5% or more, or 1% or more;

[2-64] the agent according to any one of the preceding items [2-1] to [2-62], wherein TPS is less than 50%, less than 25%, less than 10%, less than 5% or less than 1%;

[2-65] the agent according to any one of the preceding items [2-1] to [2-64], wherein tumor mutation burden (hereinafter, abbreviated as "TMB".) of cancer is high frequency (10 mutations or more per $10^6$ bases);

[2-66] the agent according to any one of the preceding items [2-1] to [2-64], wherein TMB of cancer is low frequency (less than 10 mutations per $10^6$ bases);

[2-67] the agent according to any one of the preceding items [2-1] to [2-66], if necessary, being further administered in combination with anti-histamine (e.g., diphenhydramine, chlorpheniramine, ketotifen and olopatadine, etc.), nonsteroidal anti-inflammatory drug (NSAID)(e.g., ibuprofen, indomethacin, felbinac, loxoprofen, meloxicam, ketoprofen, ibuprofen, flurbiprofen, naproxen and celecoxib, etc.) and/or antipyretic analgesics (e.g., aspirin, acetaminophen, isopropylantipyrine, ethenzamide, sazapyrine, salicylamide, sodium salicylate, thiaramide hydrochloride and lactylphenetidine, etc.);

[3-1] a method for suppressing the induction of inflammatory cytokine production in blood or tissue in suppressing the progression of, suppressing the recurrence of and/or treating cancer, comprising

(1) administering the STING agonist (preferably, an agent containing (a) the compound, N-oxide thereof, prodrug thereof, pharmaceutically acceptable salt thereof or solvate thereof, described in the preceding items [1] to [26], or (b) the compound, N-oxide thereof, pharmaceutically acceptable salt thereof or solvate thereof, described in the preceding items [1-1] to [1-44], as an active ingredient), or

(2) administering the STING agonist in combination with one or two or more kinds of anti-neoplastic agents (preferably, an anti-PD-1 antibody, anti-PD-L1 antibody or anti-CTLA-4 antibody), and

further administering an adrenal corticosteroid to a patient in need thereof;

[4-1] a method for suppressing the progression of, suppressing the recurrence of and/or treating cancer, comprising further administering an adrenal corticosteroid

(1) when administering the STING agonist (preferably, an agent containing (a) the compound, N-oxide thereof, prodrug thereof, pharmaceutically acceptable salt thereof or solvate thereof, described in the preceding items [1] to [26], or (b) the compound, N-oxide thereof, pharmaceutically acceptable salt thereof or solvate thereof, described in the preceding items [1-1] to [1-44], as an active ingredient), or
(2) when administering the STING agonist in combination with one or two or more kinds of anti-neoplastic agents (preferably, an anti-PD-1 antibody, anti-PD-L1 antibody or anti-CTLA-4 antibody),

to a patient in need of suppressing the progression of, suppressing the recurrence of and/or treating cancer;
[5-1] an adrenal corticosteroid for suppressing the induction of inflammatory cytokine production in blood or tissue, in suppressing the progression of, suppressing the recurrence of and/or treating cancer, comprising

(1) administering the STING agonist (preferably, an agent containing (a) the compound, N-oxide thereof, prodrug thereof, pharmaceutically acceptable salt thereof or solvate thereof, described in the preceding items [1] to [26], or (b) the compound, N-oxide thereof, pharmaceutically acceptable salt thereof or solvate thereof, described in the preceding items [1-1] to [1-44], as an active ingredient), or
(2) administering the STING agonist in combination with one or two or more kinds of anti-neoplastic agents (preferably, an anti-PD-1 antibody, anti-PD-L1 antibody or anti-CTLA-4 antibody); and

[6-1] use of an adrenal corticosteroid in manufacturing an agent for suppressing the induction of inflammatory cytokine production in blood or tissue, being further administered in suppressing the progression of, suppressing the recurrence of and/or treating cancer, comprising

(1) administering the STING agonist (preferably, an agent containing (a) the compound, N-oxide thereof, prodrug thereof, pharmaceutically acceptable salt thereof or solvate thereof, described in the preceding items [1] to [26], or (b) the compound, N-oxide thereof, pharmaceutically acceptable salt thereof or solvate thereof, described in the preceding items [1-1] to [1-44], as an active ingredient), or
(2) administering the STING agonist in combination with one or two oe more kinds of antineoplastic agents (preferably, an anti-PD-1 antibody, anti-PD-L1 antibody or anti-CTLA-4 antibody).

Furthermore, in another embodiment, the present invention relates to a STING agonist, being administered along with an anti-neoplastic agent in suppressing the progression of, suppressing the recurrence of and/or treating cancer. In other words, constitution of the invention regarding the STING agonist is as follows.

[7-1] An agent for suppressing the progression of, suppressing the recurrence of and/or treating cancer, containing a STING agonistic compound as an active ingredient, being administered along with an anti-neoplastic agent;
[7-2] the agent according to the preceding item [7-1], wherein the cancer is solid cancer or hematological cancer;
[7-3] the agent according to the preceding item [7-2], wherein the solid cancer is any one or more of cancer described in the preceding item [2-54] or [2-55];
[7-4] the agent according to the preceding item [7-2], wherein the hematological cancer is any one or more of cancer described in the preceding item [2-56];
[7-5] the agent according to any one of the preceding items [7-1] to [7-4], wherein the STING agonistic compound is

(1) the compound, N-oxide thereof, prodrug thereof, pharmaceutically acceptable salt thereof or solvate thereof, described in the preceding items [1] to [26], or
(2) the compound, N-oxide thereof, pharmaceutically acceptable salt thereof or solvate thereof, described in the preceding items [1-1] to [1-44];

[7-6] the agent according to any one of the preceding items [7-1] to [7-4], wherein the STING agonistic compound is selected from compounds described in any one of the preceding items [2-8] to [2-14];
[7-7] the agent according to any one of the preceding items [7-1] to [7-5], which if the STING agonistic compound is

(1) the compound, N-oxide thereof, prodrug thereof, pharmaceutically acceptable salt thereof or solvate thereof, described in the preceding items [1] to [26], or
(2) the compound, N-oxide thereof, pharmaceutically acceptable salt thereof or solvate thereof, described in the preceding items [1-1] to [1-44], is prescribed at the usage and/or dosage selected from any one of the preceding items [2-15] to [2-20];

[7-8] the agent according to any one of the preceding items [7-1] to [7-7], wherein the STING agonistic compound

and anti-neoplastic agent are administered in a separate formulation;

[7-9] the agent according to any one of the preceding items [7-1] to [7-8], wherein the anti-neoplastic agent is administered before administration of the STING agonistic compound;

[7-10] the agent according to any one of the preceding items [7-1] to [7-8], wherein the STING agonistic compound is administered before administration of the anti-neoplastic agent;

[7-11] the agent according to any one of the preceding items [7-1] to [7-8], wherein there is the term during which the STING agonistic compound and anti-neoplastic agent are administered concomitantly;

[7-12] the agent according to any one of the preceding items [7-1] to [7-8], wherein the STING agonistic compound and anti-neoplastic agent are administered concomitantly;

[7-13] the agent according to any one of the preceding items [7-1] to [7-7], wherein the STING agonistic compound and anti-neoplastic agent are administered in one formulation thereof;

[7-14] the agent according to any one of the preceding items [7-1] to [7-13], wherein the anti-neoplastic agent is one or two or more kinds of anti-neoplastic agents selected from those described in any one of the preceding items [2-21] to [2-27];

[7-15] the agent according to any one of the preceding items [7-1] to [7-13], wherein the anti-neoplastic agent is an anti-PD-1 antibody, anti-VEGFR2 antibody, anti-CD47 antibody, nucleotide analog, platinum preparation, antifolate, DNMT inhibitor, BCL-2 inhibitor, BTK inhibitor or pyridine metabolism inhibitor;

[7-16] the agent according to the preceding item [7-15], wherein the anti-PD-1 antibody is any one selected from Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM244C8, GLS010, CS1003, BAT-1306, AK105, AK103, BI 754091, LZM009, CMAB819, Sym021, SSI-361, JY034, HX008, ISU106, and CX-188;

[7-17] the agent according to the preceding item [7-15], being prescribed at the usage and/or dosage corresponding to those described in the preceding items [2-28] to [2-30], respectively, if the anti-PD-1 antibody is any of Nivolumab, Pembrolizumab and Cemiplimab-rwlc;

[7-18] the agent according to the preceding item [7-15], wherein the anti-VEGFR2 antibody is any one of antibody selected from Ramucirumab, Alacizumab, Alacizumab pegol, Olinvacimab and AMG596;

[7-19] the agent according to the preceding item [7-15], wherein the anti-CD47 antibody is ALX148;

[7-20] the agent according to the preceding item [7-15], wherein the nucleotide analog is Gemcitabine;

[7-21] the agent according to the preceding item [7-15], wherein the platinum preparation is Cisplatin, Carboplatin, Nedaplatin or Oxaliplatin;

[7-22] the agent according to the preceding item [7-15], wherein the antifolate is Pemetrexed, Leucovorin or Methotrexate;

[7-21] the agent according to the preceding item [7-15], wherein the DNMT inhibitor is Azacitidine;

[7-22] the agent according to the preceding item [7-15], wherein the BCL-2 inhibitor is Navitoclax or Venetoclax;

[7-23] the agent according to the preceding item [7-15], wherein the BTK inhibitor is Ibrutinib or Acalabrutinib; and

[7-24] the agent according to the preceding item [7-15], wherein the pyridine metabolism inhibitor is TS-1 (registered trademark), 5-fluorouracil, UFT, Carmofur, Doxifluridine, FdUrd, Cytarabine or Capecitabine.

[Advantage effects of invention]

Since the compound pertaining to the present invention has the agonistic activity to STING, it can be used as an active ingredient of the agent for suppressing the progression of, suppressing the recurrence of and/or treating cancer or infectious disease.

[Brief description of drawings]

[Figure 1] It shows the anti-tumor activity of the compound pertaining to the present invention (the compound shown in Example 1) in a subcutaneously colon cancer cell line MC38 tumor-bearing mouse model. A vehicle and the compound (open square: 3 mg/kg) (n=6) were administered 7 days after MC38 transplantation, respectively, and the change in tumor volume was continuously measured until 26 days after transplantation.

[Figure 2] It shows the antitumor activities of the compounds pertaining to the present invention (each compound shown in Examples 10, 10 (1) and 10 (2)) in a subcutaneously colon cancer cell line MC38 tumor-bearing mouse model. A vehicle and the respective compounds (n=8) were administered 7 days after MC38 transplantation, respectively, and the change in tumor volume was continuously measured until 28 days after transplantation.

[Figure 3] It shows the antitumor activities of the compounds pertaining to the present invention (each compound shown in Examples 10 (3) to 10 (6)) in a subcutaneously colon cancer cell line MC38 tumor-bearing mouse model. A vehicle and the respective compounds (n=6) were administered 8 days after MC38 transplantation, respectively, and the change in tumor volume was continuously measured until 30 days after transplantation.

[Figure 4] It shows the effect of dexamethasone on the induction of cytokine production by the compound pertaining to the present invention (the compound produced in Example 10(1) (hereinafter, may be abbreviated as "Compound A")) in a subcutaneously colon cancer cell line MC38 tumor-bearing mouse model. In the figure, "Ex.10(1)" represents Compound A, and "Dex" represents dexamethasone. The numbers in the upper part of vertical column charts represent the average values of each cytokine level, and the circle marks are plots representing the cytokine production of each individual.

[Figure 5] It shows the effect of dexamethasone on the anti-tumor activity of Compound A in a subcutaneously colon cancer cell line MC38 tumor-bearing mouse model. References in the legend represent the same meanings as described above.

[Figure 6] It shows the respective anti-tumor effects of Compound A alone, anti-PD-1 antibody (4H2 clone) alone and combination thereof in a subcutaneously colon cancer cell line MC38 tumor-bearing mouse model, respectively. In the legend, "Anti-PD-1" represents the anti-PD-1 antibody, and other references in the legend represent the same meanings as described above.

[Figure 7] It shows the results of evaluating the anti-tumor effects in Figure 6 on individual mouse in each administration group. In the figure, "TF" represents tumor free, the numerator represents the number of individuals with complete tumor remission, and the denominator represents the number of individuals evaluated.

[Figure 8] It shows the effect of dexamethasone on the anti-tumor effect of Compound A and anti-PD-1 antibody (4H2) co-administered in a subcutaneously colon cancer cell line MC38 tumor-bearing mouse model. References in the legend represent the same meanings as described above.

[Figure 9] It shows the respective anti-tumor effects of Compound A alone, anti-PD-1 antibody (4H2) alone, and combination thereof in a subcutaneously melanoma cell line B16F10 tumor-bearing mouse model. In the figure, "rIgG1" represents a control antibody, and other references in the legend represent the same meanings as described above.

[Figure 10] It shows the respective anti-tumor effects of Compound A alone, anti-VEGFR2 antibody (DC 101 clone) alone, and combination thereof in a subcutaneously melanoma cell line B16F10 tumor-bearing mouse model. In the figures, "rIgG1" represents a control antibody, "Anti-VEGFR2" represents the anti-VEGFR2 antibody, and other references in the legends represent the same meanings as above.

[Figure 11] It shows the anti-tumor activity of the compound produced in Example 3 (hereinafter, may be abbreviated as "Compound B") against human acute myeloid leukemia cell lines. In the table, "source" represents the source of the cell line.

[Figure 12] It shows the anti-tumor effect of Compound A in a subcutaneously tumor-bearing model using immunodeficient mice transplanted with human acute myeloid leukemia cell line MV4-11. All references in the figure represent the same meanings as described above.

[Figure 13] They show the respective anti-tumor effects against human acute myeloid leukemia cell lines (KG-1$\alpha$ (upper panel) and THP-1 (lower panel)) in combination with Compound B and BCL-2 inhibitor Venetoclax. In the figure, the horizontal axis represents the evaluated concentration of Venetoclax ("Blank" represents the absence of Venetoclax), and the "signal intensity" on the vertical axis represents the absorbance value (450 nm), and the lower the value, the higher the anti-tumor effect. The legend in the upper right corner represents each evaluated concentration of Compound B. "DMSO" represents a control for Compound B.

[Figure 14] They show the anti-tumor effects against human acute myeloid leukemia cell line CMK in combination of Compound B with pyridine metabolism inhibitor Cytarabine (upper panel) and DNMT inhibitor Azacitidine (lower panel), respectively. The horizontal axis in the upper panel represents the evaluated concentration of Cytarabine, and the horizontal axis in the lower panel represents the evaluated concentration of Azacitidine, and other symbols represent the same meanings as described above.

[Figure 15] They show the respective anti-tumor effects against human B-cell lymphoma cell lines (DOHH2 (upper panel) and OCI-Ly3 (lower panel)) in combination with Compound B and BCL-2 inhibitor Navitoclax. In the figures, the horizontal axis represents the evaluated concentration of Navitoclax, and other symbols represent the same meanings as described above.

[Description of Embodiments]

The inventions relate to

(1) a method for suppressing the progression of, suppressing the recurrence of and/or treating cancer, comprising

further administering in combination with an adrenal corticosteroid when administering the STING agonist,
(2) a method for suppressing the progression of, suppressing the recurrence of and/or treating cancer, comprising further administering in combination with an adrenal corticosteroid when administering the STING agonist in combination with one or more kinds of anti-neoplastic agents, or
(3) use of an adrenal corticosteroid for suppressing the induction of inflammatory cytokine production in blood or tissue, in suppressing the progression of, suppressing the recurrence of and/or treating cancer, comprising (a) administering the STING agonist, or (b) administering the STING agonist in combination with one or more kinds of anti-neoplastic agents.

In other words, the present invention is characterized by further administering in combination with the adrenal corticosteroid to maximize the effect in suppressing the progression of, suppressing the recurrence of and/or treating cancer while reducing the induction of cytokine production caused by the STING agonist administered to suppress the progression of, suppress the recurrence of and/or treat cancer to acceptable level.

[STING agonistic compound]

The STING agonistic compounds pertaining to the present invention are not particularly limited as long as being a compound with the STING agonistic activity, but preferable the compounds pertaining to the present invention, examples of which include (1) the compound, N-oxide thereof, prodrug thereof, pharmaceutically acceptable salt thereof or solvate thereof, described in any one of the preceding items [1] to [26], in the present specification, and (2) the compound, N-oxide thereof, pharmaceutically acceptable salt thereof or solvate thereof, described in any one of the preceding items [1-1] to [1-44].

In the specification of the present invention, the interpretation of the groups used in the general formula (I) or the like and the general formula (I-1) or the like, representing the compounds pertaining to the present invention, respectively, are as follows.

In the specification of the present invention, examples of the "halogen atom" include a fluorine atom, chlorine atom, bromine atom and iodine atom.

In the specification of the present invention, examples of the "C1-4 alkyl group" include a methyl group, ethyl group, *n*-propyl group, isopropyl group, *n*-butyl group, isobutyl group, *sec*-butyl group and *t*ert-butyl group.

In present specification, examples of the "linear C1-4 alkylene group" include a methylene group, ethylene group, *n*-propylene group and n-butylene group.

In the present specification, examples of the "linear or branched chain C1-4 alkylene group" include a methylene group, ethylene group, *n*-propylene group, isopropylene group, *n*-butylene group, isobutylene group, *sec*-butylene group and *tert*-butylene group.

In the specification of the present invention, examples of the "C1-5 alkyl group" include a methyl group, ethyl group, *n*-propyl group, isopropyl group, *n*-butyl group, isobutyl group, *sec*-butyl group, *tert*-butyl group, pentyl group, isopentyl group and 2,3-dimethylpropyl group.

In the specification of the present invention, the "C1-3 alkylene group" is a methylene group, ethylene group or propylene group.

In the specification of the present invention, examples of the "C1-4 alkoxy group" include a methoxy group, ethoxy group, *n*-propoxy group, isopropoxy group, *n*-butoxy group, isobutoxy group, *sec*-butoxy group, *tert*-butoxy group and the like.

In the specification of the present invention, examples of the "C1-4 haloalkyl group" include a fluoromethyl group, chloromethyl group, bromomethyl group, iodomethyl group, difluoromethyl group, trifluoromethyl group, 1-fluoroethyl group, 2-fluoroethyl group, 2-chloroethyl group, pentafluoroethyl group, 1-fluoropropyl group, 2-chloropropyl group, 3-fluoropropyl group, 3-chloropropyl group, 4,4,4-trifluorobutyl group and 4-bromobutyl and the like.

In the specification of the present invention, examples of the "C1-4 haloalkoxy group" include a trifluoromethoxy group, trichloromethoxy group, chloromethoxy group, bromomethoxy group, fluoromethoxy group, iodomethoxy group, difluoromethoxy group, dibromomethoxy group, 2-chloroethoxy group, 2,2,2-trifluoroethoxy group, 2,2,2-trichloroethoxy group, 3-bromopropoxy group, 3-chloropropoxy group, 2,3-dichloropropoxy group and the like.

In the specification of the present invention, examples of the "C3-6 cycloalkyl group" include a cyclopropyl group, cyclobutyl group, cyclopentyl group and cyclohexyl group.

In the specification of the present invention, examples of the "C3-7 cycloalkyl group" include a cyclopropyl group, cyclobutyl group, cyclopentyl group, cyclohexyl group and cycloheptyl group.

In the specification of the present invention, examples of the "C3-7 cycloalkylene group" include a cyclopropylene group, cyclobutylene group, cyclopentylene group, cyclohexylene group and cycloheptylene group.

In the specification of the present invention, examples of the "C1-4 alkoxycarbonyl group" include a methoxycarbonyl group, ethoxycarbonyl group, *n*-propoxycarbonyl group, isopropoxycarbonyl group, *n*-butoxycarbonyl group,

isobutoxycarbonyl group, *sec*-butoxycarbonyl group, *tert*-butoxycarbonyl group and the like.

In the specification of the present invention, examples of the "C5-6 monocyclic carbocycle" include a cyclopentane, cyclohexane, cyclopentene, cyclohexene, cyclopentadiene, cyclohexadiene, benzene and the like.

In the specification of the present invention, examples of the "5 to 7-membered monocycle" include a cyclopentane, cyclohexane, cyclopentene, cyclohexene, cyclopentadiene, cyclohexadiene, benzene, cycloheptane, cycloheptene, cycloheptadiene, pyrrole, oxazole, isoxazole, thiazole, isothiazole, pyrroline, pyrrolidine, dihydrooxazole, tetrahydrooxazole, dihydroisoxazole, tetrahydroisoxazole, dihydrothiazole, tetrahydrothiazole, dihydroisothiazole, tetrahydroisothiazole, imidazole, pyrazole, furazan, oxadiazole, thiadiazole, imidazoline, imidazolidine, pyrazoline, pyrazolidine, dihydrofurazan, tetrahydrofurazan, dihydrooxadiazole, tetrahydrooxadiazole, dihydrothiadiazole, tetrahydrothiadiazole, triazole, triazoline, triazolidine, tetrazole, tetrazoline, tetrazolidine, furan, dihydrofuran, tetrahydrofuran, oxolane, dioxolane, thiophene, dihydrothiophene, tetrahydrothiophene, dithiolane, pyridine, oxazine, thiazine, dihydropyridine, tetrahydropyridine, piperidine, dihydrooxazine, tetrahydrooxazine, dihydrothiazine, tetrahydrothiazine, morpholine, thiomorpholine, pyrazine, pyrimidine, pyridazine, oxadiazine, thiadiazine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrooxadiazine, tetrahydrooxadiazine, dihydrothiadiazine, tetrahydrothiadiazine, pyran, dihydropyran, tetrahydropyran, oxothiane, dioxothiane, oxathiane, dioxane, thiopyran, dihydrothiopyran, tetrahydrothiopyran, dithiane, azepine, diazepine, oxepin, thiepine, oxazepine, oxadiazepine, thiazepine, thiadiazepine, dihydroazepine, tetrahydroazepine, perhydroazepine, dihydrodiazepine, tetrahydrodiazepine, perhydrodiazepine, dihydrooxepin, tetrahydrooxepin, perhydrooxepin, dihydrothiepine, tetrahydrothiepine, perhydrothiepine, dihydrooxazepine, tetrahydrooxazepine, perhydrooxazepine, dihydrooxadiazepine, tetrahydrooxadiazepine, perhydrooxadiazepine, dihydrothiazepine, tetrahydrothiazepine, perhydrothiazepine, dihydrothiadiazepine, tetrahydrothiadiazepine, perhydrothiadiazepine and the like.

In the specification of the present invention, examples of the "8 to 10-membered bicycle" include a pentalene, perhydropentalene, indene, perhydroindene, indane, azulene, perhydroazulene, naphthalene, dihydronaphthalene, tetrahydronaphthalene, perhydronaphthalene, thienopyrazole, thienoimidazole, pyrazolothiazole, indole, isoindole, indolizine, benzofuran, isobenzofuran, benzothiophene, isobenzothiophene, indazole, purine, benzoxazole, benzothiazole, benzimidazole, imidazopyridine, benzofurazan, benzothiadiazole, benzotriazole, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydrobenzoxazole, perhydrobenzoxazole, dihydrobenzothiazole, perhydrobenzothiazole, dihydrobenzoimidazole, perhydrobenzoimidazole, dioxaindane, benzodithiolane, dithianaphthalene, quinoline, isoquinoline, quinolidine, phthalazine, pteridine, naphthyridine, quinoxaline, quinazoline, cinnoline, chromene, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzooxathiane, dihydrobenzoxazine, dihydrobenzothiazine, pyrazinomorpholine, benzodioxane, chroman, benzodithiane and the like.

In the specification of the present invention, examples of the "5 to 6-membered monocyclic heterocycle containing 1 to 4 heteroatoms selected from an oxygen atom, nitrogen atom and sulfur atom" include a pyrrole, oxazole, isoxazole, thiazole, isothiazole, pyrroline, pyrrolidine, dihydrooxazole, tetrahydrooxazole, dihydroisoxazole, tetrahydroisoxazole, dihydrothiazole, tetrahydrothiazole, dihydroisothiazole, tetrahydroisothiazole, imidazole, pyrazole, furazan, oxadiazole, thiadiazole, imidazoline, imidazolidine, pyrazoline, pyrazolidine, dihydrofurazan, tetrahydrofurazan, dihydrooxadiazole, tetrahydrooxadiazole, dihydrothiadiazole, tetrahydrothiadiazole, triazole, triazoline, triazolidine, tetrazole, tetrazoline, tetrazolidine, furan, dihydrofuran, tetrahydrofuran, oxolane, dioxolane, thiophene, dihydrothiophene, tetrahydrothiophene, dithiolane, pyridine, oxazine, thiazine, dihydropyridine, tetrahydropyridine, piperidine, dihydrooxazine, tetrahydrooxazine, dihydrothiazine, tetrahydrothiazine, morpholine, thiomorpholine, pyrazine, pyrimidine, pyridazine, oxadiazine, thiadiazine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrooxadiazine, tetrahydrooxadiazine, dihydrothiadiazine, tetrahydrothiadiazine, pyran, dihydropyran, tetrahydropyran, oxothiane, dioxothiane, oxathiane, dioxane, thiopyran, dihydrothiopyran, tetrahydrothiopyran, dithiane and the like.

In the specification of the present invention, examples of the "5 to 6-membered monocyclic aromatic heterocycle containing 1 to 4 heteroatoms selected from an oxygen atom, nitrogen atom and sulfur atom" include a pyrrole, imidazole, triazole, tetrazole, pyrazole, furan, thiophene, oxazole, isoxazole, thiazole, isothiazole, furazan, oxadiazole, thiadiazole, pyridine, pyrazine, pyrimidine, pyridazine and the like.

In the specification of the present invention, examples of the "5 to 6-membered monocyclic aromatic nitrogen-containing heterocycle containing 1 to 4 nitrogen atoms and without any other heteroatoms" include a pyrrole, imidazole, triazole, tetrazole, pyrazole, pyridine, pyrazine, pyrimidine, pyridazine and the like.

In the specification of the present invention, examples of the "3 to 7-membered monocyclic non-aromatic heterocycle" include an oxirane, aziridine, thiirane, azetidine, oxetane, thietane, pyrroline, pyrrolidine, imidazoline, imidazolidine, triazoline, triazolidine, tetrazoline, tetrazolidine, pyrazoline, pyrazolidine, dihydrofuran, tetrahydrofuran, dihydrothiophene, tetrahydrothiophene, dihydrooxazole, tetrahydrooxazole, dihydroisoxazole, tetrahydroisoxazole, dihydrothiazole, tetrahydrothiazole, dihydroisothiazole, tetrahydroisothiazole, dihydrofurazan, tetrahydrofurazan, dihydrooxadiazole, tetrahydrooxadiazole, dihydrothiadiazole, tetrahydrothiadiazole, oxolane, dioxolane, dithiolane, pyran, thiopyran, oxazine, oxadiazine, thiazine, thiadiazine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydropyran, tetrahydropyran, dihydrothiopyran, tetrahydrothiopyran, dihydrooxazine, tetrahydrooxazine, dihydrooxadiazine, tetrahydrooxadiazine, dihydrothiazine, tetrahydrothiazine, dihydrothiadiazine, tetrahydrothiadiazine, morpholine, thiomorpholine, oxothiane, dioxothiane, oxathiane, dioxane, dithiane, azepine, diazepine, oxepin, thiepine, oxazepine, oxadiazepine, thiazepine, thiadiazepine, dihydroazepine, tetrahydroazepine, perhydroazepine, dihydrodiazepine, tetrahydrodiazepine, perhydrodiazepine, dihydrooxepin, tetrahydrooxepin, perhydrooxepin, dihydrothiepine, tetrahydrothiepine, perhydrothiepine, dihydrooxazepine, tetrahydrooxazepine, perhydrooxazepine, dihydrooxadiazepine, tetrahydrooxadiazepine, perhydrooxadiazepine, dihydrothiazepine, tetrahydrothiazepine, perhydrothiazepine, dihydrothiadiazepine, tetrahydrothiadiazepine, perhydrothiadiazepine and the like.

In the specification of the present invention, the group represented by "*t*-Bu" represents a *tert*-butyl group.

In the specification of the present invention, examples of the "free radical group producing a compound represented by the general formula (I) or N-oxide thereof, as a result of decomposition in vivo" include the group defined as $R^{FR}$.

Ring A in the general formula (I), (I-1), (II) or (II-1) pertaining to the present invention is preferably a 5 to 6-membered monocyclic aromatic heterocycle containing 1 to 4 heteroatoms selected from an oxygen atom, nitrogen atom and sulfur atom, more preferably pyrazole, triazole (e.g., 1,2,3-triazole and 1,2,4-triazole), tetrazole, oxazole, isoxazole, imidazole, thiazole or isothiazole, and furthermore preferably, pyrazole, while Ring B of the general formula (I) or (I-1) pertaining to the present invention is preferably (i) a C5-6 monocyclic carbocycle or (ii) 5 to 6-membered monocyclic heterocycle containing 1 to 4 heteroatoms selected from an oxygen atom, nitrogen atom and sulfur atom, and more preferably a benzene ring.

Furthermore, Z in the general formula (I) or (I-1) pertaining to the present invention is preferably an oxygen atom, Y is preferably -CH=, and X is preferably a nitrogen atom.

$L^2$ in the general formula (I) or the like, the formula (Ib), the general formula (I-1) or the like or the formula (Ib-1) pertaining to the present invention is preferably a bond or C1-3 alkylene group, and more preferably, a bond, and $L^1$ is preferably -O-, -CONH-, -CO-, -COz-, -S-, -SOz- or -SO-, and more preferably -CONH- (provided that the left side of the group is attached to Ring B), -CO-, $-CO_2-$, -S-, $-SO_2-$ or -SO-, $R^1$ is preferably a hydrogen atom, hydroxyl group, C1-4 alkyl group or carboxy group, more preferably a hydrogen atom or C1-4 alkyl group, and furthermore preferably a hydrogen atom, methyl group, ethyl group or n-propyl group, $R^2$ and $R^{2c}$ are preferably nitro groups and $NR^{2a}R^{2b}$ and $NR^{2d}R^{2e}$, respectively, more preferably amino groups, and $R^3$ is preferably a hydrogen atom, halogen atom or hydroxyl group, and more preferably a halogen atom.

In the general formula (I) or the like, the formula (Ib), the general formula (I-1) or the like or the formula (Ib-1) pertaining to the present invention, m is preferably 1 and p and pa are preferably an integer of 0 or 1, and more preferably 0. In the formula (Ib) or (Ib-1) or the general formula (II), (II-1), (III) or (III-1) pertaining to the present invention, n is preferably 1 or 2.

$R^{2a}$, $R^4$ and $R^6$ in the general formula (I) or the like pertaining to the present invention are preferably hydrogen atoms. On the other hand, $R^{2d}$, $R^{4a}$ and $R^{6a}$ in the general formula (I-1) or the like are preferably hydrogen atoms or $R^{FR}$s. Herein, two or more of $R^{2d}$, $R^{4a}$ and $R^{6a}$ may represent $R^{FR}$s, preferably two or more of $R^{2d}$, $R^{4a}$ and $R^{6a}$ do not represent $R^{FR}$s, simultaneously. Furthermore, any one of $R^{2d}$, $R^{4a}$ and $R^{6a}$ in the general formula (I-1) or the like represents $R^{FR}$, more preferably $R^{2d}$ and $R^{6a}$ are hydrogen atoms and $R^{4a}$ represents $R^{FR}$.

Preferable examples of the phosphonooxyalkyl groups which may be represented by $R^{FR}$ in the general formula (I-1) or the like include

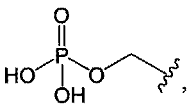, 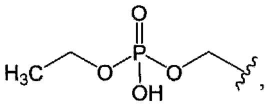, 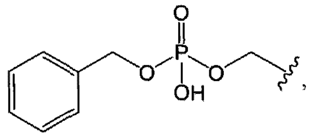, 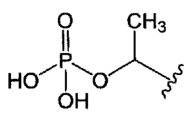,

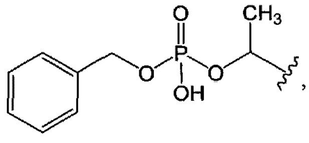, 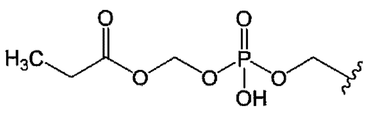, 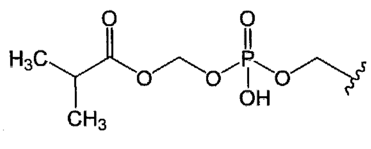,

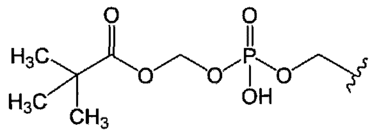 and 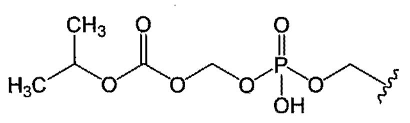,

more preferably $-CH_2OP(=O)(OH)_2$.

Preferable examples of other groups which may be represented by $R^{FR}$ in the general formula (I-1) or the like include

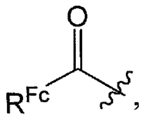, 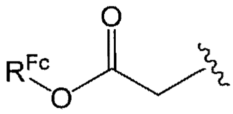, 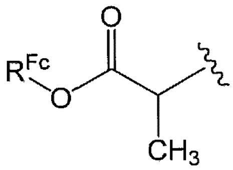, 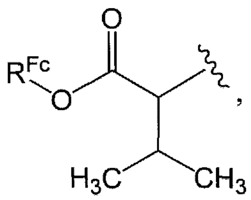,

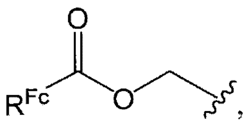, 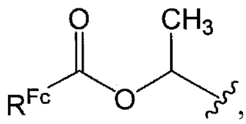, 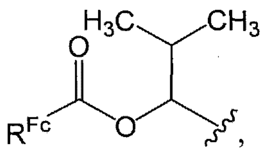,

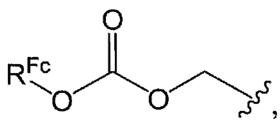, 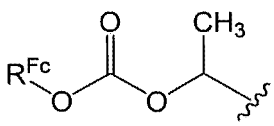 and 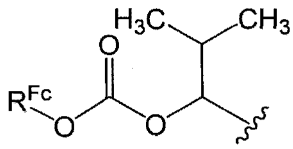

[wherein $R^{Fc}$ represents the same meaning as described above.], and furthermore preferable examples of $R^{Fc}$ include

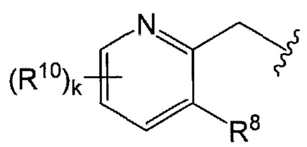 and 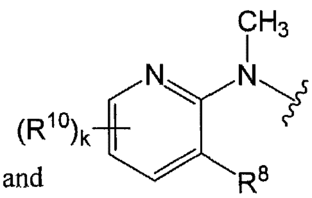

[wherein all symbols represent the same meanings as described above.].

Another preferable examples of $R^{Fc}$ include

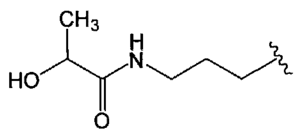, 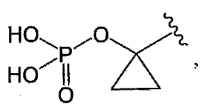, 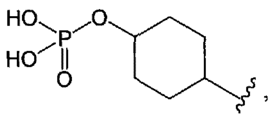, 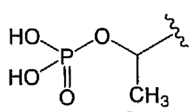,

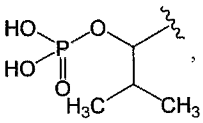, 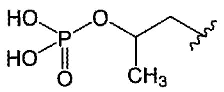, 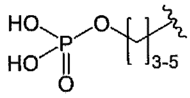, 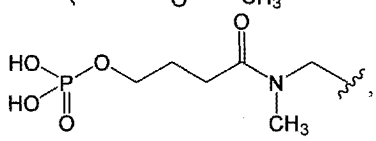,

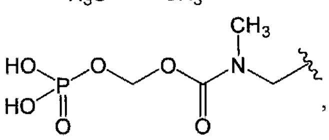, 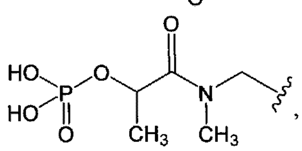, 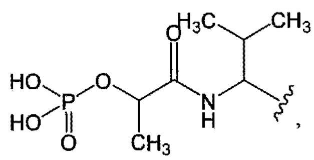,

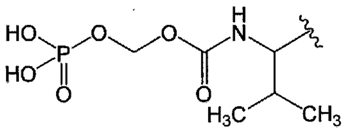, 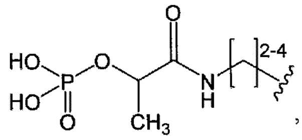,

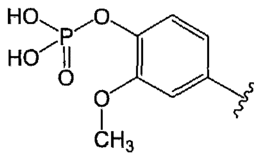, 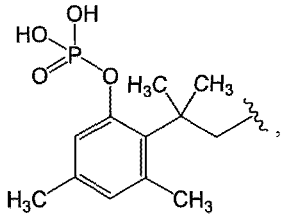, 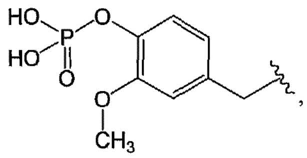,

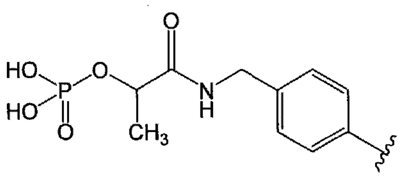, 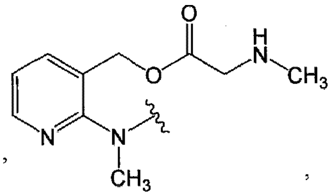, 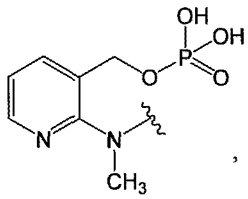,

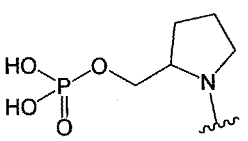, 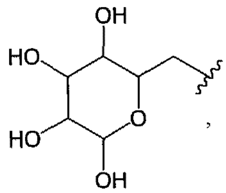, 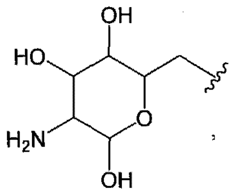, 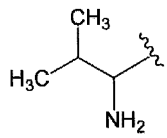 and

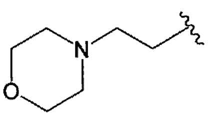,

and the like.

W in the formula (Ib), formula (Ib-1), general formula (II), general formula (II-1), general formula (III) or general formula (III-1) pertaining to the present invention is preferably -CH=, and V is preferably -CH=.

U in the formula (Ib), formula (Ib-1), general formula (II) or general formula (II-1) pertaining to the present invention is preferably a carbon atom.

T in the general formula (I), (I-1), (II) or (II-1) pertaining to the present invention is preferably a nitrogen atom.

The compound represented by the general formula (I), N-oxide thereof, prodrug thereof, pharmaceutically acceptable salt thereof or solvate thereof, pertaining to the present invention is preferably a compound represented by the general formula (II), N-oxide thereof, prodrug thereof, pharmaceutically acceptable salt thereof, or solvate thereof, more preferably a compound represented by the general formula (III), N-oxide thereof, prodrug thereof, pharmaceutically acceptable salt thereof or solvate thereof.

Furthermore, preferable examples of the compounds represented by the general formula (I), N-oxides thereof, prodrugs thereof, pharmaceutically acceptable salts thereof, or solvates thereof include the compounds (1) to (35) described in the preceding item [26], N-oxides thereof, prodrugs thereof, pharmaceutically acceptable salts thereof, or solvates thereof.

In addition, the compound represented by the general formula (I-1), N-oxide thereof, pharmaceutically acceptable salt thereof or solvate thereof, pertaining to the present invention is preferably the compound represented by the general formula (II-1), N-oxide thereof, pharmaceutically acceptable salt thereof or solvate thereof, more preferably a compound represented by the general formula (III-1), N-oxide thereof, pharmaceutically acceptable salt thereof or solvate thereof.

Furthermore, preferable examples of the compounds represented by the general formula (I-1), N-oxides thereof, pharmaceutically acceptable salts thereof, or solvates thereof include the compounds (1) to (57) described in the preceding item [1-39], N-oxides thereof, pharmaceutically acceptable salts thereof, or solvates thereof. Furthermore, the solvates of the compounds (1) to (57) described in the preceding item [1-39] are preferably hydrates of the compounds (1) to (57) described in the preceding item [1-39] or pharmaceutically acceptable salts thereof (e.g., alkali metal salts

(e.g., lithium salt, sodium salt and potassium salt, etc.)).

[Isomers of the compounds pertaining to the present invention]

Unless otherwise specified in the present invention, examples of isomers include all of them. Examples of alkyl groups include straight and branched ones. Furthermore, the present invention includes all of geometric isomers (E-form, Z-form, cis-form, trans-form) in double bonds, rings or condensed rings, optical isomers due to the presence of an asymmetric carbon atom and the like (R or S-form, α or β configuration, enantiomers, diastereomers), optically active substances having optical activity (D, L, d or 1 isomers), polar substances by chromatographic separation (high polar substances or low polar substances), equilibrium compounds, rotamers and mixtures or racemic mixtures thereof in an arbitrary ratio. Forthermore, the present invention also includes all isomers due to tautomers.

Furthermore, the optical isomers in the present invention are not limited to 100% pure ones, and may contain less than 50% other optical isomers.

In the present invention, unless otherwise specified, as being apparent to those skilled in the art, the symbols

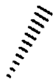

represents that it is connected to the other side of the paper (that is, α arrangement),

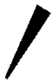

represents that it is connected to the front side of the paper (that is, β arrangement),

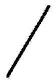

represents that it is α-configuration, β-configuration or a mixture thereof in an arbitrary ratio, and



represents a single bond or double bond.

[N-oxide forms of the compounds pertaining to the present invention]

The compound represented by the general formula (I) or the like or the general formula (I-1) or the like can be converted into an N-oxide form thereof by publically known methods. The N-oxide form means a compound represented by the general formula (I) or the like or the general formula (I-1) or the like in which the nitrogen atom is oxidized. Furthermore, these N-oxide forms can become prodrugs thereof, pharmaceutically acceptable salts thereof or solvates thereof, as described in the respective pragraphs below [Prodrugs of the compounds pertaining to the present invention], [Salts of the compounds pertaining to the present invention] and [Solvates the compounds pertaining to the present invention].

[Prodrugs of the compounds pertaining to the present invention]

The compound represented by the general formula (I) or the like or N-oxide thereof can be converted into a prodrug thereof by publically known methods. The prodrug is a compound which is converted into, for example, the compound represented by the general formula (I) or the like or N-oxide thereof by a reaction with enzymes or gastric acid or the like in vivo. For example, the compound represented by the general formula (I-1) or the like or N-oxide thereof in which any one of $R^{2d}$, $R^{4a}$ and $R^{6a}$ is the preceding $R^{FR}$ can be administered as a prodrug of the compound represented by the general formula (I) or the like or N-oxide thereof, and preferable examples of the prodrugs include the respective compounds in the items (14), (18), (19), (32), (37) to (39), (41), (42) and (45) to (57), described in the preceding item [1-39]. The prodrugs of the compounds represented by the general formula (I) or the like or N-oxides thereof may be

changed to the corresponding compounds represented by the general formula (I) or the like or N-oxide thereof under physiological conditions as described in Hirokawa Shoten, 1990, "Development of Pharmaceuticals", Volume 7, "Molecular Design," pages 163-198.

Examples of other prodrugs of the compound represented by the general formula (I) or the like or N-oxide thereof include, if the compound represented by the general formula (I) or the like or N-oxide thereof has a 5 to 6-membered monocyclic aromatic nitrogen-containing heterocycle containing 1 to 4 nitrogen atoms and without any other heteroatoms, the compounds in which a nitrogen atom on the nitrogen-containing heterocycle was acylated, alkylated or phosphorylated (e.g., a compound in which the nitrogen atom on the nitrogen-containing heterocycle in the compound represented by the general formula (I) or the like was eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated or *tert*-butylated, etc.), if the compound represented by the general formula (I) or the like has an amino group, the compounds in which the amino group was acylated, alkylated or phosphorylated (e.g., the compound in which the amino group in the compound represented by the general formula (I) or the like was eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated or *tert*-butylated, etc.), if the compound represented by the general formula (I) or the like has a hydroxyl group, the compounds in which the hydroxyl group was acylated, alkylated, phosphorylated or borated (e.g., the compound in which the hydroxyl group in the compound represented by the general formula (I) or the like was acetylated, palmitoylated, propanoylated, pivaloylated, succinylated, fumarylated, alanylated or dimethylaminomethylcarbonylated, etc.), and if the compound represented by the general formula (I) or the like has a carboxy group, the compounds in which the carboxy group was esterified or amidated (e.g., the compound in which the carboxy group of the compound represented by the general formula (I) or the like was ethylesterified, phenylesterified, carboxymethylesterified, dimethylaminomethylesterified, pivaloyloxymethylesterified, 1-{(ethoxycarbonyl)oxy}ethylesterified, phthalidylesterified, (5-methyl-2-oxo-1,3-dioxolen-4-yl)methylesterified, 1-{[(cyclohexyloxy)carbonyl]oxy}ethylesterified or methylamidated, etc.) and the like. These compounds per se can be produced by publically known methods. In addition, the prodrug of the compound represented by the general formula (I) or the like or N-oxide form thereof may become a pharmaceutically acceptable salt thereof or solvate thereof, as described in the respective paragraphs below [Salts of the compounds pertaining to the present invention] and [Solvates of the compounds pertaining to the present invention].

[Salts of the compounds pertaining to the present invention]

The compound represented by the general formula (I) or the like, N-oxide thereof or prodrug thereof and the compound represented by the general formula (I-1) or the like or N-oxide thereof can be converted into the corresponding pharmaceutically acceptable salt by publically known methods. Herein, examples of the pharmaceutically acceptable salts include an alkali metal salt (e.g., lithium salt, sodium salt and potassium salt, etc.), alkaline earth metal salt (e.g., calcium salt, magnesium salt and barium salt, etc.), ammonium salt, organic amine salt (e.g., aliphatic amine salt (e.g., methylamine salt, dimethylamine salt, cyclopentylamine salt, trimethylamine salt, triethylamine salt, dicyclohexylamine salt, monoethanolamine salt, diethanolamine salt, triethanolamine salt, procaine salt, meglumine salt, diethanolamine salt, tris(hydroxymethyl)aminomethane salt and ethylenediamine salt, etc.), aralkylamine salt (e.g., benzylamine salt, phenethylamine salt, *N, N*-dibenzylethylenediamine salt and benetamine salt, etc.), heterocyclic aromatic amine salt (e.g., piperidine salt, pyridine salt, picoline salt, quinoline salt and isoquinoline salt, etc.), quaternary ammonium salt (e.g., tetramethylammonium salt, tetraethylammonium salt, benzyltrimethylammonium salt, benzyltriethylammonium salt, benzyltributylammonium salt, methyltrioctylammonium salt and tetrabutylammonium salt, etc.), basic amino acid salt (e.g., arginine salt, lysine salt, etc.) and *N*-methyl-*D*-glucamine salts, etc.), acid adduct salt (e.g., inorganic acid salt (e.g. hydrochloride salt, hydrobromide salt, hydroiodide salt, sulphate, phosphate and nitrate etc.) and organic acid salt (e.g., acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate and gluconate, etc.), etc.) and the like. The pharmaceutically acceptable salt is preferably water-soluble.

In particular, among the compounds represented by the general formula (I-1) or the like in which any one of $R^{2d}$, $R^{4a}$ and $R^{6a}$ is the above-mentioned phosphonooxyalkyl group, examples thereof forming a salt along with the same group include the above-mentioned alkali metal salt, the above-mentioned alkaline earth metal salt, zinc salt, ammonium salt, organic amine salt and the like, and among these salts, the alkali metal salt is preferably a sodium salt and potassium salt, the alkaline earth metal salt is preferably a calcium salt, and the organic amine salt is preferably a basic amino acid salt (e.g., arginine salt (e.g., L-arginine salt) and lysine salt (e.g., L-lysine salt), etc.), meglumine salt, tris(hydroxymethyl)aminomethane salt and the like.

[Solvates of the compounds pertaining to the present invention]

The compound represented by the general formula (I) or the like, N-oxide thereof, prodrug thereof or pharmaceutically acceptable salt thereof and the compound represented by the general formula (I-1) or the like, N-oxide thereof or pharmaceutically acceptable salt thereof can also be converted into a solvate by publically known methods. The solvate is preferably low toxicity and water soluble. Examples of suitable solvates include a solvate with a solvent such as water and alcohols (e.g., ethanol etc.). Herein, a hydrate may be in the form of, for example, a polyhydrate such as a monohydrate to pentahydrate, or low hydrate such as a hemihydrate. Examples of the forms of the hydrates of the compound pertaining to the present invention include a monohydrate, dihydrate, trihydrate and di- to tri-hydrate. Furthermore, examples of the forms of these hydrates include a clathrate hydrate. These hydrates can be obtained by precipitating the compound represented by the general formula (I) or the like, N-oxide thereof, prodrug thereof or pharmaceutically acceptable salts thereof, or the compound represented by the general formula (I-1) or the like, N-oxide thereof or pharmaceutically acceptable salt thereof from, for example, a water-containing organic solvent.

[Crystal]

Each crystal of the compound represented by the general formula (I) or the like, N-oxide thereof, prodrug thereof, pharmaceutically acceptable salt thereof or solvate thereof, and the compound represented by the general formula (I-1) or the like, N-oxide thereof, pharmaceutically acceptable salt thereof, or solvate thereof can be identified by the X-ray powder diffraction spectral data and physicochemical data such as differential scanning calorimetry (DSC). However, due to the nature of X-ray power diffraction spectral data, the diffraction angle ($2\theta$) and overall pattern are important in determining the crystalline identity, and its relative intensity can vary slightly depending on the direction of crystalline growth, particle size and measurement conditions, and the DSC data can also vary slightly depending on the measurement conditions.

[Co-crystal]

The compound represented by the general formula (I) or the like, N-oxide thereof, prodrug thereof, pharmaceutically acceptable salt thereof or solvate thereof, and the compound represented by the general formula (I-1) or the like, N-oxide thereof, pharmaceutically acceptable salt thereof, or solvate thereof can be co-crystallized with an appropriate co-crystal forming agent. The co-crystal is preferably a pharmaceutically acceptable one which can be co-crystallized with a pharmaceutically acceptable co-crystal forming agent. A co-crystal is defined as a crystal in which two or more different molecules are formed by intermolecular interactions different from ionic bonds. Furthermore, the co-crystal may be a complex of a neutral molecule and salt. The co-crystal can be prepared by publically known methods, for example, by melt crystallization, recrystallization from solvent or by physically grinding components together. Examples of the appropriate co-crystal forming agents include those described in WO2006/007448, such as 4-aminobenzoic acid, 4-aminopyridine, adenine, alanine, acetylsalicylic acid and the like.

[Radioisotopes of the compounds pertaining to the present invention]

The compound represented by the general formula (I) or the like, N-oxide thereof, prodrug thereof, pharmaceutically acceptable salt thereof or solvate thereof and the compound represented by the general formula (I-1) or the like, N-oxide thereof, pharmaceutically acceptable salt thereof or solvate thereof may be labeled with an isotope or the like (e.g., $^{2}H$, $^{3}H$, $^{11}C$, $^{13}C$, $^{14}C$, $^{13}N$, $^{15}N$, $^{15}O$, $^{17}O$, $^{18}O$, $^{35}S$, $^{18}F$, $^{36}Cl$, $^{123}I$, $^{125}I$, etc.). Examples thereof include the compound in which all or part of hydrogen atoms constituting one or more groups among $R^1$, $R^2$, $R^3$, $R^4$, $R^5$, $R^6$ and $R^7$ in the general formula (I) or $R^1$, $R^{2c}$, $R^3$, $R^{4a}$, $R^5$, $R^{6a}$ and $R^7$ in the general formula (I-1) were replaced with deuterium atoms or tritium atoms, for example, 4-(4-amino-2-fluoro-5-(methoxy-$d_3$)phenyl)-7-(1*H*-pyrazol-4-yl)isoxazolo[4,5-*c*]pyridin-3-amine and the like. In the present specification, "methyl-$d_3$" and "methoxy-$d_3$" represent a triduteriomethyl group and triduteriomethoxy group, respectively.

[Method for producing compounds pertaining to the present invention]

The compound pertaining to the present invention can be produced by appropriately improving publically known methods, for example, the methods described in Comprehensive Organic Transformations: A Guide to Functional Group Preparations, 2nd Edition (Richard C. Larock, John Wiley & Sons Inc, 1999), methods below, methods shown in Examples and the like or combination thereof.

Among the compounds represented by the general formula (I) or the like, the compound represented by the general formula (IV)

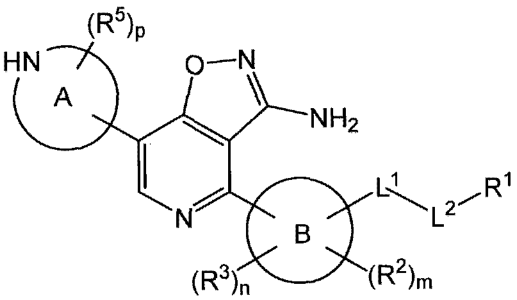

(IV)

[wherein all symbols represent the same meanings as described above.], can be produced by the method represented by the following Reaction Scheme 1.

## Reaction Scheme 1

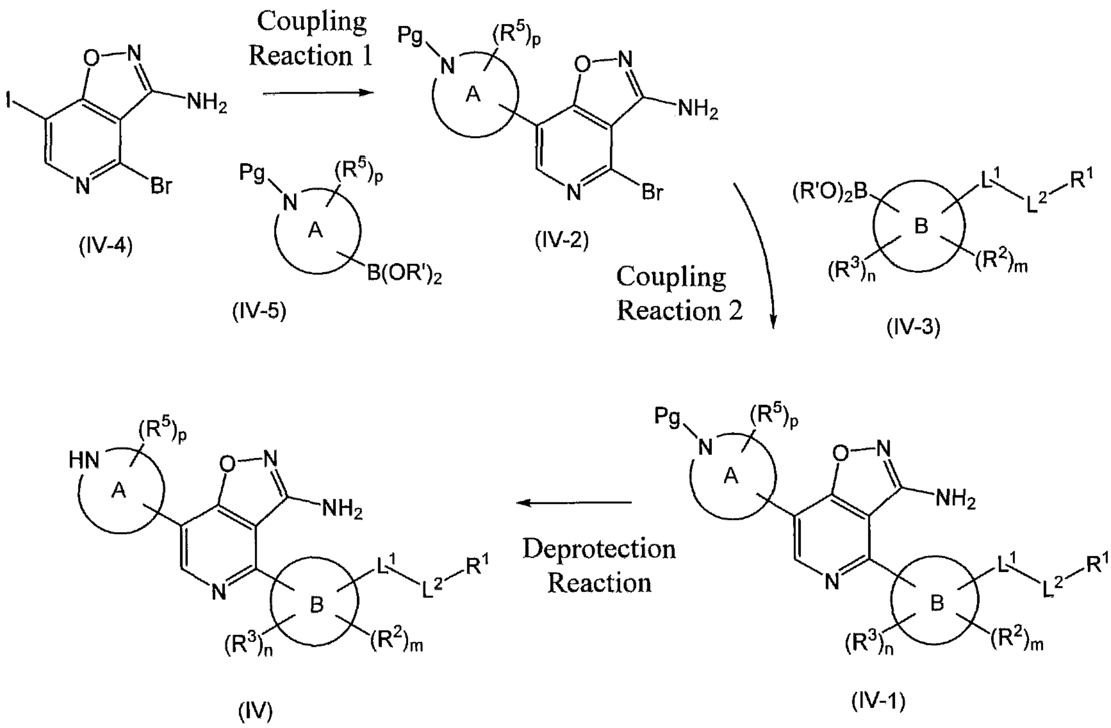


[wherein Pg represents a protecting group for an amino group (e.g., a tert-butoxycarbonyl group, benzyloxycarbonyl group, fluorenylcarbonyl group, trityl group, o-nitrobenzenesulfenyl group, acetyl group or the like), and R' each independently represents a hydrogen atom, C1-5 alkyl group, C3-6 cycloalkyl group, hydroxyl group or halogen atom, herein if R' represents a C1-5 alkyl group, two R's may form a dioxaborolane ring along with adjacent oxygen atom and boron atom, and other symbols represent the same meanings as described above.].

Coupling Reaction 1 in Reaction Scheme 1 can be carried out by publically known Suzuki coupling reaction, for example, at 0 to 200 °C, under the presence or absence of 0.01 to 100 mol% of a palladium catalyst (e.g., tetrakistriphenylphosphine palladium, bis(triphenylphosphine)palladium(II)dichloride, tris(dibenzylideneacetone)dipalladium, palladium acetate, palladium acetylacetonate, [1,1'-bis(diphenylphosphino)ferrocene]dichloropalladium dichloromethane complex or bis[di-*tert*-butyl(4-dimethylaminophenyl)phosphine]palladium, etc.) and 0.01 to 400 mol% of a phosphine ligand (e.g., triphenylphosphine, tri-tert-butylphosphine, tricyclohexylphosphine, di(1-adamantyl)-*n*-butylphosphine or the like), in an organic solvent (e.g,, dichloromethane, chloroform, dioxane, ethyl acetate, methanol, ethanol, isopropyl alcohol, tetrahydrofuran, dimethylformamide or *N*-methylpyrrolidone, etc.) alone or a mixed solvent with water, under the presence or absence of 1 to 10 equivalents of a base (e.g., potassium carbonate, sodium carbonate, cesium carbonate, sodium hydroxide, potassium hydroxide, sodium phosphate, potassium triethylamine phosphate, *N, N*-diisopropylethylamine or the like), in the presence of 1 to 10 equivalents of a boric acid reagent.

Furthermore, Coupling Reaction 1 can also be carried out by publically known coupling reactions using an organometallic reagent, for example, Negishi reaction using a zinc reagent instead of a boric acid reagent, Stille reaction using a tin reagent instead of the boric acid reagent, Hiyama coupling using a silicon reagent instead of the boric acid reagent, and Kumada reaction using a Grignard reagent instead of the boric acid reagent and a nickel catalyst instead of a palladium catalyst are also performed.

Coupling Reaction 2 in Reaction Scheme 1 is also performed by publically known Suzuki coupling reaction, Negishi reaction, Stille reaction, Hiyama coupling, Kumada reaction, or the like.

The deprotection reaction in Reaction Scheme 1 can be carried out by a publically known deprotection reaction under acidic conditions, for example, at 0 to 100 °C in an organic solvent (e.g., dichloromethane, chloroform, dioxane, ethyl acetate, methanol, isopropyl alcohol, tetrahydrofuran or anisole, etc.), in an organic acid (e.g., acetic acid, trifluoroacetic acid, methanesulfonic acid or *p*-tosylic acid, etc.) or inorganic acid (e.g., hydrochloric acid or sulfuric acid, etc.) or a mixture thereof (e.g., hydrogen bromide/acetic acid etc.), and in the presence or absence of 2,2,2-trifluoroethanol.

The compound represented by the general formula (I-1) or the like in which none of $R^{2d}$, $R^{4a}$ and $R^{6a}$ represents the preceding $R^{FR}$ may be produced by the method represented by the preceding Reaction Scheme 1.

Furthermore, among the compounds represented by the general formula (I-1) or the like, the compound represented by the general formula (V)

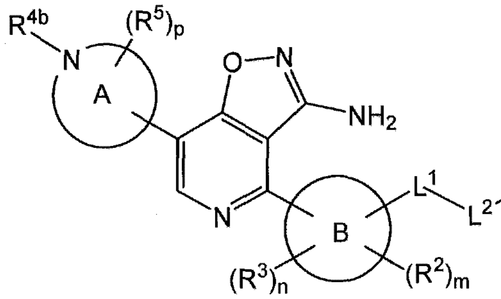

(V)

[wherein $R^{4b}$ represents $-(CR^{Fb}{}_2)_qOP(=O)(OR^{Fa'})_2$, $-(CR^{Fb}{}_2O)_rC(=O)R^{Fc}$, $-(CR^{Fb}{}_2O)_rC(=O)OR^{Fc}$ or $-CR^{Fb}{}_2C(=O)OR^{Fc}$, $R^{Fa'}$ each independently represents a hydrogen atom, C1-4 alkyl group, C3-6 cycloalkyl group, $-(CH_2)_2OH$, $-CR^{Fb}{}_2OC(=O)$-(C1-4 alkyl), $-CR^{Fb}{}_2OC(=O)O$-(C1-4 alkyl), benzyl group or protected group, and other symbols represent the same meanings as described above.] is produced by subjecting the compound represented by the general formula (IV) to the following alkylation reaction, and if $R^{Fa'}$ is a protecting group, being subjected it to a deprotection reaction, if necessary.

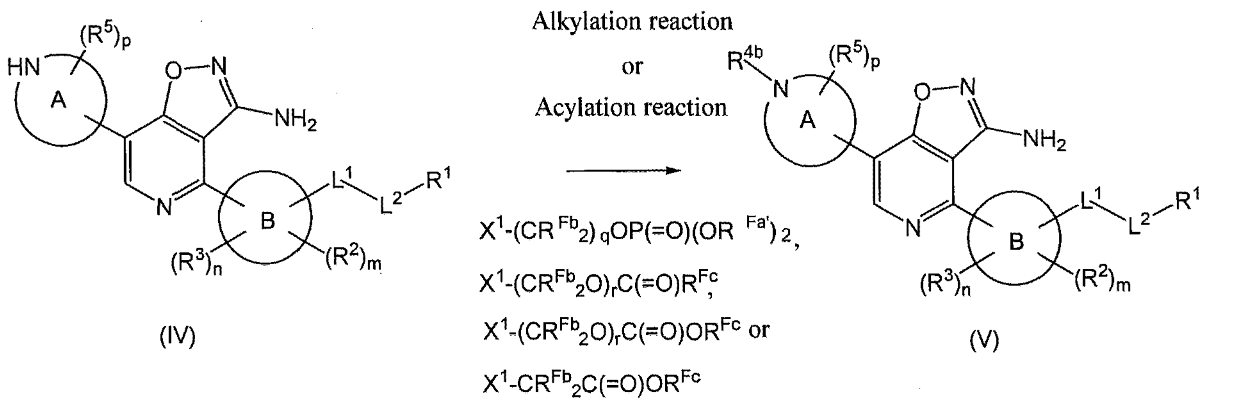


(IV) (V)

[wherein $X^1$ represents a halogen atom or trichloromethyl group, and other symbols represent the same meanings as described above.].

Herein, the alkylation reaction is publically known, and for example, is carried out by reacting $X^1(CR^{Fb})_qOP(=O)(OR^{Fa'})_2$, $X^1(CR^{Fb}{}_2O)_rC(=O)R^{Fc}$, $X^1(CR^{Fb}{}_2O)_rC(=O)OR^{Fc}$ or $X^1CR^{Fb}{}_2C(=O)OR^{Fc}$ with the compound represented by the general formula (IV), in an organic solvent (e.g., dichloromethane, chloroform, dioxane, ethyl acetate, methanol, ethanol, isopropyl alcohol, tetrahydrofuran, dimethylformamide, *N*-methylpyrrolidone and dimethyl sulfoxide, etc.), in the presence of an inorganic base (potassium carbonate, sodium carbonate, cesium carbonate, sodium hydroxide

or potassium hydroxide, etc.) or organic base (e.g., triethylamine, *N,N*-diisopropylamine, lithium diisopropylamide, imidazole, lithium bis(trimethylsilyl)amide, sodium bis(trimethylsilyl)amide, potassium bis(trimethylsilyl)amide, *tert*-butylimino-tris(dimethylamino)phosphorane, *tert*-butylimino-tri(pyridino)phosphorane or 1,4-diazabicyclo[2.2.2]octane, etc.). Furthermore, if $R^{Fa'}$ is a protecting group, the deprotection reaction of the same $R^{Fa'}$ is also publically known, and for example, it can be carried out by publically known deprotection reaction under acidic conditions or hydrogenation reaction in the presence of palladium-carbon catalyst or the like. In addition, if $R^{Fa'}$ represents a protecting group, it corresponds to a protective group for a hydroxyl group, and examples thereof include a methyl group, trityl group, methoxymethyl group, 1-ethoxyethyl group, methoxyethoxymethyl group, 2-tetrahydropyranyl group, trimethylsilyl group, triethylsilyl group, *tert*-butyldimethylsilyl group, *tert*-butyldiphenylsilyl group, acetyl group, pivaloyl group, benzoyl group, benzyl group, *p*-methoxybenzyl group, allyloxycarbonyl group or 2,2,2-trichloroethoxycarbonyl group or the like. Further, the hydrogenation reaction in the presence of a palladium-carbon catalyst or the like is carried out, for example, at room temperature to 120 °C, under a hydrogen gas atmosphere of 1 to 20 atm, in an organic solvent (e.g., methanol, ethanol, tetrahydrofuran, dioxane, ethyl acetate or isopropyl alcohol, etc.), in the presence of 0.01 to 100 mol% of catalyst (e.g., palladium-carbon, platinum-carbon, palladium hydroxide-carbon or rhodium-carbon, etc.).

The compound represented by the general formula (IV-4) in Reaction Scheme 1 can be produced by the method represented by the following Reaction Scheme 2.

Reaction Scheme 2

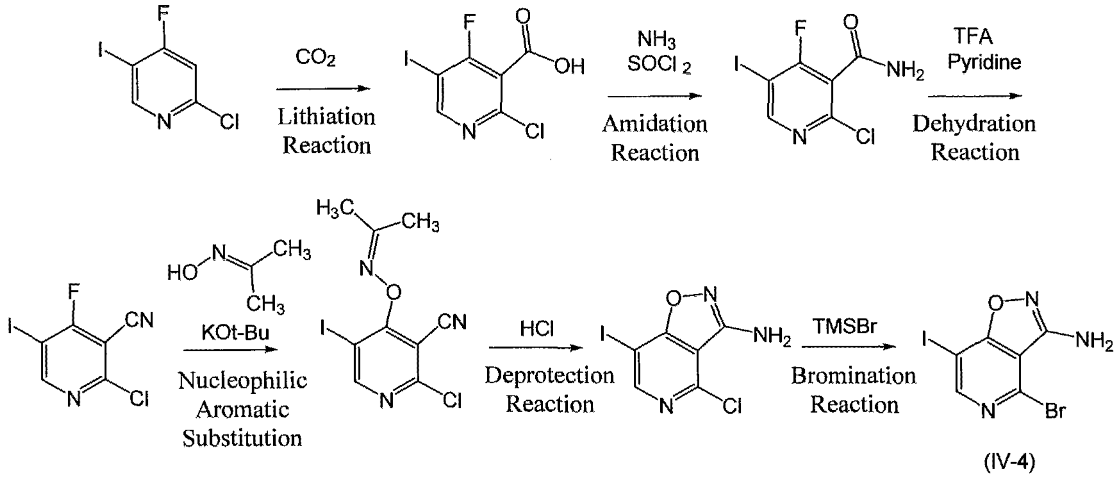


(IV-4)

The lithiation reaction in Reaction Scheme 2 can be carried out by publically known methods, for example, by reacting a base (e.g., lithium diisopropylamide, *n*-butyllithium or *tert*-butyllithium, etc.) in an organic solvent (e.g., tetrahydrofuran, diethylether, dioxane, dichloromethane, dichloroethane, *n*-hexane or toluene, or a mixed solvent thereof, etc.), at - 78 °C to room temperature, followed by addition of carbon dioxide (e.g., carbon dioxide gas or dry ice, etc.), and then reacting it at -78 °C to room temperature.

The amidation reaction in Reaction Scheme 2 can be carried out by publically known methods, for example, by reacting it to an acid halide agent (e.g., oxalyl chloride or thionyl chloride, etc.) at -78 °C to reflux temperature in an organic solvent (e.g., chloroform, dichloromethane, diethylether, tetrahydrofuran or dimethoxyethane, etc.) or under solvent-free condition, and then reacting the obtained acid halide at -78 °C to reflux temperature, with addition of ammonia (e.g., ammonia gas, ammonia water or ammonia methanol solution, etc.), in the presence or absence of a base (e.g., pyridine, triethylamine, dimethylaniline or *N, N*-dimethylaminopyridine, etc.).

The dehydration reaction in Reaction Scheme 2 can be carried out by publically known methods, for example, by reacting it at -78 °C to the reflux temperature, in the presence or absence of a solvent (e.g., chloroform, dichloromethane, diethylether, tetrahydrofuran or dimethoxyethane, etc.), in the presence or absence of a base (e.g., pyridine, triethylamine, dimethylaniline, *N,N*-dimethylaminopyridine or *N,N*-diisopropylethylamine, etc.), in the presence of a dehydrating agent (e.g., thionylchloride, trifluoroacetic anhydride, acetic anhydride, diphosphorus pentoxide or (methoxycarbonylsulfamoyl)triethylammonium hydroxide inner salt, etc.).

The nucleophilic aromatic substitution reaction in Reaction Scheme 2 can be carried out by publically known methods, for example, by reacting it at room temperature to 120 °C, in an organic solvent (e.g., *N,N*-dimethylacetamide, *N,N*-dimethylformamide, tetrahydrofuran, acetonitrile, 2-propanol or dimethyl sulfoxide or a mixed solvent thereof, etc.), in the presence of 1 to 10 equivalents of acetoxime and a base (e.g., *tert*-butoxy potassium, *tert*-butoxy sodium, potassium carbonate, cesium carbonate, sodium hydrogen carbonate or tripotassium phosphate, etc.).

The deprotection reaction in Reaction Scheme 2 can be carried out by publically known methods, for example, a deprotection reaction under acidic condition. For example, it can be carried out at 0 to 100 °C, in an organic solvent (e.g., dichloromethane, chloroform, dioxane, ethylacetate, methanol, isopropyl alcohol, tetrahydrofuran or anisole, etc.), in an organic acid (e.g., acetic acid, trifluoroacetic acid, methanesulfonic acid or *p*-tosylic acid, etc.) or an inorganic acid (e.g., hydrochloric acid or sulfuric acid, etc.) or a mixture thereof (e.g., hydrogen bromide/acetic acid etc.) in the presence or absence of 2,2,2-trifluoroethanol.

The bromination reaction in Reaction Scheme 2 can be carried out by publically known methods, for example, it can be carried out at -78 °C to 100 °C, in an organic solvent (e.g., dichloromethane, chloroform, tetrahydrofuran, acetonitrile, dioxane, ethylacetate or acetic acid, etc.), in the presence or absence of 1 to 10 equivalents of a brominating agent (e.g., trimethylsilylbromide (TMSBr), bromine, hydrobromic acid or phosphorus tribromide, etc.) and 0.1 to 100 mol% of catalyst (e.g., copper (II) bromide or lithium bromide, etc.).

Further, the reactants used in the reaction process to produce the compound represented in the general formula (V) from the compound represented in the general formula (IV) can be produced according to publically known methods, or can be produced by the method below, respectively.

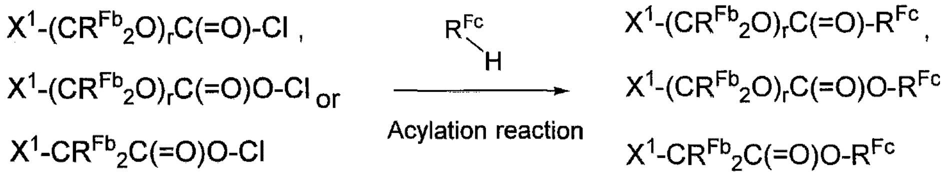


or

[wherein all symbols represent the same meanings as described above.].

Herein, the acylation reaction is publically known, for example, it can be carried out by reacting the compound represented by $R^{Fc}$-H (or a salt thereof) at -78 to 100 °C, in the presence of an inorganic base (potassium carbonate, sodium carbonate, cesium carbonate, sodium hydroxide or potassium hydroxide, etc.) or organic base (e.g., triethylamine, *N,N*-diisopropylamine, lithium diisopropylamide, imidazole, lithium bis(trimethylsilyl)amide, sodium bis(trimethylsilyl)amide, potassium bis(trimethylsilyl)amide, *tert*-butylimino-tris(dimethylamino)phosphorane, *tert*-butylimino-tris(pyrrolidino)phosphorane or 1,4-diazabicyclo[2.2.2]octane, etc.), in an organic solvent (e.g., dichloromethane, chloroform, dioxane, ethyl acetate, methanol, ethanol, isopropyl alcohol, tetrahydrofuran, dimethylformamide, *N*-methylpyrrolidone and dimethyl sulfoxide, etc.).

In each reaction in the present specification, the compounds used as a starting material, compounds or reagents to be added, for example, the compound represented by the general formula (IV-3) or general formula (IV-5) and the compound used in the alkylation reaction, acylation reaction or Reaction Scheme 2 are publically known or can be produced according to publically known methods or methods described in Examples.

Among the compounds used in the present invention, the compounds having optical activity can be produced by using starting materials or reagents having optical activity, by optically resolving a racemic intermediate and then conducing to the compound to be used in the present invention, or by optically resolving a racemic compound. This method of optical resolution is publically known, and examples thereof include a method or the like to form a salt/complex with other optically active compounds and perform recrystallization, and then to isolate the desired compound or directly separate using a chiral column or the like.

In each reaction in the present specification, the reaction involving heating can be performed using a water bath, oil bath, sand bath or microwave, as being apparent to those skilled in the art.

In each reaction in the present specification, a solid-phase supported reagent supported on a high-molecular polymer (e.g., polystyrene, polyacrylamide, polypropylene or polyethylene glycol, etc.) may be used as appropriate.

In each reaction in the present specification, the reaction products can be purified by conventional purification methods, for example, methods such as distillation under normal pressure or reduced pressure, high performance liquid chromatography using silica gel or magnesium silicate, thin layer chromatography, ion exchange resin, scavenger resin or column chromatography, washing, recrystallization and the like. Purification may be carried out for each reaction or may be carried out after completion of several reactions.

[Other STING agonistic compounds]

Examples of the STING compounds which may be used in the present invention also include the following compounds, in addition to the compounds pertaining to the present invention described above.

Those are, any of STING agonistic compounds described in the specification of the patent application selected from the group consisting of WO2015/185565, WO2017/093933, WO2017/175147, WO2017/175156, WO2019/069275,

WO2019/069270, WO2019/069269, WO2016/096174, WO2017/186711, WO2019/129880, WO2014/093936, WO2014/189805, WO2014/189806, WO2016/145102, WO2017/075477, WO2017/106740, WO2018/009466, WO2018/198076, WO2018/200812, WO2017/027645, WO2017/027646, WO2018/067423, WO2018/118665, WO2018/118664, WO2018/208667, WO2019/027858, WO2019/027857, WO2019/125974, WO2019/195124, WO2019/195063, WO2017/011622, WO2016/164619, WO2019/046511, WO2019/051489, WO2019/051488, WO2017/161349, WO2018/009648, WO2018/013887, WO2018/013908, WO2019/046511, WO2019/051489, WO2019/051488, WO2019/161171, WO2020/014127, WO2018/013924, WO2018/060323, WO2018/172206, WO2019/185476, WO2019/185477, WO2017/123669, WO2017/123657, WO2018/045204, WO2020/010092, WO2020/010155, WO2018/100558, WO2019/092660, WO2019/180683, WO2018/098203, WO2018/138685, WO2018/138684, WO2019/118839, WO2020/016782, WO2018/065360, WO2018/152450, WO2018/152453, WO2019/232392, WO2018/156625, US2017/0146519, WO2018/234808, WO2018/234807, WO2018/234805, WO2019/243823, WO2019/243825, WO2019/023459, WO2019/046500, WO2019/046498, WO2019/046496, WO2019/074887, WO2019/160884, WO2019/173587, WO2019/043634, US2017/0050967, WO2019/165032, WO2019/158731, WO2019/134705, WO2019/134707, WO2019/123338, WO2019/123339, WO2019/123340, WO2019/122202, WO2019/100061, WO2020/010451, WO2020/006432, WO2019/238786, WO2019/227007, WO2019/219820, WO2019/211799, WO2019/193542, WO2019/193533, WO2019/193543, WO2019/123340, WO2019/123339, WO2019/123338, WO2019/183578, WO2019/175776, WO2019/170912, WO2020/028565, WO2020/028566, WO2020/038387, WO2020/042995, WO2020/050406, WO2020/057546, WO2020/072492, WO2020/074004, WO2020/092127, WO2020/106736, WO2020/117623, WO2020/117624, WO2020/117625, WO2020/115676, WO2020/124059, WO2020/135715, WO2020/146237, WO2020/151682, WO2020/156363, WO2020/163415, WO2020/178768, WO2020/178769, WO2020/178770, WO2020/202091, WO2020/194160, WO2020/221038, WO2020/232375, WO2020/232378, WO2020/227421, WO2020/252240, WO2020/236586, WO2020/243519, WO2020/249773, WO2021/009362, WO2021/009365, WO2021/000770, WO2021/014365, WO2021/013234, WO2021/013250, WO2021/026009, WO2021/035257, WO2021/035258, WO2021/037179 and WO2021/042024 can also be used in the present invention. In particular, the STING agonistic compound known, for the skilled persons, as the name of ADU-S100 (CAS registered number 1638241-89-0), MK-1454, MK-2118, SB11285, GSK3745417, BMS-986301, E7766, TAK-676, CRD5500, MAVU-104, SYNB1891, SB11325, SB11396, TTI-10001, exoSTING, VTX-001, SRCB-0074, ISMA-101 or BI-13874456 can also be used.

Herein, the STING agonistic compound described in the patent application identified as WO2018/067423, which can be used in the present invention, is preferably a compound identified by CAS registered number selected from the group consisting of 2218503-83-2, 2218505-09-8, 2218505-08-7, 2218503-88-7, 2218504-006, 2218504-44-8, 2218504-06-2 and 2218504-10-8.

Furthermore, The STING agonistic compound described in the patent application identified as WO2018/100558, which can be used in the present invention, is preferably a compound identified by CAS registry number selected from the group consisting of 2228934-37-8, 2228891-92-5, 2228891-91-4, 2228891-93-6, 2228891-97-0, 2228891-94-7, 2228892-02-0, 2228892-01-9, 2228893-53-4, 2228892-08-6, 2228892-16-6, 2228892-15-5, 2228892-09-7, 2228892-61-1, 2228892-60-0, 2228892-59-7, 2228892-69-9, 2228892-68-8, 2228892-94-0, 2228892-93-9, 2228893-00-1, 2228892-99-5, 2228893-32-9, 2228893-31-8, 2228893-13-6, 2228893-12-5, 2228893-17-0, 2228893-16-9, 2228893-44-3 and 2228893-43-2.

Furthermore, the STING agonistic compound described in the patent application identified as WO2018/060323, which can be used in the present invention, is preferably a compound identified by CAS registry number selected from the group consisting of 2211044-08-3, 2211044-07-2, 2308490-32-4, 2211044-10-7, 2308490-31-3, 2211044-12-9, 2308490-29-9 and 2211044-14-1.

Similarly, the STING agonistic compound described in the patent application identified as WO2017/093933, which can be used in the present invention, is preferably a compound identified by CAS registry number selected from the group consisting of 2099072-25-8, 2099072-26-9, 2099072-21-4, 2099072-22-5, 2099073-79-5, 2099072-28-1, 2099072-29-2, 2099072-30-5, 2099072-27-0, 2099072-31-6, 2099072-23-6, 2099072-24-7, 2099072-32-7, 2099072-33-8 and 2099072-34-9.

The STING agonistic compound which can be used in the present invention is preferably the following compound

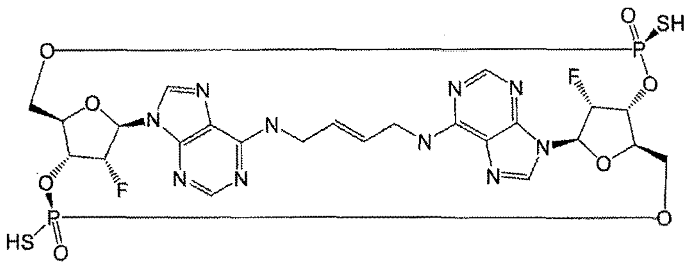

, as well.

[Prescriptions for compounds pertaining to the present invention]

When the STING agonistic compound is

(1) the compound, N-oxide thereof, prodrug thereof, pharmaceutically acceptable salt thereof or solvate thereof, according to any one of the preceding items [1] to [26], or
(2) the compound, N-oxide thereof, pharmaceutically acceptable salt thereof or solvate thereof, according to any one of the preceding items [1-1] to [1-44],

the STING agonist is administered to an adult at about 0.03 to about 10.0 mg/kg (body weight) of the compound per dose or about 2.4 to about 800 mg per dose every 1, 2, 3, 4, 6 or 8 weeks by intravenous injection or intravenous drip infusion. As for specific dosages of the STING agonist, it can be, for example,

(a) administered to an adult at 0.03 mg/kg, 0.04 mg/kg, 0.05 mg/kg, 0.06 mg/kg, 0.07 mg/kg, 0.08 mg/kg, 0.09 mg/kg or 0.1 mg/kg (body weight) of the STING agonistic compound per dose,
(b) administered to an adult at 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg (body weight) of the STING agonistic compound per dose,
(c) administered to an adult at 1.1 mg/kg, 1.2 mg/kg, 1.3mg/kg, 1.4 mg/kg, 1.5 mg/kg, 1.6 mg/kg, 1.7 mg/kg, 1.8 mg/kg, 1.9 mg/kg or 2.0 mg/kg (body weight) of the STING agonistic compound per dose, or
(d) administered to an adult at 2.1 mg/kg, 2.2 mg/kg, 2.3mg/kg, 2.4 mg/kg, 2.5 mg/kg, 2.6 mg/kg, 2.7 mg/kg, 2.8 mg/kg, 2.9 mg/kg or 3.0 mg/kg (body weight) of the STING agonistic compound per dose.

As for another embodiment of specific dosage of the STING agonist, it can be, for example,

(a1) administered to an adult at 2.4 mg, 3.2 mg, 4.0 mg, 4.8 mg, 5.6 mg, 6.4 mg, 7.2 mg or 8.0 mg of the STING agonistic compound per dose,
(b1) administered to an adult at 16.0 mg, 24.0 mg, 32.0 mg, 40.0 mg, 48.0 mg, 56.0 mg, 64.0 mg, 72.0 mg or 80.0 mg of the STING agonistic compound per dose,
(c1) administered to an adult at 88.0 mg, 96.0 mg, 104.0 mg, 112.0 mg, 120.0 mg, 128.0 mg, 136.0 mg, 144.0 mg, 152 mg or 160.0 mg of the STING agonistic compound per dose, or
(d1) administered to an adult at 168.0 mg, 176 mg, 184 mg, 192.0 mg, 200.0 mg, 208.0 mg, 216.0 mg, 224.0 mg, 232 mg or 240.0 mg of the STING agonistic compound per dose.

As for the frequency and methods for administration of the STING agonist, it can be administered, for example, every 1, 2, 3, 4, 6 or 8 weeks by intravenous injection or intravenous drip infusion, and administered continuously at any of the above dosing intervals until physicians determines that the administration is unnecessary or until the administration is discontinued due to the occurrence of adverse events.
On the other hand, as for the methods for administration of the STING agonist, it may be administered, not continuously, an arbitrary time selected from 1 to 16 times, for example, only once, only twice, only three times, only four times, only six times, only eight times, only ten times, only twelve times, only fourteen times or only sixteen times.
The usage and dosage of the STING agonist pertaining to the present invention is preferably to be administered to an adult at an arbitrary dosage of 0.03 to 0.3 mg/kg (body weight) of the STING agonistic compound per dose every 1, 2, 3 or 4 weeks by intravenous drip infusion.

[Anti-neoplastic agent]

Examples of anti-neoplastic agents which can be used in the present invention include an alkylating agent (e.g., Dacarbazine, Nimustine, Temozolomide, Fotemustine, Bendamustine, Cyclophosphamide, Ifosfamide, Carmustine, Chlorambucil and Procarbazine, etc.), platinum preparation (e.g., Cisplatin, Carboplatin, Nedaplatin and Oxaliplatin, etc.), antimetabolite (e.g., antifolate (e.g., Pemetrexed, Leucovorin and Methotrexate, etc.), pyridine metabolism inhibitor (e.g., TS-1 (registered trademark), 5-fluorouracil, UFT, Carmofur, Doxifluridine, FdUrd, Cytarabine and Capecitabine, etc.), purine metabolism inhibitor (e.g., Fludarabine, Cladribine and Nelarabine, etc.), ribonucleotide reductase inhibitor, nucleotide analog (e.g., Gemcitabine etc.)), topoisomerase inhibitor (e.g., Irinotecan, Nogitecan and Etoposide, etc.), microtubule polymerization inhibitor (e.g., Vinblastine, Vincristine, Vindesine, Vinorelbine and Eribulin, etc.), microtubule depolymerization inhibitor (e.g., Docetaxel and Paclitaxel, etc.), antitumor antibiotic (e.g., Bleomycin, Mitomycin C, Doxorubicin, Daunorubicin, Idarubicin, Etoposide, Mitoxantrone, Vinblastine, Vincristine, Peplomycin, Amrubicin, Aclarubicin. and Epirubicin, etc.), cytokine preparation (e.g., IFN-$\alpha$2a, IFN-$\alpha$2b, peg-IFN-$\alpha$2b, natural IFN-$\beta$ and Interleukin-2, etc.), anti-hormonal drug (e.g., Tamoxifen, Fulvestrant, Goserelin, Leuprorelin, Anastrozole, Letrozole and Exemestane, etc.), molecular targeting drug, tumor immunotherapeutic drug, other antibody drugs and the like. Herein, the antineoplastic agent pertaining to the present invention is preferably one without any concerns of developing cytokine release syndrome when administered by itself alone.

Herein, examples of the molecular targeting drugs include an ALK inhibitor (e.g., Crizotinib, Ceritinib, Ensartinib, Alectinib and Lorlatinib, etc.), BCR-ABL inhibitor (e.g., Imatinib and Dasatinib, etc.), EGFR inhibitor (e.g., Erlotinib, EGF816, Afatinib, Osimertinib mesilate, Gefitinib and Rociletinib, etc.), B-RAF inhibitor (e.g., Sorafenib, Vemurafenib, TAK-580, Dabrafenib, Encorafenib, LXH254, Emurafenib and Zanubrutinib, etc.), VEGFR inhibitor (e.g., Bevacizumab, Apatinib, Lenvatinib, Aflibercept and Axitinib, etc.), FGFR inhibitor (e.g., AZD4547, Vofatmab, Roblitinib and Pemigatinib, etc.), c-MET inhibitor (e.g., Savolitinib, Merestinib, Capmatinib, Capmatinib and Glesatinib, etc.), AXL inhibitor (e.g., ONO-7475 and Bemcentinib, etc.), MEK inhibitor (e.g., Cobimetinib, Binimetinib, Selumetinib and Trametinib, etc.), CDK inhibitor (e.g., Dinaciclib, Abemaciclib, Palbociclib and Trilaciclib, etc.), BTK inhibitor (e.g., Ibrutinib and Acalabrutinib, etc.), BCL-2 inhibitor (e.g., Navitoclax and Venetoclax, etc.), PI3K-$\delta/\gamma$ inhibitor (e.g., Umbralisib, Parsaclisib and IPI-549, etc.), JAK-1/2 inhibitor (e.g., Itacitinib and Ruxolitinib, etc.), ERK inhibitor (e.g., SCH 900353 etc.), TGFbR1 inhibitor (e.g., Galunisertib etc.), Cancer cell sternness kinase inhibitor (e.g., Amcasertib), FAK inhibitor (e.g., Defactinib), Syk/FLT3 dual inhibitor (e.g., Mivavotinib etc.), ATR inhibitor (e.g., Ceralasertib etc.), Wee1 kinase inhibitor (e.g., Adavosertib etc.), multi-tyrosine kinase inhibitor (e.g., Sunitinib, Pazopanib, Cabozantinib, Regorafenib, Nintedanib, Sitravatinib and Midostaurin, etc.), mTOR inhibitor (e.g., Temsirolimus, Everolimus, Vistusertib, Irinotecan, etc.), HDAC inhibitor (e.g., Vorinostat, Romidepsin, Entinostat, Chidamide, Mocetinostat, Citarinostat, Panobinostat and Valproate, etc.), PARP inhibitor (e.g., Niraparib, Olaparib, Veliparib, Rucaparib and Beigene-290, etc.), aromatase inhibitor (e.g., Exemestane and Letrozole, etc.), EZH2 inhibitor (e.g., Tazemetostat etc.), Galectin-3 inhibitor (e.g., Belapectin etc.), STAT3 inhibitor (e.g., Napabucasin etc.), DNMT inhibitor (e.g., Azacitidine etc.), SMO inhibitor (e.g., Vismodegib etc.), HSP90 inhibitor (e.g., XL888 etc.), $\gamma$-tubulin specific inhibitor (e.g., Glaziovianin A and Plinabulin, etc.), HIF2$\alpha$ inhibitor (e.g., PT2385 etc.), glutaminase inhibitor (e.g., Telaglenastat etc.), E3 ligase inhibitor (e.g., Avadomide etc.), NRF2 activator (e.g., Omaveloxolone etc.), arginase inhibitor (e.g., CB-1158 etc.) , cell cycle inhibitor (e.g., Trabectedin etc.), Ephrin B4 inhibitor (e.g., sEphB4-HAS etc.), IAP antagonist (e.g., Birinapant etc.), anti-HER2 antibody (e.g., Pertuzumab, Margetuximab, Disitamab, Disitamab vedotin, Gancotamab, Timigutuzumab, Zanidatamab, Zenocutuzumab, Trastuzumab, Trastuzumab beta, Trastuzumab deruxtecan, Trastuzumab duocarmazine, Trastuzumab emtansine, R48 and ZW33), anti-HER1 antibody (e.g., Cetuximab, Cetuximab sarotalocan, Panitumumab, Necitumumab, Nimotuzumab, Depatuxizumab, Depatuxizumab mafodotin, Futuximab, Laprituximab Laprituximab emtansine, Matuzumab, Modotuximab, Petosemtamab, Tomuzotuximab, Losatuxizumab, Losatuxizumab vedotin, Serclutamab, Serclutamab talirine, Imgatuzumab, Futuximab and Zalutumurnab, etc.), anti-HER3 antibody (e.g., Duligotuzumab, Elgemtumab, Istiratumab, Lumretuzumab, Zenocutuzumab, Patritumab, Patritumab deruxtecan, and Seribantumab, etc.), anti-CD40 antibody (e.g., Bleselumab, Dacetuzumab, Iscalimab, Lucatumumab, Mitazalimab, Ravagalimab, Selicrelumab, Teneliximab, ABBV-428, and APX005M, etc.), anti-CD70 antibody (e.g., Cusatuzumab, Vorsetuzumab, Vorsetuzumab mafodotin and ARGX-110, etc.), anti-VEGF antibody (e.g., Bevacizumab, Bevacizumab beta, Ranibizumab, Abicipar pegol, Aflibercept, Brolucizumab, Convercept, Dilpacimab, Faricimab, Navicixizumab, Varisacumab and IMC -1C11, etc.), anti-VEGFR1 antibody (e.g., Icrucumab, etc.), anti-VEGFR2 antibody (e.g., Ramucirumab, Alacizumab, Alacizumab pegol, Olinvacimab, Pegdinetanib and AMG596, etc.), anti-CD20 antibody (e.g., Rituximab, Blontuvetmab, Epitumomab, Ibritumomab tiuxetan, Ocaratuzumab, Ocrelizumab, Technetium ($^{99}$mTc) nofetumomab merpentan, Tositumomab, Veltuzumab, Ofatumumab, Ublituximab, Obinutuzumab and Nofetumomab, etc.), anti-CD30 antibody (e.g., Brentuximab Vedotin and Iratumumab, etc.), anti-CD38 antibody (e.g., Daratumumab, Isatuximab, Mezagitamab, AT13/5 and MOR202, etc.), anti-TNFRSF10B antibody (e.g., Benufutamab, Conatumumab, Drozitumab, Lexatumumab, Tigatuzumab, Eftozanenmin alfa and DS-8273a, etc.), anti-TNFRSF10A antibody (e.g. Mapatumumab etc.), anti-MUC1 antibody (e.g., Cantuzumab, Cantuzumab ravtansine, Clivatuzumab, Clivatuzumab tetraxetan, Yttrium ($^{90}$Y) clivatuzumab tetraxetan, Epitumomab, Epitumomab

cituxetan, Sontuzumab, Gatipotuzumab, Nacolomab, Nacolomab tafenatox, 7F11C7, BrE-3, CMB-401, CTM01 and HMFG1, etc.), anti-MUC5AC antibody (e.g., Ensituximab etc.), anti-MUC16 antibody (e.g., Oregovomab, Abagovomab, Igovomab, and Sofituzumab vedotin, etc.), anti-DLL4 antibody (e.g., Demcizumab, Dilpacimab, Navicixizumab and Enoticumab, etc.), anti-fucosyl GM1 antibody (e.g., BMS-986012 etc.), anti-gpNMB antibody (e.g., Glembatumumab vedotin etc.), anti-Mesothelin antibody (e.g., Amatuximab, Anetumab ravtansine, Anetumab corixetan, RG7784 and BMS-986148, etc.), anti-MMP9 antibody (e.g., Andecaliximab etc.), anti-GD2 antibody (e.g., Dinutuximab, Dinutuximab beta, Lorukafusp alfa, Naxitamab, 14G2a, MORAb-028, Surek, TRBs07 and ME361, etc.), anti-MET antibody (e.g., Emibetuzumab, Onartuzumab, Telisotuzumab and Telisotuzumab vedotin, etc.), anti-FOLR1 antibody (e.g., Farletuzumab, Mirvetuximab and Mirvetuximab soravtansine, etc.), anti-CD79b antibody (e.g., Iladatuzumab, Iladatuzumab vedotin, and Polatuzumab vedotin, etc.), anti-DLL3 antibody (e.g., Rovalpituzumab and Rovalpituzumab Tesirine, etc.), anti-CD51 antibody (e.g., Abituzumab, Etaracizumab, and Intetumumab, etc.), anti-EPCAM antibody (e.g., Adecatumumab, Catumaxomab, Edrecolomab, Oportuzumab monatox, Citatuzumab bogatox and Tucotuzumab celmoleukin, etc.), anti-CEACAM5 antibody (e.g., Altumomab, Arcitumomab, Cergutuzumab amunaleukin, Labetuzumab, Labetuzumab govitecan, $^{90}$Y-cT84.66, AMG211, BW431/26, CE25B7, COL-1, and T84.66 M5A, etc.), anti-CEACAM6 antibody (e.g., Tinurilimab etc.), anti-FGFR2 antibody (e.g., Aprutumab, Aprutumab ixadotin, and Bemarituzumab, etc.), anti-CD44 antibody (e.g. Bivatuzumab mertansine etc.), anti-PSMA antibody (e.g. Indium ($^{111}$In) capromab pendetide, $^{177}$Lu-J591 and ES414, etc.), anti-Endoglin antibody (e.g. Carotuximab etc.), anti-IGF1R antibody (e.g., Cixutumumab, Figitumumab, Ganitumab, Dalotuzumab, Teprotumumab, and Robatumumab, etc.), anti-TNFSF11 antibody (e.g., Denosumab), anti-GUCY2C antibody (e.g., Indusatumumab vedotin), anti-SLC39A6 antibody (e.g., Ladiratuzumab vedotin etc.), anti-SLC34A2 antibody (e.g., Lifastuzumab vedotin etc.), anti-NCAM1 antibody (e.g. Lorvotuzumab mertansine and N901, etc.), anti-ganglioside GD3 antibody (e.g., Ecromeximab and Mitumomab, etc.), anti-AMHR2 antibody (e.g., Murlentamab etc.), anti-CD37 antibody (e.g., Lilotomab, Lutetium ($^{177}$lu) lilotomab satetraxetan, Naratuximab, Naratuximab emtansine and Otlertuzumab, etc.), anti-IL1RAP antibody (e.g., Nidanilimab etc.), anti-PDGFR2 antibody (e.g. Olaratumab and Tovetumab, etc.), anti-CD200 antibody (e.g., Samalizumab etc.), anti-TAG-72 antibody (e.g., Anatumomab mafenatox, Minretumomab, Indium ($^{111}$In) satumomab pendetide, CC49, HCC49 and M4, etc.), anti-SLITRK6 antibody (e.g., Sirtratumab vedotin etc.), anti-DPEP3 antibody (e.g., Tamrintamab pamozirine etc.), anti-CD19 antibody (e.g., Axicabtagene ciloleucel, Coltuximab ravtansine, Denintuzumab mafodotin, Inebilizumab, Loncastuximab, Loncastuximab tesirine, Obexelimab, Tafasitamab, Taplitumomab, Paptox, and huAnti-B4, etc.), anti-NOTCH2/3 antibody (e.g., Tarextumab etc.), anti-tenascin C antibody (e.g., Tenatumomab etc.), anti-AXL antibody (e.g., Enapotamab Enapotamab vedotin, and Tilvestamab, etc.), anti-STEAP1 antibody (e.g., Vandortuzumab vedotin etc.), anti-CTAA16 antibody (e.g., Technetium ($^{99}$mTc) votumumab etc.), anti-CLDN18 antibody (e.g., Zolbetuximab etc.), anti-GM3 antibody (e.g., Racotumomab, FCGR1 and H22, etc.), anti-PSCA antibody (e.g., MK-4721 etc.), anti-FN extra domain B antibody (e.g., AS1409 etc.), anti-HAVCR1 antibody (e.g., CDX-014 etc.), anti-TNFRSF4 antibody (e.g., MEDI6383 etc.), anti-HER1-MET bispecific antibody (e.g., Amivantamab etc.), anti-EPCAM-CD3 bispecific antibody (e.g., Solitomab and Catumaxomab, etc.), anti-Ang2-VEGF bispecific antibody (e.g., Vanucizumab etc.), anti-HER2-CD3 bispecific antibody (e.g. Ertumaxomab etc.), anti-HER3-IGF1R bispecific antibody (e.g. Istiratumab etc.), anti-PMSA-CD3 bispecific antibody (e.g. Pasotuxizumab), anti-HER1-LGRS bispecific antibody (e.g. Petosemtamab etc.), anti-SSTR2-CD3 bispecific antibody (e.g., Tidutamab etc.), anti-CD30-CD16A bispecific antibody (e.g., AFM13 etc.), anti-CEA-CD3 bispecific antibody (e.g., Cibisatamab and RO6958688, etc.), anti-CD3-CD19 bispecific antibody (e.g., Duvortuxizumab and Blinatumomab, etc.), anti-IL3RA-CD3 bispecific antibody (e.g., Flotetuzumab and Vibecotamab, etc.), anti-GPRC5D-CD3 bispecific antibody (e.g., Talquetamab etc.), anti-CD20-CD3 bispecific antibody (e.g., Plamotamab, Odronextamab, Mosunetuzumab, Glofitamab, Epcoritamab and REGN1979, etc.), anti-TNFRSF17-CD3 bispecific antibody (e.g., Teclistamab etc.), anti-CLEC12A-CD3 bispecific antibody (e.g., Tepoditamab etc.), anti-HER2-HER3 bispecific antibody (e.g., Zenocutuzumab etc.), anti-FAP antibody/IL-2 fusion protein (e.g., RO6874281 etc.), anti-CEA antibody/IL-2 fusion protein (e.g., Cergutuzumab amunaleukin etc.), and the like.

Furthermore, examples of tumor immunotherapeutic drugs include an anti-PD-1 antibody (e.g., Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, GLS010, CS1003, BAT-1306, AK105, AK103, BI 754091, LZM009, CMAB819, Sym021, SSI-361, JY034, HX008, ISU106 and CX-188, etc.), anti-PD-L1 antibody (e.g., Atezolizumab, Avelumab, Durvalumab, Manelimab, Pacmilimab, Envafolimab, Cosibelimab, Sugemalimab, BMS-936559, STI-1014, HLX20, SHR-1316, MSB2311, BGB-A333, KL-A167, AK106, AK104, ZKAB001, FAZ053, CBT-502 and JS003, etc.), PD-1 antagonist (e.g., AUNP-12 and each compound of BMS-M1 to BMS-M10 (see WO2014/151634, WO2016/039749, WO2016/057624, WO2016/077518, WO2016/100285, WO2016/100608, WO2016/126646, WO2016/149351, WO2017/151830 and WO2017/176608), BMS-1, BMS-2, BMS-3, BMS-8, BMS-37, BMS-200, BMS-202, BMS-230, BMS-242, BMS-1001 and BMS -1166 (see WO2015/034820, WO2015/160641, WO2017/066227 and Oncotarget. 2017 Sep 22; 8 (42): 72167-72181.), each compound of Incyte-1 to Incyte-6 (see WO2017/070089, WO2017/087777, WO2017/106634, WO2017/112730, WO2017/192961 and WO2017/205464),

CAMC-1 to CAMC-4 (see WO2017/202273, WO2017/202274, WO2017/202275 and WO2017/202276), RG_1 (see WO2017/118762) and DPPA-1 (see Angew. Chem. Int. Ed. 2015, 54, 11760-11764), etc.), PD-L1/VISTA antagonist (e.g., CA-170), PD-L1/TIM3 antagonist (e.g., CA-327), anti-PD-L2 antibody, PD-L1 fusion protein, PD-L2 fusion protein (e.g., AMP-224 etc.), anti-CTLA-4 antibody (e.g., Ipilimumab, Zalifrelimab, Nurulimab and Tremelimumab, etc.), anti-LAG-3 antibody (e.g., Relatlimab, Ieramilimab, Fianlimab, Encelimab and Mavezelimab, etc.), anti-TIM3 antibody (e.g., MBG453 and Cobolimab, etc.), anti-KIR antibody (e.g., Lirilumab, IPH2101, LY3321367 and MK-4280, etc.), anti-BTLA antibody, anti-TIGIT antibody (e.g., Tiragolumab, Etigilimab, Vibostolimab and BMS-986207, etc), anti-VISTA antibody (e.g., Onvatilimab etc.), anti-CD137 antibody (e.g., Urelumab and Utomilumab, etc.), anti-CSF-1R antibody/CSF-1R inhibitor (e.g., Cabiralizumab, Emactuzumab, LY3022855, Axatilimab, MCS-110, IMC-CS4, AMG820, Pexidartinib, BLZ945 andARRY-382, etc.), anti-OX40 antibody (e.g., MED16469, Ivuxolimab, MEDI0562, MEDI6383, Efizonerimod, GSK3174998, BMS-986178 and MOXR0916, etc.), anti-HVEM antibody, anti-CD27 antibody (e.g., Varlilumab etc.), anti-GITR antibody/GITR fusion protein (e.g., Efaprinermin alfa, Efgivanermin alfa, MK-4166, INCAGN01876, GWN323 and TRX-518, etc.), anti-CD28 antibody, anti-CCR4 antibody (e.g., Mogamulizumab etc.), anti-B7-H3 antibody (e.g., Enoblituzumab, Mirzotamab, Mirzotamab clezutoclax and Omburtamab, etc.), anti-ICOS agonist antibody (e.g., Vopratelimab and GSK3359609, etc.), anti-CD4 antibody (e.g., Zanolimumab and IT1208, etc.), anti-DEC-205 antibody/NY-ESO-1 fusion protein (e.g., CDX-1401), anti-SLAMF7 antibody (e.g., Azintuxizumab, Azintuxizumab vedotin and Elotuzumab etc.), anti-CD73 antibody (e.g., Oleclumab and BMS-986179, etc.), PEGylated IL-2 (Bempegaldesleukin), IDO inhibitor (e.g., Epacadostat, Indoximod and Linrodostat, etc.), TLR agonist (e.g., Motolimod, CMP-001, G100, Tilsotolimod, SD-101 and MEDI9197, etc.), adenosine A2A receptor antagonist (e.g., Preladenant, AZD4635, Taminadenant and Ciforadenant, etc.), anti-NKG2A antibody (e.g., Monalizumab etc.), anti-CSF-1 antibody (e.g., PD0360324 etc.), immunopotentiator (e.g., PV-10 etc.), IL-15 super agonist (e.g., ALT-803 etc.), soluble LAG3 (e.g., Eftilagimod alpha etc.), anti-CD47 antibody/CD47 antagonist (e.g., ALX148 etc.) and IL-12 antagonist (e.g., M9241 etc.) and the like. Incidentally, Nivolumab can be produced according to the method described in WO2006/121168, Pembrolizumab can be produced according to the method described in WO2008/156712, BMS-936559 can be produced according to the method described in WO2007/005874, and Ipilimumab can be produced according to the method described in WO2001/014424.

Furthermore, examples of other antibody drugs include an anti-IL-1β antibody (e.g., Canakinumab etc.), anti-CCR2 antibody (e.g., Plozalizumab etc.) and the like.

The tumor immunotherapeutic drug pertaining to the present invention can be administered, for example, in the following usage and dosage. That is, it can be administered intravenously (e.g., intravenous drip infusion) at about 1 to about 21 mg/kg (body weight) per dose or about 80 to about 1680 mg per dose, of an active ingredient of the tumor immunotherapeutic drug, every 1 to 8 weeks, over about 30 minutes to about 60 minutes or about 60 minutes or more. Herein, examples of single dosages based on body weight include 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 12 mg/kg, 14 mg/kg, 15 mg/k, 20 mg/kg and 21 mg/kg, while examples of single dosage include 200 mg, 240 mg, 250 mg, 280 mg, 300 mg, 320 mg, 350 mg, 360 mg, 400 mg, 420 mg, 450 mg, 480 mg, 500 mg, 540 mg, 560 mg, 600 mg, 640 mg, 700 mg, 720 mg, 750 mg, 800 mg, 840 mg, 900 mg, 1000 mg, 1080 mg, 1100 mg, 1120 mg, 1200 mg, 1600 mg and 1680 mg. Furthermore, examples of the dosage intervals include 1 week, 2 weeks, 3 weeks, 4 weeks, 6 weeks and 8 weeks, and examples of single dosing terms include about 30 minutes, about 60 minutes and about 60 minutes or more.

Herein, if the active ingredient of tumor immunotherapeutic drug is Nivolumab, which is an anti-PD-1 antibody, it can be administered to an adult at (1) 1 mg/kg (body weight) per dose every 3 weeks, (2) 3 mg/kg (body weight) per dose every 2 weeks, (3) 2 mg/kg (body weight) per dose every 3 weeks, (4) 80 mg/kg per dose every 3 weeks, (5) 240 mg/kg per dose every 2 weeks, (6) 360 mg/kg per dose every 3 weeks, or (7) 480 mg/kg per dose every 4 weeks, of Nivolumab, by intravenous drip infusion.

In particular, it can be administered to patients with malignant melanoma at 3 mg/kg (body weight) per dose every 2 weeks or 2 mg/kg (body weight) per dose every 3 weeks, of Nivolumab, by intravenous drip infusion, and administered to each patient with non-small cell lung cancer, renal cell carcinoma, classic Hodgkin's lymphoma, head and neck cancer, gastric cancer and malignant pleural mesothelioma, at 3 mg/kg (body weight) of Nivolumab per dose every 2 weeks by intravenous drip infusion. As another usage and dosage, for example, it can be administered to each patient with malignant melanoma, non-small cell lung cancer, renal cell carcinoma, urothelial cancer, MSI-H-positive colorectal cancer, gastric cancer, esophageal cancer, hepatocellular carcinoma, small cell lung cancer and malignant pleural mesothelioma, at 240 mg per dose every 2 weeks or 480 mg per dose every 4 weeks, of Nivolmab, by intravenous drip infusion. Furthermore, as yet another usage and dosage, for example, to patients with malignant melanoma, in combination with Ipilimumab (3 mg/kg (body weight) per dose once per day every 3 weeks four times by intravenous drip infusion), it can be administered at 1 mg/kg (body weight) of Nivolumab per dose four times every 3 weeks by intravenous drip infusion, and then administered at 3 mg/kg (body weight) of Nivolumab per dose every 2 weeks by intravenous drip infusion, or it can be administered at 80 mg of Nivolumab per dose four times every 3 weeks by intravenous drip infusion, and then administered at 240 mg per dose every 2 weeks or 480 mg per dose every 4 weeks,

of Nivolumab, by intravenous drip infusion. Furthermore, for example, to each patient with renal cell carcinoma or MSI-H-positive colorectal cancer, in combination with Ipilimumab (1 mg/kg (body weight) per dose once per day every 3 weeks four times by intravenous drip infusion), it can be administered at 240 mg of Nivolumab per dose four times every 3 weeks by intravenous drip infusion, and then administered at 240 mg per dose every 2 weeks or 480 mg per dose every 4 weeks, of Nivolumab, by intravenous drip infusion. On the other hand, for example, to patients with non-small cell lung cancer, in combination with other anti-neoplastic agents, it can be administered at 240 mg per dose every 2 weeks or 360 mg per dose every 3 weeks, of Nivolumab, by intravenous drip infusion.

If the active ingredient of tumor immunotherapeutic drug is Pembrolizumab, which is an anti-PD-1 antibody, it can be administered to an adult at (1) 200 mg per dose every 3 weeks, (2) 400 mg per dose every 6 weeks, or (3) 2 mg/kg (body weight) per dose (up to 200 mg per dose), of Pembrolizumab, by intravenous drip infusion. In particular, to each patient with malignant melanoma, non-small cell lung cancer, small cell lung cancer, classical Hodgkin lymphoma, head and neck cancer, MSI-H-positive solid cancer or colorectal cancer, urothelial cancer, cervical cancer, endometrial cancer, primary mediastinal B-cell lymphoma, hepatocellular carcinoma, gastric cancer, esophageal cancer and Merkel cell carcinoma, it can be administered at 200 mg per dose every 3 weeks or 400 mg per dose every 6 weeks, of Pembrolizumab, by intravenous drip infusion. Furthermore, to patients with renal cell carcinoma, in combination with Axitinib, it can be administered at the same usage and dosage. Furthermore, as another usage and dosage, for example, to each patient with pediatric classic Hodgkin lymphoma, aged 2 years or older, MSI-H-positive solid cancer or colorectal cancer, primary mediastinal B-cell lymphoma and Merkel cell carcinoma, it can be administered at 2 mg/kg (body weight) of Pembrolizumab per dose (up to 200 mg per dose) every 3 weeks by intravenous drip infusion.

If the active ingredient of tumor immunotherapeutic drug is Cemiplimab-rwlc, which is an anti-PD-1 antibody, it can be administered to an adult at 350 mg/kg of Cemiplimab-rwlc per dose every 3 weeks by intravenous drip infusion. In particular, to patients with spinous cell carcinoma, it can be administered at the same dosage and usage.

If the active ingredient of tumor immunotherapeutic drug is Avelumab, which is an anti-PD-L1 antibody, it can be administered to an adult at 10 mg/kg (body weight) of Avelumab per dose every 2 weeks by intravenous drip infusion. In particular, to patients with Merkel cell carcinoma, it can be administered at 10 mg/kg (body weight) of Avelumab per dose every 2 weeks by intravenous drip infusion. Furthermore, to patients with renal cell carcinoma, in combination with Axitinib, it can be administered at the same usage and dosage.

If the active ingredient of tumor immunotherapeutic drug is Atezolizumab, which is an anti-PD-L1 antibody, it can be administered to an adult at (1) 840 mg per dose every 2 weeks, (2) 1200 mg per dose every 3 weeks, or (3) 1680 mg per dose every 4 weeks, ofAtezolizumab by intravenous drip infusion. In particular, to each patient with non-small cell lung cancer or small cell lung cancer, previously treated with chemotherapy, urothelial cancer and hepatocellular carcinoma, it is administered at the same usage and dosage above, herein, to patients with non-small cell lung cancer, previously untreated with chemotherapy, in combination with other anti-neoplastic agents (Bevacizumab, Paclitaxel and Carboplatin), or to patients with non-small cell lung cancer, previously untreated with chemotherapy, in combination with other anti-neoplastic agents (Carboplatin and Etoposide), it can be administered at 1200 mg per dose every 3 weeks, respectively. Furthermore, to patients with triple negative breast cancer, in combination with Paclitaxel, it can be administered at 840 mg of Atezolizumab per dose every 2 weeks by intravenous drip infusion.

If the active ingredient of tumor immunotherapeutic drug is Durvalumab, which is an anti-PD-L1 antibody, it can be administered to an adult at 10 mg/kg (body weight) of Durvalumab per dose every 2 weeks by intravenous drip infusion. In particular, to each patient with non-small cell lung cancer and urothelial cancer, it can be administered at the same usage and dosage.

If the active ingredient of tumor immunotherapeutic drug is Ipilimumab, which is an anti-CTLA-4 antibody, it can be administered to an adult at (1) 3 mg/kg (body weight) per dose once per day or (2) 1 mg/kg (body weight) per dose once per day, of Ipilimumab, four times every 3 weeks by intravenous drip infusion. In particular, to patients with malignant melanoma, it can be administered alone or in combination with Nivolumab at 3 mg/kg (body weight) of Ipilimumab per dose once per day four times every 3 weeks by intravenous drip infusion, and to each patient with renal cell carcinoma and MSI-positive colorectal cancer, in combination with Nivolumab, it can be administered at 1 mg/kg (body weight) of Ipilimumab per dose once per day four times every 3 weeks by intravenous drip infusion.

[Adrenal corticosteroids]

Examples of the adrenal corticosteroids which can be used in the present invention include one or more kinds of agents containing an active ingredient selected from cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone

palmitate, dexamethasone sodium metasulfobenzoate, paramethasone, paramethasone acetate, betamethasone, betamethasone dipropionate, betamethasone valerate, betamethasone acetate, betamethasone butyrate propionate and betamethasone sodium phosphate.

The adrenal corticosteroids which is used in the present invention are preferably those which can be applied in the form of injection which is expected to have the inhibitory effect in a short-term on the induction of cytokine production in blood or tissues induced in a short time by STING agonistic compounds, and preferable examples thereof include hydrocortisone sodium phosphate, hydrocortisone sodium succinate, methylprednisolone sodium succinate, prednisolone sodium succinate, dexamethasone, dexamethasone sodium phosphate, betamethasone sodium phosphate and the like. On the other hand, examples of adrenal corticosteroids which can inhibit the induction of cytokine production by oral administration include an agent containing dexamethasone as the active ingredient.

The usage and dosage of adrenal corticosteroid in the present invention varies, according to the judgment of the treating physician based on the type of adrenal corticosteroid used and the patient's symptoms, but in principle, if the active ingredient of the adrenal corticosteroid is

(a) hydrocortisone sodium phosphate, it can be administered to an adult at 100 to 1000 mg of hydrocortisone per dose, 1 to 4 times per day by intravenous injection or intravenous drip infusion,
(b) hydrocortisone sodium succinate, it can be administered to an adult
(b1) at 50 to 100 mg of hydrocortisone per dose, 1 to 4 times per day by intravenous injection or intravenous drip infusion, or
(b2) in an emergency, at 100 to 200 mg of hydrocortisone per dose by intravenous injection or intravenous drip infusion,
(c) prednisolone sodium succinate, it can be administered to an adult
(c1) at 10 to 50 mg of prednisolone per dose every 3 to 6 hours by intravenous injection, or
(c2) at 20 to 100 mg of prednisolone per dose once or twice per day by intravenous drip infusion,
(d) methylprednisolone sodium succinate, it can be administered slowly to an adult at 125 to 2000 mg of methylprednisolone per dose by intravenous injection or intravenous drip infusion,
(e) dexamethasone sodium phosphate, it can be administered to an adult
(e1) at 1.65 to 6.6 mg of dexamethasone per dose every 3 to 6 hours by intravenous injection, or
(e2) at 1.65 to 8.3 mg of dexamethasone per dose once or twice per day by intravenous drip infusion,
(f) betamethasone sodium phosphate, it can be administered to an adult
(f1) at 2 to 8 mg of betamethasone per dose every 3 to 6 hours by intravenous injection, or
(f2) at 2 to 10 mg of betamethasone per dose once or twice per day by intravenous drip infusion.

On the other hand, if the active ingredient of the adrenal corticosteroid is dexamethasone, it can be administered orally to an adult at 0.5 to 8 mg of betamethasone per day in 1 to 4 divided doses.

The timings of administering the adrenal corticosteroid pertaining to the present invention can be, for example, before administration of the STING agonist per each administration thereof, for example, an arbitrary time between immediately before and about 2 hours before administration thereof, and preferably about 30 minutes, about 1 hour, about 90 minutes or about 2 hours before administration thereof. Furthermore, it can be administered, per each administration of the STING agonist, after administration thereof, for example, just after administration thereof, or simultaneously per each administration of the STING agonist.

On the other hand, if the adrenal corticosteroid pertaining to the present invention is administered orally, it can be administered, per each administration of the STING agonist, at an arbitrary timing at least one day before administration thereof.

When administering the adrenal corticosteroid pertaining to the present invention, it may be combined with an anti-histamine drug (e.g., diphenhydramine, chlorpheniramine, ketotifen and olopatadine, etc.), nonsteroidal anti-inflammatory drug (NSAID) (e.g., ibuprofen, indomethacin, felbinac, loxoprofen, meloxicam, ketoprofen, ibuprofen, flurbiprofen, naproxen and celecoxib, etc.) and/or an anti-fever analgesic (e.g., aspirin, acetaminophen, isopropylantipyrine, ethenzamide, sazapyrine, salicylamide, sodium salicylate, thiaramide hydrochloride and lactylphenetidine, etc.).

[Combination with Other Drugs]

The STING agonist in the present invention may be administered with or instead of an adrenal corticosteroid, with IL-6 inhibitor (e.g., Tocilizumab and Sarilumab) or TNF-alpha inhibitor (e.g., Infliximab, Adalimumab, Etanercept, and Golimumab).

[Application of the Present Invention]

The types of cancers to which the STING agonist of the present invention may be applied are not limited and include all solid tumors and hematological cancers. Herein, among solid cancers, examples of epithelial cell cancers include malignant melanoma (e.g., malignant melanoma in skin, oral mucosal epithelium, or orbit, etc.), non-small cell lung cancer (e.g., squamous non-small cell lung cancer and non-squamous non-small cell lung cancer), small cell lung cancer, head and neck cancer (e.g., oral cancer, nasopharyngeal cancer, oropharyngeal cancer, hypopharyngeal cancer, laryngeal cancer, salivary gland cancer and tongue cancer), renal cell cancer (e.g., clear cell renal cell cancer), breast cancer, ovarian cancer (e.g., serous ovarian cancer and ovarian clear cell adenocarcinoma), nasopharyngeal cancer, uterine cancer (e.g., cervical cancer and endometrial cancer), anal cancer (e.g., anal canal cancer), colorectal cancer (e.g., MSI-H and/or dMMR positive colorectal cancer), rectal cancer, colon cancer, hepatocellular carcinoma, esophageal cancer, gastric cancer, esophagogastric junction cancer, pancreatic cancer, urine urothelial cancer (e.g., bladder cancer, upper urinary tract cancer, ureteral cancer, renal pelvis cancer and urethral cancer), prostate cancer, fallopian tube cancer, primary peritoneal cancer, malignant pleural mesothelioma, gallbladder cancer, bile duct cancer, biliary tract cancer, skin cancer (e.g., uveal melanoma and Merkel cell carcinoma), testicular cancer (germ cell tumor), vaginal cancer, vulvar cancer, penile cancer, small intestine cancer, endocrine system cancer, thyroid cancer, parathyroid cancer, adrenal carcinoma, spinal tumor, neuroblastoma, medulloblastoma, ocular retinoblastoma, neuroendocrine tumor, brain tumor (e.g., glioma (e.g., glioblastoma and gliosarcoma) and meningioma), squamous cell carcinoma and the like.

Further, among solid cancers, examples of sarcomas include bone/soft tissue sarcomas (e.g., Ewing sarcoma, pediatric rhabdomyosarcoma, endometrial leiomyosarcoma, chondrosarcoma, lung sarcoma, osteosarcoma and congenital fibrosarcoma), Kaposi's sarcoma and the like.

Furthermore, examples of hematological cancers include multiple myeloma, malignant lymphoma (e.g., non-Hodgkin lymphoma (e.g., B-cell non-Hodgkin's lymphoma (e.g., precursor B-cell lymphoblastic lymphoma, precursor B-cell acute lymphoblastic leukemia, chronic B-lymphoid leukemia, (small lymphocytic lymphoma), B-cell precursor leukemia, B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B-cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM-monoclonal gammopathy of undetermined significance, $\mu$ heavy chain disease, $\lambda$ heavy chain disease, $\alpha$ heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma with MYC and BCL2 and/or BCL6 rearrangement, and high-grade B-cell lymphoma, not otherwise specified and B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroavacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the gastrointestinal tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with T follicular helper phenotype, and anaplastic large cell lymphoma, ALK positive, anaplastic large cell lymphoma, ALK negative or breast implant-associated anaplastic large-cell lymphoma)) and Hodgkin lymphoma (e.g., classic Hodgkin lymphoma (e.g., nodular sclerosis, mixed cellularity, lymphocyte-rich and lymphopenic) and nodular lymphoid predominant Hodgkin

lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome and chronic myelogenous leukemia), central nervous system malignant lymphoma, myeloproliferative syndromes and the like.

Furthermore, the types of cancers to which the STING agonist of the present invention may be applied also include pediatric cancers and unknown primary cancers.

Furthermore, the STING agonist in the present invention may also be prescribed to (a) a patient with cancer on which the therapeutic effects of other anti-neoplastic agents are insufficient or not sufficient, or patient with cancer worsened after treatment with other anti-neoplastic agents, (b) a patient with incurable or unresectable, metastatic, recurrent, refractory and/or distant metastatic cancer, (c) a patient with cancer of which TPS or CPS is 50% or more, 25% or more, 10% or more, 5% or more or 1% or more, (d) a patient with MSI-H or dMMR cancer (e) a patient with BRAF V600E mutation-positive malignant melanoma or non-small cell lung cancer, (f) a patient with EGFR gene mutation-positive or ALK fusion gene-positive cancer, or (g) a patient with TMB high frequency cancer.

Herein, "other anti-neoplastic agents" are the anti-neoplastic agents pertaining to the present invention, which include agents exemplified as alkylating agents, platinum preparations, antimetabolite antagonists (e.g., folate metabolism, pyridine metabolism inhibitors and purine metabolism inhibitors), ribonucleotide reductase inhibitors, nucleotide analogs, topoisomerase inhibitors, microtubule polymerization inhibitors, microtubule depolymerization inhibitors, antitumor antibiotics, cytokine preparations, anti-hormonal drugs, molecular targeting drugs, and tumor immunotherapeutic drugs. Furthermore, examples of the meanings of "the therapeutic effects of other anti-neoplastic agents are insufficient or not sufficient" include the case to be determined as "stable (SD)" or "progression (PD)" according to Response Evaluation Criteria in Solid Tumors: RECIST by even treatment with already-existing anti-cancer drugs.

Furthermore, the STING agonist in the present invention may also be prescribed to (h) a patent with cancer with no history of treatment with other anti-neoplastic agents, (i) a patient with cancer in which TPS or CPS is less than 50%, less than 25%, less than 10%, less than 5% or less than 1%, (j) a patient with cancer without MSI-H and/or dMMR or with MSI-L, (k) a patient with BRAF V600 wild type malignant melanoma or non-small cell lung cancer, (1) a patient with EGFR gene mutation-negative and/or ALK fusion gene-negative non-small cell lung cancer, or (m) a patient with TMB low frequency cancer.

Furthermore, the STING agonist in the present invention can also be applied as a postoperative adjuvant therapy for preventively suppressing the recurrence or metastasis after surgical resection of cancer or preoperative adjuvant therapy, being performed before surgical resection.

In the present specification, examples of the term "treating cancer" include therapies (a) to decrease the proliferation of cancer cells, (b) to reduce symptoms caused by cancer, to improve the quality of life of a patient with cancer, (c) to reduce the dosage of other already administered anti-cancer drugs or cancer therapeutic adjuvants and/or (d) to prolong the survival of a patient with cancer. And, the term "suppressing the progress of cancer" means delaying the progress of cancer, stabilizing symptoms associated with cancer, and reversing the progress of symptoms. The term "suppressing the recurrence of cancer" means to prevent the recurrence of cancer in a patient of which cancer lesion had been completely or substantially eliminated or removed by cancer therapy or cancer resection surgery.

[Combination or Combination Preparation]

In order to (a) suppress the progression of, suppress the recurrence of and/or enhance the therapeutic effect on cancer, (b) decrease the dosage of other combined anti-neoplastic agents, (c) reduce the side effects of other combined anti-neoplastic agents, and/or (d) enhance the immunoenhancing effects of other combined anti-neoplastic agents, that is, as an adjuvant, the STING agonist in the present invention may be prescribed in combination with one or more kinds of other anti-neoplastic agents. In the present invention, the formulation which is prescribed in combination with other anti-neoplastic agents may be of a combination preparation which both components are mixed in one preparation or of separated preparations. The combination can compensate the effects in preventing, suppressing the progression of, suppressing the recurrence of and/or treating cancer with the anti-neoplastic agents, and maintain or reduce the dosage or frequency of administration thereof. If the STING agonist in the present invention and other anti-neoplastic agents are administered separately, both may be administered simultaneously for a certain period, and then only the STING agonist in the present invention or other anti-neoplastic agents may be administered alone. Furthermore, the STING agonist in the present invention may be administered initially, followed by administration with other anti-neoplastic agents, or other anti-neoplastic agents may be administered initially, followed by administration with the STING agonist in the present invention. In the above administration, there may be a certain period in which both drugs are administered, simultaneously. Furthermore, the methods for administering each drug may be the same or different. Depending on the nature of drugs, it can also be provided as a kit containing the STING agonist in the present invention and other anti-neoplastic agents. Herein, the dosage of other anti-neoplastic agents can be appropriately selected based on a dosage clinically used. Furthermore, other anti-neoplastic agents may be administered in combination of two or more kinds

thereof at an appropriate ratio. Furthermore, examples of other anti-neoplastic agents include those which would be found in the future, as well as those which have been found to date.

[Formulation]

The STING agonist the present invention is almost used as an injection or infusion solution for parenteral administration.

The injection or infusion solution for parenteral administration may be in any form of an aqueous solution, suspension or emulsion, or may be formulated as a solid agent along with pharmaceutically acceptable carrier such that it will used with being dissolved, suspended, or emulsified in a solvent (e.g., distilled water for injection, saline, dextrose solution, and isotonic solution (e.g., solution of sodium chloride, potassium chloride, glycerin, mannitol, sorbitol, boric acid, borax or propylene glycol), etc.) at the time of use. Herein, examples of the pharmaceutically acceptable carriers include a stabilizer (e.g., various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate and dibutylhydroxytoluene, etc.), solubilizer (e.g., alcohols (e.g., ethanol etc.), polyols (e.g., propylene glycol and polyethylene glycol, etc.), nonionic surfactants (e.g., Polysorbate 20 (registered trademark), Polysorbate 80 (registered trademark) and HCO-50, etc.), etc.), suspending agent (e.g., glyceryl monostearate, aluminum monostearate, methyl cellulose, carboxymethyl cellulose, hydroxymethyl cellulose and sodium lauryl sulfate, etc.), emulsifier (e.g., gum arabic, sodium alginate and tragacanth, etc.), soothing agent (e.g., benzyl alcohol, chlorobutanol and sorbitol, etc.), buffering agent (e.g., phosphate buffer, acetate buffer, borate buffer, carbonate buffer, citrate buffer, Tris buffer, glutamic acid buffer and epsilon aminocaproic acid buffer, etc.), preservative (e.g., methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid and borax, etc.), antiseptic agent (e.g., benzalkonium chloride, parahydroxybenzoic acid and chlorobutanol, etc.), pH adjuster (e.g., hydrochloric acid, sodium hydroxide, phosphoric acid and acetic acid, etc.), antioxidant and the like. As the antioxidants, (1) aqueous antioxidant such as ascorbic acid, cysteine hydrochloride, sodium bisulfate, sodium metabisulfite, and sodium sulfite, (2) oil-soluble antioxidant such as ascorbyl palmitate, butylated hydroxy anisole, butylated hydroxy toluene, lecithin, propyl gallate, and $\alpha$-tocopherol, and (3) metal chelating agent such as citric acid, ethylenediaminetetraacetic acid, sorbitol, tartaric acid, and phosphoric acid or the like can be used.

The injection or infusion solution can be produced by performing sterilization in the final process, or aseptic manipulation, e.g., performing sterilization by filtration with a filter or the like, and subsequently filling an aseptic container. The injection or infusion solution may be used by dissolving the vacuum dried or lyophilized aseptic powder (which may include a pharmaceutically acceptable carrier powder) in an appropriate solvent at the time of use.

The contents of all patent documents and non-patent documents or references explicitly cited in the present specification may be incorporated herein as part of the present specification.

The present invention will be described in more detail by the following Examples, but the scope of the present invention is not limited thereto. Various changes or modifications can be made by those skilled in the art based on the description of the present invention, and these changes or modifications are also included in the present invention.

[Example]

Hi-flash SI or Hi-flash NH in parentheses shown in the section of medium pressure preparative liquid chromatography represents the type of column used (Hi-flash SI: silica gel (manufactured by Yamazen Co., Ltd.), Hi-flash NH: aminopropyl group-supporting silica gel (manufactured by Yamazen Co., Ltd.)).

LC-MS / ELSD was performed under the following conditions:
[Column: YMC Triart C18 (particle size: $1.9 \times 10^{-6}$ m; column length: $30 \times 2.0$ mm ID); flow rate: 1.0 mL/min; column temperature: 40 °C; mobile phase (A): 0.1 % trifluoroacetic acid solution; mobile phase (B): 0.1% trifluoroacetic acid-acetonitrile solution; gradient (show the ratio of mobile phase (A): mobile phase (B)): [0 min] 95: 5; [0.1 min] 95: 5; [1.2 min] 5:95; [1.4 min] 5:95; [1.41 min] 95: 5; [1.5 min] 95: 5; and detector: UV (PDA), ELSD, MS]

Numerical values shown at NMR are the $^1$H-NMR-measured values (chemical shift values) when the measurement solvent described in the parentheses is used.

The compound names used in the present specification are named by using computer programs: ACD/Name (registered trademark) (version 6.00, manufactured by Advanced Chemistry Development Inc.), Chemdraw Ultra (version 12.0, manufactured by Cambridge Soft) or Lexichem Toolkit (version 1.4.2, manufactured by OpenEye Scientific Software), which generally names according to IUPAC rules, or named according to the IUPAC nomenclature.

Reference Example 1: Lithium 2-chloro-4-fluoro-5-iodonicotinate

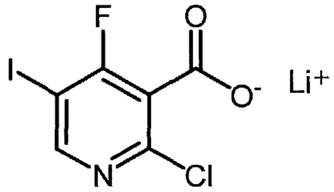

2-chloro-4-fluoro-5-iodopyridine (CAS No. 1370534-60-3) (13.4 g) was dissolved in tetrahydrofuran (hereinafter, abbreviated as THF) (50 mL) and cooled to -78 °C. Then, lithium diisopropylamide (1 mol/L THF solution, 50 mL) was added dropwise thereto over 30 minutes. After stirring at -78 °C for 1.5 hours, finely crushed dry ice (11.4 g) was added thereto, which was stirred at -78 °C for 30 minutes. The reaction solution was warmed to room temperature and the resulting precipitate was collected by filtration to give the titled compound (16.5 g) having the following physical property value.
LCMS retention time (min): 0.63;
MS (ESI, Pos.): 302 $(M + H)^+$;
$^1$H-NMR (DMSO-$d_6$): δ 8.44 (d, J=9.0Hz, 1H).

Reference Example 2: 2-chloro-4-fluoro-5-iodonicotinonitrile

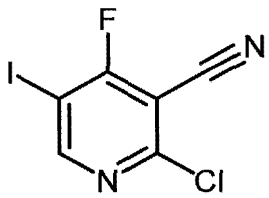

A mixture of the compound (16.0 g) prepared in Reference Example 1, *N, N*-dimethylformamide (hereinafter, abbreviated as DMF) (0.20 mL) and thionyl chloride (38.0 mL) was stirred at 80 °C for 3.5 hours. The reaction solution was concentrated, and the THF solution dissolving the residue therefrom (100 mL) was cooled to 0 °C, to which saturated aqueous ammonia (28%, 10.8 mL) was added dropwise with stirring. After stirring for 30 minutes, tap water was added to the reaction mixture, which was extracted with ethyl acetate. The organic layer was washed with saturated saline, dried over sodium sulfate, and concentrated. The residue therefrom was used in the next step without purification.

The crude product obtained by the above operation was dissolved in THF (174 mL), pyridine (21.1 mL) and trifluoroacetic anhydride (10.9 mL) were added thereto under ice cooling, and the mixture thereof was stirred at 0 °C for 1 hour. A saturated aqueous sodium hydrogen carbonate solution was added thereto, and the mixture thereof was extracted with ethyl acetate. The organic layer was washed with saturated saline, dried over sodium sulfate, and concentrated. The residue therefrom was purified by silica gel chromatography (Hi-flash SI) (hexane: ethyl acetate = 0: 100 to 70: 30) to give the titled compound (5.82 g) having the following physical property value.
LCMS retention time (min): 0.92;
MS (ESI, Pos.): 283 $(M + H)^+$;
$^1$H-NMR ($CDCl_3$): δ 8.83 (d, J=7.7Hz, 1H).

Reference Example 3: 2-chloro-5-iodo-4-((propan-2-ylideneamino)oxy)nicotinonitrile

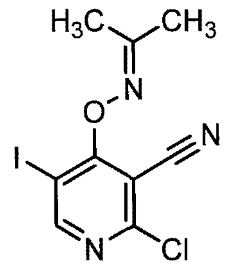

Sodium *tert*-butoxide (9.02 g) was added to the THF solution (100 mL) dissolving propan-2-one oxime (6.86 g) at room temperature, and the mixture thereof was stirred for 1 hour (hereinafter, this solution is referred to as an oxime solution). The oxime solution was added dropwise to THF solution (90 mL) dissolving the compound (26.5 g) produced in Reference Example 2 over 15 minutes under ice cooling. After the temperature of the reaction solution was raised to room temperature, it was further stirred for 30 minutes. A saturated ammonium chloride aqueous solution was added thereto, and the mixture thereof was extracted with ethyl acetate. The organic layer was washed with saturated saline, dried over sodium sulfate, and concentrated. The residue therefrom was purified by silica gel column chromatography (Hi-flash SI) (hexane: ethyl acetate = 70: 30) to give the titled compound (31.1 g) having the following physical property value.
LCMS retention time (min): 1.02;
$^1$H-NMR ($CDCl_3$): δ 8.67 (s, 1H), 2.21 (s, 3H), 2.13 (s, 3H).

Reference Example 4: 4-chloro-7-iodoisoxazolo[4,5-c]pyridin-3-amine

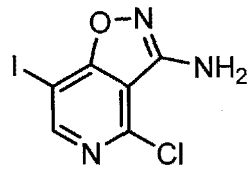

5 mol/L hydrochloric acid (70 mL) was added to ethanol solution (70 mL) dissolving the compound (4.66 g) produced in Reference Example 3, and the mixture thereof was stirred at 70 °C for 1 hour. The solid formed in the reaction solution was collected by filtration to give the titled compound (2.93 g) having the following physical property value.
LCMS retention time (min): 0.76;
MS (ESI, Pos.): 296 $(M + H)^+$;
$^1$H-NMR (DMSO-$d_6$): δ 8.65 (s, 1H), 6.59 (s, 2H).

Reference Example 5: 4-bromo-7-iodoisoxazolo[4,5-c]pyridin-3-amine

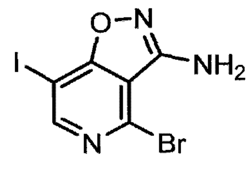

Bromotrimethylsilane (14.9 mL) was added to propionitrile solution (55.5 mL) dissolving the compound (5.55 g) produced in Reference Example 4 at room temperature, which was stirred at 105 °C for 3 hours. The reaction solution was cooled to room temperature, saturated aqueous sodium hydrogen carbonate solution was added thereto, and the mixture thereof was extracted with ethyl acetate. The organic layer was washed with saturated saline, dried over sodium sulfate, and concentrated. To the residue therefrom, hexane-ethyl acetate mixed solvent (4: 1, 50 mL) was added, and the mixture thereof was stirred for 30 minutes. The precipitate therein was collected by filtration to give the titled compound (4.78 g) having the following physical property value.
LCMS retention time (min): 0.81;

MS (ESI, Pos.): 340 $(M + H)^+$;
$^1$H-NMR ($CDCl_3$): δ 8.64 (s, 1H), 6.48 (s, 2H).

Reference Example 6: 4-bromo-7-(1-(tetrahydro-2H-pyran-2-yl)-1*H*-pyrazol-4-yl)isoxazolo[4,5-*c*]pyridin-3-amine

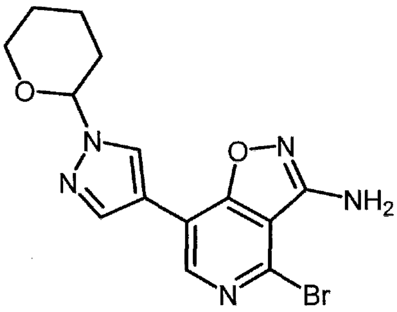

Under nitrogen atmosphere, 1-(tetrahydro-2*H*-pyran-2-yl)-4-(4,4,5,5-tetramethyl-1,3,2-dioxaborolan-2-yl)-1*H*-pyrazole (1.73 g)(CAS No. 1.003846-21-6), [1,1'-bis(diphenylphosphino)ferrocene]palladium(II) dichloride (484 mg) and 2 mol/L tripotassium phosphate aqueous solution (5.9 mL) was added to 1,4-dioxane solution (25 mL) dissolving the compound (2.01 g) prepared in Reference Example 5 (2.01 g), and the mixture thereof was stirred at 90 °C for 4 hours. The reaction solution was cooled to room temperature, diluted with ethyl acetate, and the insoluble material therein was filtered through a short silica gel pad. Water was added to the obtained filtrate, and the mixture thereof was extracted with ethyl acetate. The organic layer was washed with saturated brine, dried over sodium sulfate, and concentrated. To the residue therefrom, methanol (10 mL) was added, and the mixture thereof was stirred for 30 minutes. The precipitate therein was collected by filtration to give the titled compound (1.50 g) having the following physical property value.
LCMS retention time (min): 0.80;
MS (ESI, Pos.): 364 $(M + H)^+$.

Reference Example 7: (5-bromo-4-fluoro-2-nitrophenyl)(methyl)sulfane

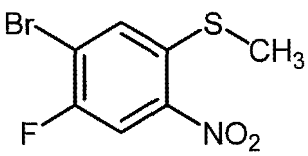

(1-bromo-2,5-fluoro-2-nitrophenyl)(methyl)sulfane (CAS No. 167415-27-2) (2.00 g) was dissolved in DMF solution (20 mL) and cooled to 0 °C. An aqueous solution (4.2 mL) dissolving sodium thiomethoxide (707 mg) was added dropwise thereto, and the mixture thereof was stirred under ice cooling for 1.5 hours. The resulting precipitate therein was collected by filtration and dried to give the titled compound (1.17 g) having the following physical property value.
LCMS retention time (min): 1.05;
$^1$H-NMR ($CDCl_3$): δ 8.06 (d, J=8.0Hz, 1H), 7.52 (d, J=6.0Hz, 1H), 2.52 (s, 3H).

Reference Example 8: 4-bromo-5-fluoro-2-(methylthio)aniline

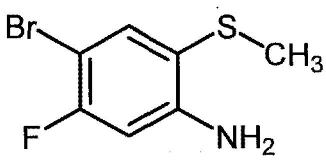

Iron powder (1.23 g) was added to acetic acid solution (12 mL) dissolving the compound (1.17 g) produced in Reference Example 7, and the mixture thereof was stirred at 90 °C for 1 hour. The reaction solution was cooled to room temperature, filtered through Celite (Registered trademark), and the obtained filtrate was concentrated. The residue therefrom was purified by silica gel column chromatography (Hi-flash NH) (hexane: ethyl acetate = 90: 10 to 70: 30) to give the titled compound (1.06 g) having the following physical property value.
LCMS retention time (min): 1.01;
MS (ESI, Pos.): 236 $(M + H)^+$;
$^1$H-NMR ($CDCl_3$): δ 7.52 (d, J=7.5Hz, 1H), 6.50 (d, J=10.5Hz, 1H), 4.45 (brs, 2H), 2.31 (s, 3H).

Reference Example 9: 4-bromo-5-fluoro-2-(methylsulfonyl) aniline

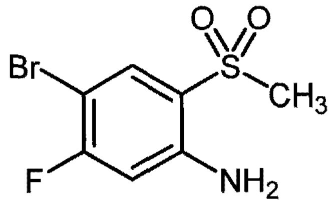

Under ice cooling, metachloroperbenzoic acid (containing about 30% water) (1.41 g) was added to dichloromethane solution (8.0 mL) dissolving the compound (500 mg) produced in Reference Example 8. After stirring it under ice-cooling for 1 hour, 10% sodium thiosulfate aqueous solution and saturated sodium hydrogencarbonate aqueous solution were added thereto to stop its reaction, and the mixture thereof was extracted with ethyl acetate. The organic layer was washed with saturated brine, dried over sodium sulfate, and concentrated. The residue therefrom was purified by silica gel column chromatography (Hi-flash SI) (hexane: ethyl acetate = 90: 10 to 50: 50) to give the titled compound (487 mg) having the following physical property value.
LCMS retention time (min): 0.82;
MS (ESI, Pos.): 268 $(M + H)^+$.

Reference Example 10: 1-(2-amino-5-bromo-4-fluorophenyl)ethan-1-one

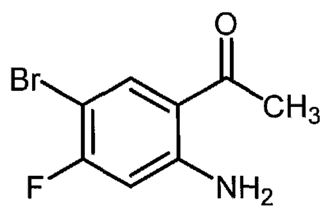

Under nitrogen atmosphere, 4-bromo-5-fluoro-2-iodoaniline (CAS No. 1219741-79-3) (810 mg), copper (I) iodide (48.8 mg), tributyl(1-ethoxyvinyl)tin (1.04 mL) and acetonitrile (10 mL) were mixed, and the mixture solution thereof was deaerated. Bis(triphenylphosphine)palladium (II) dichloride (180 mg) was added thereto, and the mixture thereof was stirred at 80 °C for 5 hours. The reaction solution was directly purified by silica gel column chromatography (Hi-flash SI) (hexane: ethyl acetate = 100: 0 to 70 :30) to give the titled compound (547 mg) having the following physical property value.
LCMS retention time (min): 0.91;
MS (ESI, Pos.): 232 $(M + H)^+$.

Reference Example 11: 2-amino-5-bromo-*N*-ethyl-4-fluorobenzamide

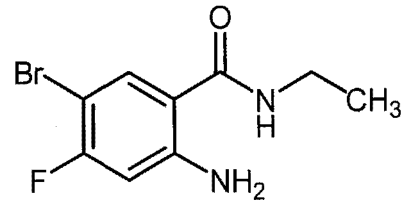

Amixture of 2-amino-5-bromo-4-fluorobenzoic acid (CAS No. 143945-65-7) (2.20 g), 1-[bis(dimethylamino)methylene]-1*H*-1,2,3-triazolo[4,5-*b*]pyridinium 3-oxidehexafluorophosphate (HATU: CAS No. 148893-10-1) (4.60 g), *N,N*-diisopropylethylamine (2.4 mL) and DMF (47 mL) was stirred at room temperature for 2 hours. To the reaction solution, saturated aqueous sodium hydrogen carbonate solution was added, and the mixture thereof was extracted with ethyl acetate. The organic layer was washed with saturated saline, dried over sodium sulfate, and concentrated. The residue therefrom was purified by silica gel column chromatography (Hi-flash SI) (hexane: ethyl acetate = 90: 10 to 60: 40) to give the titled compound (2.08 g) having the following physical property value.
LCMS retention time (min): 0.84;
MS (ESI, Pos.): 261 $(M + H)^+$.

Reference Example 12: 5-fluoro-2-(methylsulfonyl)-4-(4,4,5,5-tetramethyl-1,3,2-dioxaborolan-2-yl)aniline

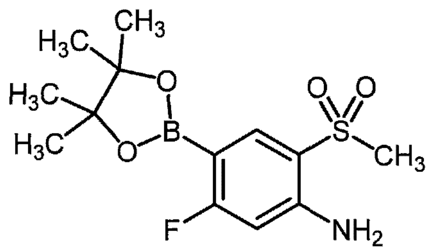

Under nitrogen atmosphere, 1,4-dioxane (8.0 mL) was added to a mixture of the compound (487 mg) produced in Reference Example 9, bis(pinacolato)diboron (922 mg) and potassium acetate (713 mg), and the mixture thereof was degassed. [1,1'-bis(diphenylphosphino)ferrocene]dichloropalladium dichloromethane complex (148 mg) was added thereto, and the mixture thereof was stirred at 90 °C overnight. The reaction mixture was filtered through Celite (registered trademark), and the obtained filtrate was concentrated. The residue therefrom was purified by silica gel column chromatography (Hi-flash SI) (hexane: ethyl acetate = 100: 0 to 80: 20) to give the titled compound (342 mg) having the following physical property value.
LCMS retention time (min): 0.91;
MS (ESI, Pos.): 316 $(M + H)^+$.

Reference Examples 12(1) to 12(5)

In place of 4-bromo-5-fluoro-2-(methylsulfonyl)aniline of Reference Example 9, the bromoaryl compound corresponding thereto was used, and by subjecting it to the same operation as in Reference Example 12, the compounds having the following physical property value were obtained.

Reference Example 12(1): methyl 2-amino-4-fluoro-5-(4,4,5,5-tetramethyl-1,3,2-dioxaborolan-2-yl)benzoate

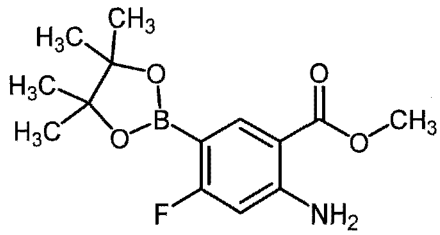

LCMS retention time (minutes): 1.04;
MS (ESI, Pos.): 296 $(M + H)^+$.

Reference Example 12(2): 5-fluoro-2-(methylthio)-4-(4,4,5,5-tetramethyl-1,3,2-dioxaborolan-2-yl)aniline

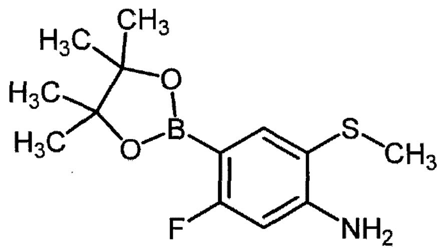

LCMS holding time (min): 1.06;
MS (ESI, Pos.): 284 $(M + H)^+$.

Reference Example 12(3): 1-(2-amino-4-fluoro-5-(4,4,5,5-tetramethyl-1,3,2-dioxaborolan-2-yl)phenyl)ethan-1-one

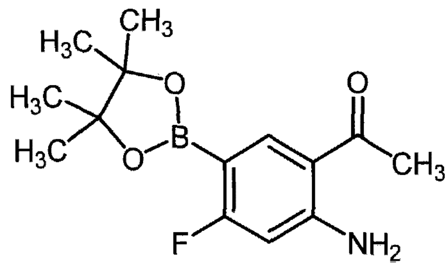

LCMS retention time (min): 0.99;
MS (ESI, Pos.): 280 $(M + H)^+$.

Reference Example 12(4): 2-amino-*N*-ethyl-4-fluoro-5-(4,4,5,5-tetramethyl-1,3,2-dioxaborolan-2-yl)benzamide

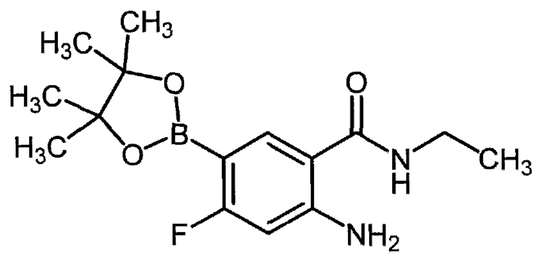

LCMS retention time (minutes): 0.91;
MS (ESI, Pos.): 309 $(M + H)^+$.

Reference Example 12(5): 2-amino-4-chloro-*N*-ethyl-5-(4,4,5,5-tetramethyl-1,3,2-dioxaborolan-2-yl)benzamide

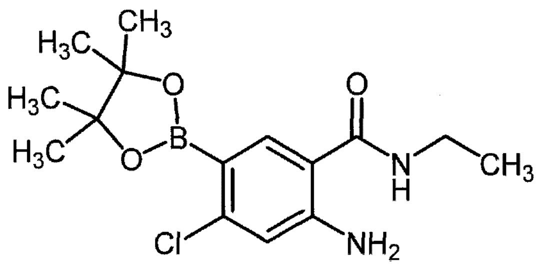

LCMS holding time (minutes): 1.14;
MS (ESI, Pos.): 325 $(M + H)^+$.

Reference Example 13: methyl 2-amino-5-(3-amino-7-(1-(tetrahydro-2*H*-pyran-2-yl)-1*H*-pyrazol-4-yl)isoxazolo[4,5-c]pyridin-4-yl)-4-fluorobenzoate

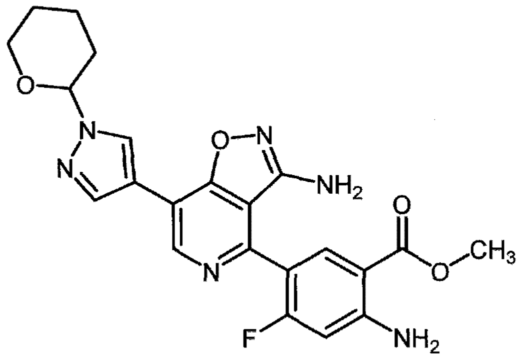

Under nitrogen atmosphere, the boronic acid ester (89.1 mg) produced in Reference Example 12(1) and bis[di-*tert*-butyl(4-dimethylaminophenyl)phosphine]palladium (19.4 mg) and 2 mol/L sodium carbonate aqueous solution (0.27 mL) were added to DMF solution (1.37 mL) dissolving the compound (100 mg) produced in Reference Example 6, and the mixture thereof was stirred at 110 °C for 2 hours. After cooling it to room temperature, tap water was added thereto, and the mixture thereof was extracted with ethyl acetate. The organic layer was washed with saturated saline, dried over sodium sulfate, and concentrated. The residue therefrom was purified by silica gel column chromatography (Hi-flash SI) (hexane: ethyl acetate = 95: 5 to 20: 80) to give the titled compound (110 mg) having the following physical property value.
LCMS retention time (min): 0.75;
MS (ESI, Pos.): 453 $(M + H)^+$.

Example 1: methyl 2-amino-5-(3-amino-7-(1*H*-pyrazol-4-yl)isoxazolo[4,5-*c*]pyridin-4-yl)-4-fluorobenzoate

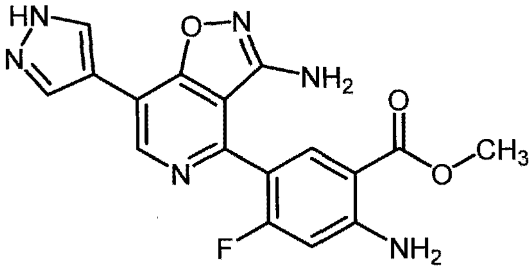

Trifluoroacetic acid (4.0 mL) was added to dichloromethane solution (4.0 mL) dissolving the compound (388 mg) produced in Reference Example 13, and the mixture thereof was stirred at 40 °C for 5 hours. To the reaction solution, saturated sodium hydrogen carbonate was added, and the mixture thereof was extracted with ethyl acetate. The organic layer was washed with saturated saline, dried over sodium sulfate, and concentrated. The residue therefrom was purified by silica gel column chromatography (Hi-flash SI) (hexane: ethyl acetate = 90: 10 to 0: 100) to give the titled compound (19.6 mg) having the following physical property value.
LCMS retention time (min): 0.59;
MS (ESI, Pos.): 369 $(M + H)^+$;
$^1$H-NMR ($CD_3OD$): δ 8.86 (s, 1H), 8.34 (s, 2H), 8.05 (d, J=8.5Hz, 1H), 6.66 (d, J=12.5Hz, 1H), 3,85 (s, 3H).

Example 2: 4-(4-amino-2-fluoro-5-methoxyphenyl)-7-(1*H*-pyrazol-4-yl)isoxazolo[4,5-*c*]pyridin-3-amine hydrochloride

Under nitrogen atmosphere, 5-fluoro-2-methoxy-4-(4,4,5,5-tetramethyl-1,3,2-dioxaborolan-2-yl)aniline (CAS No. 1326283-60-6) (224 mg), bis[tri-tert-butylphosphine]palladium (65.9 mg), and 2 mol/L tripotassium phosphate aqueous solution (1.1 mL) were added to 1,4-dioxane solution (7.1 mL) dissolving the compound (235 mg) produced in Reference Example 6, and the mixture thereof was stirred at 110 °C for 3 hours. The reaction solution was directly purified by silica gel column chromatography (Hi-flash SI) (hexane: ethyl acetate = 100: 0 to 0: 100) to give 4-(4-amino-2-fluoro-5-methoxyphenyl)-7-(1-(tetrahydro-2*H*-pyran-2-yl)-1*H*-pyrazol-4-yl)isoxazolo[4,5-*c*]pyridin-3-amine (108 mg) was obtained.

Hydrochloric acid (10% methanol solution, 2.0 mL) was added to THF solution (2.2 mL) dissolving this compound (108 mg), and the mixture thereof was stirred at room temperature for 2 hours. To the reaction solution, saturated sodium hydrogen carbonate was added, and the mixture thereof was extracted with ethyl acetate-methanol (9: 1). The organic layer was washed with saturated saline, dried over sodium sulfate and concentrated. The residue therefrom was purified by silica gel column chromatography (Hi-flash SI) (ethyl acetate: methanol = 100: 0 to 90:10). After concentration, the obtained residue was dissolved in methanol (5.0 mL), hydrochloric acid (10% methanol solution, 0.8 mL) was added thereto, and the mixture thereof was concentrated. To the obtained residue, ethyl acetate (50 mL) was added, and the mixture thereof was stirred under heating reflux for 1 hour and then concentrated to give the titled compound (87 mg) having the following physical property value.
LCMS retention time (min): 0.54;
MS (ESI, Pos.): 341 $(M + H)^+$;
$^1$H-NMR ($CD_3OD$): δ 8.96 (s, 1H), 8.44 (s, 2H), 7.11 (d, J=6.5Hz, 1H), 6.70 (d, J=12.0Hz, 1H), 3.93 (s, 3H).

Example 3: 1-(2-amino-5-(3-amino-7-(1*H*-pyrazol-4-yl)isoxazolo[4,5-*c*]pyridin-4-yl)-4-fluorophenyl)ethan-1-one (Compound B)

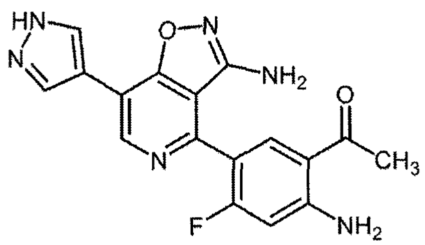

Under nitrogen atmosphere, the boronate ester (10.7 g) prepared in Reference Example 12 (3), butyl di-1-adamantylphosphine (984 mg), palladium acetate (308 mg), potassium iodide (456 mg) and 2 mol/L tripotassium phosphate aqueous solution (28 mL) were added to 1-methyl-2-pyrrolidone solution (hereinafter, abbreviated as NMP) (100 mL) dissolving the compound (10.0 g) produced in Reference Example 6, and the mixture thereof was stirred at 50 to 60 °C for 45 hours. After allowing the reaction solution to cool, insoluble matters therein were removed by filtration while washing with NMP. To the obtained filtrate, tap water (240 mL) was added little by little, and the mixture thereof was stirred for 40 minutes, and the precipitated solid therein was collected by filtration. The obtained solid was sequentially washed with acetonitrile (80 mL, twice) and methyl *tert*-butyl ether (80 mL, twice) by slurry washing, and then filtered and dried to give 1-(2-amino-5-(3-amino-7-(1-(tetrahydro-2*H*-pyran-2-yl)- 1H-pyrazol-4-yl)isoxazolo[4,5-*c*]pyridin-4-yl)-4-fluorophenyl)ethan-1-one (8.52 g).

To this compound (6.00 g), methanol (24 mL) and methanesulfonic acid (3.96 g) were added, and the mixture thereof was stirred at 55 °C for 3 hours. The reaction mixture was allowed to cool to room temperature, and triethylamine (18 mL) was added thereto, and the mixture thereof was stirred at 55 °C for 2.5 hours. After allowing it to cool, the resulting precipitate was filtered to obtain a beige powder. To the powder, methanol (40 mL) was added, and the mixture thereof was washed by slurry washing at room temperature, filtered, and dried to obtain the titled compound (4.50 g) having the following physical property value.
LCMS retention time (min): 0.56;
MS (ESI, Pos.): 353 $(M + H)^+$;
$^1$H-NMR (DMSO-$d_6$): δ 13.3 (s, 1H), 8.97 (s, 1H), 8.47 (s, 1H), 8.23 (s, 1H), 7.98 (d, J=8.5Hz, 1H), 7.71 (brs, 2H), 6.67 (d, J=13.0Hz, 1H), 5.73 (s, 2H), 2.52 (s, 3H).

Example 4: 4-(4-amino-2-fluoro-5-(methylthio)phenyl)-7-(1*H*-pyrazol-4-yl)isoxazolo[4,5-*c*]pyridin-3-amine hydrochloride

To THF solution (1.5 mL) dissolving 4-(4-amino-2-fluoro-5-(methylthio)phenyl)-7-(1-(tetrahydro-2*H*-pyran-2-yl)-1*H*-pyrazol-4-yl)isoxazolo[4,5-c]pyridin-3-amine (76.6 mg) obtained by using the boronate ester produced in Reference Example 12(2) in place of methyl 2-amino-4-fluoro-5-(4,4,5,5-tetramethyl-1,3,2-dioxaborolan-2-yl)benzoate prepared in Reference Example 12(1) and subjecting it to the same operation as that in Reference Example 13, hydrochloric acid (10% methanol solution, 1.1 mL) was added thereto at room temperature, and the mixture thereof was stirred for 1 hour. After the reaction, the resulting precipitate was collected by filtration to obtain the titled compound (76.1 mg) having the following physical property value.
LCMS retention time (min): 0.60;
MS (ESI, Pos.): 357 $(M + H)^+$;
$^1$H-NMR ($CD_3OD$): δ 9.05 (s, 1H), 8.53 (s, 2H), 7.76 (d, J=8.0Hz, 1H), 6.78 (d, J=13.0Hz, 1H), 2.41 (s, 3H).

Examples 4(1) to 4(16)

In place of 4-(4-amino-2-fluoro-5-(methylthio)phenyl)-7-(1-(tetrahydro-2*H* pyran-2-yl)-1*H*-pyrazol-4-yl)isoxazolo[4,5-*c*]pyridin-3-amine, the compound corresponding thereto was prepared by a procedure similar to that described in Example 4, and then subjected to a procedure similar to that described in Example 4, following that, to give the compounds having the following physical property values.

Example 4(1): 4-(4-amino-3-methoxyphenyl)-7-(1*H*-pyrazol-4-yl)isoxazolo[4,5-*c*]pyridin-3-amine hydrochloride

LCMS retention time (Min): 0.51;
MS (ESI, Pos.): 323 $(M + H)^+$;
$^1$H-NMR ($CD_3OD$): δ 8.99 (s, 1H), 8.49 (s, 2H), 7.52 (s, 1H), 7.42 (d, J=7.0Hz, 1H), 7.30 (d, J=8.5Hz, 1H), 4.05 (s, 3H).

Example 4(2): 4-(4-amino-2-fluoro-5-(methoxy-$d_3$)phenyl)-7-(1*H*-pyrazol-4-yl)isoxazolo[4,5-c]pvridine-3-amine hydrochloride

LCMS retention time (min): 0.55;
MS (ESI, Pos.): 344 $(M + H)^+$;
$^1$H-NMR ($CD_3OD$): δ 9.09 (s, 1H), 8.56 (s, 2H), 7.29 (d, J=5.5, 1H), 6.95 (d, J=9.0Hz, 1H).

Example 4(3): 4-(4-amino-2-fluoro-5-(methylsulfonyl)phenyl)-7-(1*H*-pyrazol-4-yl)isoxazolo[4,5-*c*]pyridin-3-amine hydrochloride

LCMS retention time (min): 0.55;
MS (ESI, Pos.): 389 $(M + H)^+$;
$^1$H-NMR ($CD_3OD$): δ 9.10 (s, 1H), 8.50 (s, 2H), 8.12 (d, J=8.0, 1H), 6.90 (d, J=12.5Hz, 1H), 3.17 (s, 3H).

Example 4(4): 4-(4-amino-2-fluoro-3-methoxyphenyl)-7-(1*H*-pyrazol-4-yl)isoxazolo[4,5-*c*]pyridin-3-amine hydrochloride

HPLC retention time (min): 0.55;
MS (ESI, Pos.): 341 $(M + H)^+$;
$^1$H-NMR ($CD_3OD$): δ 9.04 (s, 1H), 8.49 (s, 2H), 7.24 (dd, J=8.5, 7.5, 1H), 6.84 (d, J=8.5Hz, 1H), 3.99 (s, 3H).

Example 4(5): 2-amino-5-(3-amino-7-(1*H*-pyrazol-4-yl)isoxazolo[4,5-*c*]pyridin-4-yl)-4-fluorobenzamide hydrochloride

LCMS retention time (min): 0.50;
MS (ESI, Pos.): 354 $(M + H)^+$;
$^1$H-NMR (DMSO-$d_6$): δ 9.03 (s, 1H), 8.40 (s, 2H), 7.92 (d, J=9.0, 1H), 7.79 (br s, 1H), 7.23 (br s, 1H), 6.64 (d, J=12.0Hz, 1H), 5.98 (br s, 2H).

Example 4(6): ethyl 2-amino-5-(3-amino-7-(1*H*-pyrazol-4-yl)isoxazolo[4,5-*c*]pyridin-4-yl)-4-fluorobenzoate hydrochloride

LCMS retention time (min): 0.69;
MS (ESI, Pos.): 383 $(M + H)^+$;
$^1$H-NMR (DMSO-$d_6$): δ 9.01 (s, 1H), 8.38 (s, 2H), 7.99 (d, J=8.5, 1H), 7.30 (br s, 1H), 6.72 (d, J=13.0Hz, 1H), 5.83 (br s, 2H), 4.28 (q, J=7.0Hz, 2H), 1.29 (t, J=7.0Hz, 3H).

Example 4(7): 1-(2-amino-5-(3-amino-7-(1*H*-pyrazol-4-yl)isoxazolo[4,5-*c*]pyridin-4-yl)-4-fluorophenyl)propan-1-one hydrochloride

LCMS retention time (min): 0.77;
MS (ESI, Pos.): 367 $(M + H)^+$;
$^1$H-NMR ($CD_3OD$): δ 8.94 (s, 1H), 8.38 (s, 2H), 8.20 (d, J=8.5Hz, 1H), 6.66 (d, J=13.0Hz, 1H), 2.94 (q, J=7.0Hz, 2H), 1.09 (t, J=7.0Hz, 3H).

Example 4(8): 2-amino-5-(3-amino-7-(1*H*-pyrazol-4-yl)isoxazolo[4,5-*c*]pyridin-4-yl)-*N*-ethyl-4-fluorobenzamide hydrochloride

LCMS retention time (min): 0.70;
MS (ESI, Pos.): 382 $(M + H)^+$;
$^1$H-NMR ($CD_3OD$): δ 8.98 (s, 1H), 8.41 (s, 2H), 7.85 (d, J=8.0Hz, 1H), 6.65 (d, J=13.0Hz, 1H), 3.29 (q, J=7.0Hz, 2H), 1.11 (t, J=7.0Hz, 3H).

Example 4(9): 1-(2-amino-5-(3-amino-7-(1*H*-pyrazol-4-yl)isoxazolo[4,5-*c*]pyridin-4-yl)phenyl)ethan-1-one hydrochloride

LCMS retention time (min): 0.67;
MS (ESI, Pos.): 335 $(M + H)^+$;
$^1$H-NMR ($CD_3OD$): δ 8.86 (s, 1H), 8.37 (s, 2H), 8.29 (d, J=2.0Hz, 1H), 7.64 (dd, J=9.0, 2.0Hz, 1H), 6.95 (d, J=9.0Hz, 1H), 2.56 (s, 3H).

Example 4(10): methyl 2-amino-5-(3-amino-7-(1*H*-pyrazol-4-yl)isoxazolo[4,5-*c*]pyridin-4-yl)benzoate hydrochloride

LCMS retention time (Min): 0.71;
MS (ESI, Pos.): 351 $(M + H)^+$;
$^1$H-NMR (DMSO-$d_6$): δ 9.00 (s, 1H), 8.45 (s, 2H), 8.21 (s, 1H), 7.71 (d, J=9.0Hz, 1H), 7.01 (d, J=9.0Hz, 1H), 6.02 (br s, 2H), 3.84 (s, 3H).

Example 4(11): 2-amino-5-(3-amino-7-(1*H*-pyrazol-4-yl)isoxazolo[4,5-*c*]pyridin-4-yl)-*N*-propylbenzamide hydrochloride

LCMS retention time (min): 0.70;
MS (ESI, Pos.): 378 $(M + H)^+$;
$^1$H-NMR ($CD_3OD$): δ 8.86 (s, 1H), 8.38 (s, 2H), 7.95 (d, J=2.0Hz, 1H), 7.60 (dd, J=8.5, 2.0Hz, 1H), 6.94 (d, J=8.5Hz, 1H), 3.26-3.13 (m, 2H), 1.54 (q, J=7.0Hz, 2H), 0.90 (t, J=7.0Hz, 3H).

Example 4(12): 1-(2-amino-5-(3-amino-7-(1*H*-pyrazol-4-yl)isoxazolo[4,5-*c*]pyridin-4-yl)4-fluorophenyl)butan-1-one hydrochloride

LCMS retention time (min): 0.90;
MS (ESI, Pos.): 381 $(M + H)^+$;
$^1$H-NMR ($CD_3OD$): δ 8.94 (s, 1H), 8.37 (s, 2H), 8.20 (d, J=8.0Hz, 1H), 6.65 (d, J=13.0Hz, 1H), 2.88 (t, J=7.0Hz, 2H), 1.65 (q, J=7.5Hz, 2H), 0.90 (t, J=7.5Hz, 3H).

Example 4(13): 1-(2-amino-5-(3-amino-7-(1*H*-pyrazol-4-yl)isoxazolo[4,5-*c*]pyridin-4-yl)phenyl)butan-1-one hydrochloride

LCMS retention time (min): 0.87;
MS (ESI, Pos.): 363 $(M + H)^+$;
$^1$H-NMR ($CD_3OD$): δ 8.86 (s, 1H), 8.38 (s, 2H), 8.33 (d, J=2.0Hz, 1H), 7.63 (dd, J=9.0, 2.0Hz, 1H), 6.96 (d, J=9.0Hz, 1H), 2.96 (t, J=7.5 Hz, 2H), 1.70-1.64 (m, 2H), 0.92 (t, J=7.0Hz, 3H).

Example 4(14): 2-hydroxyethyl 2-amino-5-(3-amino-7-(1*H*-pyrazol-4-yl)isoxazolo[4,5-*c*]pyridin-4-yl)-4-fluorobenzoate hydrochloride

LCMS retention time (min): 0.76;
MS (ESI, Pos.): 399 $(M + H)^+$;
$^1$H-NMR ($CD_3OD$): δ 8.97 (s, 1H), 8.39 (s, 2H), 8.30 (d, J=8.5 Hz, 1H), 6.69 (d, J= 3.0 Hz, 1H), 4.26 (t, J=5.0 Hz, 2H), 3.74 (t, J=5.0 Hz, 2H).

Example 4(15): 2-amino-5-(3-amino-7-(1*H*-pyrazol-4-yl)isoxazolo[4,5-*c*]pyridin-4-yl)-*N*-methylbenzamide hydrochloride

LCMS retention time (min): 0.69;
MS (ESI, Pos.): 350 $(M + H)^+$;
$^1$H-NMR (DMSO-$d_6$): δ 9.04 (s, 1H), 8.56 (d, J=4.5 Hz, 1H), 8.52 (s, 2H), 8.18 (d, J=2.0Hz, 1H), 7.64 (dd, J=8.5, 2.0Hz, 1H), 6.94 (d, J=8.5Hz, 1H), 6.22 (s, 2H), 2.78 (d, J=4.5Hz, 3H).

Example 4(16): 4-(4-amino-2-chloro-5-(methylthio)phenyl)-7-(1*H*-pyrazol-4-yl)isoxazolo[4.5-*c*]pyridin-3-amine hydrochloride

LCMS retention time (min): 0.63;
MS (ESI, Pos.): 373 $(M + H)^+$;
$^1$H-NMR (DMSO-$d_6$): δ 9.08 (s, 1H), 8.43 (s, 2H), 7.40 (s, 1H), 6.93 (s, 1H), 2.37 (s, 3H).

Examples 4(17) to 4(24)

In place of 4-(4-amino-2-fluoro-5-(methylthio)phenyl)-7-(1-(tetrahydro-2*H*-pyran-2-yl)-1*H*-pyrazol-4-yl)isoxazolo[4,5-*c*]pyridin-3-amine, the compound corresponding thereto was prepared by a procedure similar to that described in Example 4, and purified by reverse phase HPLC (used column: YMC Triart C18 (30 mm × 75 mm); mobile phase: 0.1% TFA/water/acetonitrile = 95: 5 to 60: 40) to obtain the compounds having the following physical property values.

Example 4(17): 2-amino-5-(3-amino-7-(1*H*-pyrazol-4-yl)isoxazolo[4,5-*c*]pyridin-4-yl)-4-fluoro-N-methylbenzamide trifluoroacetate

LCMS retention time (min): 0.66;
MS (ESI, Pos.): 368 $(M + H)^+$;
$^1$H-NMR (DMSO-$d_6$): δ (rotamer mixture) 8.98 (s, 1H), 8.35 (s, 2H), 8.27-8.21 (m, 1H), 7.76 (d, J=8.5Hz, 1H), 6.61 (d, J=12.5Hz, 1H), 5.69 (br s, 2H), 2.71 (s, 1.5H), 2.69 (s, 1.5H).

Example 4(18): 4-(4-amino-5-(ethylthio)-2-fluorophenyl)-7-(1*H*-pyrazol-4-yl)isoxazolo[4,5-c]pyridin-3-amine trifluoroacetate

LCMS retention time (min): 0.64;
MS (ESI, Pos.): 371 $(M + H)^+$;

$^1$H-NMR ($CD_3OD$): δ 8.97 (s, 1H), 8.43 (s, 2H), 7.69 (d, J=8.0Hz, 1H), 6.73 (d, J=12.5Hz, 1H), 2.81 (q, J=7.0Hz, 2H), 1.25 (t, J=7.0Hz, 3H).

Example 4(19): 2-amino-5-(3-amino-7-(1*H*-pyrazol-4-yl)isoxazolo[4,5-*c*]pyridin-4-yl)-4-fluoro-N propylbenzamide trifluoroacetate

LCMS retention time (min): 0.84;
MS (ESI, Pos.): 396 $(M + H)^+$;
$^1$H-NMR ($CD_3OD$): δ 8.82 (s, 1H), 8.31 (s, 2H), 7.67 (d, J=8.0 Hz, 1H), 7.56 (d, J=12.5 Hz, 1H), 3.26-3.13 (m, 2H), 1.53-1.48 (m, 2H), 0.86 (t, J=7.0 Hz, 3H).

Example 4(20): 2-amino-5-(3-amino-7-(1*H*-pyrazol-4-yl)isoxazolo[4,5-*c*]pyridin-4-yl)benzamide trifluoroacetate

LCMS retention Time (min): 0.67;
MS (ESI, Pos.): 336 $(M + H)^+$;
$^1$H-NMR ($CD_3OD$): δ 8.79 (s, 1H), 8.29 (s, 2H), 7.95 (d, J=2.0Hz, 1H), 7.55 (dd, J=8.5, 2.0Hz, 1H), 6.88 (d, J=8.5 Hz, 1H).

Example 4(21): 2-amino-5-(3-amino-7-(1*H*-pyrazol-4-yl)isoxazolo[4,5-*c*]pyridin-4-yl)-*N*-ethylbenzamide trifluoroacetate

LCMS retention time (min): 0.55;
MS (ESI, Pos.): 364 $(M + H)^+$;
$^1$H-NMR (DMSO-$d_6$): δ 8.96 (s, 1H), 8.37 (s, 2H), 8.30 (t, J=6.0Hz, 1H), 7.96 (d, J=2.5Hz, 1H), 7.57 (dd, J=11.5, 2.5Hz, 1H), 6.88 (d, J=11.5Hz, 1H), 5.84 (brs, 2H), 3.25 (qd, J=9.0, 6.0Hz, 2H), 1.10 (t, J=9.0Hz, 3H).

Example 4(22): 1-(2-amino-5-(3-amino-7-(1*H*-pyrazol-4-yl)isoxazolo[4,5-*c*]pyridin-4-yl)phenyl)propan-1-one trifluoroacetate

HPLC retention time (min): 0.62;
MS (ESI, Pos.): 349 $(M + H)^+$;
$^1$H-NMR (DMSO-$d_6$): δ 8.97 (s, 1H), 8.37 (s, 2H), 8.33 (s, 1H), 8.25 (d, J=2.0Hz, 1H), 7.76 (dd, J=9.0, 2.0Hz, 1H), 6.96 (d, J=9.0Hz, 1H), 6.46 (br s, 2H), 5.94 (brs, 2H), 3.06 (q, J=7.0Hz, 2H), 1.10 (t, J=7.0Hz, 3H).

Example 4(23): 2-amino-5-(3-amino-7-(1*H*-pyrazol-4-yl)isoxazolo[4,5-*c*]pyridin-4-yl)-4-chloro-*N*-ethylbenzamide trifluoroacetate

LCMS retention time (min): 0.59;
MS (ESI, Pos.): 398 $(M + H)^+$;
$^1$H-NMR (DMSO-$d_6$): δ 9.00 (s, 1H), 8.38 (s, 2H), 8.32 (t, J=6.5Hz, 1H), 7.71 (s, 1H), 6.95 (s, 1H), 5.54 (brs, 2H), 3.23 (qd, J=10.0, 6.5Hz, 2H), 1.08 (t, J=10.0Hz, 3H).

Example 4(24): 4-(2-fluoro-5-methoxy-4-nitrophenyl)-7-(1*H*-pyrazol-4-yl)isoxazolo[4,5-c]pyridin-3-amine trifluoroacetate

LCMS retention time (min): 0.92;
MS (ESI, Pos.): 371 $(M + H)^+$.

Example 4 (25): 4-(4-amino-2-fluoro-5-(trifluoromethyl)phenyl)-7-(1*H*-pyrazol-4-yl)isoxazolo[4,5-*c*]pyridine-3-amine

In place of 4-(4-amino-2-fluoro-5-(methylthio)phenyl)-7-(1-(tetrahydro-2*H*-pyran-2-yl)-1*H*-pyrazol-4-yl)isoxazolo[4,5-*c*]pyridin-3-amine, the compound corresponding thereto was prepared by a procedure similar to that described in Example 4, and purified by reverse phase HPLC (column used: Xtimate C18 (25 mm × 150 mm); mobile phase: 0.225% formic acid/water/acetonitrile = 75: 25 to 45: 55) to obtain the titled compound having the following physical property value.
LCMS retention time (min): 0.90;
MS (ESI, Pos.): 379 $(M + H)^+$;
$^1$H-NMR (DMSO-$d_6$): δ 8.93 (s, 1H), 8.39 (s, 2H), 7.69 (d, J=7.5Hz, 1H), 6.78 (d, J=12.5Hz, 1H).

Example 4 (26): 1-(2-amino-5-(3-amino-7-(1*H*-pyrazol-4-yl)isoxazolo[4,5-*c*]pyridin-4-yl)-4-fluorophenyl)ethan-1-one hydrochloride

By a procedure similar to that described in Example 4, using the compound produced in Reference Example 6 (200 mg) and the compound produced in Reference Example 12 (3) (168 mg), the titled compound having the following physical property value was obtained. LCMS retention time (min): 0.56;
MS(ESI, Pos.): 353$(M+H)^+$;
$^1$H-NMR (DMSO-$d_6$): δ9.05(s, 1H), 8.42(s, 2H), 8.09(d, J=9.0Hz, 1H), 6.71(d, J=15.0Hz, 1H), 2.51(s,3H).

Example 5: 4-(4-amino-2-fluoro-5-(methylsulfinyl)phenyl)-7-(1*H*-pyrazol-4-yl)isoxazolo[4,5-c]pyridin-3-amine trifluoroacetate

The compound (17.2 mg) prepared in Example 4, sodium perborate tetrahydrate (6.16 mg), acetic acid (0.5 mL) and methanol (0.2 mL) were mixed, and the mixture thereof was stirred at 50 °C for 6 hours. The reaction solution was purified by reverse phase HPLC (used column: YMC Triart C18 (30 mm × 75 mm); mobile phase: 0.1% TFA/water/acetonitrile = 95: 5 to 60: 40) to obtain the titled compound (5.0 mg) having the following physical property value.
LCMS retention time (min): 0.50;
MS (ESI, Pos.): 373 $(M + H)^+$;
$^1$H-NMR (DMSO-$d_6$): δ 8.99 (s, 1H), 8.37 (s, 2H), 7.56 (d, J=8.0Hz, 1H), 6.66 (d, J=12.5Hz, 1H), 2.79 (s, 3H).

Example 6: 2-amino-5-(3-amino-7-(1*H*-pyrazol-4-yl)isoxazolo[4,5-*c*]pyridin-4-yl)-4-fluorobenzoic acid

THF (0.2 mL) and methanol (0.1 mL) were added to the compound (20 mg) produced in Example 1, and 2.0 mol/L sodium hydroxide aqueous solution (81 μL) was added in dropwise thereto at room temperature, and the mixture thereof was stirred for 3 hours. The reaction solution was neutralized and purified by reverse phase HPLC (used column: YMC Triart C18 (30 mm × 75 mm); mobile phase: 0.1% TFA/water/acetonitrile = 95: 5 to 60: 40) to obtain the titled compound (12.1 mg) having physical property value.
LCMS retention time (min): 0.53;
MS (ESI, Pos.): 355 $(M + H)^+$;
$^1$H-NMR (DMSO-$d_6$): δ 8.97 (s, 1H), 8.35 (s, 2H), 7.93 (d, J=8.5Hz, 1H), 7.23 (br s, 1H), 6.67 (d, J=12.5Hz, 1H), 5.72 (br s, 2H).

Example 7: 4-(4-amino-2-fluoro-5-methoxyphenyl)-7-(3-methyl-1*H*-pyrazol-4-yl)isoxazolo[4,5-*c*]pyridin-3-amine trifluoroacetate

In place of 1-(tetrahydro-2*H*-pyran-2-yl)-4-(4,4,5,5-tetramethyl-1,3,2-dioxaborolan-2-yl)-1*H*-pyrazole, (1-(*tert*-butoxycarbonyl)-3-methyl-1*H*-pyrazol-4-yl)boronic acid was subjected to the same operations as those in Reference Example 6 → Reference Example 13 → Example 2, to obtain the titled compound having the following physical property value. LCMS retention time (min): 0.56;
MS (ESI, Pos.): 355 $(M + H)^+$.

Example 8: 1-(2-amino-5-(3-amino-7-(1*H*-pyrazol-4-yl)isoxazolo[4,5-*c*]pyridin-4-yl)-4-hydroxyphenyl)ethan-1-one trifluoroacetate

To 1,3-dimethyl-2-imidazolidinone solution (3 mL) dissolving 1-(2-amino-5-(3-amino-7-(1-(tetrahydro-2H-pyran-2-yl)-1*H*-pyrazol-4-yl)isoxazolo[4,5-*c*]pyridin-4-yl)-4-fluorophenyl)ethan-1-one (150 mg) prepared in the processes described in Example 3, acetohydroxamic acid (258 mg) and potassium carbonate (618 mg) were added, and the mixture thereof was stirred at 80 °C for 5 hours. After cooling it to room temperature, tap water (15 mL) was added thereto, and the mixture thereof was extracted with ethyl acetate (20 mL), washed with saturated saline, and then concentrated. The residue therefrom was purified by silica gel column chromatography (Hi-flash NH) (ethyl acetate: methanol = 100: 0 to 50: 50), to obtain 1-(2-amino-5-(3-amino-7-(1-(tetrahydro-2*H*-pyran-2-yl)-1*H*-pyrazol-4-yl)isoxazolo[4,5-*c*]pyridin-4-yl)-4-hydroxyphenyl)ethan-1-one (50 mg).

To this compound (50 mg), methanol (2.0 mL) and methanesulfonic acid (34 mg) were added, and the mixture thereof was stirred at room temperature for 64 hours. The precipitate generated by the reaction was collected by filtration, dissolved in dimethyl sulfoxide and purified by reverse phase HPLC (used column: YMC Triart C18 (30 mm × 75 mm); mobile phase: 0.1% TFA/water/acetonitrile = 95: 5 to 60: 40) to obtain the titled compound (23.1 mg) having the following physical property value.
LCMS retention time (min): 0.55;

MS (ESI, Pos.): 351 $(M + H)^+$.

Reference Example 14: 5-bromo-2-chloro-3-fluoroisonicotinonitrile

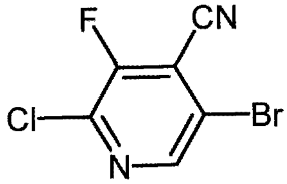

In place of 2-chloro-4-fluoro-5-iodopyridine, 5-bromo-2-chloro-3-fluoropyridine (CAS No. 831203-13-5) was subjected to the similar operations as those in Reference Example 1 → Reference Example 2, to obtain the titled compound having the following physical property value.
$^1$H-NMR (DMSO-$d_6$): δ 8.81 (s, 1H).

Reference Example 15: 5-(4-amino-2-fluoro-5-methoxyphenyl)-2-chloro-3-fluoroisonicotinonitrile

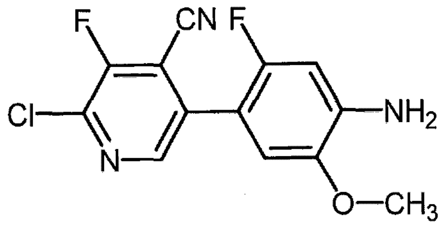

Under argon atmosphere, 5-fluoro-2-methoxy-4-(4,4,5,5-tetramethyl-1,3,2-dioxaborolan-2-yl)aniline (CAS No. 1326283-60-6)(70.0 mg), [1,1'-bis(diphenylphosphino)ferrocene]palladium(II) dichloride (19.0 mg) and 2mol/L tripotassium phosphate aqueous solution (0.40 mL) were added to 1,4-dioxane solution (2.0 mL) dissolving the compound (61.0 mg) prepared in Reference Example 14, and the mixture thereof was stirred at 90 °C for 3 hours. After allowing it to cool, to the reaction solution, water was added, and the mixture thereof was extracted with ethyl acetate. The organic layer was washed with saturated brine, dried over sodium sulfate, and concentrated. The residue therefrom was purified by silica gel column chromatography (Hi-flash SI) (hexane: ethyl acetate = 100: 0 to 30: 70) to obtain the titled compound (45.0 mg) having the following physical property value. MS (ESI, Pos.): 296 $(M + H)^+$;
$^1$H-NMR ($CDCl_3$): δ8.41 (s, 1H), 6.76 (d, J=6.5Hz, 1H), 6.55 (d, J=11.5Hz, 1H), 4.25 (s, 2H), 3.88 (s, 3H).

Reference Example 16: 5-(4-amino-2-fluoro-5-methoxyphenyl)-3-fluoro-2-(1-(tetrahydro-2*H*-pyran-2-yl)-1*H*-pyrazol-4-yl)isonicotinonitrile

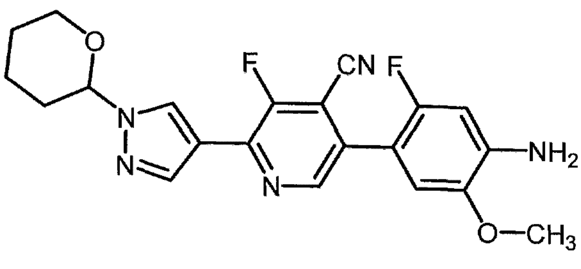

Under argon atmosphere, 4-(5,5-dimethyl-1,3,2-dioxaborinan-2-yl)-1-(tetrahydro-2*H*-pyran-2-yl)-1*H*-pyrazole (CAS No. 1072944-26-3)(40.0 mg), [1,1'-bis(diphenylphosphino)ferrocene]palladium(II) dichloride (11.0 mg) and 2 mol/L

tripotassium phosphate aqueous solution (0.20 mL) were added to 1,4-dioxane solution (2.0 mL) dissolving the compound (45.0 mg) produced in Reference Example 15, and the mixture thereof was stirred at 110 °C for 6 hours. After allowing it to cool, to the reaction solution, water was added, and the mixture thereof was extracted with ethyl acetate. The organic layer was washed with saturated brine, dried over sodium sulfate, and concentrated. The residue therefrom was purified by silica gel column chromatography (Hi-flash SI) (hexane: ethyl acetate = 100: 0 to 15: 85) to obtain the titled compound (30.0 mg) having the following physical property value.
MS (ESI, Pos.): 412 $(M + H)^+$;
$^1$H-NMR ($CDCl_3$): δ 8.57 (dd, J=1.5, 0.5Hz, 1H), 8.30 (d, J=2.0Hz, 1H), 8.23 (d, J=1.0 Hz, 1H), 6.81 (d, J=6.5Hz, 1H), 6.56 (d, J=11.0Hz, 1H), 5.49-5.44 (m, 1H), 4.19 (s , 2H), 4.13-4.07 (m, 1H), 3.89 (s, 3H), 3.79-3.71 (m, 1H), 2.17-2.05 (m, 3H), 1.80-1.62 (m, 3H).

Reference Example 17: 4-(4-amino-2-fluoro-5-methoxyphenyl)-7-(1-(tetrahydro-2*H*-pyran-2-yl)-1*H*-pyrazol-4-yl)isoxazolo[5,4-*c*]pyridin-3-amine

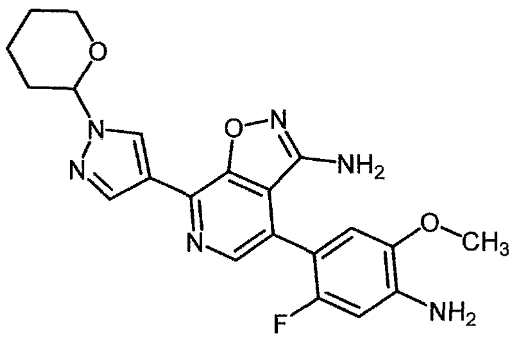

Under nitrogen atmosphere, potassium *tert*-butoxide (89.0 mg) was added to DMF solution (1.0 mL) dissolving acetohydroxamic acid (59 mg) at room temperature, and the mixture thereof was stirred for 30 minutes. To this mixed solution, DMF solution (2.0 mL) dissolving the compound (65 mg) produced in Reference Example 16 was added in dropwise, and the mixture thereof was stirred at room temperature for 16 hours. To the reaction solution, water was added, and the mixture thereof was extracted with ethyl acetate. The organic layer was washed with saturated brine, dried over sodium sulfate, and concentrated. The residue therefrom was purified by silica gel column chromatography (Hi-flash SI) (hexane: ethyl acetate = 100: 0 to 15: 85) to give the titled compound (22.0 mg) having the following physical property value.
MS (ESI, Pos.): 425 $(M + H)^+$;
$^1$H-NMR ($CDCl_3$): δ 8.56 (s, 1H), 8.42 (s, 1H), 8.31 (d, J=0.5Hz, 1H), 6.76 (d, J=6.5Hz, 1H), 6.59 (d, J=10.5Hz, 1H), 5.52-5.47 (m, 1H), 4.14-4.08 (m, 1H), 4.33 (s, 2H), 4.15 (s, 2H), 3.87 (s, 3H), 3.79-3.70 (m, 1H), 2.16-2.04 (m, 3H), 1.75-1.50 (m, 3H).

Example 9: 4-(4-amino-2-fluoro-5-methoxyphenyl)-7-(1*H*-pyrazol-4-yl)isoxazolo[5,4-*c*]pyridin-3-amine hydrochloride

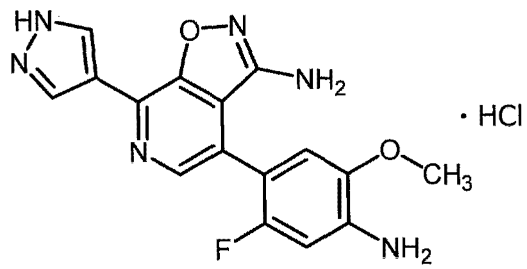

Hydrochloric acid (1.25 mol/L methanol solution) (0.64 mL) was added to THF solution (1.0 mL) dissolving the compound (20.0 mg) produced in Reference Example 17 at room temperature, and the mixture thereof was stirred for 3 hours. The reaction solution was concentrated to give the titled compound (5.8 mg) having the following physical

property value. LCMS retention time (min): 0.66;
MS (ESI, Pos.): 341 $(M + H)^+$;
$^1$H-NMR ($CD_3OD$): δ 8.45 (s, 2H), 8.24 (d, J=1.0) Hz, 1H), 6.92 (d, J=7.0 Hz, 1H), 6.70 (d, J=11.5Hz, 1H), 3.89 (s, 3H).

Reference Example 18: methyl 2-amino-5-(3-amino-7-(1-(((di-*tert*-butoxyphosphoryl)oxy)methyl)-1*H*-pyrazol-4-yl)isoxazolo[4,5-*c*]pyridin-4-yl)-4-fluorobenzoate

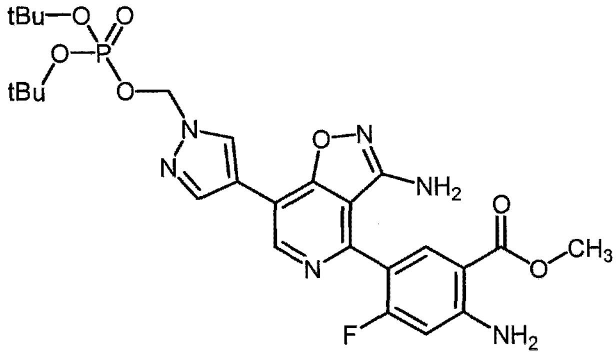

Cesium carbonate (6.61 g) and di-*tert*-butyl-chloromethyl phosphate (1.41 mL) were added to DMF solution (41 mL) dissolving the compound (1.49 g) prepared in Example 1, and the mixture thereof was heated at 50 °C for 5 hours. To the reaction solution, saturated aqueous sodium hydrogen carbonate solution was added, and the mixture thereof was extracted with ethyl acetate. The organic layer was washed with saturated brine, dried over sodium sulfate and concentrated. The residue therefrom was purified by silica gel column chromatography (Hi-flash SI) (hexane: ethyl acetate = 90: 10 to 0: 100) to give the titled compound (1.14 g) having the following physical property value.
LCMS retention time (min): 0.84;
MS (ESI, Pos.): 591 $(M + H)^+$.

Example 10: methyl 2-amino-5-(3-amino-7-(1-((phosphonooxy)methyl)-1*H*-pyrazol-4-yl)isoxazolo[4,5-*c*]pyridin-4-yl)-4-fluorobenzoate

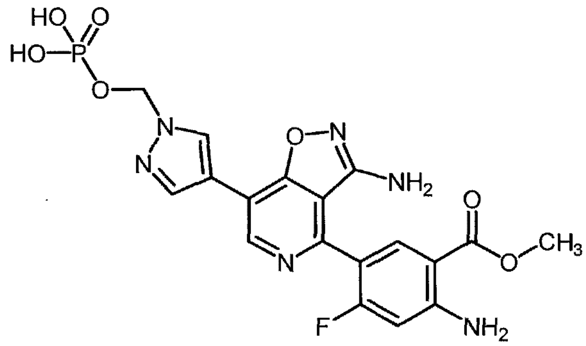

Purified water (6.8 mL) and acetic acid (13.5 mL) were added to the compound (1.09 g) produced in Reference Example 18, and the mixture thereof was stirred at 50 °C overnight. The precipitate deposited by the reaction was collected by filtration. The obtained filtrate was purified by reverse phase HPLC (used column: YMC Triart C18 (50 mm × 100 mm); mobile phase: 0.1% TFA/water/acetonitrile = 95: 5 to 50: 50) and concentrated to give the titled compound (536 mg) having the following physical property value.
LCMS retention time (min): 0.51;
MS (ESI, Pos.): 479 $(M + H)^+$;
$^1$H-NMR (DMSO-$d_6$): δ 8.99 (s, 1H), 8.63 (s, 1H), 8.35 (s, 1H), 7.94 (d, J=8.5Hz, 1H), 7.21 (brs, 2H), 6.71 (d, J=12.5Hz, 1H), 5.91 (d, J=10.0Hz, 2H), 5.75 (brs, 2H), 3.82 (s, 3H).

Example 10(1): (4-(4-(5-acetyl-4-amino-2-fluorophenyl)-3-aminoisoxazolo[4,5-*c*]pyridin-7-yl)-1*H*-pyrazol-1-yl)methyl dihydrogen phosphate (Compound A)

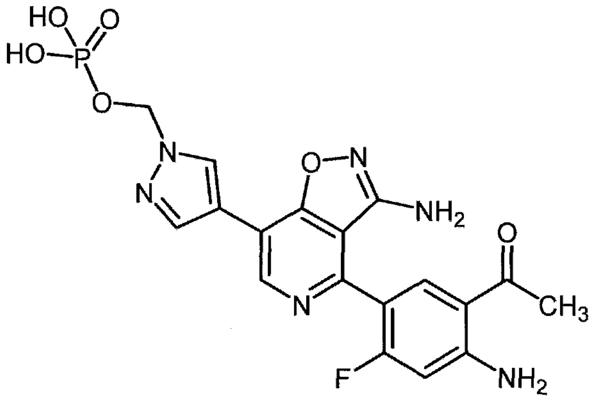

Cesium carbonate (91.9 mg) and di-tert-butyl-chloromethylphosphate (20 μL) were added to DMF solution (0.5 mL) dissolving the compound (24 mg) produced in Example 3, and the mixture thereof was stirred at room temperature for 8 hours. To the reaction solution, tap water was added, and the mixture thereof was extracted with ethyl acetate. The organic layer was washed with saturated saline, dried over sodium sulfate and concentrated. To the obtained residue, dichloromethane (0.30 mL) and trifluoroacetic acid (0.12 mL) were added, and the mixture thereof was stirred at 40 °C overnight. The reaction solution was diluted with DMSO and purified by reverse phase HPLC (used column: YMC Triart C18 (30 mm × 75 mm); mobile phase: 0.1% TFA/water/acetonitrile = 95: 5 to 60: 40) to give the titled compound (3.9 mg) having the following physical property value.
LCMS retention time (min): 0.50;
MS (ESI, Pos.): 463 $(M + H)^+$;
$^1$H-NMR (DMSO-$d_6$): δ 8.98 (s, 1H), 8.61 (s, 1H), 8.33 (s, 1H), 7.97 (d, J=8.5Hz, 1H), 7.70 (brs, 2H), 6.65 (d, J=13.0Hz, 1H), 5.89 (d, J=10.0Hz, 2H), 5.76 (brs, 2H), 2.51 (s, 3H).

Example 10(2): ethyl 2-amino-5-(3-amino-7-(1-((phosphonooxy)methyl)-1*H*-pyrazol-4-yl)isoxazolo[4,5-*c*]pyridin-4-yl)-4-fluorobenzoate

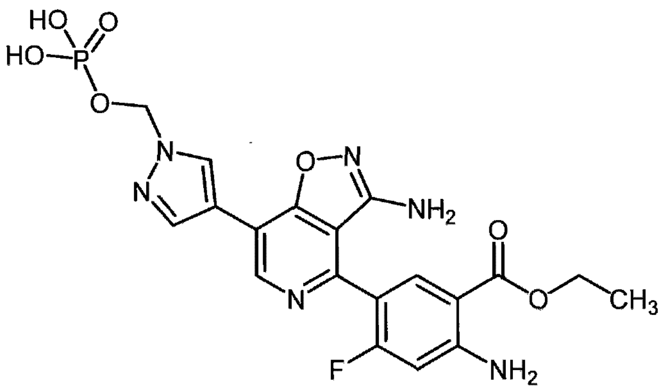

Cesium carbonate (128 mg) and di-*tert*-butyl-chloromethylphosphate (27 μL) were added to DMF solution (0.5 mL) dissolving the compound (36 mg) produced in Example 4(6), and the mixture thereof was stirred at room temperature overnight. To the reaction solution, tap water was added, and the mixture thereof was extracted with ethyl acetate. The organic layer was washed with saturated saline, dried over sodium sulfate and concentrated. To the obtained residue, dichloromethane (0.30 mL) and trifluoroacetic acid (0.18 mL) were added, and the mixture thereof was stirred at 40 °C for 3.5 hours. The reaction solution was diluted with DMSO and purified by reverse phase HPLC (used column: YMC Triart C18 (30 mm × 75 mm); mobile phase: 0.1% TFA/water/acetonitrile = 95: 5 to 60: 40) to give the titled compound

(15.0 mg) having the following physical property value.
LCMS retention time (min): 0.55;
MS (ESI, Pos.): 493 $(M + H)^+$;
$^1$H-NMR (DMSO-$d_6$): δ 8.97 (s, 1H), 8.60 (s, 1H), 8.31 (s, 1H), 7.93 (d, J=8.5Hz, 1H), 7.19 (br s, 2H), 6.67 (d, J=12.5Hz, 1H), 5.87 (d, J=10.0Hz, 2H), 5.73 (brs, 2H), 4.26 (q, J=7.0Hz, 2H), 1.27 (t, J=7.0Hz, 3H).

Examples 10(3) to 10(7)

In place of the compound prepared in Example 1, the compound corresponding thereto was subjected to the similar procedures as those of Reference Example 18 → Example 10, to obtain the compounds having the following physical property values.

Example 10(3): (4-(3-amino-4-(4-amino-5-(ethylcarbamoyl)-2-fluorophenyl)isoxazolo[4,5-*c*]pyridin-7-yl)-1*H*-pyrazol-1-yl)methyl dihydrogen phosphate

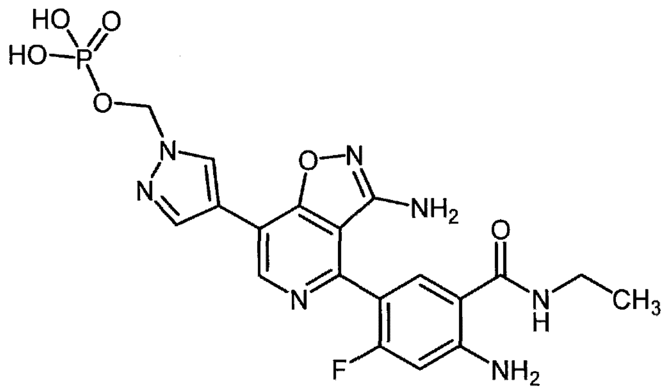

LCMS retention time (min): 0.50;
MS (ESI, Pos.): 492 $(M + H)^+$;
$^1$H-NMR (DMSO-$d_6$): δ 8.99 (s, 1H), 8.61 (s, 1H), 8.33 (s, 1H), 8.27 (t, J=5.5Hz, 1H), 7.78 (d, J=8.5Hz, 1H), 7.13 (brs, 2H), 6.60 (d, J=12.5Hz, 1H), 5.88 (d, J=10.0Hz, 2H), 5.65 (brs, 2H), 3.26-3.18 (m, 2H), 1.07 (t, J=7.0Hz, 3H).

Example 10(4): (4-(3-amino-4-(4-amino-2-fluoro-5-(methylthio)phenyl)isoxazolo[4,5-*c*]pyridin-7-yl)-1*H*-pyrazol-1-yl)methyl dihydrogen phosphate

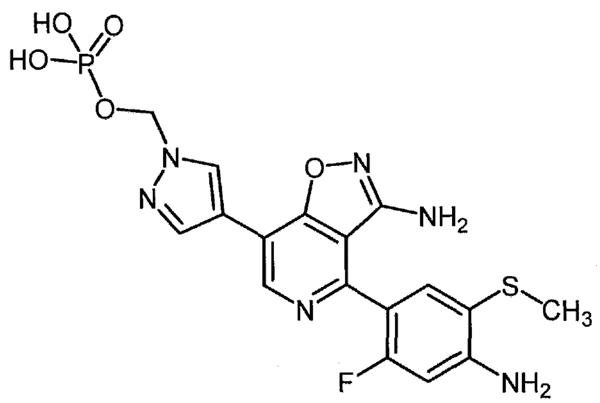

LCMS holding time (minutes): 0.52;
MS (ESI, Pos.): 467 $(M + H)^+$;
$^1$H-NMR (DMSO-$d_6$): δ 8.96 (s, 1H), 8.60 (s, 1H), 8.32 (s, 1H), 7.40 (d, J=8.5Hz, 1H), 6.62 (d, J=12.5Hz, 1H), 5.95 (brs,

2H), 5.88 (d, J=10.0Hz, 2H), 5.64 (s, 2H), 2.32 (s, 3H).

Example 10(5): (4-(3-amino-4-(4-amino-2-fluoro-5-propionylphenyl)isoxazolo[4,5-*c*]pyridin-7-yl)-1*H*-pyrazol-1-yl)methyl dihydrogen phosphate

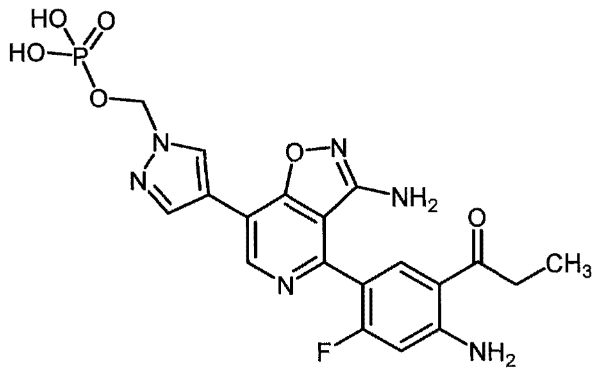

LCMS holding time (minutes):0.53;
MS(ESI, Pos.): 477(M+H)$^+$;
$^1$H-NMR(DMSO-$d_6$): δ 8.98 (s, 1H), 8.61(s, 1H), 8.33(s, 1H), 7.99 (d, J=8.5Hz, 1H), 7.70 (brs, 2H), 6.66 (d, J=12.0Hz, 1H), 5.88 (d, J=10.0Hz, 2H), 5.75 (brs, 2H), 2.96 (q, J=7.5Hz, 2H), 1.05 (t, J=7.5Hz, 3H).

Example 10(6): (4-(4-(3-acetyl-4-aminophenyl)-3-aminoisoxazolo[4,5-*c*]pyridin-7-yl)-1*H*-pyrazol-1-yl)methyl dihydrogen phosphate

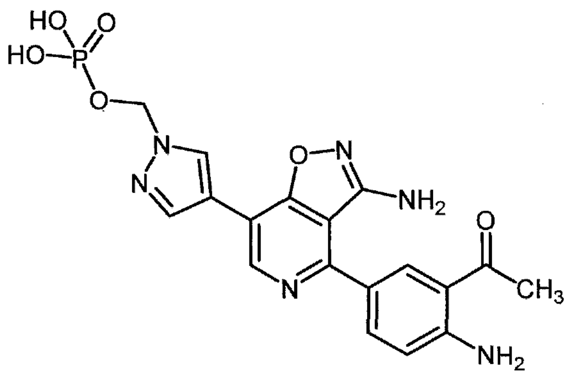

LCMS holding time (minutes): 0.48;
MS (ESI, Pos.): 445 (M + H)$^+$;
$^1$H-NMR (DMSO-$d_6$): δ 8.95 (s, 1H), 8.58 (s, 1H), 8.31 (s, 1H), 8.19 (d, J=2.0Hz, 1H), 7.77 (dd, J=8.5, 2.0Hz, 1H), 7.58 (brs, 2H), 6.92 (d, J=8.5Hz, 1H), 5.92-5.85 (m, 4H), 2.54 (s, 3H).

Example 10(7): (4-(3-amino-4-(4-amino-2-fluoro-5-(methylsulfonyl)phenyl)isoxazolo[4,5-*c*]pyridin-7-yl)-1*H*-pyrazol-1-yl)methyl dihydrogen phosphate

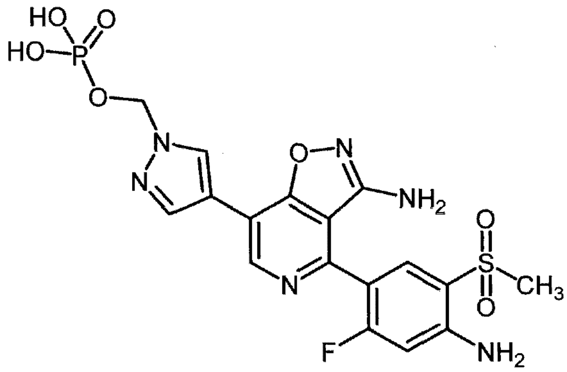

LCMS holding time (minutes): 0.668;
MS(ESI, Pos.): 499(M+H)$^+$;
$^1$H-NMR(DMSO-$d_6$): δ 9.00 (s, 1H), 8.63 (s, 1H), 8.35 (s, 1H), 7.74 (d, J=8.0Hz, 1H), 6.79 (d, J=12.0Hz, 1H), 6.64 (brs, 2H), 5.91 (d, J=12.0Hz, 2H), 5.83 (brs, 2H), 3.18 (s, 3H).

Example 10 8): acetate or acetic acid solvate of (4-(3-amino-4-(4-amino-5-(ethylcarbamoyl)-2-chlorophenyl)isoxazolo[4,5-*c*]pyridin-7-yl)-1*H*-pyrazol-1-yl)methyl dihydrogen phosphate

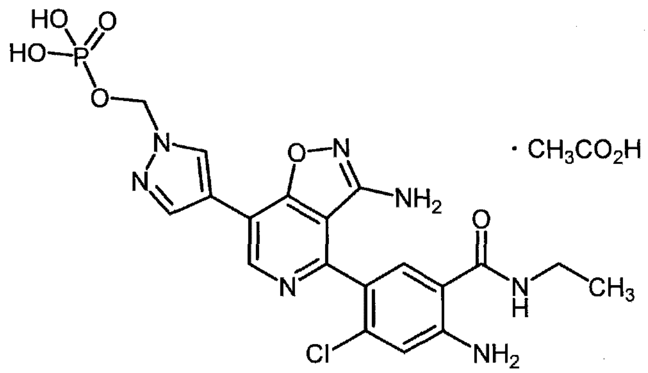

By subjecting the compound (421 mg) produced in Example 4 (23) to the same operation as in Reference Example 18, (4-(3-amino-4-(4-amino-2-chloro-5-(ethylcarbamoyl)phenyl)isoxazolo[4,5-*c*]pyridin-7-yl)-1*H*-pyrazol-1-yl)methyl di-*tert*-butyl phosphate (430 mg) was obtained. Acetic acid (19.3 mL) and purified water (3.4 mL) were added to this compound (379 mg), and the mixture thereof was stirred at 60 °C for 5 hours. The precipitate obtained therein was collected by filtration and dried to obtain the titled compound (304 mg) having the following physical property value and being in the form of acetate or acetic acid solvate.
LCMS retention time (min): 0.706;
MS (ESI, Pos.): 508 (M + H)$^+$;
$^1$H-NMR(DMSO-$d_6$): δ 9.01 (s, 1H), 8.64 (s, 1H), 8.36 (s, 1H), 8.33-8.29 (m, 1H), 7.70 (s, 1H), 7.01 (brs, 2H), 6.94 (s, 1H), 5.90 (d, J = 10.0Hz, 2H) , 5.53 (brs, 2H), 3.24-3.18 (m, 2H), 1.91 (s, 3H), 1.07 (t, J=7.0Hz, 3H).

Example 10(9): hydrate of (4-(4-(5-acetyl-4-amino-2-fluorophenyl)-3-aminoisoxazolo[4,5-*c*]pyridin-7-yl)-1*H*-pyrazol-1-yl)methyl dihydrogen phosphate

By subjecting the compound (100 mg) produced in Example 3 to the same operation as in Reference Example 18, (4-(4-(5-acetyl-4-amino-2-fluorophenyl)-3-aminoisoxazolo[4,5-*c*]pyridin-7-yl)-1*H*-pyrazol-1-yl)methyl di-*tert*-butyl phosphate (112 mg) was obtained. Acetic acid (0.20 mL) and purified water (0.05 mL) were added to this compound (25 mg), and the mixture thereof was stirred at 60 °C overnight. The precipitate obtained therein was collected by filtration and dried to obtain the titled compound (18.0 mg) of Example 10(1), being in the form of hydrate, having the following physical property value.
LCMS retention time (min): 0.50;
MS (ESI, Pos.): 463 (M + H)$^+$;

$^1$H-NMR (DMSO-$d_6$): δ 8.98 (s, 1H), 8.61 (s, 1H), 8.33 (s, 1H), 7.97 (d, J=8.5Hz, 1H), 7.70 (brs, 2H), 6.65 (d, J=13.0Hz, 1H), 5.89 (d, J=10.0Hz, 2H), 5.76 (brs, 2H), 2.51 (s, 3H).

Example 10(10): (4-(4-(5-acetyl-4-amino-2-fluorophenyl)-3-aminoisoxazolo[4,5-*c*]pyridin-7-yl)-1*H*-pyrazol-1-yl)methyl monohydrogenphosphate monopotassium salt

0.25M aqueous potassium acetate solution (0.43 mL, 1 equivalent) was added to acetic acid solution (1.25 mL) dissolving the compound (50 mg) prepared in Example 10(1), and the mixture thereof was stirred at room temperature for 8 hours. The obtained suspension was collected by filtration and dried under reduced pressure to obtain the titled compound (43.5 mg), being in crystalline form, having the following physical property value.
LCMS retention time (min): 0.49;
MS (ESI, Pos.): 463 $(M + H)^+$;
$^1$H-NMR (DMSO-$d_6$ + $CD_3OD$): δ 8.94 (s, 1H), 8.60 (s, 1H), 8.21 (s, 1H), 7.99 (d, J=8.5Hz, 1H), 7.70 (brs, 2H), 6.66 (d, J=13.0Hz, 1H), 5.69 (d, J=9.5Hz, 2H), 2.52 (s, 3H).

Example 10(11): trifluoroacetate or trifluoroacetic acid solvate of ethyl 2-amino-5-(3-amino-7-(1-((phosphonooxy)methyl)-1*H*-pyrazol-4-yl)isoxazolo[4,5-*c*]pyridin-4-yl)-4-fluorobenzoate

Cesium carbonate (128 mg) and di-*tert*-butyl-chloromethyl phosphate (27 μL) were added to DMF solution (0.5 mL) dissolving the compound prepared in Example 4(6) (2.00 g), and the mixture thereof was stirred at room temperature overnight. To the reaction solution, tap water was added, and the mixture thereof was extracted with ethyl acetate. The organic layer was washed with saturated saline, dried over sodium sulfate and concentrated. The residue therefrom was purified by silica gel column chromatography (Hi-flash SI) (hexane: ethyl acetate = 90: 10 to 0: 100) to obtain ethyl 2-amino-5-(3-amino-7-(1-(((di-*tert*-butoxyphosphoryl)oxy)methyl)-1*H*-pyrazol-4-yl)isoxazolo[4,5-*c*]pyridin-4-yl)-4-fluorobenzoate (2.12 g). Purified water (10 mL), ethanol (10 mL) and trifluoroacetic acid (5.3 mL) were sequentially added thereto, and the mixture thereof was stirred at 40 °C. After 2 hours, ethanol (5 mL) was added thereto, and the mixture thereof was cooled to room temperature. The precipitate obtained therein was collected by filtration with washing with ethanol and dried under reduced pressure to obtain the titled compound (1.81 g) having the following physical property value.
LCMS retention time (min): 0.55;
MS (ESI, Pos.): 493 $(M + H)^+$;
$^1$H-NMR (DMSO-$d_6$):δ 9.02 (s, 1H), 8.65 (s, 1H), 8.36 (s, 1H), 7.98 (d, J=8.5Hz, 1H), 7.32 (brs, 2H), 6.70 (d, J=12.5Hz, 1H), 5.91 (d, J=10.0Hz, 2H), 5.79 (brs, 2H), 4.28 (q, J=7.0Hz, 2H), 1.29 (t, J=7.0Hz, 3H).

Example 10(12): acetate or acetic acid solvate of ethyl 2-amino-5-(3-amino-7-(1-((phosphonooxy)methyl)-1*H*-pyrazol-4-yl)isoxazolo[4,5-*c*]pyridin-4-yl)-4-fluorobenzoate

Purified water (30 mL) and acetic acid (20 mL) were added to the compound (2.13 g) produced in Example 10(2), and the mixture thereof was stirred at 60 °C for 4 hours. The solvent was distilled off under reduced pressure and diluted with ethanol (30 mL). The reaction solution was stirred overnight and collected by filtration to give the titled compound (1.50 g) having the following physical property value.
LCMS retention time (min): 0.55;
MS (ESI, Pos.): 493 $(M + H)^+$;
$^1$H-NMR (DMSO-$d_6$): δ 8.99 (s, 1H), 8.62 (s, 1H), 8.34 (s, 1H), 7.96 (d, J=8.5Hz, 1H), 7.21 (brs, 2H), 6.69 (d, J=13.0Hz, 1H), 5.91 (d, J=10.0Hz, 2H), 5.74 (brs, 2H), 4.27 (q, J=7.0Hz, 2H), 1.92 (s, 3H), 1.29 (t, J=7.0Hz, 3H).

Example 10(13): 1-(4-(5-(5-acetyl-4-amino-2-fluorophenyl)-3-aminoisoxazolo[4,5-*c*]pyridin-7-yl)-1*H*-pyrazol-1-yl)ethyl dihydrogen phosphate

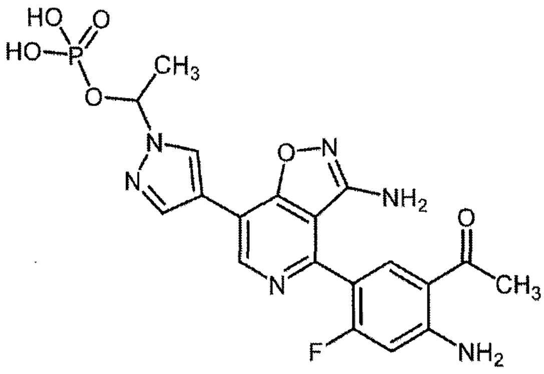

In place of di-*tert*-butyl-chloromethylphosphate, di-*tert*-butyl-chloroethyl phosphate (CAS No. 1567347-31-2) was subjected to the similar procedures as those of Reference Example 18 → Example 10, to obtain the titled compound having the following physical property value.
LCMS retention time (min): 0.51;
MS(ESI, Pos.): 477(M+H)$^+$;
$^1$H-NMR(DMSO-$d_6$):δ 8.96(s, 1H), 8.56(s, 1H), 8.27(s, 1H), 7.96(d, J=8.4Hz, 1H), 7.70(brs, 2H), 6.65(d, J=13Hz, 1H), 6.34-6.29(m, 1H), 5.75(brs, 2H), 2.51(s, 3H), 1.80(d, J=6Hz, 3H).

Reference Example 19: 4-chloro-7-iodoisothiazolo[4,5-*c*]pyridin-3-amine

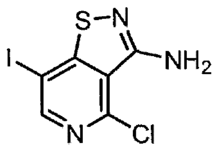

Under nitrogen atmosphere, dimethyl sulfoxide was added to sodium sulfide (138 mg), and the mixture thereof was stirred for 10 minutes, and then the compound (500 mg) produced in Reference Example 2 was added thereto, and the mixture thereof was stirred at room temperature for 30 minutes. After cooling it to 10 °C, aqueous ammonia was added thereto, and the mixture thereof was stirred for 30 minutes. *N*-chlorosuccinimide (248 mg) was added thereto, and the mixture thereof was stirred for 30 minutes, and further *N*-chlorosuccinimide (472 mg) was further added thereto, and the mixture thereof was stirred for 30 minutes. Saturated aqueous sodium thiosulfate solution (5 mL) and tap water (15 mL) were added thereto, and the resulting precipitate was collected by filtration. The precipitate was dried at 50 °C for 1.5 hours, dissolved in ethyl acetate and washed with tap water. It was dried over sodium sulfate and concentrated to give the titled compound (286 mg) having the following physical property value.
LCMS retention time (min): 0.88;
MS (ESI, Pos.): 312 (M + H)$^+$.

Reference Example 20: 4-bromo-7-iodoisothiazolo[4,5-*c*]pyridin-3-amine

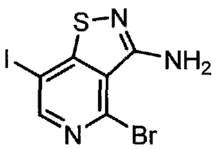

Under nitrogen atmosphere, propionitrile (2.4 mL) and bromotrimethylsilane (0.61 mL) were added to the compound (240 mg) produced in Reference Example 19, and the mixture thereof was stirred at 100 °C for 20 hours. After cooling it to 0 °C, methyl *tert*-butyl ether (7.2 mL) was added thereto, and the mixture thereof was stirred for 1.5 hours. The resulting precipitate was collected by filtration to give the titled compound (317 mg) having the following physical property value.

LCMS retention time (min): 0.91;
MS (ESI, Pos.): 356 $(M + H)^+$.

Reference Example 21: 4-bromo-7-(1-(tetrahydro-2*H*-pyran-2-yl)-1*H*-pyrazol-4-yl)isothiazolo[4,5-*c*]pyridin-3-amine

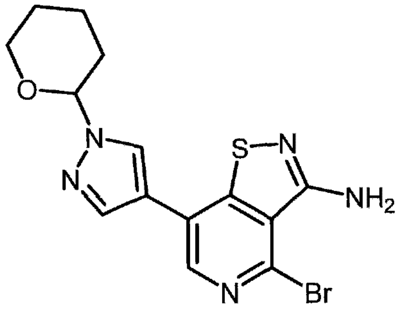

Under nitrogen atmosphere, to a mixture of the compound (384 mg) produced in Reference Example 20, 1-(tetrahydro-2*H*-pyran-2-yl)-4-(4,4,5,5-tetramethyl-1,3,2-dioxaborolan-2-yl)-1*H*-pyrazole (315 mg) and [1,1'-bis(diphenylphosphino)ferrocene]dichloropalladium dichloromethane complex (66 mg), 1,4-dioxane (4.6 mL) and 2 mol/L tripotassium phosphate aqueous solution (1.6 mL) were added, and the mixture thereof was stirred at 105 °C for 29 hours. After cooling it to room temperature, ethyl acetate and city water were added thereto, and the mixture thereof was filtered through Celite (trade name). The mixture was extracted with ethyl acetate and then concentrated. The residue therefrom was purified by silica gel column chromatography (Hi-flash DIOL) (ethyl acetate: hexane = 75: 25 to 50: 50) to give the titled compound (75.7 mg) having the following physical property value.
LCMS retention time (min): 0.86;
MS (ESI, Pos.): 380 $(M + H)^+$.

Example 11: 1-(2-amino-5-(3-amino-7-(1*H*-pyrazol-4-yl)isothiazolo[4,5-*c*]pyridin-4-yl)-4-fluorophenyl)ethan- 1 -one trifluoroacetate

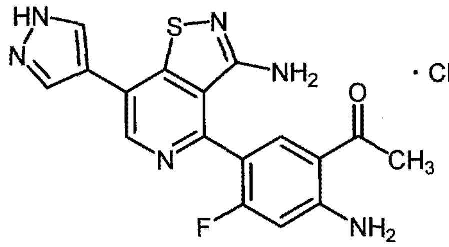

· $CF_3CO_2H$

Under nitrogen atmosphere, the compound (69 mg) produced in Reference Example 12(3), butyl di-1-adamantylphosphine (8.9 mg), palladium acetate (2.8 mg), potassium iodide (2.7 mg) and 2 mol/L tripotassium phosphate aqueous solution (0.17 mL) were added to NMP solution (1.25 mL) dissolving the compound (75 mg) produced in Reference Example 21, and the mixture thereof was stirred at 80 °C for 18 hours. After allowing the reaction solution to cool, it was directly purified by silica gel column chromatography (Hi-flash DIOL) (ethyl acetate: hexane = 80: 20 to 50: 50) to obtain 1-(2-amino-5-(3-amino-7-(1-(tetrahydro-2*H*-pyran-2-yl)-1*H*-pyrazol-4-yl)isothiazolo[4,5-*c*]pyridin-4-yl)-4-fluorophenyl)ethan-1-one (13 mg).

To this compound (13 mg), methanol (0.65 mL) and methanesulfonic acid (8.3 mg) were added, and the mixture thereof was stirred at room temperature for 3 hours. After allowed the reaction mixture to cool to room temperature, triethylamine (8.8 mg) was added thereto, and the mixture thereof was concentrated, and the residue therefrom was purified by reverse-phase HPLC (used column: YMC Triart C18 (30 mm × 75 mm); mobile phase: 0.1% TFA/water/acetonitrile = 95: 5 to 60: 40) to give the titled compound (3.0 mg) having the following physical property value.
LCMS retention time (min): 0.60;
MS (ESI, Pos.): 369 $(M + H)^+$;
$^1$H-NMR (DMSO-$d_6$): δ 8.83 (s, 1H), 8.32 (brs, 1H), 8.10 (brs, 1H), 7.94 (d, J=8.5Hz, 1H), 7.69 (brs, 2H), 6.67 (d, J=13.0Hz,

1H), 5.87 (s, 2H), 2.49 (s, 3H).

Reference Example 22: chloromethyl 1,4'-bipiperidine-1'-carboxylate

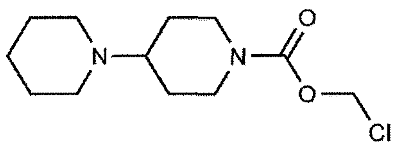

1,4'-Bipiperidine (CAS No. 4897-50-1) (300 mg) was dissolved in dichloromethane (8.0 mL), then which was cooled to 0 °C, and then N,N-diisopropylethylamine (0.40 mL) and chloromethyl chloroformate (CAS No. 22128-62-7) were added thereto, and the mixture thereof was stirred for 30 minutes. Saturated sodium bicarbonate solution was added to the reaction solution, then which was exracted by ethyl acetate. The organic layer thereof was washed with saturated brine, dried over sodium sulfate, and concentrated. The residue therefrom was purified by silica gel column chromatography (Hi-flash SI) (hexane: ethyl acetate = 90: 10 to 20: 80)to give the titled compound (452 mg) having the following physical property value.
LCMS retention time (min): 0.49;
MS(ESI, Pos.): 262, 264$(M+H)^+$.

Reference Example 22(1): chloromethyl (3-(((di-*tert*-butoxyphosphoryl)oxy)methyl)pyridin-2-yl)(methyl)carbamate

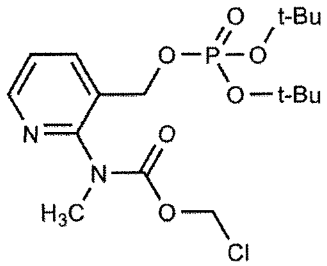

A solution of 2-(methylamino)pyridine-3-methanol (CAS No. 32399-12-5) (100 mg) in dichloromethane (3.6 mL) was cooled to 0°C, and *N*,*N*-diisopropylethylamine (0.16 mL) and chloromethyl chloroformate (63.7 μL) were added thereto. After being stirred at the same temperature for 30 minutes, di-tert-butyl *N*,*N*-diisopropylphosphoramidite (0.30 mL) (CAS No. 137348-86-8) and 1H-tetrazole (65.9 mg) (CAS No.27988-97-2) were added thereto, and the mixture thereof was stirred for another 30 minutes. The reaction was stopped with 10% aqueous sodium sulfite solution, saturated aqueous sodium hydrogen sulfate solution was added thereto, and the aqueous layer thereof was extracted with ethyl acetate. The organic layer thereof was washed with saturated brine, dried over sodium sulfate, and concentrated. The residue therefrom was purified by silica gel column chromatography (Hi-flash SI, amino type) (hexane: ethyl acetate = 90: 10 to 20: 80)to give the titled compound (165 mg) having the following physical property value.
LCMS retention time (min): 0.92;
MS(ESI, Pos.): 423, 425$(M+H)^+$.

Reference Example 22(2): (2-(methyl((2,2,2-trichloroethoxy)carbonyl)amino)pyridin-3-yl)methyl-*N*-(*tert*-butoxycarbonyl)-*N*-methylglycinate

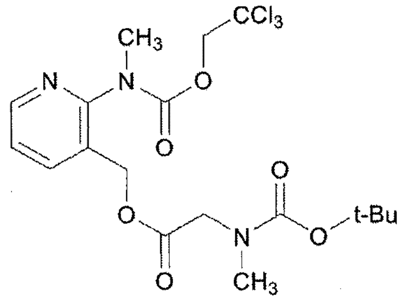

2-(methylamino)pyridine-3-methanol (CAS No. 32399-12-5) (300 mg) was dissolved in dichloromethane (6.0 mL), and *N*,*N*-diisopropylethylamine (0.46 mL) was added thereto, and the mixture thereof was cooled to 0°C. 2,2,2-trichloroethyl chloroformate (CAS No. 17341-93-4) (0.33 mL) was added in dropwise thereto, and the mixture thereof was stirred at the same temperature for 30 minutes. To this solution, 1-(3-dimethylaminopropyl)-3-ethylcarbodiimide hydrochloride (CAS No. 25952-53-8) (624 mg), 4-dimethylaminopyridine (CAS No. 1122-58-3) (80 mg) and *N*-α-(*tert*-butoxycarbonyl)sarcosine (CAS No. 13734-36-6) (493 mg) were added, and the mixture thereof was stirred at 0°C for 1 hour. To this solution, saturated ammonium chloride solution was added, and the solution thereof was divided, and the organic layer thereof was washed twice with saturated ammonium chloride solution, and concentrated. The residue therefrom was purified by silica gel column chromatography (Hi-flash SI) (hexane: ethyl acetate = 80: 20 to 60: 40)to give the titled compound (514 mg) having the following physical property value.
LCMS retention time (min): 1.03;
MS(ESI, Pos.): 484, 486$(M+H)^+$.

Reference Example 22(3): 2,2,2-trichloroethyl (3-(((di-*tert*-butoxyphosphoryl)oxy)methyl)pyridin-2-yl) (methyl) carbamate

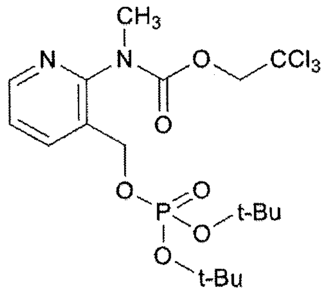

2-(methylamino)pyridine-3-methanol (CAS No. 32399-12-5) (200 mg) was dissolved in dichloromethane (8.0 mL), and *N*,*N*-diisopropylethylamine (0.33 mL) was added thereto, and the mixture thereof was cooled to 0°C. 2,2,2-trichloroethyl chloroformate (CAS No. 17341-93-4) (0.20 mL) was added in dropwise thereto, and the mixture thereof was stirred at the same temperature for 1 hour. To this solution, di-*tert*-butyl *N,N*-diisopropylphosphoramidite (0.59 mL) (CAS No. 137348-86-8) and 1*H*-tetrazole (132 mg). (CAS No. 27988-97-2) were added, and the mixture thereof was stirred for another 1 hour. To this reaction solution, 30% aqueous hydrogen peroxide (0.33 mL) was added under 0°C, the temperature thereof was raised to room temperature, and the mixture thereof was stirred for 1 hour. The reaction was stopped with 10% aqueous sodium sulfite solution, saturated aqueous sodium hydrogen sulfate solution was added thereto, and the aqueous layer thereof was extracted with ethyl acetate. The organic layer thereof was dried over sodium sulfate and concentrated. The residue therefrom was purified by silica gel column chromatography (Hi-flash SI, diol type) (hexane: ethyl acetate = 90: 10 to 70: 30)to give the titled compound (109 mg) having the following physical property value.
LCMS retention time (min): 1.07;
MS(ESI, Pos.): 507$(M+H)^+$.

Example 12: (4-(4-(5-acetyl-4-amino-2-fluorophenyl)-3-aminoisoxazolo[4,5-*c*]pyridin-7-yl)-1*H*-pyrazol-1-yl)methyl [1,4'-bipiperidine]-1'-carboxylate

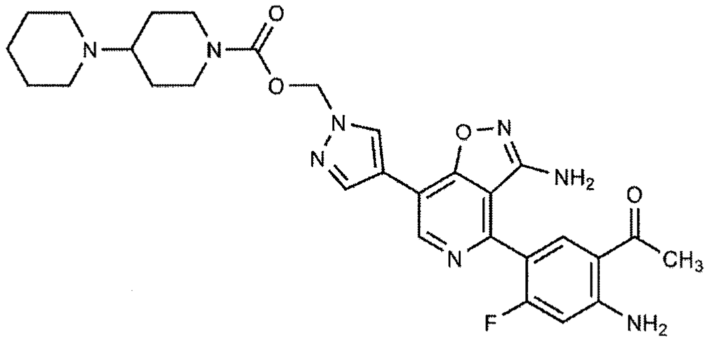

The compound produced in Example 3 (100 mg) and the compound produced in Reference Example 22 (111 mg) were dissolved in DMF (1.0 mL), and cesium carbonate (462 mg) and potassium iodide (94 mg) were added thereto, and the mixture thereof was stirred for 1 hour at room temperature. The reaction solution was filtered through Celite to remove insoluble material, and then concentrated under reduced pressure. The residue therefrom was purified by silica gel column chromatography (Hi-flash SI, amino type) (hexane: ethyl acetate = 90: 10 to 20: 80)to give the titled compound (74 mg) having the following physical property value.
LCMS retention time (min): 0.63;
MS(ESI, Pos.): 577(M+H)$^+$;
$^1$H-NMR(DMSO-$d_6$):δ8.99(s, 1H), 8.59(s, 1H), 8.34(s, 1H), 7.97(d, J=9.0Hz, 1H), 7.71(brs, 2H), 6.66(d, J=12.0Hz, 1H), 6.12(brs, 2H), 5.78(brs, 2H), 4.07-3.88(m, 2H), 2.89-2.69(m, 3H), 2.52(s, 3H), 2.41-2.30(m, 4H), 1.68-1.60(m, 2H), 1.51-1.21(m, 8H).

Example 12(1): (4-(5-(5-acetyl-4-amino-2-fluorophenyl)-3-aminoisoxazolo[4,5-*c*]pyridin-7-yl)-1*H*-pyrazol-1-yl)methyl methyl(3-((phosphonooxy)methyl)pyridin-2-yl)carbamate

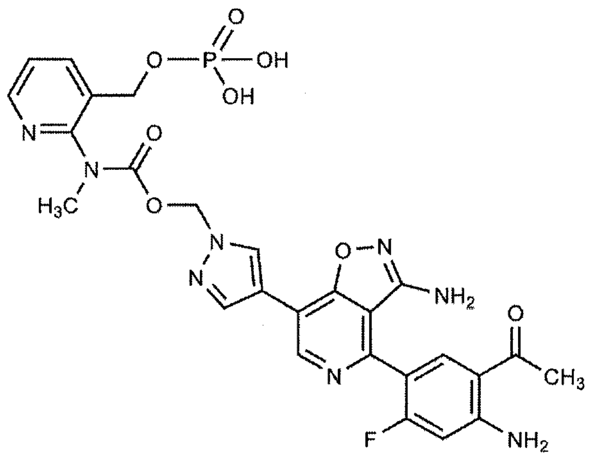

By the same procedures as those in Example 12→Example 10(8) using the compound produced in Example 3 (74.3 mg) and Reference Example 22(1) (134 mg), the titled compound (71 mg) having the following physical property value was obtained.
LCMS retention time (min): 0.57;
MS(ESI, Pos.): 627(M+H)$^+$;
$^1$H-NMR(DMSO-$d_6$):δ8.98(s, 1H), 8.58(brs, 1H), 8.45-8.41(m, 1H), 8.31(m, 1H), 7.99-7.90(m, 2H), 7.70(brs, 2H), 7.47-7.42(m, 1H), 6.66(d, J= 12Hz, 1H), 6.10(brs, 2H), 5.78(brs, 2H), 4.90-4.62(m, 2H), 3.20(s, 3H), 2.52(s, 3H).

Reference Example 23: (2-((1-(4-(4-(5-acetyl-4-amino-2-fluorophenyl)-3-aminoisoxazolo[4,5-*c*]pyridin-7-yl)-1*H*-pyrazol-1-yl)ethoxy)carbonyl)(methyl)amino)pyridin-3-yl)methyl *N*-(tert-butoxycarbonyl)-*N*-methylglycinate

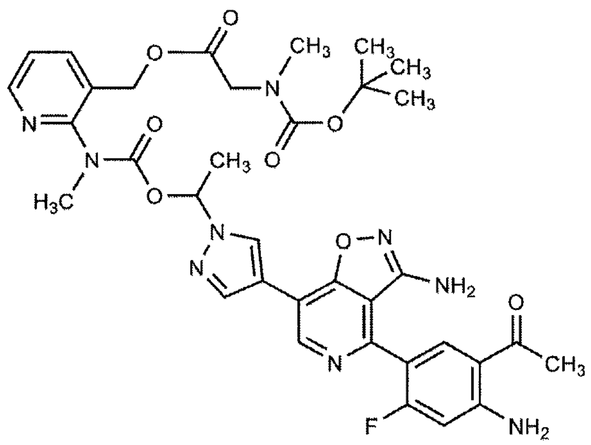

The compound produced in Example 3 (100 mg) and (2-((1-chloroethoxy)carbonyl)(methyl)amino)-3-pyridinyl)methyl (methyl(((2-methyl-2-propanyl)oxy)carbonyl)amino)acetate (Cas No. 338990-31-1) (177 mg) were subjected to the same procedure as that in Example 12 to obtain the titled compound (53.7 mg) having the following physical property value.
LCMS retention time (min): 0.87;
MS(ESI, Pos.): 733(M+H)+.

Example 12(2): (2-(((1-(4-(5-(5-acetyl-4-amino-2-fluorophenyl)-3-aminoisoxazolo[4,5-*c*]pyridin-7-yl)-1*H*-pyrazol-1-yl)ethoxy)carbonyl)(methyl)amino)pyridin-3-yl)methyl methylglycinate

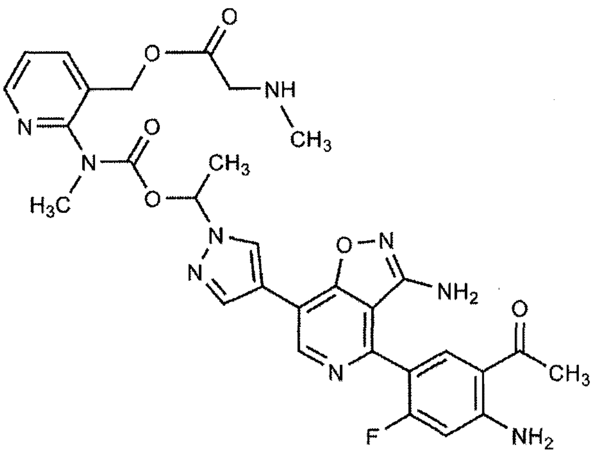

To a solution of the compound produced in Reference Example 23 (48.9 mg) in dichloromethane (1.0 mL), hydrochloric acid (4.0 M, 1,4-dioxane solution) was added, and the mixture thereof was stirred at room temperature for 10 minutes. The reaction solution was filtered and the filtrate therefrom was neutralized with saturated sodium bicarbonate solution. The aqueous layer thereof was extracted with ethyl acetate, and the organic layer thereof was washed with saturated brine and dried over sodium sulfate to obtain the titled compound (26 mg) having the following physical property value.
LCMS retention time (min): 0.63;
MS(ESI, Pos.): 632(M+H)+.

Example 12(3): 2-((2-((((4-(4-(5-acetyl-4-amino-2-fluorophenyl)-3-aminoisoxazolo[4,5-*c*]pyridin-7-yl)-1*H*-pyrazol-1yl)methoxy)carbonyl)(methyl)amino)pyridin-3-yl)methoxy)-*N*-methyl-2-oxoethane-1-ammonium chloride

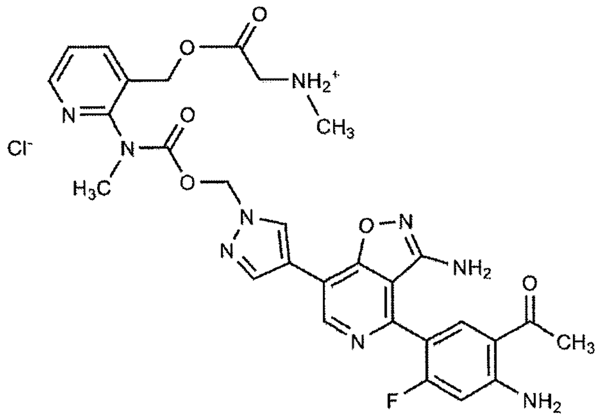

The compound produced in Example 3 (125 mg) and (2-(chloromethoxycarbonyl(methyl)amino)-3-pyridyl)methyl 2-(tert-butoxycarbonyl(methyl)amino)acetate (185 mg) were subjected to the same procedure as those in Reference Example 23→Example 12(2), and the precipitate obtained after the reaction was filtered off and dried to obtain the titled compound (22 mg) having the following physical property value.
LCMS retention time (min): 0.61;
MS(ESI, Pos.): 618(M+H)$^+$;
$^1$H-NMR(DMSO-$d_6$):δ9.19-9.09(m, 3H), 8.70(brs, 1H), 8.56(dd, J=4.7, 1.8Hz, 1H), 8.45(brs, 1H), 8.07(m, 2H), 7.81(brs, 2H), 7.53(dd, J=7.7, 4.7Hz, 1H), 6.75(d, J=12.8Hz, 1H), 6.40-5.83(m, 4H), 5.26-5.03(m, 2H), 4.09(t, J=5.69Hz, 2H), 3.30(brs, 3H), 2.62(m, 3H), 2.58(s, 3H).

Example 12(4): (4-(4-(5-acetyl-4-amino-2-fluorophenyl)-3-aminoisoxazolo[4,5-*c*]pyridin-7-yl)-1*H*-pyrazol-1-yl)methyl (2-morpholinoethyl)carbonate hydrochloride

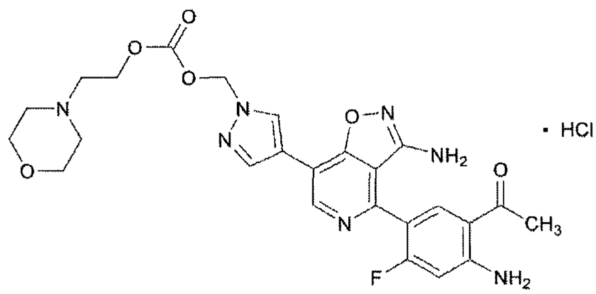

2-morpholinoethanol (CAS No. 622-40-2) (167 mg) was dissolved in dichloromethane (3.34 mL), pyridine (123 μL) was added thereto, and the mixture thereof was cooled to 0°C. Chloromethyl chloroformate (CAS No. 22128-62-7) (121 μL) was added in dropwise thereto, and the mixture thereof was stirred at room temperature for 30 minutes. To this solution, saturated sodium bicarbonate solution was added, and the mixture thereof was extracted with dichloromethane. The organic layer thereof was washed with water, dried over sodium sulfate, and concentrated. The residue therefrom was dissolved in DMF (4.0 mL) and the compound produced in Example 3 (200 mg), cesium carbonate (370 mg) and potassium iodide (19 mg) were added thereto, and the mixture thereof was stirred at room temperature for 18 hours. The reaction solution was filtered to remove insoluble material. Water was added to this filtrate, which was extracted with ethyl acetate, and concentrated. The residue therefrom was purified by reversed-phase HPLC (column used: YMC Triart C18 (50 mm × 100 mm); mobile phase: 0.1% TFA/water/acetonitrile = 95: 5 to 50: 50), and concentrated. The crude obtained therefrom was dissolved in THF (4 mL), 4 mol/L hydrogen chloride dioxane solution (284 μL) was added thereto, and the resulting solid was filtered off and dried to obtain the titled compound (307 mg) having the following physical property value.
LCMS retention time (min): 0.59;
MS(ESI, Pos.): 540(M+H)$^+$;
$^1$H-NMR(DMSO-$d_6$):δ10.47(brs, 1H), 9.03(s, 1H), 8.72(s, 1H), 8.42(s, 1H), 8.00(d, J=8.3Hz, 1H), 7.76(brs, 2H), 6.68(d, J=12.8Hz, 1H), 6.27(s, 2H), 5.84(brs, 2H), 4.57-4.52(m, 2H), 4.00-3.92(m, 2H), 3.76-3.66(m, 2H), 3.20-3.06(m, 2H),

2.52(s, 3H).

Reference Example 24: (4-(4-(5-acetyl-4-amino-2-fluorophenyl)-3-aminoisoxazolo(4,5-*c*]pyridin-7-yl)-1*H*-pyrazol-1-yl)methyl(((2R,3R,4S,5R)-3,4,5,6-tetrakis((trimethylsilyl)oxy)tetrahydro-2*H*-pyran-2-yl)methyl) carbonate

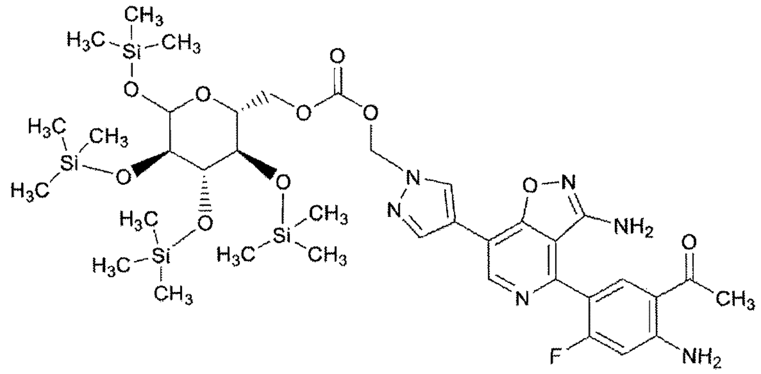

The compound produced in Example 3 (700 mg) and chloromethyl(3,4,5,6-tetrakis(trimethylsilyloxy)tetrahydropyran-2-yl)methylcarbonate (1.45 g) were dissolved in DMF (14 mL). To this solution, cesium carbonate (842 mg) and potassium iodide (165 mg) were added, and the mixture thereof was stirred at room temperature for 19 hours. Water was added to the reaction solution, which was extracted with ethyl acetate. The organic layer thereof was washed with water, dried over sodium sulfate, and concentrated. The residue therefrom was purified by silica gel column chromatography (Hi-flash SI) (hexane: ethyl acetate = 70: 30 to 40: 60) to obtain the titled compound (792 mg) with the following physical property value.
LCMS retention time (min): 1.32;
MS(ESI, Pos.): 877(M+H)$^+$.

Example 12(5): (4-(4-(5-acetyl-4-amino-2-fluorophenyl)-3-aminoisoxazolo[4,5-*c*]pyridin-7-yl)-1*H*-pyrazol-1-yl)methyl (((2R,3S,4S,5R)-3,4,5,6-tetrahydroxytetrahydro-2*H*-pyran-2-yl)methyl) carbonate

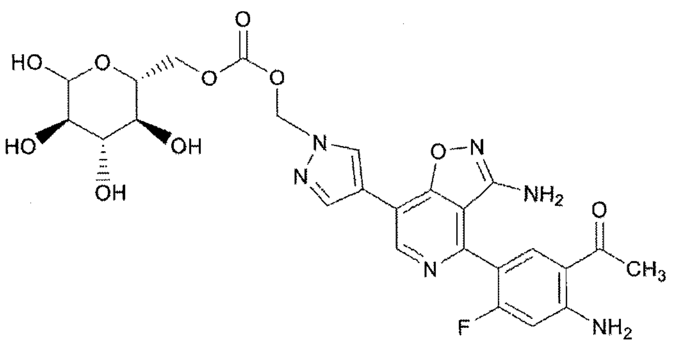

The compound produced in Reference Example 24 (132 mg) was dissolved in THF (1.32 mL). To this solution, 1 mol/L hydrochloric acid (132 μL) was added, and then THF (0.66 mL) was added thereto, and the mixture thereof was stirred at room temperature for 30 minutes. The resulting precipitate was filtered off and dried to obtain the titled compound (88 mg) with the following physical property value.
LCMS retention time (min): 0.56;
MS(ESI, Pos.): 589(M+H)$^+$;
$^1$H-NMR(DMSO-$d_6$):δ9.03(s, 1H), 8.71(s, 1H), 8.41(s, 1H), 8.02(d, J=8.3Hz, 1H), 7.77(brs, 2H), 6.68(d, J=12.8Hz, 1H), 6.22(brs, 2H), 5.85(brs, 2H), 4.89(d, J=3.7Hz, 1H), 4.44-4.28(m, 2H), 4.23-4.13(m, 2H), 3.83-3.76(m, 1H), 2.52(s, 3H).

Example 12(6): (4-(4-(5-acetyl-4-amino-2-fluorophenyl)-3-aminoisoxazolo[4,5-*c*]pyridin-7-yl)-1*H*-pyrazol-1-yl)methyl (((2R,3S,4R,5R)-5-amino-3,4,6-trihydroxytetrahydro-2*H*-pyran-2-yl)methyl) carbonate hydrochloride

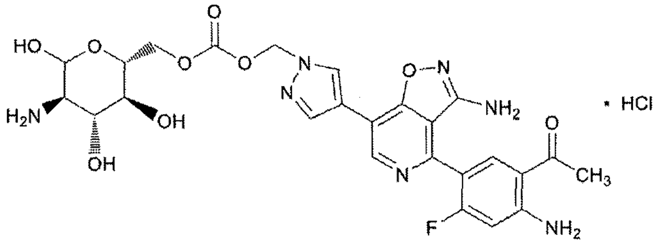

In place of chloromethyl(3,4,5,6-tetrakis(trimethylsilyloxy)tetrahydropyran-2-yl)methylcarbonate, ((2R,3R,4R,5R)-5-(tert-butoxycarbonylamino)-3,4,6-tris(trimethylsilyloxy)tetrahydropyran-2-yl)methyl chloromethylcarbonate was subjected to the same procedure as those in Reference Example 24→Example 12(5) to obtain the titled compound having the following physical property value.
LCMS retention time (min): 0.56;
MS(ESI, Pos.): 588(M+H)$^+$;
$^1$H-NMR(DMSO-$d_6$):δ9.03(s, 1H), 8.71(s, 1H), 8.42(s, 1H), 8.00(d, J=8.2Hz, 1H), 7.73(brs, 2H), 6.69(d, J=12.7Hz, 1H), 6.23(brs, 2H), 5.85(brs, 2H), 5.18(m, 1H), 4.46-4.38(m, 1H), 4.28-4.19(m, 1H), 3.88-3.81(m, 1H), 3.22-3.14(m, 1H), 2.94-2.86(m, 1H), 2.53(s, 3H).

Reference Example 25: (2-(4-(4-(5-acetyl-4-amino-2-fluorophenyl)-3-aminoisoxazolo[4,5-*c*]pyridin-7-yl)-*N*-methyl-1*H*-pyrazol-1-carboxamide)pyridin-3-yl)methyl *N*-(*tert*-butoxycarbonyl)-*N*-methylglycinate

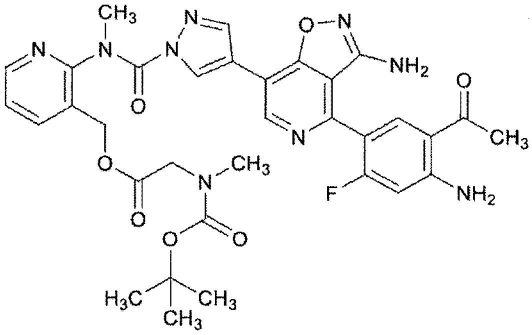

The compound produced in Example 3 (200 mg) and the compound produced in Reference Example 22(3) (434 mg) were dissolved in DMF (4.0 mL), and diazabicycloundecene (259 mg) was added thereto. The reaction solution was raised to 50 °C and stirred for 4 hours, then lowered to room temperature and stirred for another 14.5 hours. This solution was purified by reversed-phase HPLC (column used: YMC Triart C18 (50 mm × 100 mm); mobile phase: 0.1% TFA/water/acetonitrile = 95: 5 to 50: 50) to obtain the titled compound (177 mg) with the following physical property value.
LCMS retention time (min): 0.90;
MS(ESI, Pos.): 688(M+H)$^+$.

Example 12(7): (2-(4-(4-(5-acetyl-4-amino-2-fluorophenyl)-3-aminoisoxazolo[4,5-*c*]pyridin-7-yl)-*N*-methyl-1*H*-pyrazole-1-carboxamido)pyridin-3-yl)methyl methyl glycinate

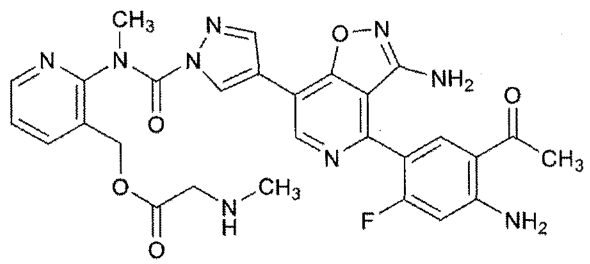

The compound produced in Reference Example 24 (177 mg) was dissolved in dichloromethane (7.0 mL), and 4 mol/L hydrogen chloride dioxane solution (64 μL) was added thereto, and the mixture thereof was stirred at room temperature for 2 hours. To this solution, heptane (1.7 mL) was added in dropwise, and the resulting solid was filtered off and dried. The resulting solid was dissolved in purified water (16 mL) and neutralized by adding saturated sodium bicarbonate solution. It was extracted with ethyl acetate and the organic layer thereof was concentrated. The residue therefrom was dissolved in THF (5 mL) and heptane (10 mL) was added in dropwise thereto. The resulting solid was filtered off and dried to obtain the titled compound (307 mg) with the following physical property value.
LCMS retention time (min): 0.62;
MS(ESI, Pos.): 588(M+H)$^+$;
$^1$H-NMR(DMSO-$d_6$):δ8.99(s, 1H), 8.91(s, 1H), 8.37(m, 1H), 8.17(brs, 1H), 8.00-7.95(m, 2H), 7.72(brs, 2H), 7.44(dd, J=7.6, 4.7Hz, 1H), 6.66(d, J=12.8Hz, 1H), 5.80(brs, 2H), 5.21(brs, 2H), 3.40(brs, 3H), 2.53(s, 3H), 2.25(s, 3H).

Reference Example 26: (2-(4-(4-(5-acetyl-4-amino-2-fluorophenyl)-3-aminoisoxazolo[4,5-*c*]pyridin-7-yl)-*N*-methyl-1*H*-pyrazole-1-carboxamide)pyridin-3-yl(methyl di-*tert*-butyl phosphate

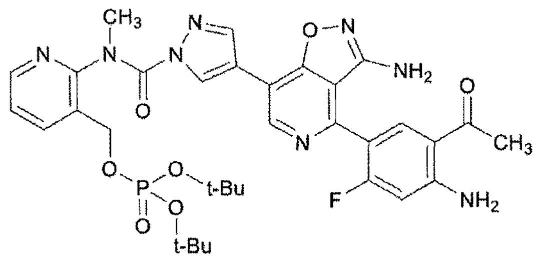

In place of Reference Example 22(2), the compound produced in Reference Example 22(3) was subjected to the same procedure as that in Reference Example 25 to obtain the titled compound having the following physical property value.
LCMS retention time (min): 0.89;
MS(ESI, Pos.): 709(M+H)$^+$.

Example 12(8): (2-(4-(4-(5-acetyl-4-amino-2-fluorophenyl)-3-aminoisoxazolo[4,5-*c*]pyridin-7-yl)-*N*-methyl-1*H*-pyrazole-1-carboxamide)pyridin-3-yl)methyl dihydrogen phosphate

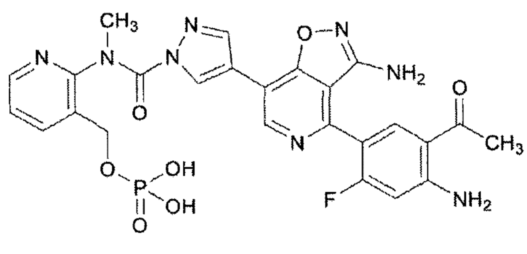

The compound produced in Reference Example 26 was subjected to the same procedure as that in Example

10(1) to obtain the titled compound having the following physical property value.
LCMS retention time (min): 0.57;
MS(ESI, Pos.): 597(M+H)+;
$^1$H-NMR(DMSO-$d_6$):δ8.99(s, 1H), 8.91(s, 1H), 8.32(m, 1H), 8.17(brs, 1H), 8.02-7.96(m, 2H), 7.73(brs, 2H), 7.46(dd, J=7.6, 4.8Hz, 1H), 6.66(d, J=12.8Hz, 1H), 5.80(brs, 2H), 5.01(brs, 2H), 3.37(s, 3H), 2.52(s, 3H).

Example 12(9): (4-(4-(5-acetyl-4-amino-2-fluorophenyl)-3-aminoisoxazolo[4,5-*c*]pyridin-7-yl)-1*H*-pyrazole-1-carbonyl)glycine

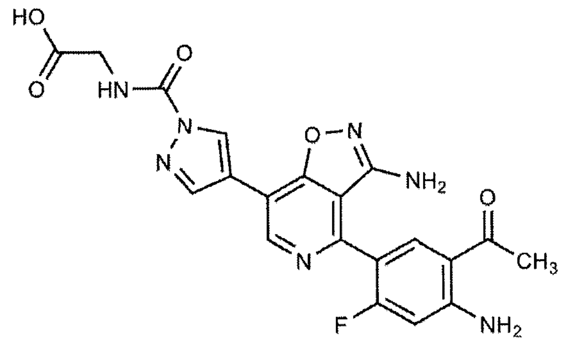

To a solution of the compound produced in Example 3 (100 mg) in 1,3-dimethyl-2-imidazolidinone (hereinafter, "DMI") (2.0 mL), *tert*-butyl 2-isocyanatoacetate (CAS No. 113238-61-2) (89 mg) was added, and the mixture thereof was stirred at room temperature for 16 hours. Water was added to the reaction solution, which was extracted with ethyl acetate. The organic layer thereof was washed with saturated brine, dried over sodium sulfate, and concentrated. The residue therefrom was dissolved in dichloromethane (1 mL), trifluoroacetic acid (0.2 mL) was added thereto, and the mixture thereof was stirred at room temperature for 6 hours. The solvent was distilled off to obtain the titled compound (89 mg) having the following physical property value.
LCMS retention time (min): 0.64;
MS(ESI, Pos.): 454(M+H)+;
$^1$H-NMR(DMSO-$d_6$):δ 9.12(s, 1H), 8.97(s, 1H), 8.92(t, J=6Hz, 1H), 8.57(s, 1H), 8.00(d, J=8.5Hz, 1H), 7.73(brs, 2H), 6.67(d, J=12.5Hz, 1H), 5.85(brs, 2H), 3.96(d, J=6Hz, 2H), 2.51(s, 3H).

Example 12(10): methyl 4-(4-(5-acetyl-4-amino-2-fluorophenyl)-3-aminoisoxazolo(4,5-*c*]pyridin-7-yl)-1*H*-pyrazole-1-carboxylate

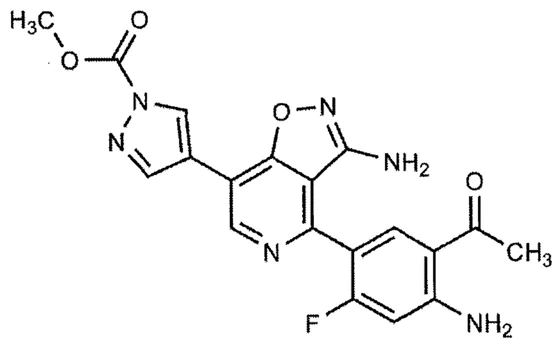

To a solution of the compound produced in Example 3 (50 mg) in DMI (1.0 mL), methyl chloroformate (CAS No. 79-22-1) (67 mg) was added, and the mixture thereof was stirred at room temperature for 1 hour. Water was added to the reaction solution, and the precipitate therefrom was filtered off to obtain the titled compound (55 mg) with the following physical property value.
LCMS retention time (min): 0.69;
MS(ESI, Pos.): 411(M+H)+;

$^1$H-NMR(DMSO-$d_6$):δ 9.10(s, 1H), 8.94(s, 1H), 8.60(s, 1H), 7.98(d, J=8.6Hz, 1H), 7.73(brs, 2H), 6.66(d, J=13Hz, 1H), 5.83(brs, 2H), 4.05(s, 3H), 2.51(s, 3H).

Example 12(11): ((((4-(4-(5-acetyl-4-amino-2-fluorophenyl)-3-aminoisoxazolo[4,5-*c*]pyridin-7-yl)-1*H*-pyrazol-1-yl)methoxy)(hydroxy)phosphoryl)oxy)methyl isopropyl carbonate

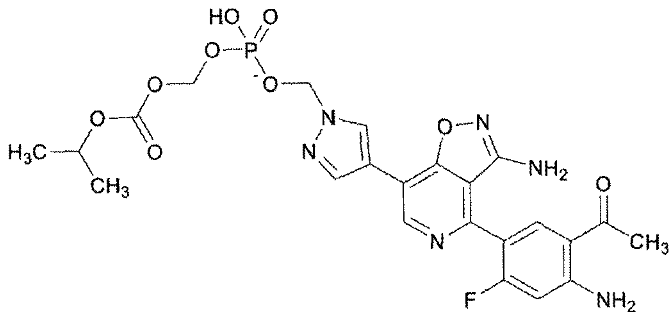

The compound produced in Example 10 (1) (100 mg) was dissolved in DMF (2.0 mL), and to this solution, *N*,*N'*-dicyclohexyl-4-morpholinecarboxamidine (CAS No. 4975-73-9) (254 mg) and chloromethyl isopropyl carbonate (CAS No. 35180-01-9) (198 mg) were added, and the mixture thereof was stirred at room temperature for 24 hours. The reaction solution was filtered to remove insoluble material, and the filtrate therefrom was purified by reversed-phase HPLC (column used: YMC Triart C18 (50 mm × 100 mm); mobile phase: 0.1% TFA/water/acetonitrile = 95: 5 to 50: 50) to obtain the titled compound (52 mg) having the following physical property value.
LCMS retention time (min): 0.06;
MS(ESI, Pos.): 579(M+H)$^+$;
$^1$H-NMR(DMSO-$d_6$):δ9.02(s, 1H), 8.65(s, 1H), 8.37(s, 1H), 8.01(d, J=8.4Hz, 1H), 7.75(brs, 2H), 6.68(d, J=12.8Hz, 1H), 5.93(d, J=10.3Hz, 2H), 5.81(brs, 2H) , 5.47(d, J=13.3Hz, 2H), 4.81(qq, J=6.2, 6.2Hz, 1H), 2.52(s, 3H), 1.23(d, J=6.2Hz, 6H).

[Pharmacological Example]

Example 13: Effect on IRF (Interferon regulatory factor) pathway

The cells mainly described at lines 12-20 of the right column on page 183 in Mol Immunol. 2017, Vol. 90, p. 182-189 were suspended in RPMI medium to prepare 2×10$^6$ cells/mL of cell suspension. 50 μL of the cell suspensions were dispensed into a 96-well plate, to which 50 μL of 6 to 20,000 nmol/L compound solutions were added. After adding the compound, the mixture thereof was incubated at 37 °C for about 24 hours. After incubation, 10 μL of cell suspensions were collected from each well, which were mixed with 50 μL of Quanti-luc (Invivogen). Then, the activation of the IRF pathway was measured by detecting luminescence using a microplate reader (Molcular Devices).
EC50 values of the compounds of the present invention shown in each Example are shown below.

[Table 1]

| Example No. | EC50 (pmol/L) |
|---|---|
| 2 | 1.93 |
| 4 | 0.09 |
| 4(3) | 0.95 |
| 1 | 0.18 |
| 4(5) | 0.08 |
| 3 | 0.04 |
| 4(16) | 1.00 |
| 4(6) | 0.04 |

(continued)

| Example No. | EC50 (pmol/L) |
|---|---|
| 4(7) | 0.02 |
| 4(8) | 0.02 |
| 4(9) | 0.13 |
| 4(15) | 0.10 |

Example 14: Verification of STING agonistic activity

The cells described at lines 37-39 on page 18 in the publication of PCT application with application number PCT/EP2017/59781 were suspended in RPMI medium to prepare $2 \times 10^6$ cells/mL cell suspension. 50 μL of cell suspensions were dispensed into a 96-well plate, to which 50 μL of 6 to 20,000 nM compound solutions were further added, followed by incubation at 37 °C for about 24 hours. 10 μL of the cell suspensions were collected from each well, which were mixed with 50 μL of Quanti-luc (Invivogen), and then the activity of the IRF pathway was measured by detecting luminescence using a microplate reader.

The compound pertaining to the present invention shown in Example 1 showed no IRF activating effect. Therefore, it was shown that the IRF activating effect of the compound pertaining to the present invention exemplified in Example 1 is based on the agonistic activity on STING by the compound pertaining to the present invention.

Example 15: Evaluation of IDO1 inhibitory activity

The evaluation of IDO 1 inhibitory activity was carried out using the IDO 1 Fluorogenic Inhibitor Screening Assay Kit (BPS Bioscience). Specifically, IDO1 Fluorogenic Reaction Solution was dissolved, of which 180 μL were added to each well. Then, 10 μL of the compounds at the respective concentrations of 0.6, 2, 6, 20, 60 and 200 μmol/L were added thereto. Further, after adding 10 μL of IDO1 His-Tag solution thereto, and the mixture thereof was incubated at room temperature for 1 hour, and then 20 μL of Fluorescence Solution was added thereto, and the mixture thereof was incubated at 37 °C for 4 hours. After standing them at room temperature for 10 minutes, the fluorescence was measured using a microplate reader (excitation: 400 nm, emission: 510 nm).

The compound pertaining to the present invention produced in Example 1 showed no IDO1 inhibitory activity.

Example 16: Evaluation of inhibitory activity against various kinases

4 μmol/L of the test substance solution (the compound pertaining to the present invention produced in Example 1) (at 4 times the final concentration) was prepared by dissolving it to the assay buffer (20 mmol/L HEPES, 0.01% Triton X-100, 1 mmol/L DTT, pH 7.5). 4 μmol/L of the substrate/ATP/metal solution (at 4 times the final concentration) was prepared by dissolving it to the kit buffer (20 mmol/L HEPES, 0.01% Triton X-100, 5 mmol/L DTT, pH 7.5). Various kinase solutions at twice the final concentration were prepared by dissolving them to the assay buffer. 5 μL of the test substance solution, 5 μL of the substrate/ATP/metal solution and 10 μL of the kinase solution were mixed in wells of a polypropylene 384-well plate, and the mixture thereof was reacted at room temperature for 1 to 5 hours. The reaction was stopped by adding 70 μL of the termination buffer (QuickScout Screening Assist MSA; Carna Biosciences). The substrate peptide and phosphorylated peptide in the reaction solution were separated and quantified by LabChip system (Perkin Elmer). The kinase reaction was evaluated based on the product ratio (P / (P + S)) calculated from the peak height (S) of the substrate peptide and the peak height (P) of the phosphorylated peptide. The various kinases used in this evaluation are as follows:

BTK, KDR, each subtype of PKCα to τ, each CDK of CDK2 to 9, FAK, TIE2, RAF1 and BRAF.

The compound pertaining to the present invention produced in Example 1 showed no significantly inhibit activities against any of the evaluated kinases.

Example 17: Evaluation of anti-tumor effect in a subcutaneously colon cancer cell line MC38 tumor-bearing mouse model

Colon cancer cell line MC38 derived from C57/BL6 mice were subcutaneously transplanted to right flank region of syngeneic mice (C57/BL6, female, 6 weeks old (Charles River Japan)) (herein, the day of transplantation was designated as Day 0) to prepare subcutaneously MC38 tumor-bearing mice. Seven days after transplantation, the subcutaneously MC38 tumor-bearing mice were grouped based on tumor volume, and the Vehicle group (n=8) and group to which the compound shown in Example 1 (3 mg/kg, n=6) was administered were set. The changes in tumor volume

were continuously measured until 26 days after transplantation (Day 26). The tumor volume was calculated by the following formula:

$$[\text{Tumor volume (mm}^3)] = [\text{major axis (mm)}] \times [\text{minor axis (mm)}]^2 \times 0.5$$

Figure 1 showed its results.

The compound prepared in Example 1 almost completely suppressed the tumor growth at the dose of 3 mg/kg.

Example 18: Evaluation of anti-tumor effect in a subcutaneously colon cancer cell line MC38 tumor-bearing mouse model

Colon cancer cell line MC38 derived from C57/BL6 mice were subcutaneously transplanted to right flank region of syngeneic mice (C57/BL6, female, 6 weeks old (Charles River Japan)) (herein, the day of transplantation was designated as Day 0) to prepare subcutaneous MC38 tumor-bearing mice. They were grouped based on tumor volume 7 or 8 days after transplantation, and the Vehicle (n=8 or 6) and the respective compounds of Examples 10 and 10(1) to 10(6) (1, 1, 1, 10, 3, 1 and 1 mg/kg, respectively, n=8 or 6) were administered thereto. The changes in tumor volume were measured serially until 28 or 30 days after transplantation (Day 28 or 30). The tumor volumes were calculated from the formula shown in Example 17.

Figures 2 and 3 show results thereof.

All of the compounds prepared in Examples 10 and 10(1) to 10(6), respectively, suppressed tumor growth at the above doses. That is, in each group to which the compounds prepared in Examples 10 and 10(1) to 10(6), respectively, were administered, the median tumor volumes were less than 500 $mm^3$ even 30 days after transplantation.

Example 19: Conversion of a prodrug to an active body thereof

The solutions of the example compounds in Table 2 below (50 μmol/L, 50% acetonitrile with 5% DMSO) were added to plasma (245 μL), respectively, such that the final concentrations thereof become 1 μmol/L. After keeping them the temperature at 37°C for 60 minutes, 40 μL of the reaction solutions were taken therefrom, and added to 200 μL of acetonitrile/ethanol (7:3) containing Candesartan (internal standard).

The concentration of the compound prepared in Example 3 (Compound B) in the plasma resulting from the above reaction was analyzed by LC-MS/MS according to the following method.

[LC-MS system]
Prominence UFLCXR (Shimadzu Corporation), API5000 (AB Sciex)
[Pretreatment]
The whole amount was transferred to a filter plate for protein removal and filtered by suction, and the filtrate thereof was diluted 2-fold with water.
[LC column] Shim-pack XR-ODSII 2.0 mm ID × 75 mm (Shimadzu Corporation)
[LC condition]
Column temperature: 40°C
Mobile phase: A: 0.2% formic acid in 5 mM ammonium acetate solution; and
B: acetonitrile; and
Gradient program:

Time (min) 0→1.5→3.0→3.1→4;
Mobile phase B (%) 10→90→90→10→10; and
Flow rate: 0.5 mL/min.

[MS conditions]
Electrospray, positive mode, multiple reaction monitoring;
MS monitor ion:

Compound B: m/z353.0→m/z311.2, DP: 80, CE: 35; and
Candesartan (internal standard): m/z309.1→m/z163.0, DP: 71, CE: 21.

A regression formula was calculated from the peak area ratio (peak area of Compound B/peak area of Candesartan) of the real sample and the standard sample for the calibration curve in the same matrix, and the peak area ratio of the real sample was substituted into the regression formula to calculate the quantitative value. 60 minutes later,

the concentration of Compound B in the sample was compared, and the conversion rate (%) thereof was calculated using the following formula:

$$\text{Conversion rate (\%)} = \text{Concentration of Compound B in the sample after 60 minutes } (\mu mol/L) \times 100$$

[Table 2]

| Example No. | Plasma conversion rate | |
|---|---|---|
| | Mouse | Human |
| 10 (1) | +++ | +++ |
| 12 (5) | +++ | +++ |
| 12 (11) | + | + |
| 12 (3) | +++ | +++ |
| 12 (1) | + | ++ |
| 12 (6) | +++ | +++ |
| 12 | + | + |
| 12 (4) | +++ | +++ |
| 12 (9) | + | + |
| 12 (2) | +++ | +++ |
| 12 (10) | +++ | +++ |

In the table, "+" represents that the conversion rate of each example compound in the table to Compound B is less than 25%, "++" represents that the conversion rate is more than 25% and less than 50%, and "+++" represents that the conversion rate is more than 50%.

It was confirmed that the example compounds in the above table, which are prodrugs, were converted into the compounds prepared in Example 3, which are active bodies thereof in plasma.

Example 20: Evaluation of the effect of dexamethasone on anti-tumor activity in a subcutaneously colon cancer cell line MC38 tumor-bearing mouse model

Colon cancer cell line MC38 derived from C57/BL6 mice were subcutaneously transplanted to right flank region of syngeneic mice (C57/BL6, female, 6 weeks old (Charles River Japan)) (herein, the day of transplantation was designated as Day 0) to prepare subcutaneously MC38 tumor-bearing mice. They were grouped based on tumor volume 7 days after transplantation, and the following drugs were administered intravenously once 8 days after transplantation to the subcutaneously MC38 tumor-bearing mice. Dexamethasone was administered intravenously once 1 hour before administration of Compound A.

- Vehicle (n=6);
- Compound prepared in Example 10 (1) (Compound A) (0.3 mg/kg; n=6),
- Compound A (1 mg/kg; n=6),
- Compound A (0.3 mg/kg) and dexamethasone (10 mg/kg) (n=6), and
- Compound A (1 mg/kg) and dexamethasone (10 mg/kg) (n=6).

Then, the changes in tumor volume were measured serially until 25 days after transplantation (Day 25), respectively. The tumor volumes were calculated from the formula shown in Example 17. Furthermore, Blood was collected 1 hour after administration of the compound, and the amounts of cytokines such as IL-6 and IFN-β in plasma were measured. Figures 4 and 5 show results thereof.

The pretreatment of dexamethasone decreased the production of IL-6 in plasma when used in combination with Compound A, compared to that when administration of the compound alone, but did not affect the anti-tumor effect

of the compound.

Example 21: Evaluation of the effect of dexamethasone on anti-tumor activity in a subcutaneously colon cancer cell line MC38 tumor-bearing mouse model

Colon cancer cell line MC38 derived from C57/BL6 mice were subcutaneously transplanted to right flank region of syngeneic mice (C57/BL6, female, 6 weeks old (Charles River Japan)) (herein, the day of transplantation was designated as Day 0) to prepare subcutaneously MC38 tumor-bearing mice. They were grouped based on tumor volume 7 days after transplantation, and the following drugs were administered to the subcutaneously MC38 tumor-bearing mice, respectively. Compound A was administered intravenously once 8 days after transplantation, and the anti-PD-1 antibody 4H2 was administered intraperitoneally 4 times every 6 days since 8 days after transplantation.

- Vehicle (n=9),
- Compound A (0.1 mg/kg; n=9),
- Anti-PD-1 antibody 4H2 (10 mg/kg (but 20 mg/kg only in the first dosage); n=9), and
- Compound A (0.1 mg/kg) and anti-PD-1 antibody 4H2 (10 mg/kg (but 20 mg/kg only in the first dosage); n=9).

The anti-PD-1 antibody 4H2 can be obtained according to the method described in WO2006/121168.
Then, the changes in tumor volume were measured serially until 28 days after transplantation (Day 28), respectively. The tumor volumes were calculated from the formula shown in Example 17. Figures 6 and 7 show results thereof.
At the above dosage, Compound A enhanced the anti-tumor effect observed with anti-PD-1 antibody alone, and showed the tumor-reducing effect even 28 days after transplantation. In addition, the number of patients with complete tumor remission is 2 out of 9 in case of the anti-PD-1 antibody alone, but 5 out of 9 in case of combination of Compound A and the anti-PD-1 antibody.

Example 22: Evaluation of the effect of dexamethasone on anti-tumor activity in a subcutaneously colon cancer cell line MC38 tumor-bearing mouse model

Colon cancer cell line MC38 derived from C57/BL6 mice were subcutaneously transplanted to right flank region of syngeneic mice (C57/BL6, female, 6 weeks old (Charles River Japan)) (herein, the day of transplantation was designated as Day 0) to prepare subcutaneously MC38 tumor-bearing mice. They were grouped based on tumor volume 7 days after transplantation, and the following drugs were administered to the subcutaneously MC38 tumor-bearing mice, respectively. Compound A was administered intravenously 4 times every 7 days since 7 days after transplantation, and dexamethasone was administered intravenously immediately before each administration of Compound A. The anti-PD-1 antibody 4H2 was administered intraperitoneally 4 times every 6 days since 7 days after transplantation.

- Vehicle (n=8),
- Anti-PD-1 antibody 4H2 (10 mg/kg, (but 20 mg/kg only in the first dosage)) (n=8),
- Anti-PD-1 antibody 4H2 (10 mg/kg (but 20 mg/kg only in the first dosage)) (n=8) and dexamethasone (0.1 mg/kg) (n=8),
- Compound A (0.1 mg/kg),
- Compound A (0.1 mg/kg) and dexamethasone (0.1 mg/kg) (n=8),
- Compound A (0.1 mg/kg) and anti-PD-1 antibody 4H2 (10 mg/kg (but 20 mg/kg only in the first dosage)) (n=8), and
- Compound A (0.1 mg/kg), anti-PD-1 antibody 4H2 (10 mg/kg (but 20 mg/kg only in the first dosage)) and dexamethasone (0.1 mg/kg) (n=8).

Then, the changes in tumor volume were measured serially until 28 or 35 days after transplantation (Day 28 or 35), respectively. The tumor volumes were calculated from the formula shown in Example 17. Figure 8 shows results thereof.
The pretreatment of dexamethasone decreased the production of IL-6 in plasma when further used in combination with Compound A and the anti-PD-1 antibody 4H2, compared to that when administration of the combination alone, but did not affect the anti-tumor effect of the combination.

Example 23: Evaluation of the effect of dexamethasone on anti-tumor activity in a subcutaneously melanoma cell line B16F10 tumor-bearing mice model

Melanoma cell line B16F10 derived from C57/BL6 mice were subcutaneously transplanted to right flank region

of syngeneic mice (C57/BL6, female, 6 weeks old (Charles River Japan)) (herein, the day of transplantation was designated as Day 0) to prepare subcutaneously B16F10 tumor-bearing mice. They were grouped based on tumor volume 10 days after transplantation, and the following drugs were administered to the subcutaneously B16F10 tumor-bearing mice, respectively.

[Combination experiment 1]

- Vehicle and control antibody (rIgG1) (n=8 or 6),
- Compound A (0.3 mg/kg) and control antibody (rIgG1) (n=6),
- Compound A (0.3 mg/kg) and control antibody (rIgG1) (n=6),
- Anti-PD-1 antibody 4H2 (10 mg/kg (but 20 mg/kg only in the first dose only); n=6), and
- Compound A (0.3 mg/kg) and anti-PD-1 antibody 4H2 (10 mg/kg (but 20 mg/kg only for the first dosage)) (n=6).

In the Combination Experiment 1, Compound A was administered once 10 days after transplantation while the anti-PD-1 antibody 4H2 and control antibody were administered 4 times every 6 days since 10 days after transplantation, respectively.

[Combination experiment 2]

- Vehicle and control antibody (rIgG1) (n=8 or 6),
- Compound A (0.3 mg/kg) and control antibody (rIgG1) (n=6),
- Anti-VEGFR2 antibody DC101 (BioXCell) (10 mg/kg; n=6), and
- Compound A (0.3 mg/kg) and anti-VEGFR2 antibody DC101 (10 mg/kg) (n=6).

In the Combination Experiment 2, Compound A was administered 4 times every 7 days since 10 days after transplantation while anti-VEGFR2 antibody DC101 and control antibody were administered 7 times every 3 or 4 days sonce 10 days after transplantation, respectively.

Vehicle and Compound A were administered intravenously while control antibody, anti-PD-1 antibody, and anti-VEGFR2 antibody were administered intraperitoneally, respectively.

Then, the changes in tumor volume were measured serially until 30 days after transplantation (Day 30), respectively. The tumor volumes were calculated from the formula shown in Example 17. Figures 9 and 10 show results thereof.

Compound A enhanced the anti-tumor effects of anti-PD-1 antibody and anti-VEGFR2 antibody at the above dosages, respectively.

Example 24: Evaluation of anti-tumor effect on human acute myeloid leukemia cell line

The compound prepared in Example 3 (Compound B) was evaluated for anti-tumor activity against several human acute myeloid leukemia cell lines shown in the table of Figure 13.

The cell lines above were suspended in medium (RPMI 1640 containing 10% FBS and 1% Penicillin-Streptomycin), respectively, and seeded into 96-well plates at a density of $5.0\times10^4$ cells/well. Immediately after seeding, mediums containing Compound B and Vehicle (0.1% at the final concentration of DMSO solution after addition to the medium), respectively, were added to each well and mixed well. The final concentration of Compound B after addition to the medium was prepared to be 100 to1000 nmol/L, respectively. After culturing the cell lines for 48 hours at 37 °C and 5% $CO_2$, 10 μL of Cell Counting Kit-8 (Dojindo) was added to each well, and after incubation for 60 minutes, the absorbance (450 nm) in each well was measured using a microplate reader. EC50 value was calculated from the absorbance value using Graphpad prism (Graphpad Software) to evaluate the effect of Compound B on cell proliferation. Figure 11 shows results thereof. Relative cell count was calculated according to the following formula. Relative minimum cell count represents the minimum value of the above values.

[Formula 1]

Relative cell count (%) = (Absorbance (450 nm) in addition of Compound B – blank) / (Absorbance (450 nm) in addition of Vehicle (0.1% DMSO) –blank) x 100

Compound B pertaining to the present invention showed significant anti-tumor effect against several human acute myeloid leukemia cell lines under administration alone thereof.

Example 25: Evaluation of the anti-tumor effect in a subcutaneously tumor model bearing human acute myeloid leukemia cell line MV4-11

Human acute myeloid leukemia cell line MV4-11 was transplanted subcutaneously to right flank region of immunodeficient mice (C.B-17/Icr-scid/scidJcl, female, 6 weeks old (Japan Clare)) (herein, the day of transplantation was designated as Day 0) to prepare subcutaneously MV4-11 tumor-bearing mice. They were grouped based on tumor volume 6 days after transplantation, and Compound A (0.3, 1.0, and 3.0 mg/kg, respectively; n=8) and Vehicle (n=8) were administered to the subcutaneously tumor-bearing mice, respectively. The changes in tumor volume were measured serially until 28 days after transplantation (Day 28). The tumor volumes were calculated from the formula shown in Example 17. Figure 12 shows results thereof.

Compound A suppressed tumor growth at dosages of 1.0 mg/kg and 3.0 mg/kg. Since immunodeficient mice were used in this example, which indicates that Compound A suppressed the growth of cancer cells by direct action, not through tumor immunity.

Example 26: Evaluation of anti-tumor effects in combination with various anti-neoplastic agents

The anti-tumor effects of the STING agonistic compounds pertaining to the present invention and combination thereof with anti-neoplastic agents were evaluated on several human acute myeloid leukemia cell lines (KG-1$\alpha$, THP-1 and CMK) and human B-cell lymphoma cell lines (DOHH2 and OLI-Ly3) below.

The cell lines above were suspended in medium (RPMI 1640 containing 10% FBS and 1% Penicillin-Streptomycin), respectively, and seeded into 96-well plates at a density of 2.5 to $5\times10^4$ cells/well. Immediately after seeding, mediums containing Compound B prepared, respectively, such that the final concentrations after addition to the mediums become 0, 10, 30, 100, 300, and 1000 nmol/L and anti-neoplastic agents prepared such that those become the following final concentrations, respectively, were added to each well and mixed well. As a control, DMSO solution prepared such that the final concentration becomes 0.1% after addition to the medium was added thereto, separately.

- Venetoclax (final concentrations: 0, 1, 10, 100, 1000, and 10000 nmol/L, respectively),
- Cytarabine (final concentrations: 0, 1, 10, 100, 1000, and 10000 nmol/L, respectively),
- Azacitidine (final concentrations: 0, 1, 10, 100, 1000, and 10000 nmol/L, respectively), and
- Navitoclax (final concentrations: 0, 50, 500, and 5000 nmol/L, respectively) (the concentration of Compound B in combination with Navitoclax is 1000 nmol/L).

After culturing the cell lines for 48 hours at 37 °C and 5% $CO_2$, 10 $\mu$L of Cell Counting Kit-8 (Dojindo) was added to each well, and after incubation for 60 minutes, the absorbance (450 nm) in each well was measured using a microplate reader. Figures 13, 14, and 15 show results thereof, respectively. In the figures, the "signal intensity" on the vertical axis represents the absorbance value (450 nm), and the lower the value, the higher the anti-tumor effect.

Compound B enhanced the respective anti-tumor activities of Venetoclax, Azacitidine, and Cytarabine against human acute myeloid leukemia cell lines KG-1$\alpha$, THP-1 and CMK, respectively, and also enhanced the anti-tumor activity of Navitoclax against human B-cell lymphoma cell lines DOHH2 and OLI-Ly3, respectively.

[Formulation Example]

The following components are mixed by a conventional method, then a solution thereof are sterilized by a conventional method, and 5 mL thereof are filled in ampoules and lyophilized by a conventional method to obtain 10,000 ampules containing 20 mg of the active ingredient per ampoule.

| | |
|---|---|
| - Methyl 2-amino-5-(3-amino-7-(1*H*-pyrazol-4-yl)isoxazolo[4,5-*c*]pyridin-4-yl)-4-fluorobenzoate | 200 g |
| - Mannitol | 20 g |

(continued)

| | |
|---|---|
| - Distilled water | 50 L |

[Industrial Availability]

Since the compound pertaining to the present invention have the agonistic activity to STING, a pharmaceutical agent containing it as an active ingredient is useful as an agent for suppressing the progression of, suppressing the recurrence of and/or treating cancer or infectious disease.

## Claims

1. An agent for suppressing the progression of, suppressing the recurrence of and/or treating cancer, comprising a STING agonistic compound as an active ingredient, being further administered in combination with an adrenal corticosteroid when

(1) administering the agent comprising the STING agonistic compound as an active ingredient, or
(2) administering the agent comprising the STING agonistic compound as an active ingredient, in combination with one or two or more kinds of anti-neoplastic agents.

2. The agent according to claim 1, wherein the STING agonistic compound is a compound represented by the general formula (1-1)

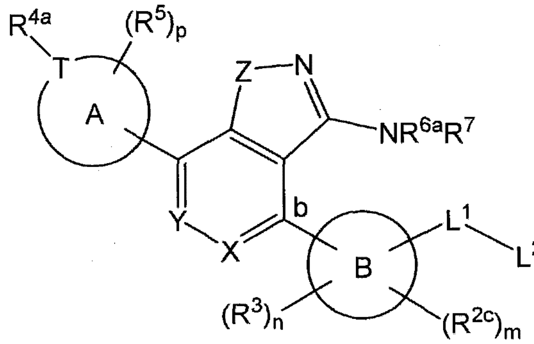

(I-1)

[wherein X and Y represent -CII= or a nitrogen atom (provided that both X and Y do not represent -CH=, simultaneously), respectively, Z represents an oxygen atom or sulfur atom, T represents a carbon atom or nitrogen atom, Ring A represents a 5 to 7-membered monocycle, Ring B represents a 5 to 7-membered monocycle or 8 to 10-membered bicycle, $L^1$ represents a bond, -O-, -CONH-, -CO-, $-CO_2-$, -S-, $-SO_2-$ or -SO-, $L^2$ represents a bond, C 1-3 alkylene group, C3-7 cycloalkylene group or phenylene group, $R^1$ represents a hydrogen atom, halogen atom, hydroxyl group, cyano group, $N(R^{1a})_2$ (herein, the two $R^{1a}$s represent each independently a hydrogen atom or C1-4 alkyl group), C1-4 alkyl group, carboxy group, C1-4 alkoxycarbonyl group, C1-4 haloalkyl group, methyl-$d_3$ group, C3-7 cycloalkyl group, phenyl group or 3 to 7-membered monocyclic non-aromatic heterocycle, $R^{2c}$ represents a hydrogen atom, hydroxyl group, halogen atom, oxo group, nitro group, cyano group, C1-4 alkoxy group or $-CH_2NR^{2d}R^{2e}$ or $NR^{2d}R^{2e}$ (herein, $R^{2d}$ is a hydrogen atom, C1-4 alkyl group or $R^{FR}$, and $R^{2e}$ represents a hydrogen atom), m represents an integer of 0 or 1, $R^3$ represents a hydrogen atom, halogen atom, hydroxyl group, C1-4 alkyl group, C1-4 alkoxy group, C1-4 haloalkyl group, C1-4 haloalkoxy group or amino group, n represents an integer of 1 to 16 (herein, if n is 2 or more, the groups represented by a plurality of $R^3$s may be the same or different), $R^{4a}$ represents a hydrogen atom, C1-4 alkyl group, carboxy group or $R^{FR}$, $R^5$ represents a C1-4 alkyl group, p represents an integer of 0 to 5 (herein, if p is two or more, the groups represented by a plurality of $R^5$s may be the same or different), $R^{6a}$ represents a hydrogen atom, C1-4 alkyl group or $R^{FR}$, wherein, $R^{FR}$ represents

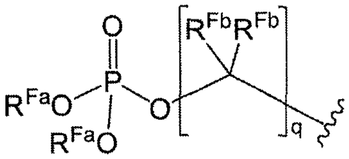

[wherein $R^{Fa}$ each independently represents a hydrogen atom, C1-4 alkyl group, C3-6 cycloalkyl group, $-(CH_2)_2OH$, $-CR^{Fb}{}_2OC(=O)$-(C1-4 alkyl), $-CR^{Fb}{}_2OC(=O)O$-(C1-4 alkyl) or benzyl group, $R^{Fb}$ each independently represents a hydrogen atom or C1-4 alkyl group, and q represents an integer of 1 or 2.], $R^7$ represents a hydrogen atom, b represents the bonding position of Ring B, provided that two or more of $R^{2d}$, $R^{4a}$ and $R^{6a}$ do not represent $R^{FR}$, simultaneously.], a pharmaceutically acceptable salt thereof or a solvate thereof.

**3.** The agent according to claim 2, wherein the compound represented by the general formula (I-1) is the compound selected from the group consisting of

(1) 4-(4-amino-2-fluoro-5-methoxyphenyl)-7-(1*H*-pyrazol-4-yl)isoxazolo[5,4-*c*]pyridin-3-amine,
(2) 4-(4-amino-2-fluoro-5-methoxyphenyl)-7-(1*H*-pyrazol-4-yl)isoxazolo[4,5-*c*]pyridin-3-amine,
(3) 4-(4-amino-3-methoxyphenyl)-7-(1*H*-pyrazol-4-yl)isoxazolo[4,5-*c*]pyridin-3-amine,
(4) 4-(4-amino-2-fluoro-5-(methylthio)phenyl)-7-(1*H*-pyrazol-4-yl)isoxazolo[4,5-*c*]pyridin-3-amine,
(5) 4-(4-amino-2-fluoro-5-(methoxy-$d_3$)phenyl)-7-(1*H*-pyrazol-4-yl)isoxazolo[4,5-*c*]pyridin-3-amine,
(6) 4-(4-amino-2-fluoro-5-(methylsulfonyl)phenyl)-7-(1*H*-pyrazol-4-yl)isoxazolo[4,5-c]pyridin-3-amine,
(7) 4-(4-amino-5-(ethylthio)-2-fluorophenyl)-7-(1*H*-pyrazol-4-yl)isoxazolo[4,5-*c*]pyridin-3-amine,
(8) 4-(4-amino-2-fluoro-5-(methylsulfinyl)phenyl)-7-(1*H*-pyrazol-4-yl)isoxazolo[4,5-*c*]pyridin-3-amine,
(9) 4-(4-amino-2-fluoro-3-methoxyphenyl)-7-(1*H*-pyrazol-4-yl)isoxazolo[4,5-*c*]pyridin-3-amine,
(10) methyl 2-amino-5-(3-amino-7-(1*H*-pyrazol-4-yl)isoxazolo[4,5-*c*]pyridin-4-yl)-4-fluorobenzoate,
(11) 2-amino-5-(3-amino-7-(1*H*-pyrazol-4-yl)isoxazolo[4,5-*c*]pyridin-4-yl)-4-fluorobenzoic acid,
(12) 2-amino-5-(3-amino-7-(1*H*-pyrazol-4-yl)isoxazolo[4,5-*c*]pyridin-4-yl)-4-fluorobenzamide,
(13) 4-(4-amino-2-fluoro-5-methoxyphenyl)-7-(3-methyl-1*H*-pyrazol-4-yl)isoxazolo[4,5-c]pyridin-3-amine,
(14) methyl 2-amino-5-(3-amino-7-(1-((phosphonooxy)methyl)-1*H*-pyrazol-4-yl)isoxazolo[4,5-*c*]pyridin-4-yl)-4-fluorobenzoate,
(15) 1-(2-amino-5-(3-amino-7-(1*H*-pyrazol-4-yl)isoxazolo[4,5-*c*]pyridin-4-yl)-4-fluorophenyl)ethan-1-one,
(16) 4-(4-amino-2-chloro-5-(methylthio)phenyl)-7-(1*H*-pyrazol-4-yl)isoxazolo[4,5-*c*]pyridin-3-amine,
(17) ethyl 2-amino-5-(3-amino-7-(1*H*-pyrazol-4-yl)isoxazolo[4,5-*c*]pyridin-4-yl)-4-fluorobenzoate,
(18) (4-(4-(5-acetyl-4-amino-2-fluorophenyl)-3-aminoisoxazolo[4,5-*c*]pyridin-7-yl)-1*H* pyrazol-1-yl)methyl dihydrogen phosphate,
(19) ethyl 2-amino-5-(3-amino-7-(1-((phosphonooxy)methyl)-1*H*-pyrazol-4-yl)isoxazolo[4,5-c]pyridin-4-yl)-4-fluorobenzoate,
(20) 2-amino-5-(3-amino-7-(1*H*-pyrazol-4-yl)isoxazolo[4,5-*c*]pyridin-4-yl)-4-fluoro-*N*-methylbenzamide,
(21) 1-(2-amino-5-(3-amino-7-(1*H*-pyrazol-4-yl)isoxazolo[4,5-*c*]pyridin-4-yl)-4-fluorophenyl)propan-1-one,
(22) 2-amino-5-(3-amino-7-(1*H*-pyrazol-4-yl)isoxazolo[4,5-*c*]pyridin-4-yl)-*N*-ethyl-4-fluorobenzamide,
(23) 1-(2-amino-5-(3-amino-7-(1*H*-pyrazol-4-yl)isoxazolo[4,5-*c*]pyridin-4-yl)phenyl)ethan-1-one,
(24) methyl 2-amino-5-(3-amino-7-(1*H*-pyrazol-4-yl)isoxazolo[4,5-*c*]pyridin-4-yl)benzoate,
(25) 2-amino-5-(3-amino-7-(1*H*-pyrazol-4-yl)isoxazolo[4,5-*c*]pyridin-4-yl)-*N*-propylbenzamide,
(26) 1-(2-amino-5-(3-amino-7-(1*H*-pyrazol-4-yl)isoxazolo[4,5-*c*]pyridin-4-yl)-4-fluorophenyl)butan-1-one,
(27) 2-amino-5-(3-amino-7-(1*H*-pyrazol-4-yl)isoxazolo[4,5-*c*]pyridin-4-yl)-4-fluoro-*N*-propylbenzamide,
(28) 1-(2-amino-5-(3-amino-7-(1*H*-pyrazol-4-yl)isoxazolo[4,5-*c*]pyridin-4-yl)phenyl)butan-1-one,
(29) 2-hydroxyethyl 2-amino-5-(3-amino-7-(1*H*-pyrazol-4-yl)isoxazolo[4,5-*c*]pyridin-4-yl)-4-fluorobenzoate,
(30) 2-amino-5-(3-amino-7-(1*H*-pyrazol-4-yl)isoxazolo[4,5-*c*]pyridin-4-yl)benzamide,
(31) 2-amino-5-(3-amino-7-(1*H*-pyrazol-4-yl)isoxazolo[4,5-*c*]pyridin-4-yl)-*N*-methylbenzamide,
(32) (4-(3-amino-4-(4-amino-5-(ethylcarbamoyl)-2-fluorophenyl)isoxazolo[4,5-*c*]pyridin-7-yl)-1*H*-pyrazol-1-yl)methyl dihydrogen phosphate,
(33) 1-(2-amino-5-(3-amino-7-(1*H*-pyrazol-4-yl)isoxazolo[4,5-*c*]pyridin-4-yl)-4-hydroxyphenyl)ethan-1-one,
(34) 2-amino-5-(3-amino-7-(1*H*-pyrazol-4-yl)isoxazolo[4,5-*c*]pyridin-4-yl)-*N*-ethylbenzamide,
(35) 1-(2-amino-5-(3-amino-7-(1*H*-pyrazol-4-yl)isoxazolo[4,5-*c*]pyridin-4-yl)phenyl)propan-1-one,
(36) 2-amino-5-(3-amino-7-(1*H*-pyrazol-4-yl)isoxazolo[4,5-*c*]pyridin-4-yl)-4-chloro-*N*-ethylbenzamide,
(37) (4-(3-amino-4-(4-amino-2-fluoro-5-(methylthio)phenyl)isoxazolo[4,5-*c*]pyridin-7-yl)-1*H*-pyrazol-1-yl)methyl dihydrogen phosphate,

(38) (4-(3-amino-4-(4-amino-2-fluoro-5-propionylphenyl)isoxazolo[4,5-*c*]pyridin-7-yl)-1*H*-pyrazol-1-yl)methyl dihydrogen phosphate,
(39) (4-(4-(3-acetyl-4-aminophenyl)-3-aminoisoxazolo[4,5-*c*]pyridin-7-yl)-1*H*-pyrazol-1-yl)methyl dihydrogen phosphate,
(40) 4-(2-fluoro-5-methoxy-4-nitrophenyl)-7-(1*H*-pyrazol-4-yl)isoxazolo[4,5-*c*]pyridin-3-amine,
(41) (4-(3-amino-4-(4-amino-2-fluoro-5-(methylsulfonyl)phenyl)isoxazolo[4,5-*c*]pyridin-7-yl)-1*H*-pyrazol-1-yl)methyl dihydrogen phosphate,
(42) (4-(3-amino-4-(4-amino-5-(ethylcarbamoyl)-2-chlorophenyl)isoxazolo[4,5-*c*]pyridin-7-yl)-1*H*-pyrazol-1-yl)methyl dihydrogen phosphate,
(43) 1-(2-amino-5-(3-amino-7-(1*H*-pyrazol-4-yl)isothiazolo[4,5-*c*]pyridin-4-yl)-4-fluorophenyl)ethan-1-one,
(44) 4-(4-amino-2-fluoro-5-(trifluoromethyl)phenyl)-7-(1*H*-pyrazol-4-yl)isoxazolo[4,5-*c*]pyridin-3-amine,
(45) ((((4-(4-(5-acetyl-4-amino-2-fluorophenyl)-3-aminoisoxazolo[4,5-*c*]pyridin-7-yl)-1*H*-pyrazol-1-yl)methoxy)(hydroxy)phosphoryl)oxy)methyl isopropyl carbonate, and
(46) 1-(4-(4-(5-acetyl-4-amino-2-fluorophenyl)-3-aminoisoxazolo[4,5-*c*]pyridin-7-yl)-1*H*-pyrazol-1-yl)ethyl dihydrogen phosphate.

**4.** The agent according to claim 2 or 3, wherein the STING agonistic compound of claim 2 or 3 is administered to an adult at 0.03 to 10.0 mg/kg (body weight) of the compound per dose every 1, 2, 3 or 4 weeks by intravenous drip infusion.

**5.** The agent according to any one of claims 1 to 4, wherein the anti-neoplastic agent is a tumor immunotherapeutic drug.

**6.** The agent according to claim 5, wherein the tumor immunotherapeutic drug is an anti-PD-1 antibody.

**7.** The agent according to claim 6, wherein the anti-PD-1 antibody is any one of antibody selected from Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab, Geptanolimab, AMP-514, STI-A1110, ENUM 388D4, ENUM 244C8, GLS010, CS1003, BAT-1306, AK105, AK103, BI 754091, LZM009, CMAB819, Sym021, SSI-361, JY034, HX008, ISU106 and CX-188.

**8.** The agent according to claim 7, wherein if the anti-PD-1 antibody is Nivolumab, it is administered to an adult at (1) 1 mg/kg (body weight) per dose every 3 weeks, (2) 3 mg/kg (body weight) per dose every 2 weeks, (3) 2 mg/kg (body weight) per dose every 3 weeks, (4) 80 mg per dose every 3 weeks, (5) 240 mg per dose every 2 weeks, (6) 360 mg per dose every 3 weeks, or (7) 480 mg per dose every 4 weeks, of Nivolumab, by intravenous drip infusion.

**9.** The agent according to claim 7, wherein if the anti-PD-1 antibody is Pembrolizumab, it is administered to an adult at (1) 200 mg per dose every 3 weeks, (2) 400 mg per dose every 6 weeks, or (3) 2 mg/kg (body weight) per dose (up to 200 mg per dose) every 3 weeks, of Pembrolizumab, by intravenous drip infusion.

**10.** The agent according to any one of claims 1 to 9, wherein the adrenal corticosteroid is administered by intravenous injection.

**11.** The agent according to claim 10, wherein the adrenal corticosteroid is administered at an arbitrary timing between just before and about 2 hours before each administration of the STING agonistic compound of claim 2 or 3.

**12.** The agent according to claim 10, wherein the adrenal corticosteroid is administered at about 30 minutes, about 1 hour, about 90 minutes or about 2 hours before each administration of the STING agonistic compound of claim 2 or 3.

**13.** The agent according to claim 10, wherein the adrenal corticosteroid is administered just after each administration of the STING agonistic compound of claim 2 or 3.

**14.** The agent according to any one of claims 1 to 9, wherein if the adrenal corticosteroid is administered orally, it is administered at an arbitrary timing on at least one day before each administration of the STING agonistic compound of claim 2 or 3.

**15.** The agent according to any one of claims 1 to 14, wherein the adrenal corticosteroid is selected from drugs comprising any of hydrocortisone sodium phosphate, hydrocortisone sodium succinate, prednisolone sodium succinate, methylprednisolone sodium succinate, dexamethasone, dexamethasone sodium phosphate and betamethasone sodium

phosphate as an active ingredient.

**16.** The agent according to claim 15, wherein if the active ingredient of the adrenal corticosteroid is hydrocortisone sodium phosphate, it is administered to an adult at 100 to 1000 mg of hydrocortisone per dose, 1 to 4 times per day by intravenous injection or intravenous drip infusion.

**17.** The agent according to claim 15, wherein if the active ingredient of the adrenal corticosteroid is hydrocortisone sodium succinate, it is administered to an adult

(1) at 50 to 100 mg of hydrocortisone per dose, 1 to 4 times per day by intravenous injection or intravenous drip infusion, or
(2) in an emergency, at 100 to 200 mg of hydrocortisone per dose by intravenous injection or intravenous drip infusion.

**18.** The agent according to claim 15, wherein if the active ingredient of the adrenal corticosteroid is prednisolone sodium succinate, it is administered to an adult at

(1) 10 to 50 mg of prednisolone per dose every 3 to 6 hours by intravenous injection, or
(2) 20 to 100 mg of prednisolone per dose once or twice per day by intravenous drip infusion.

**19.** The agent according to claim 15, wherein if the active ingredient of the adrenal corticosteroid is methylprednisolone sodium succinate, it is administered slowly to an adult at 125 to 2000 mg of methylprednisolone per dose by intravenous injection or intravenous drip infusion.

**20.** The agent according to claim 15, wherein if the active ingredient of the adrenal corticosteroid is dexamethasone sodium phosphate, it is administered to an adult at

(1) 1.65 to 6.6 mg of dexamethasone per dose every 3 to 6 hours by intravenous injection, or
(2) 1.65 to 8.3 mg of dexamethasone per dose once or twice per day by intravenous drip infusion.

**21.** The agent according to claim 15, wherein if the active ingredient of the adrenal corticosteroid is betamethasone sodium phosphate, it is administered to an adult at

(1) 2 to 8 mg of betamethasone per dose every 3 to 6 hours by intravenous injection, or
(2) 2 to 10 mg of betamethasone per dose once or twice per day by intravenous drip infusion.

**22.** The agent according to claim 15, wherein if the active ingredient of the adrenal corticosteroid is dexamethasone, it is administered orally to an adult at 0.5 to 8 mg of dexamethasone per day in 1 to 4 divided dosages.

**23.** The agent according to any one of claims 1 to 22, wherein the cancer is solid cancer or hematological cancer.

**24.** The agent according to claim 23, wherein the cancer is solid cancer, which is one or more cancers selected from malignant melanoma, non-small cell lung cancer, small cell lung cancer, head and neck cancer, renal cell cancer, breast cancer, ovarian cancer, ovarian clear cell adenocarcinoma, nasopharyngeal cancer, uterine cancer, anal cancer, colorectal cancer, rectal cancer, colon cancer, hepatocellular carcinoma, esophageal cancer, gastric cancer, esophagogastric junction cancer, pancreatic cancer, urine urothelial cancer, prostate cancer, fallopian tube cancer, primary peritoneal cancer, malignant pleural mesothelioma, gallbladder cancer, bile duct cancer, biliary tract cancer, skin cancer, testicular cancer (germ cell tumor), vaginal cancer, vulvar cancer, penile cancer, small intestine cancer, endocrine system cancer, thyroid cancer, parathyroid cancer, adrenal carcinoma, spinal tumor, neuroblastoma, medulloblastoma, ocular retinoblastoma, neuroendocrine tumor, brain tumor and squamous cell carcinoma.

**25.** The agent according to claim 23, wherein the cancer is hematological cancer, which is one or more cancers selected from multiple myeloma, malignant lymphoma, leukemia, central nervous system malignant lymphoma, myelodysplastic syndromes and myeloproliferative syndromes.

FIG. 1

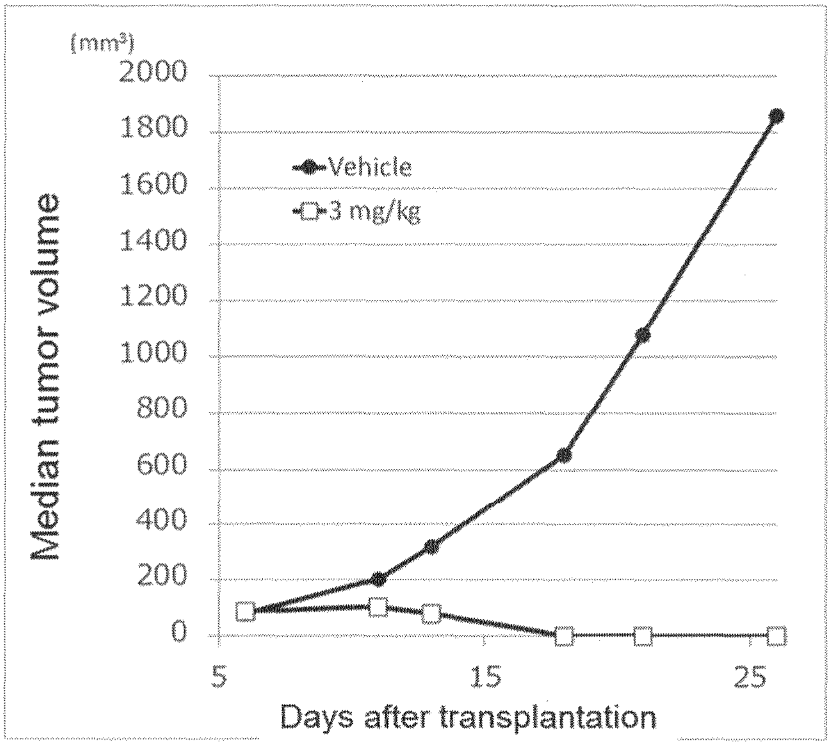
(mm³)
Median tumor volume
2000
1800
1600
1400
1200
1000
800
600
400
200
0
Vehicle
3 mg/kg
5
15
25
Days after transplantation

FIG. 2

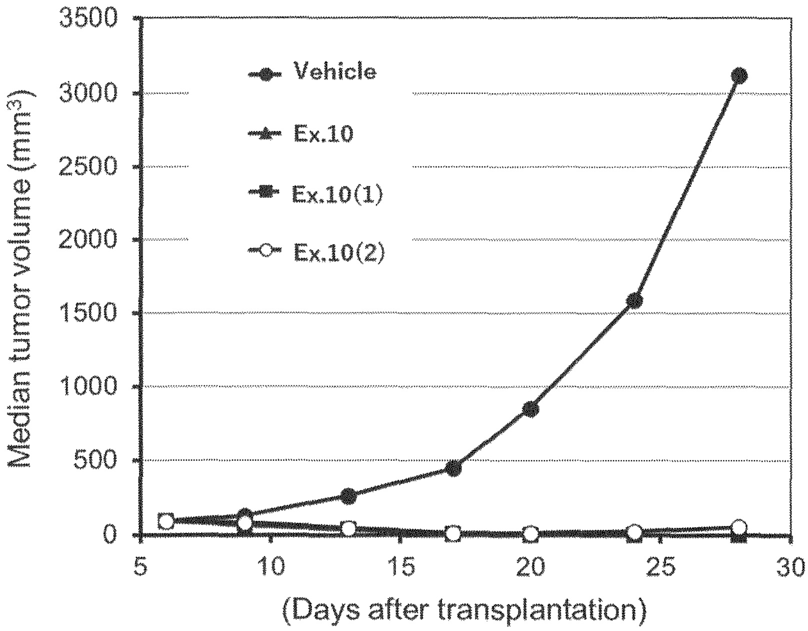
Median tumor volume (mm³)
3500
3000
2500
2000
1500
1000
500
0
Vehicle
Ex.10
Ex.10(1)
Ex.10(2)
5
10
15
20
25
30
(Days after transplantation)

FIG. 3

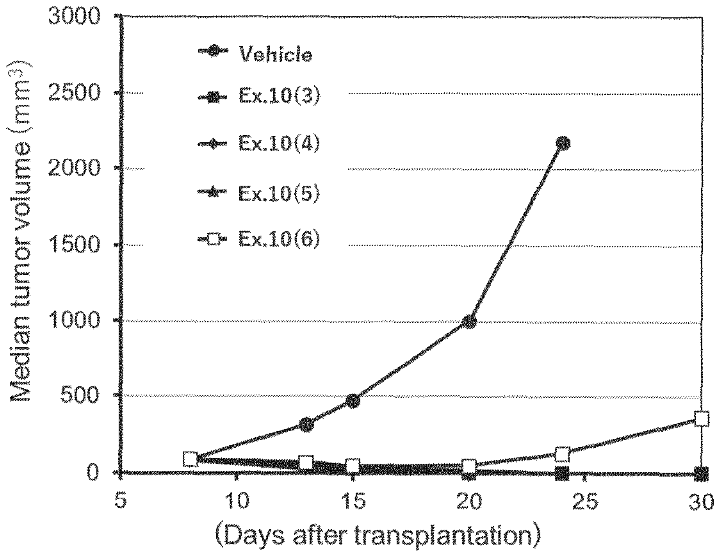
3000
2500
2000
1500
1000
500
0
Median tumor volume (mm$^3$)
Vehicle
Ex.10(3)
Ex.10(4)
Ex.10(5)
Ex.10(6)
5
10
15
20
25
30
(Days after transplantation)

FIG. 4

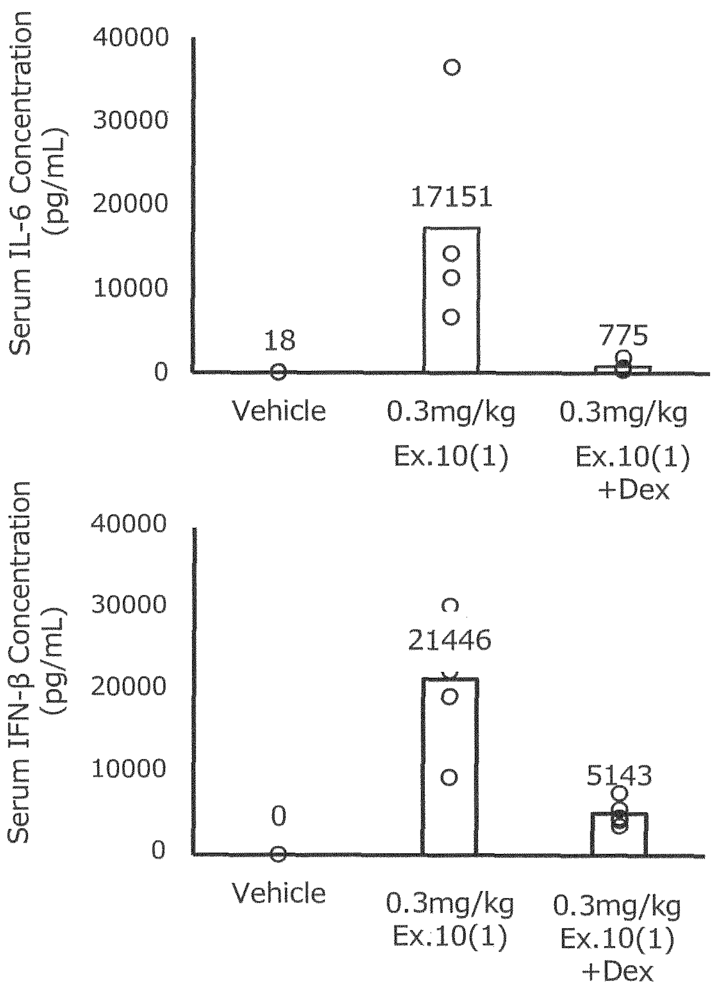
Serum IL-6 Concentration (pg/mL)
40000
30000
20000
10000
0
18
17151
775
Vehicle
0.3mg/kg Ex.10(1)
0.3mg/kg Ex.10(1) +Dex
Serum IFN-β Concentration (pg/mL)
40000
30000
20000
10000
0
0
21446
5143
Vehicle
0.3mg/kg Ex.10(1)
0.3mg/kg Ex.10(1) +Dex

*FIG. 5*

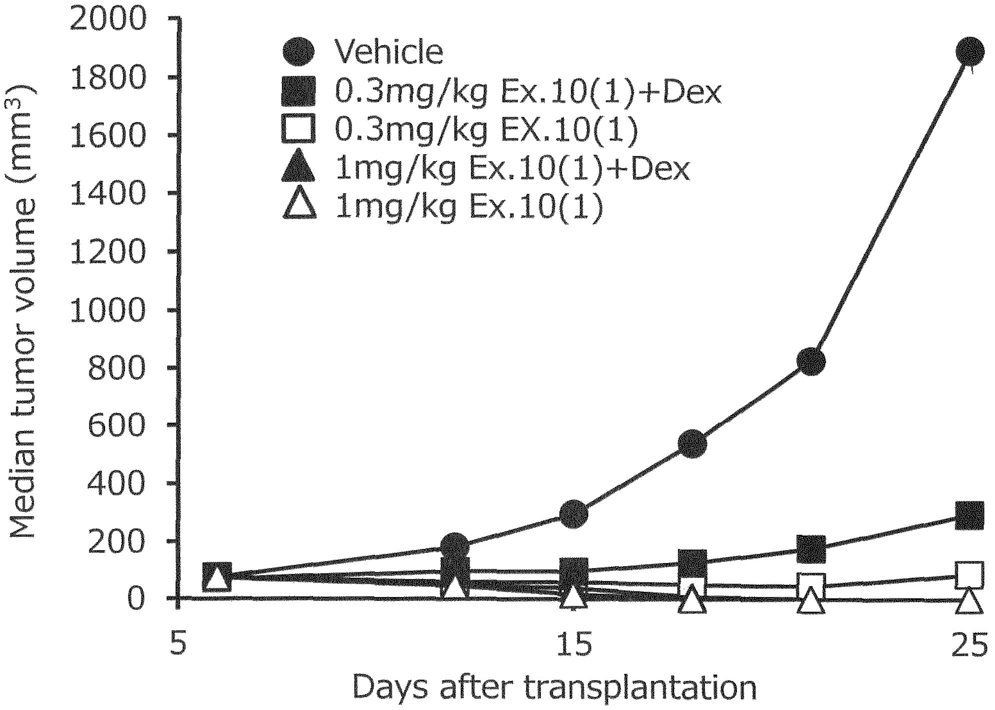
Vehicle
0.3mg/kg Ex.10(1)+Dex
0.3mg/kg EX.10(1)
1mg/kg Ex.10(1)+Dex
1mg/kg Ex.10(1)
Median tumor volume (mm3)
2000
1800
1600
1400
1200
1000
800
600
400
200
0
5
15
25
Days after transplantation

*FIG. 6*

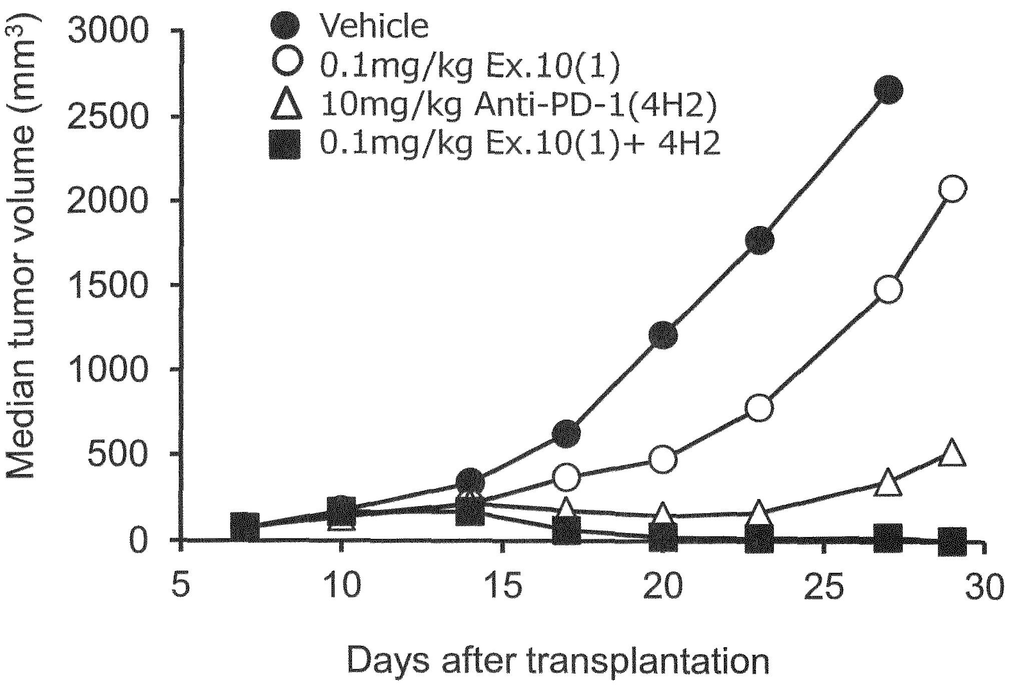
Vehicle
0.1mg/kg Ex.10(1)
10mg/kg Anti-PD-1(4H2)
0.1mg/kg Ex.10(1)+ 4H2
Median tumor volume (mm3)
3000
2500
2000
1500
1000
500
0
5
10
15
20
25
30
Days after transplantation

FIG. 7

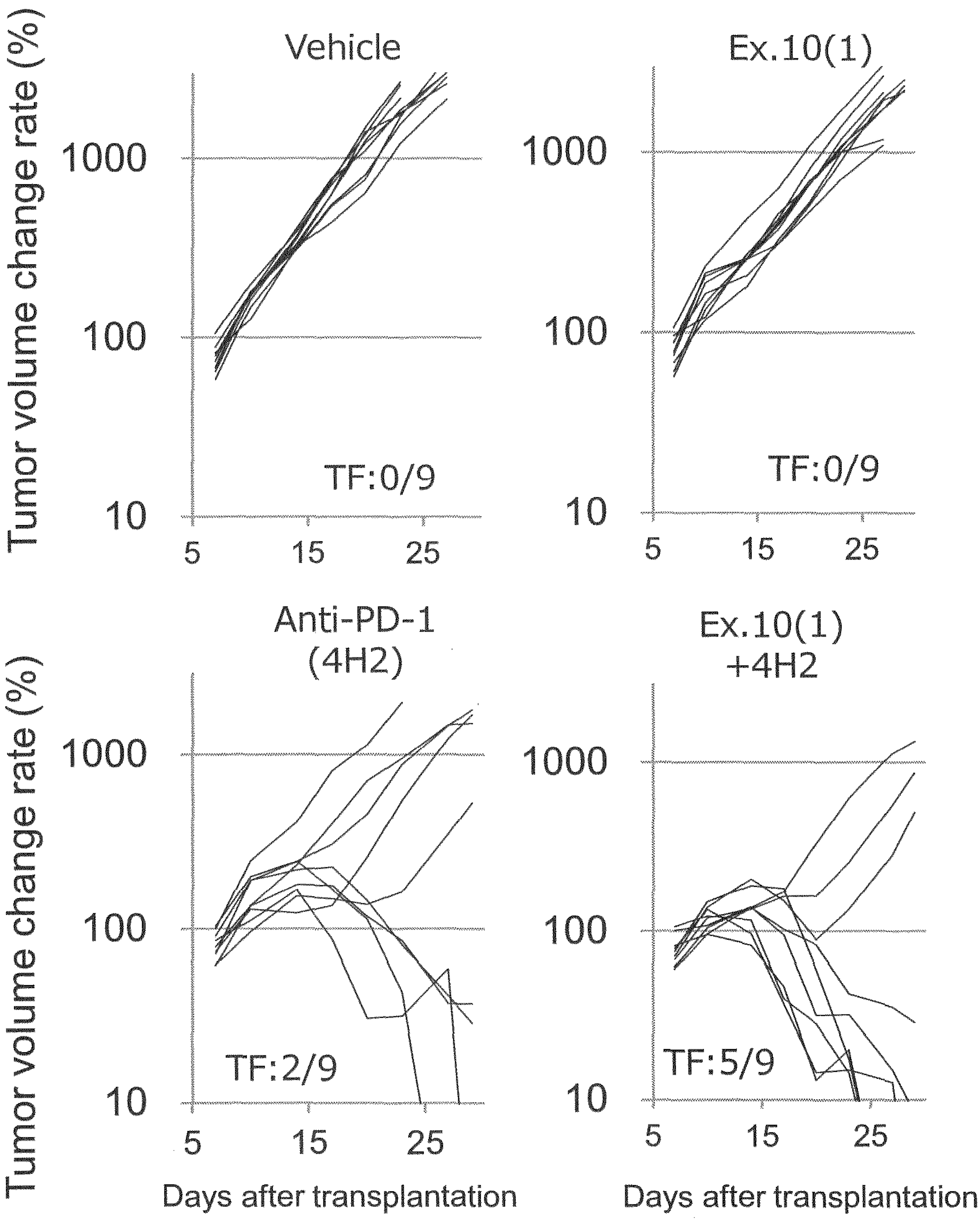
Vehicle
Ex.10(1)
Anti-PD-1
(4H2)
Ex.10(1)
+4H2
Tumor volume change rate (%)
1000
100
10
TF:0/9
TF:0/9
TF:2/9
TF:5/9
5
15
25
Days after transplantation

FIG. 8

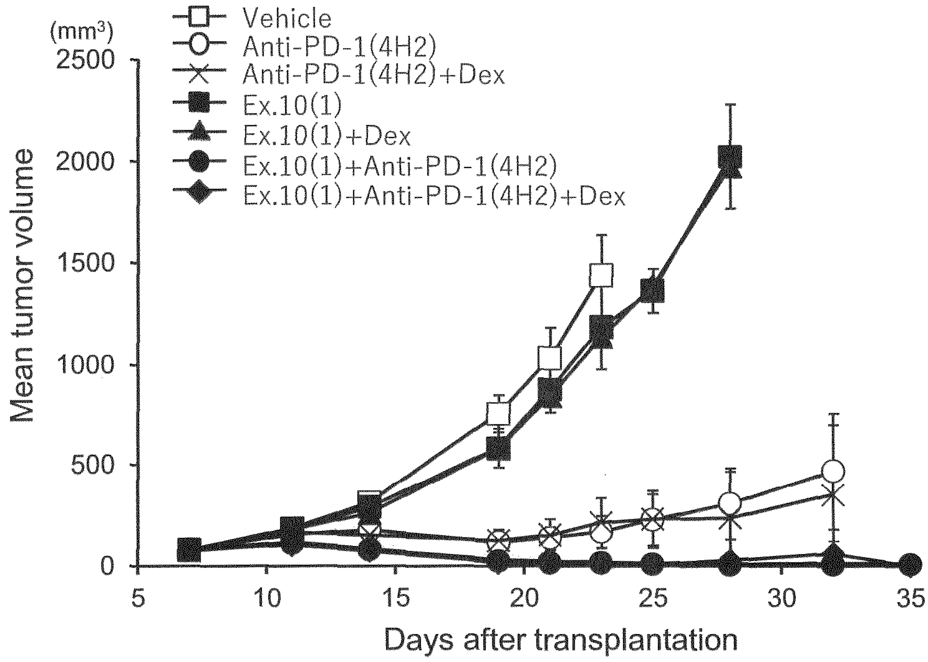
(mm³)
Vehicle
Anti-PD-1(4H2)
Anti-PD-1(4H2)+Dex
Ex.10(1)
Ex.10(1)+Dex
Ex.10(1)+Anti-PD-1(4H2)
Ex.10(1)+Anti-PD-1(4H2)+Dex
Mean tumor volume
Days after transplantation
2500
2000
1500
1000
500
0
5
10
15
20
25
30
35

FIG. 9

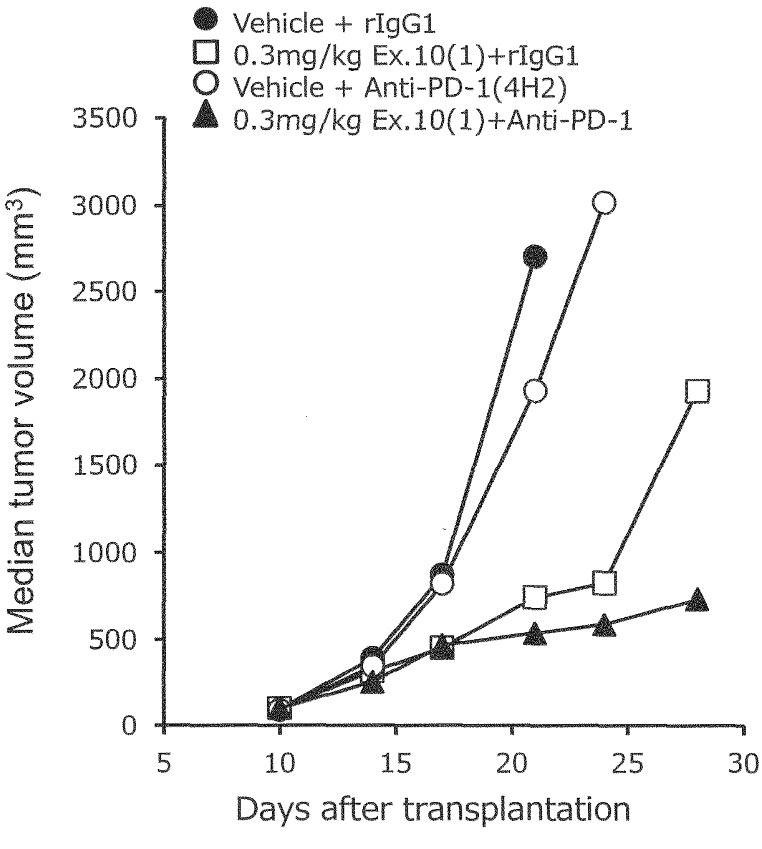
Vehicle + rIgG1
0.3mg/kg Ex.10(1)+rIgG1
Vehicle + Anti-PD-1(4H2)
0.3mg/kg Ex.10(1)+Anti-PD-1
Median tumor volume (mm³)
Days after transplantation
3500
3000
2500
2000
1500
1000
500
0
5
10
15
20
25
30

*FIG. 10*

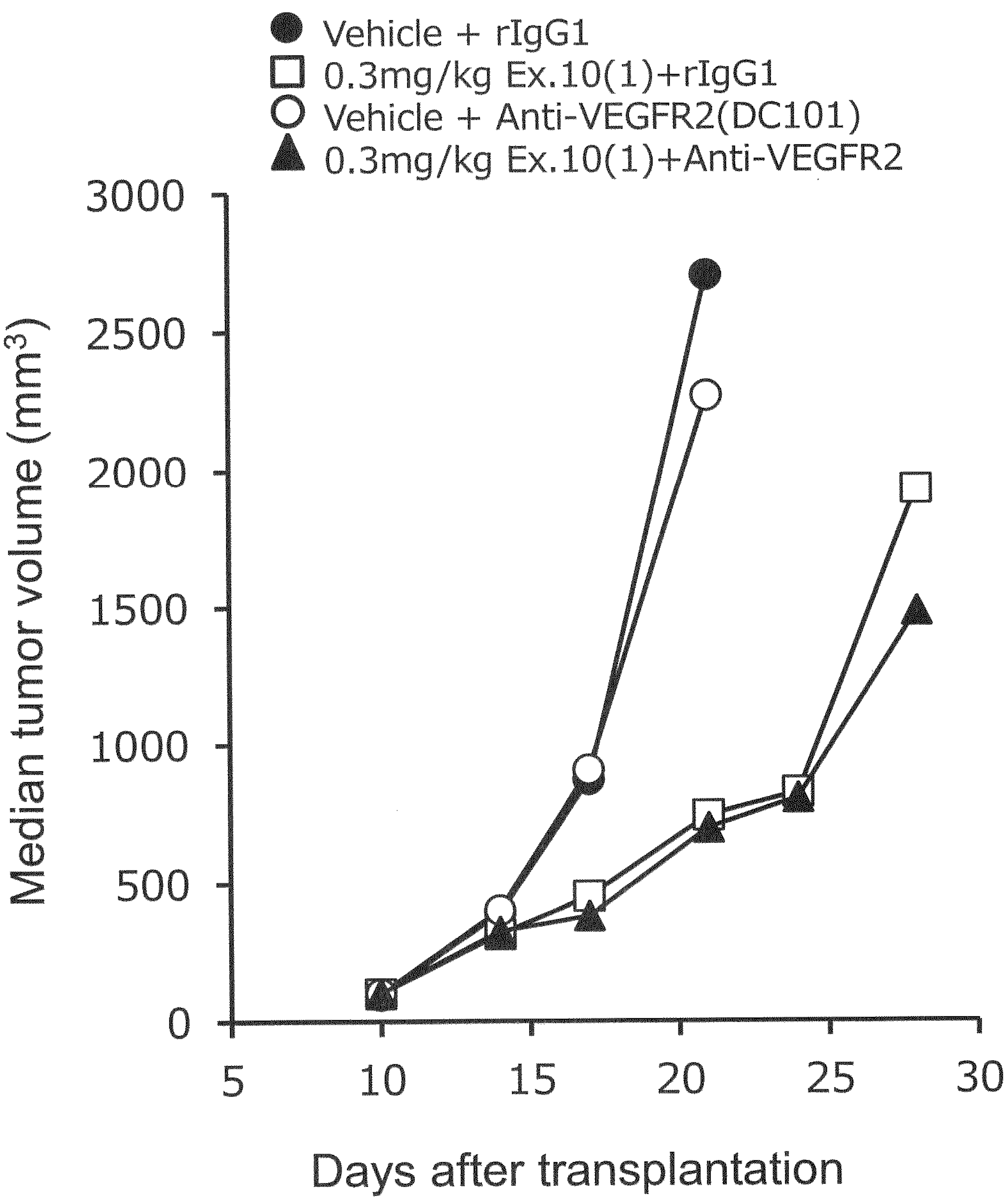
Vehicle + rIgG1
0.3mg/kg Ex.10(1)+rIgG1
Vehicle + Anti-VEGFR2(DC101)
0.3mg/kg Ex.10(1)+Anti-VEGFR2
Median tumor volume (mm3)
3000
2500
2000
1500
1000
500
0
5
10
15
20
25
30
Days after transplantation

*FIG. 11*

| FAB Classification | Names of Cell Lines | Relative minimum cell count(%) | EC50 (nM) | Source |
|---|---|---|---|---|
| eosinophilic | EOL1 | 19.4 | 27.8 | DSMZ |
| M0 | Kasumi-3 | 36.9 | 219.9 | ATCC |
| | KG-1a | 99.7 | ＞1000 | ATCC |
| M2 | SH-2 | 18.3 | 102.1 | DSMZ |
| | Kasumi-1 | 56.6 | 96.3 | ATCC |
| M4 | OCI-AML2 | 14.2 | 110.4 | DSMZ |
| | OCI-AML3 | 20.5 | 146.4 | DSMZ |
| | GDM-1 | 49.1 | 1761 | ATCC |
| | ML-2 | 7.4 | ＜100 | DSMZ |
| | HNT-34 | 35.4 | 204.8 | DSMZ |
| | PLB-985 | 84.8 | 195.8 | DSMZ |
| | OCI-AML5 | 21.4 | 24.9 | DSMZ |
| M5 | MOLM-14 | 25.1 | ＜100 | DSMZ |
| | SHI-1 | 27 | 174.1 | DSMZ |
| | SIG-M5 | 11.4 | 239.5 | DSMZ |
| | SKM-1 | 34.7 | 87.98 | DSMZ |
| | MV-4-11 | 14.4 | 116.7 | ATCC |
| M6 | OCI-M2 | 58.7 | 155.2 | DSMZ |
| | TF-1a | 70.8 | 271.6 | ATCC |
| M7 | CMK-11-5 | 37.4 | 606.5 | JCRB Cell Bank |
| | CMK-86 | 38.4 | 564.8 | JCRB Cell Bank |
| | CMK | 41.4 | 113.3 | DSMZ |
| | MEGAL | 54.2 | 298.2 | DSMZ |
| | MOLM-16 | 10.3 | ＜100 | DSMZ |

*FIG. 12*

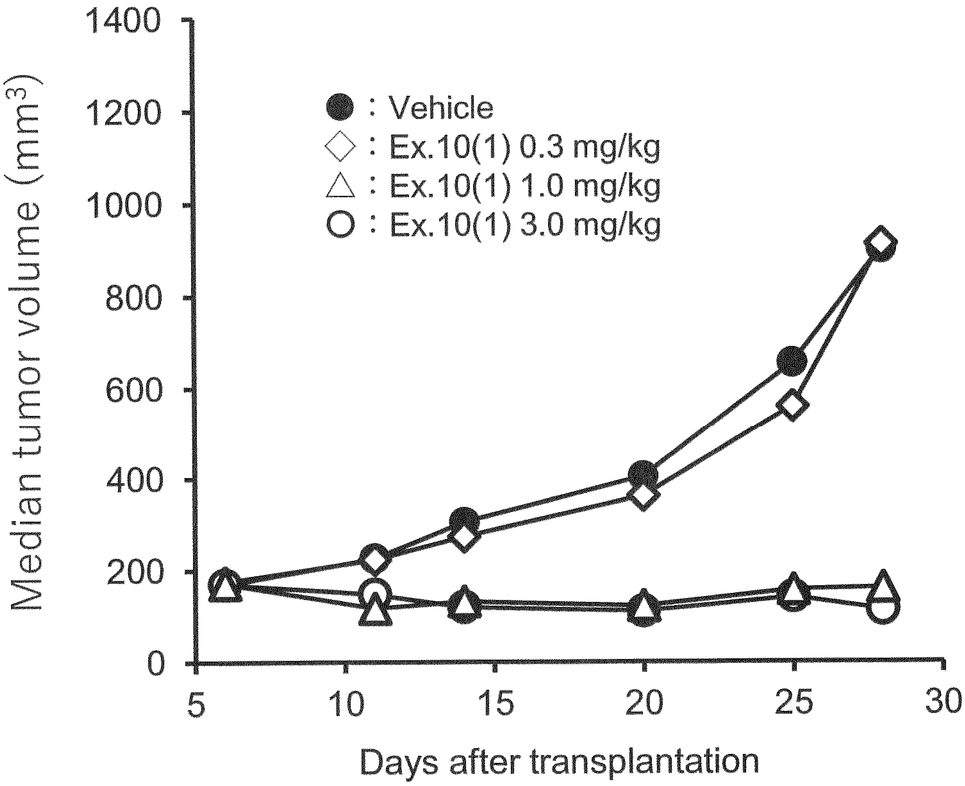
1400
1200
1000
800
600
400
200
0
Median tumor volume (mm³)
5
10
15
20
25
30
Days after transplantation
: Vehicle
: Ex.10(1) 0.3 mg/kg
: Ex.10(1) 1.0 mg/kg
: Ex.10(1) 3.0 mg/kg

FIG. 13

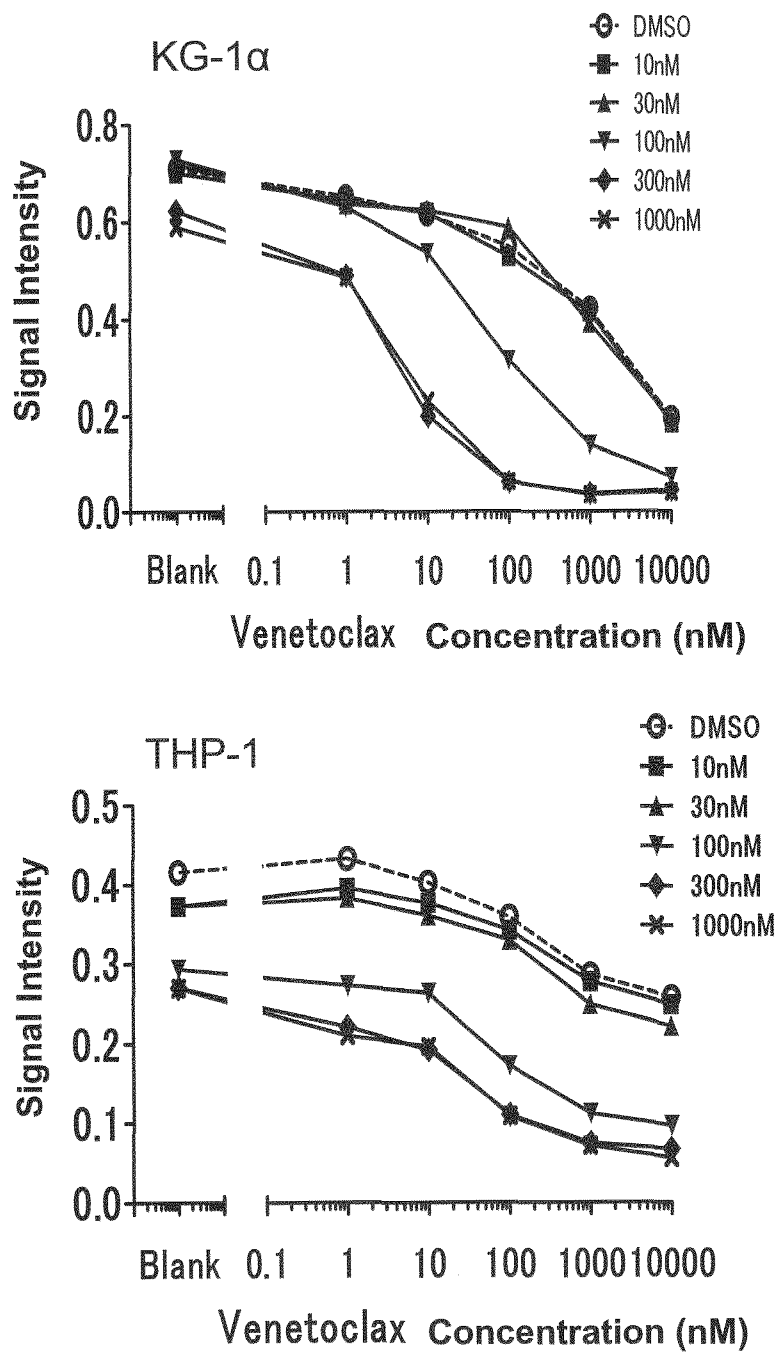
KG-1α
DMSO
10nM
30nM
100nM
300nM
1000nM
Signal Intensity
0.8
0.6
0.4
0.2
0.0
Blank
0.1
1
10
100
1000
10000
Venetoclax Concentration (nM)
THP-1
DMSO
10nM
30nM
100nM
300nM
1000nM
Signal Intensity
0.5
0.4
0.3
0.2
0.1
0.0
Blank
0.1
1
10
100
1000
10000
Venetoclax Concentration (nM)

FIG. 14

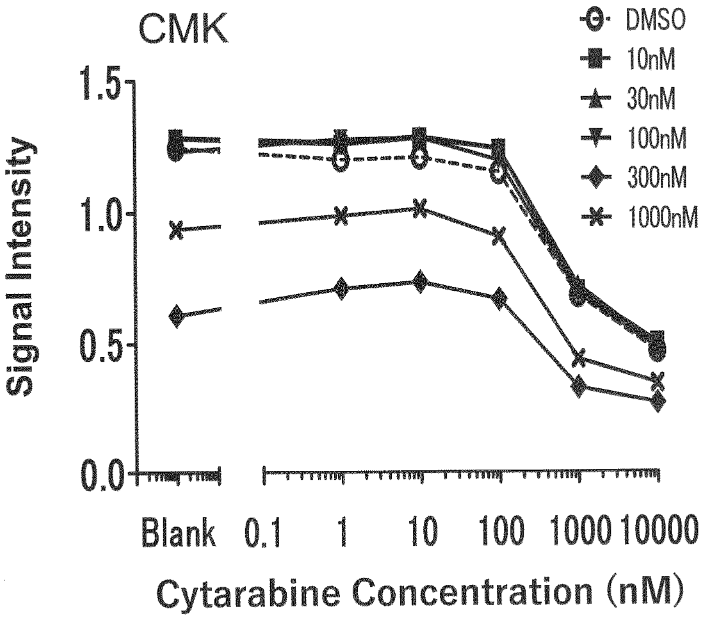
CMK
DMSO
10nM
30nM
100nM
300nM
1000nM
1.5
1.0
0.5
0.0
Signal Intensity
Blank
0.1
1
10
100
1000
10000
Cytarabine Concentration (nM)

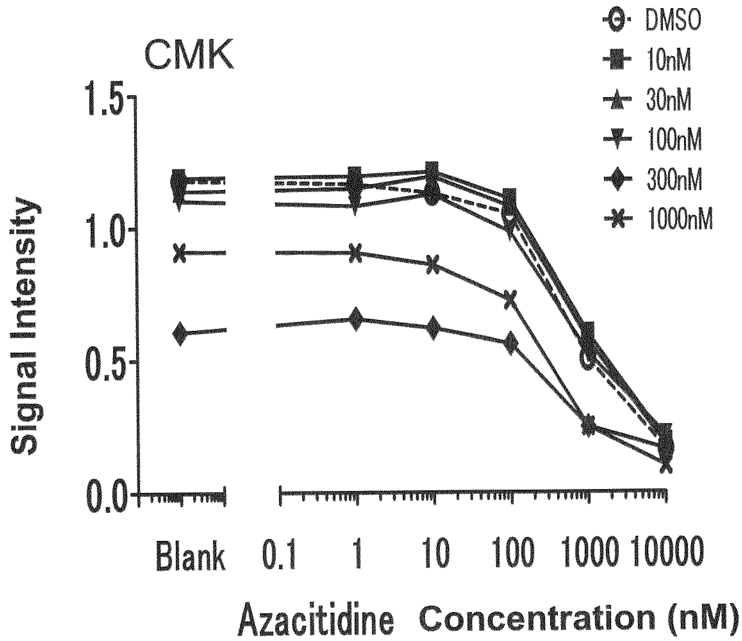
CMK
DMSO
10nM
30nM
100nM
300nM
1000nM
1.5
1.0
0.5
0.0
Signal Intensity
Blank
0.1
1
10
100
1000
10000
Azacitidine Concentration (nM)

FIG. 15

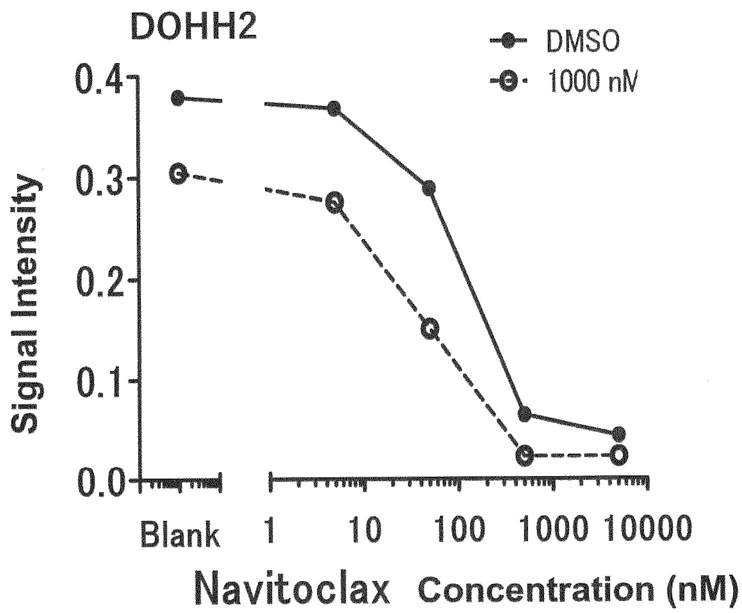
DOHH2
DMSO
1000 nM
Signal Intensity
0.4
0.3
0.2
0.1
0.0
Blank
1
10
100
1000
10000
Navitoclax Concentration (nM)

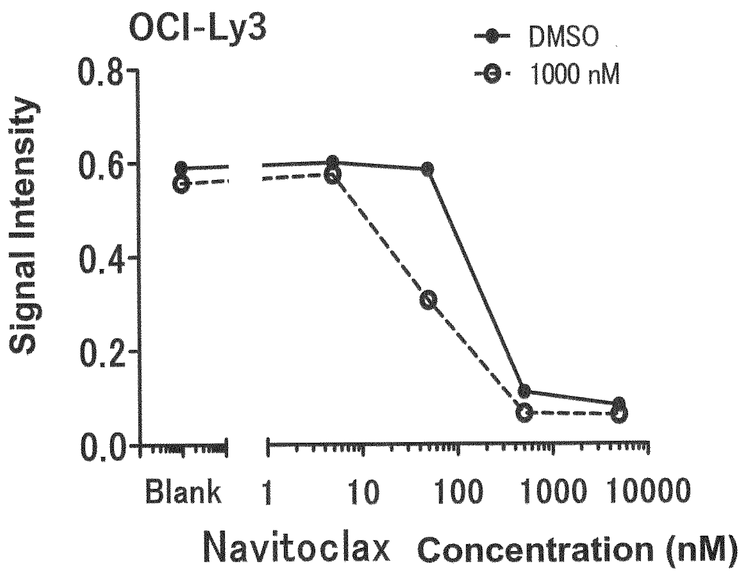
OCI-Ly3
DMSO
1000 nM
Signal Intensity
0.8
0.6
0.4
0.2
0.0
Blank
1
10
100
1000
10000
Navitoclax Concentration (nM)

**INTERNATIONAL SEARCH REPORT**

International application No. PCT/JP2021/014973

A. CLASSIFICATION OF SUBJECT MATTER

A61K 45/00(2006.01)i; A61K 31/437(2006.01)i; A61K 31/573(2006.01)i; A61K 31/661(2006.01)i; A61K 39/395(2006.01)i; A61P 35/00(2006.01)i; A61P 43/00(2006.01)i

FI: A61K45/00; A61K31/437; A61K31/573; A61K31/661; A61K39/395 U; A61P35/00; A61P43/00 111; A61P43/00 121

According to International Patent Classification (IPC) or to both national classification and IPC

B. FIELDS SEARCHED

Minimum documentation searched (classification system followed by classification symbols)

A61K45/00; A61K31/437; A61K31/573; A61K31/661; A61K39/395; A61P35/00; A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | 小坂朱 他, "Combination of a STING agonist and dexamethasone induces antitumor effects", 日本病理学会会誌, 2016, vol. 105, no. 1, p. 392, P1-58, entire text, (KOSAKA, Akemi et al., Transactiones Societatis Pathologicae Japonicae) | 1, 15<br>5-14, 16-25 |
| Y | WO 2019/027857 A1 (MERCK SHARP & DOHME CORP.) 07 February 2019 (2019-02-07) claim 1 | 5-14, 16-25 |
| Y | JP 2019-530658 A (DANA-FARBER CANCER INSTITUTE, INC.) 24 October 2019 (2019-10-24) claims 1, 16 | 5-14, 16-25 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>17 June 2021 (17.06.2021) | Date of mailing of the international search report<br>29 June 2021 (29.06.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/014973

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2018-522027 A (NOVARTIS AG) 09 August 2018 (2018-08-09) claims 1, 13 | 5-14, 16-25 |
| A | WO 2019/069275 A1 (GLAXOSMITHKLINE INTELLECTUAL PROPERTY DEVELOPMENT LIMITED) 11 April 2019 (2019-04-11) p. 6 | 1-25 |

Form PCT/ISA/210 (continuation of second sheet) (January 2015)

**INTERNATIONAL SEARCH REPORT**
Information on patent family members

International application No.
PCT/JP2021/014973

| Patent Documents referred in the Report | Publication Date | Patent Family | Publication Date |
|---|---|---|---|
| WO 2019/027857 A1 | 07 Feb. 2019 | EP 3661499 A1<br>claim 1<br>JP 2020-529421 A | |
| JP 2019-530658 A | 24 Oct. 2019 | US 2019/0083626 A1<br>WO 2018/045058 A1<br>claims 1, 16<br>EP 3506884 A1<br>KR 10-2019-0043162 A<br>CN 110121335 A | |
| JP 2018-522027 A | 09 Aug. 2018 | US 2018/0222982 A1<br>claims 1, 13<br>WO 2017/019896 A1<br>EP 3328418 A1<br>CN 108025051 A | |
| WO 2019/069275 A1 | 11 Apr. 2019 | JP 2020-536105 A<br>paragraph [0019]<br>EP 3691640 A1<br>CN 111194214 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015185565 A
- WO 2017093933 A
- WO 2017175147 A
- WO 2017175156 A
- WO 2019069275 A
- WO 2019069270 A
- WO 2019069269 A
- WO 2016096174 A
- WO 2017186711 A
- WO 2019129880 A
- WO 2014093936 A
- WO 2014189805 A
- WO 2014189806 A
- WO 2016145102 A
- WO 2017075477 A
- WO 2017106740 A
- WO 2018009466 A
- WO 2018198076 A
- WO 2018200812 A
- WO 2017027645 A
- WO 2017027646 A
- WO 2018067423 A
- WO 2018118665 A
- WO 2018118664 A
- WO 2018208667 A
- WO 2019027858 A
- WO 2019027857 A
- WO 2019125974 A
- WO 2019195124 A
- WO 2019195063 A
- WO 2017011622 A
- WO 2016164619 A
- WO 2019046511 A
- WO 2019051489 A
- WO 2019051488 A
- WO 2017161349 A
- WO 2018009648 A
- WO 2018013887 A
- WO 2018013908 A
- WO 2019161171 A
- WO 2020014127 A
- WO 2018013924 A
- WO 2018060323 A
- WO 2018172206 A
- WO 2019185476 A
- WO 2019185477 A
- WO 2017123669 A
- WO 2017123657 A
- WO 2018045204 A
- WO 2020010092 A
- WO 2020010155 A
- WO 2018100558 A
- WO 2019092660 A
- WO 2019180683 A
- WO 2018098203 A
- WO 2018138685 A
- WO 2018138684 A
- WO 2019118839 A
- WO 2020016782 A
- WO 2018065360 A
- WO 2018152450 A
- WO 2018152453 A
- WO 2019232392 A
- WO 2018156625 A
- US 20170146519 A
- WO 2018234808 A
- WO 2018234807 A
- WO 2018234805 A
- WO 2019243823 A
- WO 2019243825 A
- WO 2019023459 A
- WO 2019046500 A
- WO 2019046498 A
- WO 2019046496 A
- WO 2019074887 A
- WO 2019160884 A
- WO 2019173587 A
- WO 2019043634 A
- US 20170050967 A
- WO 2019165032 A
- WO 2019158731 A
- WO 2019134705 A
- WO 2019134707 A
- WO 2019123338 A
- WO 2019123339 A
- WO 2019123340 A
- WO 2019122202 A
- WO 2019100061 A
- WO 2020010451 A
- WO 2020006432 A
- WO 2019238786 A
- WO 2019227007 A
- WO 2019219820 A
- WO 2019211799 A
- WO 2019193542 A
- WO 2019193533 A
- WO 2019193543 A
- WO 2019183578 A

- WO 2019175776 A
- WO 2019170912 A
- WO 2020028565 A
- WO 2020028566 A
- WO 2020038387 A
- WO 2020042995 A
- WO 2020050406 A
- WO 2020057546 A
- WO 2020072492 A
- WO 2020074004 A
- WO 2020092127 A
- WO 2020106736 A
- WO 2020117623 A
- WO 2020117624 A
- WO 2020117625 A
- WO 2020115676 A
- WO 2020124059 A
- WO 2020135715 A
- WO 2020146237 A
- WO 2020151682 A
- WO 2020156363 A
- WO 2020163415 A
- WO 2020178768 A
- WO 2020178769 A
- WO 2020178770 A
- WO 2020202091 A
- WO 2020194160 A
- WO 2020221038 A
- WO 2020232375 A
- WO 2020232378 A
- WO 2020227421 A
- WO 2020252240 A
- WO 2020236586 A
- WO 2020243519 A
- WO 2020249773 A
- WO 2021009362 A
- WO 2021009365 A
- WO 2021000770 A
- WO 2021014365 A
- WO 2021013234 A
- WO 2021013250 A
- WO 2021026009 A
- WO 2021035257 A
- WO 2021035258 A
- WO 2021037179 A
- WO 2021042024 A
- WO 2006007448 A
- WO 2014151634 A
- WO 2016039749 A
- WO 2016057624 A
- WO 2016077518 A
- WO 2016100285 A
- WO 2016100608 A
- WO 2016126646 A
- WO 2016149351 A
- WO 2017151830 A
- WO 2017176608 A
- WO 2015034820 A
- WO 2015160641 A
- WO 2017066227 A
- WO 2017070089 A
- WO 2017087777 A
- WO 2017106634 A
- WO 2017112730 A
- WO 2017192961 A
- WO 2017205464 A
- WO 2017202273 A
- WO 2017202274 A
- WO 2017202275 A
- WO 2017202276 A
- WO 2017118762 A
- WO 2006121168 A
- WO 2008156712 A
- WO 2007005874 A
- WO 2001014424 A
- EP 201759781 W

**Non-patent literature cited in the description**


- **DEVAUX L.** *Curr. Opi. Microbiol.,* 2018, vol. 41, 21-28
- **CORRALES L.** *Cell Rep.,* 2015, vol. 11 (7), 1018-1030
- **SALI T.M.** *PLoS Pathog.,* vol. 11 (12), e1005324
- *CHEMICAL ABSTRACTS,* 1638241-89-0
- **HIROKAWA SHOTEN.** Molecular Design. *Development of Pharmaceuticals,* 1990, vol. 7, 163-198
- **RICHARD C. LAROCK.** Comprehensive Organic Transformations: A Guide to Functional Group Preparations. John Wiley & Sons Inc, 1999
- *CHEMICAL ABSTRACTS,* 2218503-83-2
- *CHEMICAL ABSTRACTS,* 2228934-37-8
- *CHEMICAL ABSTRACTS,* 2211044-08-3
- *CHEMICAL ABSTRACTS,* 2099072-25-8
- *Oncotarget,* 22 September 2017, vol. 8 (42), 72167-72181
- *Angew. Chem. Int. Ed.,* 2015, vol. 54, 11760-11764
- *CHEMICAL ABSTRACTS,* 1.003846-21-6
- *CHEMICAL ABSTRACTS,* 167415-27-2
- *CHEMICAL ABSTRACTS,* 1219741-79-3
- *CHEMICAL ABSTRACTS,* 143945-65-7
- *CHEMICAL ABSTRACTS,* 148893-10-1
- *CHEMICAL ABSTRACTS,* 1326283-60-6
- *CHEMICAL ABSTRACTS,* 1072944-26-3
- *CHEMICAL ABSTRACTS,* 137348-86-8

- *CHEMICAL ABSTRACTS,* 27988-97-2
- *CHEMICAL ABSTRACTS,* 32399-12-5
- *CHEMICAL ABSTRACTS,* 17341-93-4
- *CHEMICAL ABSTRACTS,* 25952-53-8
- *CHEMICAL ABSTRACTS,* 1122-58-3
- *CHEMICAL ABSTRACTS,* 13734-36-6
- *CHEMICAL ABSTRACTS,* 622-40-2
- *CHEMICAL ABSTRACTS,* 22128-62-7
- *CHEMICAL ABSTRACTS,* 4975-73-9
- *CHEMICAL ABSTRACTS,* 35180-01-9
- *Mol Immunol.,* 2017, vol. 90, 182-189